(19) Europäisches Patentamt
European Patent Office
Office européen des brevets

(11) EP 4 772 631 A1

(12) EUROPEAN PATENT APPLICATION
published in accordance with Art. 153(4) EPC

(43) Date of publication:
**08.07.2026 Bulletin 2026/28**

(21) Application number: **23950295.8**

(22) Date of filing: **01.09.2023**

(51) International Patent Classification (IPC):
***C12N 15/113*** *(2010.01)* ***A61K 31/713*** *(2006.01)*
***A61K 48/00*** *(2006.01)* ***A61P 5/00*** *(2006.01)*

(52) Cooperative Patent Classification (CPC):
**A61K 31/713; A61K 48/00; A61P 5/00; C12N 15/113**

(86) International application number:
**PCT/CN2023/116571**

(87) International publication number:
**WO 2025/043720 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Rigerna Therapeutics Co., Ltd.**
**Suzhou, Jiangsu 215128 (CN)**

(72) Inventors:
- **HUANG, Yuanyu**
  **Jiangsu 215128 (CN)**
- **FAN, Zhibin**
  **Jiangsu 215128 (CN)**
- **KONG, Lina**
  **Jiangsu 215128 (CN)**

(74) Representative: **V.O.**
**P.O. Box 87930**
**2508 DH Den Haag (NL)**

Remarks:
The complete document including Reference Table(s) and the Sequence Listing(s) can be downloaded from the EPO website

# (54) DOUBLE-STRANDED OLIGONUCLEOTIDE AND CONJUGATE FOR INHIBITING EXPRESSION OF C3 GENES, AND USES THEREOF

(57) The present application relates to the field of drugs, and in particular to a double-stranded oligonucleotide and conjugate for inhibiting the expression of C3 genes, and uses thereof. The double-stranded oligonucleotide or the conjugate comprising same can obviously inhibit the expression of C3 mRNA in HepG2 cells, can obviously inhibit the expression of C3 mRNA in animal bodies, can obviously reduce the level of C3 protein in the serum of cynomolgus monkeys, the inhibition effect being able to last for 42 days, can obviously inhibit alternative complement pathways of the serum of cynomolgus monkeys, but has no effect on classical complement pathways of the serum, can reduce the levels of C3 mRNA and protein of CFA-IgA mice, can reduce C3 deposition in a renal tissue, and can ameliorate the disease course of a patient with C3 deposition in the renal tissue. Additionally, no obvious abnormality is found in hematology and blood biochemistry of animals. The double-stranded oligonucleotide or the conjugate comprising same is beneficial to alleviating, preventing and/or treating diseases or disorders mediated by C3 genes.



Processed by Luminess, 75001 PARIS (FR)

## Description

### FIELD

The present disclosure relates to the field of pharmacy, in particular to a double-stranded oligonucleotide for inhibiting expression of C3 gene, conjugates and uses thereof.

### BACKGROUND

The complement system is a crucial component of the body's innate immunity, composed of more than 50 soluble proteins and membrane-bound proteins. The complement system plays a vital role in various life activities, such as defending the body against foreign substances, cell lysis, inflammatory responses, the dissolution of immune complexes, the clearance of apoptotic cells, and the enhancement of humoral immune responses.

Complement activation occurs through three main pathways: the classical pathway, the alternative pathway, and the mannose-binding lectin (MBL) pathway.

Given the important role of the complement system in defending against foreign substances, clearing immune complexes within the body, and participating in adaptive immunity, abnormalities in the complement system are closely associated with numerous diseases, such as kidney diseases and autoimmune disorders. For example, atypical hemolytic uremic syndrome (aHUS) is characterized by microangiopathic hemolytic anemia, thrombocytopenia, and acute renal failure. Over 50% of aHUS cases are associated with mutations or polymorphisms in complement regulatory factors such as Factor H, Factor I, MCP, C4BP, Factor B, and C3, as well as the production of autoantibodies against Factor H. These mutations or polymorphisms typically occur in a heterozygous state and affect the secretion and function of these proteins. Another example is type II membranoproliferative glomerulonephritis (MPGN), a severe class of kidney disease typically characterized by electron-dense deposits visible under electron microscopy, accompanied by the proliferation of the glomerular basement membrane and mesangial cells. Individuals with Factor H or C3 deficiency are susceptible to this disease. Furthermore, MPGN is also associated with the body's production of autoantibodies against the C3 convertase of the alternative pathway (also known as C3 nephritic factor, C3NeF). Glomerulonephritis is a disease characterized by kidney damage caused by excessive complement activation triggered by immune complexes in the renal blood vessels. C3 and its regulatory proteins, Factor I and Factor H, are closely linked to the onset and progression of glomerulonephritis. A deficiency in Factor I and Factor H leads to the dysregulation of C3. In addition, C1q knockout mice can produce autoantibodies, which make the apoptotic cells unable to be effectively cleared, so the mice are susceptible to immune complex-mediated glomerulonephritis.

Within the classical, lectin, and alternative pathways of the complement system, theuraputic targets currently under development include C1 (C1q, C1r, C1s), MASP, C2, C3, C5, and C6. For targeting the key complement protein C3, several companies are developing peptide drugs (e.g., AMY-101, APL-1) or recombinant enzymes (CB2782) for the treatment of diseases such as paroxysmal nocturnal hemoglobinuria (PNH), chronic obstructive pulmonary disease (COPD), and age-related macular degeneration (AMD). However, very few drugs targeting the key complement protein C3 are currently being developed for the treatment of kidney diseases.

Therefore, providing a double-stranded oligonucleotide that inhibits C3 gene expression is of great significance for improving the disease course in patients with C3 deposition in renal tissue, and for the alleviation, prevention, and/or treatment of diseases or conditions mediated by the C3 gene.

### SUMMARY

In view of the foregoing, the present disclosure provides a double-stranded oligonucleotide for inhibiting the expression of C3 gene, a conjugate thereof and uses thereof.

To achieve the foregoing objects, the present disclosure provides the following technical solutions:

In a first aspect, the present disclosure provides a double-stranded oligonucleotide for inhibiting expression of C3 gene, comprising a sense strand and an antisense strand;

from 5'-end to 3'-end, nucleotides of positions 2 to 19 of the antisense strand comprise a complementary region to a C3 RNA transcript, wherein the complementary region comprises at least 17 contiguous nucleotides of any one of the nucleotide sequences as set forth in SEQ ID NOs: 213 to 424 or a nucleotide sequence having 1, 2 or 3 nucleotide differences from the at least 17 contiguous nucleotides; and

the sense strand comprises at least 17 nucleotides, and the sense strand and the antisense strand are complementary or substantially complementary to form a duplex region, wherein being substantial complementary means that the sense strand and the antisense strand have no more than 3 nucleotide mismatches in the duplex region.

In some optional embodiments of the present disclosure, the antisense strand comprises a nucleotide sequence having 0, 1, 2 or 3 nucleotide differences from any one of the sequences as set forth in SEQ ID NOs: 213 to 424.

In some optional embodiments of the present disclosure, the antisense strand comprises a nucleotide sequence having 0 or 1 nucleotide difference from any one of the sequences as set forth in SEQ ID NOs: 213 to 424.

In some specific embodiments of the present disclosure, the antisense strand is selected from or comprises any one of the nucleotide sequences as set forth in SEQ ID NOs: 213 to 424.

In some optional embodiments of the present disclosure, the sense strand comprises at least 17 contiguous nucleotides of any one of the nucleotide sequences as set forth in SEQ ID NOs: 1 to 212 or a nucleotide sequence having 1, 2 or 3 nucleotide differences from the at least 17 contiguous nucleotides.

In some optional embodiments of the present disclosure, the sense strand comprises a nucleotide sequence having 0, 1, 2 or 3 nucleotide differences from any one of the sequences as set forth in SEQ ID NOs: 1 to 212.

In some optional embodiments of the present disclosure, the sense strand comprises a nucleotide sequence having 0 or 1 nucleotide difference from any one of the nucleotide sequences as set forth in SEQ ID NOs: 1 to 212.

In some specific embodiments of the present disclosure, the sense strand is selected from or comprises any one of the nucleotide sequences as set forth in SEQ ID NOs: 1 to 212.

In some specific embodiments of the present disclosure, the antisense strand is selected from or comprises any one of the nucleotide sequences as set forth in SEQ ID NOs: 213 to 424, and the sense strand is selected from or comprises any one of the nucleotide sequences as set forth in SEQ ID NOs: 1 to 212.

In some optional embodiments of the present disclosure, the antisense strand comprises a nucleotide sequence having 0 or 1 nucleotide difference from any one of the nucleotide sequences as set forth in SEQ ID NOs: 241, 242, 285, 357 or 403;

In some specific embodiments of the present disclosure, the antisense strand comprises any one of the nucleotide sequences as set forth in SEQ ID NOs: 241, 242, 285, 357 or 403.

In some optional embodiments of the present disclosure, the sense strand comprises a nucleotide sequence having 0 or 1 nucleotide difference from any one of the nucleotide sequences as set forth in SEQ ID NOs: 29, 30, 73, 145 or 191.

In some optional embodiments of the present disclosure, the sense strand comprises any one of the nucleotide sequences as set forth in SEQ ID NOs: 29, 30, 73, 145 or 191.

In some specific embodiments of the present disclosure, the antisense strand comprises any one of the nucleotide sequences as set forth in SEQ ID NOs: 241, 242, 285, 357 or 403, and the sense strand comprises any one of the nucleotide sequences as set forth in SEQ ID NOs: 29, 30, 73, 145 or 191.

In some specific embodiments of the present disclosure, the double-stranded oligonucleotide comprises one or more pairs of:

an antisense strand having a nucleotide sequence as set forth in SEQ ID NO: 241 or a nucleotide sequence having 0 or 1 nucleotide difference therefrom, and a sense strand having a nucleotide sequence as set forth in SEQ ID NO: 29 or a nucleotide sequence having 0 or 1 nucleotide difference therefrom;

an antisense strand having a nucleotide sequence as set forth in SEQ ID NO: 242 or a nucleotide sequence having 0 or 1 nucleotide difference therefrom, and a sense strand having a nucleotide sequence as set forth in SEQ ID NO: 30 or a nucleotide sequence having 0 or 1 nucleotide difference therefrom;

an antisense strand having a nucleotide sequence as set forth in SEQ ID NO: 285 or a nucleotide sequence having 0 or 1 nucleotide difference therefrom, and a sense strand having a nucleotide sequence as set forth in SEQ ID NO: 73 or a nucleotide sequence having 0 or 1 nucleotide difference therefrom;

an antisense strand having a nucleotide sequence as set forth in SEQ ID NO: 357 or a nucleotide sequence having 0 or 1 nucleotide difference therefrom, and a sense strand having a nucleotide sequence as set forth in SEQ ID NO: 145 or a nucleotide sequence having 0 or 1 nucleotide difference therefrom; and

an antisense strand having a nucleotide sequence as set forth in SEQ ID NO: 403 or a nucleotide sequence having 0 or 1 nucleotide difference therefrom, and a sense strand having a nucleotide sequence as set forth in SEQ ID NO: 191 or a nucleotide sequence having 0 or 1 nucleotide difference therefrom.

In some specific embodiments of the present disclosure, the double-stranded oligonucleotide comprises one or more pairs of:

an antisense strand having a nucleotide sequence as set forth in SEQ ID NO: 241, and a sense strand having a

nucleotide sequence as set forth in SEQ ID NO: 29;

an antisense strand having a nucleotide sequence as set forth in SEQ ID NO: 242, and a sense strand having a nucleotide sequence as set forth in SEQ ID NO: 30;

an antisense strand having a nucleotide sequence as set forth in SEQ ID NO: 285, and a sense strand having a nucleotide sequence as set forth in SEQ ID NO: 73;

an antisense strand having a nucleotide sequence as set forth in SEQ ID NO: 357, and a sense strand having a nucleotide sequence as set forth in SEQ ID NO: 145; and

an antisense strand having a nucleotide sequence as set forth in SEQ ID NO: 403, and a sense strand having a nucleotide sequence as set forth in SEQ ID NO: 191.

In some specific embodiments of the present disclosure, the double-stranded oligonucleotide comprises: an antisense strand having a nucleotide sequence as set forth in SEQ ID NO: 241 or a nucleotide sequence having 0 or 1 nucleotide difference therefrom, and a sense strand having a nucleotide sequence as set forth in SEQ ID NO: 29 or a nucleotide sequence having 0 or 1 nucleotide difference therefrom.

In some specific embodiments of the present disclosure, the double-stranded oligonucleotide comprises: an antisense strand having a nucleotide sequence as set forth in SEQ ID NO: 241, and a sense strand having a nucleotide sequence as set forth in SEQ ID NO: 29.

In some specific embodiments of the present disclosure, each nucleotide of the double-stranded oligonucleotide is independently selected from a modified or unmodified nucleotide.

In some specific embodiments of the present disclosure, substantially all nucleotides of the double-stranded oligonucleotide are independently selected from modified nucleotides, wherein "substantially all nucleotides of the double-stranded oligonucleotide" means that most but not all of the nucleotides of the double-stranded oligonucleotide are modified nucleotides, and the double-stranded oligonucleotide comprises no more than 5, 4, 3, 2 or 1 unmodified nucleotides.

In some specific embodiments of the present disclosure, all nucleotides of the double-stranded oligonucleotide are independently selected from modified nucleotides.

In some specific embodiments of the present disclosure, each nucleotide of the double-stranded oligonucleotide is independently selected from the group consisting of:

a 2'-halogen-modified nucleotide, a 2'-deoxy-modified nucleotide, a 2'-O-optionally substituted $C_{1-6}$ alkyl-modified nucleotide, a 2'-O-$(CH_2)_n$-O-$R_1$-modified nucleotide, a 2'-amino-modified nucleotide, an abasic nucleotide or a nucleotide analog, wherein the nucleotide analog is one or more selected from peptide nucleic acid (PNA), Morpholino (MNA), bridged nucleic acid (BNA), locked nucleic acid (LNA), glycol nucleic acid (GNA), threose nucleic acid (TNA) and unlocked nucleic acid (UNA);

wherein n is selected from 1 or 2, $R_1$ is selected from an optionally substituted $C_{1-6}$ alkyl or an optionally substituted $C_{1-6}$ alkoxy; when $R_1$ comprises a substituent, the substituent is selected from a halogen, a $C_{1-6}$ alkoxy, a hydroxyl and an amino.

In some specific embodiments of the present disclosure, each nucleotide of the sense strand and/or the antisense strand is independently selected from a modified or unmodified nucleotide.

In some optional embodiments of the present disclosure, substantially all nucleotides of the sense strand and/or the antisense strand are selected from modified nucleotides, wherein "substantially all nucleotides are selected from modified nucleotides" means that most but not all of the nucleotides of the strand are modified nucleotides, and the strand comprises no more than 5, 4, 3, 2 or 1 unmodified nucleotides.

In some specific embodiments of the present disclosure, all nucleotides of the sense strand and/or the antisense strand are independently selected from a modified or unmodified nucleotide.

In some specific embodiments of the present disclosure, each nucleotide of the sense strand and/or the antisense strand is independently selected from the group consisting of:

a 2'-halogen-modified nucleotide, a 2'-deoxy-modified nucleotide, a 2'-O-optionally substituted $C_{1-6}$ alkyl-modified nucleotide, a 2'-O-$(CH_2)_n$-O-$R_1$-modified nucleotide, a 2'-amino-modified nucleotide, an abasic nucleotide or a nucleotide analog, wherein the nucleotide analog is one or more selected from peptide nucleic acid (PNA), Morpholino (MNA), bridged nucleic acid (BNA), locked nucleic acid (LNA), glycol nucleic acid (GNA), threose nucleic acid (TNA)

and unlocked nucleic acid (UNA);

wherein n is selected from 1 and 2;

$R_1$ is selected from an optionally substituted $C_{1-6}$ alkyl or an optionally substituted $C_{1-6}$ alkoxy; when $R_1$ comprises a substituent, the substituent is selected from a halogen, a $C_{1-6}$ alkoxy, a hydroxyl and an amino.

Wherein, a nucleotide has a structure of:

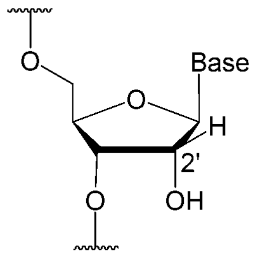

,

where Base represents a nucleoside base, and the nucleoside base of each nucleotide is selected from Uracil (U), thymine (T), cytosine (C), adenine (A), and guanine (G).

In the present diclsoure, a 2'-halogen-modified nucleotide is a nucleotide in which the 2'-hydroxyl of the ribosyl is substituted by a halogen, such as a 2'-fluoro-modified nucleotide.

In some specific embodiments of the present disclosure, the 2'-halogen-modified nucleotide is a 2'-fluoro-modified nucleotide.

In the present diclsoure, a 2'-deoxy-modified nucleotide is a nucleotide in which the 2'-hydroxyl of the ribosyl is substituted by hydrogen.

In the present diclsoure, a 2'-O-$(CH_2)_n$-O-$R_1$-modified nucleotide is a nucleotide in which the hydrogen of the 2'-hydroxyl of the ribosyl is substituted by -$(CH_2)_n$-O-$R_1$. Wherein: when n is 1, the 2'-O-$(CH_2)_n$-O-$R_1$-modified nucleotide is selected from a 2'-O-ethoxymethyl-modified nucleotide and a 2'-O-2,2,2-trifluoroethoxymethyl-modified nucleotide; when n is 2, the 2'-O-$(CH_2)_n$-O-$R_1$-modified nucleotide is selected from 2'-O-methoxyethyl-modified nucleotide (also known as a 2'-O-moe modified nucleotide).

In some optional embodiments of the present disclosure, the 2'-O-$(CH_2)_n$-O-$R_1$-modified nucleotide is selected from a 2'-O-methoxyethyl-modified nucleotide, a 2'-O-ethoxymethyl-modified nucleotide and a 2'-O-2,2,2-trifluoroethoxymethyl-modified nucleotide.

In some optional embodiments of the present disclosure, the 2'-O-$(CH_2)_n$-O-$R_1$-modified nucleotide is selected from a 2'-O-methoxyethyl-modified nucleotide.

Wherein, in the present disclosure, "optionally substituted" is used to define a variable, which may be unsubstituted, and may be substituted.

In some optional embodiments of the present disclosure, each nucleotide of the sense strand and/or the antisense strand is independently selected from the group consisting of:

a 2'-O-methyl-modified nucleotide, a 2'-fluoro-modified nucleotide, a 2'-O-methoxyethyl-modified nucleotide and a 2'-deoxy-modified nucleotide.

In some specific embodiments of the present disclosure, each nucleotide of the sense strand and/or the antisense strand is independently selected from the group consisting of:

a 2'-O-methyl-modified nucleotide, a 2'-fluoro-modified nucleotide and a 2'-O-methoxyethyl-modified nucleotide.

In some specific embodiments of the present disclosure, each nucleotide of the sense strand and/or the antisense strand is independently selected from the group consisting of:

a 2'-O-methyl-modified nucleotide, a 2'-fluoro-modified nucleotide and a 2'-deoxy-modified nucleotide.

In some specific embodiments of the present disclosure, the double-stranded oligonucleotide comprises at least one 2'-O-methoxyethyl-modified nucleotide.

In some specific embodiments of the present disclosure, the sense strand has a length selected from 18 to 21 nucleotides, and the antisense strand has a length selected from 19 to 23 nucleotides.

In some specific embodiments of the present disclosure, the sense strand has a length selected from 17 to 21 nucleotides, and the antisense strand has a length selected from 19 to 23 nucleotides.

In some specific embodiments of the present disclosure, the sense strand has a length of 19 nucleotides, and the antisense strand has a length 21 nucleotides.

In some specific embodiments of the present disclosure, the antisense strand comprises at least one 2'-O-methoxyethyl-modified nucleotide; and the sense strand comprises no more than one 2'-O-methoxyethyl-modified nucleotide.

In some specific embodiments of the present disclosure, the sense strand comprises no 2'-O-methoxyethyl-modified nucleotide; and the antisense strand comprises one 2'-O-methoxyethyl-modified nucleotide. Wherein, each nucleotide of the sense strand is independently selected from a 2'-fluoro-modified nucleotide and a 2'-O-methyl-modified nucleotide; and each nucleotide of the antisense strand is independently selected from a 2'-fluoro-modified nucleotide, a 2'-O-methyl-modified nucleotide and a 2'-O-methoxyethyl-modified nucleotide, and the antisense strand comprise one 2'-O-methoxyethyl-modified nucleotide.

In some specific embodiments of the present disclosure, the sense strand comprises one 2'-O-methoxyethyl-modified nucleotide; and the antisense strand comprises one 2'-O-methoxyethyl-modified nucleotide. Wherein, each nucleotide of the sense strand is independently selected from a 2'-fluoro-modified nucleotide, a 2'-O-methyl-modified nucleotide and a 2'-O-methoxyethyl-modified nucleotide; and each nucleotide of the antisense strand is independently selected from a 2'-fluoro-modified nucleotide, a 2'-O-methyl-modified nucleotide and a 2'-O-methoxyethyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, no more than 2 nucleotides at positions 5, 12, 13, and 18 are selected from a 2'-O-methoxyethyl-modified nucleotide, and the remaining nucleotides are independently selected from a 2'-O-methyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, no more than 1 nucleotide at positions 5, 12, 13, and 18 is selected from a 2'-O-methoxyethyl-modified nucleotide, and the remaining nucleotides are independently selected from a 2'-O-methyl-modified nucleotide.

In some specific embodiments of the present disclosure, from 5'-end to 3'-end, in the nucleotide sequence of the sense strand, nucleotides at positions 7 to 10 are selected from a 2'-fluoro-modified nucleotide, no more than 1 nucleotide at positions 5, 12, 13, and 18 is selected from a 2'-O-methoxyethyl-modified nucleotide, and the remaining nucleotides are independently selected from a 2'-O-methyl-modified nucleotide.

In some specific embodiments of the present disclosure, the sense strand has a modification pattern selected from (SS-1) to (SS-5):

(SS-1) from 5'-end to 3'-end, in the nucleotide sequence of the sense strand, the nucleotides at positions 7 to 10 are selected from a 2'-fluoro-modified nucleotide, and the remaining nucleotides are independently selected from a 2'-O-methyl-modified nucleotide;

(SS-2) from 5'-end to 3'-end, in the nucleotide sequence of the sense strand, the nucleotides at positions 7 to 10 are selected from a 2'-fluoro-modified nucleotide, nucleotide at position 5 is selected from a 2'-O-methoxyethyl-modified nucleotide, and the remaining nucleotides are independently selected from a 2'-O-methyl-modified nucleotide;

(SS-3) from 5'-end to 3'-end, in the nucleotide sequence of the sense strand, the nucleotides at positions 7 to 10 are selected from a 2'-fluoro-modified nucleotide, nucleotide at position 12 is selected from a 2'-O-methoxyethyl-modified nucleotide, and the remaining nucleotides are independently selected from a 2'-O-methyl-modified nucleotide;

(SS-4) from 5'-end to 3'-end, in the nucleotide sequence of the sense strand, the nucleotides at positions 7 to 10 are selected from a 2'-fluoro-modified nucleotide, nucleotide at position 13 is selected from a 2'-O-methoxyethyl-modified nucleotide, and the remaining nucleotides are independently selected from a 2'-O-methyl-modified nucleotide;

(SS-5) from 5'-end to 3'-end, in the nucleotide sequence of the sense strand, the nucleotides at positions 7 to 10 are selected from a 2'-fluoro-modified nucleotide, nucleotide at position 18 is selected from a 2'-O-methoxyethyl-modified nucleotide, and the remaining nucleotides are independently selected from a 2'-O-methyl-modified nucleotide.

In some specific embodiments of the present disclosure, from 5'-end to 3'-end, in the nucleotide sequence of the antisense strand, at least 4 nucleotides at positions 2, 6, 9 to 12, 14, and 16 are selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the antisense strand, at least 4 nucleotides at positions 2, 6, 9 to 12, 14, and 16 are selected from a 2'-fluoro-modified nucleotide, nucleotides at positions 2, 6, 14, and 16 are selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, at least one nucleotide at positions 8 and 15 is selected from a 2'-O-methoxyethyl-modified nucleotide, and the remaining nucleotides are independently selected from a 2'-O-methyl-modified nucleotide.

In some specific embodiments of the present disclosure, from 5'-end to 3'-end, in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, nucleotide at position 8 or 15 is selected from a 2'-O-methoxyethyl-modified nucleotide, and the remaining nucleotides are independently selected from a 2'-O-methyl-modified nucleotide.

In some specific embodiments of the present disclosure, the antisense strand has a modification pattern selected from (AS-1) to (AS-8):

(AS-1) from 5'-end to 3'-end, in the nucleotide sequence of the antisense strand, the nucleotides at positions 2, 6, 9, 14 and 16 are selected from a 2'-fluoro-modified nucleotide, nucleotide at position 8 is selected from a 2'-O-methoxyethyl-modified nucleotide, and the remaining nucleotides are independently selected from a 2'-O-methyl-modified nucleotide;

(AS-2) from 5'-end to 3'-end, in the nucleotide sequence of the antisense strand, the nucleotides at positions 2, 6, 10, 14 and 16 are selected from a 2'-fluoro-modified nucleotide, nucleotide at position 8 is selected from a 2'-O-methoxyethyl-modified nucleotide, and the remaining nucleotides are independently selected from a 2'-O-methyl-modified nucleotide;

(AS-3) from 5'-end to 3'-end, in the nucleotide sequence of the antisense strand, the nucleotides at positions 2, 6, 11, 14 and 16 are selected from a 2'-fluoro-modified nucleotide, nucleotide at position 8 is selected from a 2'-O-methoxyethyl-modified nucleotide, and the remaining nucleotides are independently selected from a 2'-O-methyl-modified nucleotide;

(AS-4) from 5'-end to 3'-end, in the nucleotide sequence of the antisense strand, the nucleotides at positions 2, 6, 12, 14 and 16 are selected from a 2'-fluoro-modified nucleotide, nucleotide at position 8 is selected from a 2'-O-methoxyethyl-modified nucleotide, and the remaining nucleotides are independently selected from a 2'-O-methyl-modified nucleotide;

(AS-5) from 5'-end to 3'-end, in the nucleotide sequence of the antisense strand, the nucleotides at positions 2, 6, 9, 14 and 16 are selected from a 2'-fluoro-modified nucleotide, nucleotide at position 15 is selected from a 2'-O-methoxyethyl-modified nucleotide, and the remaining nucleotides are independently selected from a 2'-O-methyl-modified nucleotide;

(AS-6) from 5'-end to 3'-end, in the nucleotide sequence of the antisense strand, the nucleotides at positions 2, 6, 10, 14 and 16 are selected from a 2'-fluoro-modified nucleotide, nucleotide at position 15 is selected from a 2'-O-methoxyethyl-modified nucleotide, and the remaining nucleotides are independently selected from a 2'-O-methyl-modified nucleotide;

(AS-7) rom 5'-end to 3'-end, in the nucleotide sequence of the antisense strand, the nucleotides at positions 2, 6, 11, 14 and 16 are selected from a 2'-fluoro-modified nucleotide, nucleotide at position 15 is selected from a 2'-O-methoxyethyl-modified nucleotide, and the remaining nucleotides are independently selected from a 2'-O-methyl-modified nucleotide;

(AS-8) from 5'-end to 3'-end, in the nucleotide sequence of the antisense strand, the nucleotides at positions 2, 6, 12,

14 and 16 are selected from a 2'-fluoro-modified nucleotide, nucleotide at position 15 is selected from a 2'-O-methoxyethyl-modified nucleotide, and the remaining nucleotides are independently selected from a 2'-O-methyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, and the remaining nucleotides are independently selected from a 2'-O-methyl-modified nucleotide and a 2'-O-methoxyethyl-modified nucleotide;

in the nucleotide sequence of the antisense strand, at least 4 nucleotides at positions 2, 6, 9 to 12, 14 and 16 are selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, and the remaining nucleotides are independently selected from a 2'-O-methyl-modified nucleotide and a 2'-O-methoxyethyl-modified nucleotide;

in the nucleotide sequence of the antisense strand, at least 4 nucleotides at positions 2, 6, 9 to 12, 14 and 16 are selected from a 2'-fluoro-modified nucleotide, nucleotides at positions 2, 6, 14 and 16 are selected from a 2'-fluoro-modified nucleotide, and the remaining nucleotides are independently selected from a 2'-O-methyl-modified nucleotide and a 2'-O-methoxyethyl-modified nucleotide.

In some specific embodiments of the present disclosure, from 5'-end to 3'-end, in the nucleotide sequence of the sense strand, at least 3 nucleotides at positions 7 to 10 are selected from a 2'-fluoro-modified nucleotide, no more than 2 nucleotides at positions 5, 12, 13 and 18 are selected from a 2'-O-methoxyethyl-modified nucleotide, and the remaining nucleotides are selected from a 2'-O-methyl-modified nucleotide;

in the nucleotide sequence of the antisense strand, at least 4 nucleotides at positions 2, 6, 9 to 12, 14 and 16 are selected from a 2'-fluoro-modified nucleotide, nucleotides at positions 2, 6, 14 and 16 are selected from a 2'-fluoro-modified nucleotide, at least one nucleotide at positions 8 and 15 is selected from a 2'-O-methoxyethyl-modified nucleotide, and the remaining nucleotides are selected from a 2'-O-methyl-modified nucleotide.

In some specific embodiments of the present disclosure, from 5'-end to 3'-end, in the nucleotide sequence of the sense strand, nucleotides at positions 7 to 10 are selected from a 2'-fluoro-modified nucleotide, no more than 1 nucleotide at positions 5, 12, 13 and 18 is selected from a 2'-O-methoxyethyl-modified nucleotide, and the remaining nucleotides are selected from a 2'-O-methyl-modified nucleotide;

in the nucleotide sequence of the antisense strand, at least 4 nucleotides at positions 2, 6, 14 and 16 are selected from a 2'-fluoro-modified nucleotide, any one of nucleotides at positions 9 to 12 is selected from a 2'-fluoro-modified nucleotide, at least one nucleotide at positions 8 and 15 is selected from a 2'-O-methoxyethyl-modified nucleotide, and the remaining nucleotides are selected from a 2'-O-methyl-modified nucleotide.

In some specific embodiments of the present disclosure, the sense strand and the antisense strand of the double-stranded oligonucleotide have a modification pattern selected from (ds-1) to (ds-8):

(ds-1) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-2) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-3) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-4) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 12, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-5) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-6) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-7) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide; and

(ds-8) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 12, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-9) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-10) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide,

and the remaining nucleotides are selected from a 2'-O-methyl-modified nucleotide;

(ds-11) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-12) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-13) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-14) from 5'-end to 3'-end, in the nucleotide sequence of the sense strand, the nucleotides at positions 7 to 10 are selected from a 2'-fluoro-modified nucleotide, the nucleotide at position 5 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-15) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-16) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-17) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-18) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-19) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-20) from 5'-end to 3'-end, in the nucleotide sequence of the sense strand, the nucleotides at positions 7 to 10 are selected from a 2'-fluoro-modified nucleotide, the nucleotide at position 12 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-21) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-22) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-23) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-24) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-25) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-26) from 5'-end to 3'-end, in the nucleotide sequence of the sense strand, the nucleotides at positions 7 to 10 are selected from a 2'-fluoro-modified nucleotide, the nucleotide at position 13 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-27) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-28) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-29) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-30) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-31) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-32) from 5'-end to 3'-end, in the nucleotide sequence of the sense strand, the nucleotides at positions 7 to 10 are selected from a 2'-fluoro-modified nucleotide, the nucleotide at position 18 is selected from a 2'-O-methoxyethyl-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide.

In some specific embodiments of the present disclosure, from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, at least 4 of the nucleotides at positions 2, 6, 14, and 16 are selected from a 2'-fluoro-modified nucleotide, any one of nucleotides at positions 9 to 12 is selected from a 2'-fluoro-modified nucleotide, at least one nucleotide at positions 8 and 15 is selected from a 2'-O-methoxyethyl-modified nucleotide, and the remaining nucleotides are selected from a 2'-O-methyl-modified nucleotide.

In some specific embodiments of the present disclosure, from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, at least 4 of the nucleotides at positions 2, 6, 14, and 16 are selected from a 2'-fluoro-modified nucleotide, any one of nucleotides at positions 9 to 12 is selected from a 2'-fluoro-modified nucleotide, the nucleotide at position 8 or 15 is selected from a 2'-O-methoxyethyl-modified nucleotide, and the remaining nucleotides are selected from a 2'-O-methyl-modified nucleotide.
In some specific embodiments of the present disclosure, the sense strand and the antisense strand of the double-stranded oligonucleotide have a modification pattern selected from (ds-1) to (ds-8):

(ds-1) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-2) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-3) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-4) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 12, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-5) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-6) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;

(ds-7) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide; and

(ds-8) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and

in the nucleotide sequence of the antisense strand, the nucleotides at positions 2, 6, 12, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide.

In some specific embodiments of the present disclosure, the sense strand and/or the antisense strand independently comprise one or more internucleotide phosphorothioate linkages.

In some specific embodiments of the present disclosure, the sense strand comprises two contiguous internucleotide phosphorothioate linkages at 5'-end.

In some specific embodiments of the present disclosure, the antisense strand comprises two contiguous internucleotide phosphorothioate linkages at 5'-end, and two contiguous internucleotide phosphorothioate linkages at 3'-end.

In some specific embodiments of the present disclosure, the sense strand comprises two internucleotide phosphorothioate linkages at 5'-end, and the antisense strand comprises two internucleotide phosphorothioate linkages at 5'-end and two internucleotide phosphorothioate linkages at 3'-end.

In some optional embodiments of the present disclosure, the sense strand or the antisense strand comprises a 3'-overhang having at least 1 nucleotide.

In some optional embodiments of the present disclosure, the antisense strand comprises a 3'-overhang having at least 1 nucleotide.

In some optional embodiments of the present disclosure, the antisense strand comprises a 3'-overhang having 2 nucleotides.

In some specific embodiments of the present disclosure, each nucleotide of the double-stranded oligonucleotide is independently selected from a modified nucleotide;

wherein the sense strand is selected from any one of the sequences as set forth in SEQ ID NOs: 887 to 907; and/or the antisense strand is selected from any one of the sequences as set forth in SEQ ID NOs: 908 to 943; wherein

the sense strand, from 5'-end to 3'-end,

SEQ ID NO: 887: CmsGmsAmAmGmCmUfCfAfUfGmAmAmUmAmUmAmUmUm;

SEQ ID NO: 888: CmsGmsAmAmG(moe)CmUfCfAfUfGmAmAmUmAmUmAmUmUm;

SEQ ID NO: 889: CmsGmsAmAmGmCmUfCfAfUfGmA(moe)AmUmAmUmAmUmUm;

SEQ ID NO: 890: CmsGmsAmAmGmCmUfCfAfUfGmAmA(moe)UmAmUmAmUmUm;

SEQ ID NO: 891: CmsGmsAmAmGmCmUfCfAfUfGmAmAmUmAmUmAmT(moe)Um;

SEQ ID NO: 892: CmsAmsGmAmGmAmAfAfUfUfCmUmAmCmUmAmCmAmUm;

SEQ ID NO: 893: CmsAmsGmAmG(moe)AmAfAfUfUfCmUmAmCmUmAmCmAmUm;

SEQ ID NO: 894: CmsAmsGmAmGmAmAfAfUfUfCmT(moe)AmCmUmAmCmAmUm;

SEQ ID NO: 895: CmsAmsGmAmGmAmAfAfUfUfCmUmA(moe)CmUmAmCmAmUm;

SEQ ID NO: 896: CmsAmsGmAmGmAmAfAfUfUfCmUmAmCmUmAmCmA(moe)Um;

SEQ ID NO: 897: GmsAmsGmAmAmUmUfGfCfUfUmCmAmUmAmCmAmAmAm;

SEQ ID NO: 898: GmsAmsGmAmA(moe)UmUfGfCfUfUmCmAmUmAmCmAmAmAm;

SEQ ID NO: 899: GmsAmsGmAmAmUmUfGfCfUfUmC(moe)AmUmAmCmAmAmAm;

SEQ ID NO: 900: GmsAmsGmAmAmUmUfGfCfUfUmCmA(moe)UmAmCmAmAmAm;

SEQ ID NO: 901: GmsAmsGmAmAmUmUfGfCfUfUmCmAmUmAmCmAmA(moe)Am;

SEQ ID NO: 902: CmsAmsAmCmUmCmAfCfCfUfGmUmAmAmUmAmAmAmUm;

SEQ ID NO: 903: CmsAmsAmCmT(moe)CmAfCfCfUfGmUmAmAmUmAmAmAmUm;

SEQ ID NO: 904: CmsAmsAmCmUmCmAfCfCfUfGmT(moe)AmAmUmAmAmAmUm;

SEQ ID NO: 905: CmsAmsAmCmUmCmAfCfCfUfGmUmA(moe)AmUmAmAmAmUm;

SEQ ID NO: 906: CmsAmsAmCmUmCmAfCfCfUfGmUmAmAmUmAmAmA(moe)Um;

SEQ ID NO: 907: AmsAmsAmUmUmCmUfAfCfUfAmCmAmUmCmUmAmUmAm;

and

the antisense strand, from 5'-end to 3'-end,

SEQ ID NO: 908: AmsAfsUmAmUmAfUmUmCfAmUmGmAmGfC(moe)UfUmCmGmsUmsAm;

SEQ ID NO: 909: AmsAfsUmAmUmAfUmUmCmAfUmGmAmGfC(moe)UfUmCmGmsUmsAm;

SEQ ID NO: 910: AmsAfsUmAmUmAfUmUmCmAmUfGmAmGfC(moe)UfUmCmGmsUmsAm;

SEQ ID NO: 911: AmsAfsUmAmUmAfUmT(moe)CmAfUmGmAmGfCmUfUmCmGmsUmsAm;

SEQ ID NO: 912: AmsUfsGmUmAmGfUmAmGfAmAmUmUmUfC(moe)UfCmUmGmsUmsAm;

SEQ ID NO: 913: AmsUfsGmUmAmGfUmAmGmAfAmUmUmUfC(moe)UfCmUmGmsUmsAm;

SEQ ID NO: 914: AmsUfsGmUmAmGfUmAmGmAmAfUmUmUfC(moe)UfCmUmGmsUmsAm;

SEQ ID NO: 915: AmsUfsGmUmAmGfUmA(moe)GmAfAmUmUmUfCmUfCmUmGmsUmsAm;

SEQ ID NO: 916: UmsUfsUmGmUmAfUmGmAfAmGmCmAmAfT(moe)UfCmUmCmsCmsUm;

SEQ ID NO: 917: UmsUfsUmGmUmAfUmGmAmAfGmCmAmAfT(moe)UfCmUmCmsCmsUm;

SEQ ID NO: 918: UmsUfsUmGmUmAfUmGmAmAmGfCmAmAfT(moe)UfCmUmCmsCmsUm;

SEQ ID NO: 919: UmsUfsUmGmUmAfUmG(moe)AmAfGmCmAmAfUmUfCmUmCmsCmsUm;

SEQ ID NO: 920: AmsUfsUmUmAmUfUmAmCfAmGmGmUmGfA(moe)GfUmUmGmsAmsUm;

SEQ ID NO: 921: AmsUfsUmUmAmUfUmAmCmAfGmGmUmGfA(moe)GfUmUmGmsAmsUm;

SEQ ID NO: 922: AmsUfsUmUmAmUfUmAmCmAmGfGmUmGfA(moe)GfUmUmGmsAmsUm;

SEQ ID NO: 923: AmsUfsUmUmAmUfUmA(moe)CmAfGmGmUmGfAmGfUmUmGmsAmsUm;

SEQ ID NO: 924: AmsAfsUmAmUmAfUmT(moe)CmAmUfGmAmGfCmUfUmCmGmsUmsAm;

SEQ ID NO: 925: AmsUfsGmUmAmGfUmA(moe)GmAmAfUmUmUfCmUfCmUmGmsUmsAm;

SEQ ID NO: 926: AmsUfsUmUmAmUfUmA(moe)CmAmGfGmUmGfAmGfUmUmGmsAmsUm;

SEQ ID NO: 927: AmsUfsGmUmAmGfUmAmGmAmAmUfUmUfC(moe)UfCmUmGmsUmsAm;

SEQ ID NO: 928: UmsAfsUmAmGmAfUmGmUmAmGmUfAmGfAmAfUmUmUmsCmsUm;

SEQ ID NO: 929: UmsAfsUmAmGmAfUmGmUfAmGmUmAmGfA(moe)AfUmUmUmsCmsUm;

SEQ ID NO: 930: UmsAfsUmAmGmAfUmGmUmAmGmUfAmGfA(moe)AfUmUmUmsCmsUm;

SEQ ID NO: 931: AmsAfsUmAmUmAfUmUmCfAmUmGmAmGfCmUfUmCmGmsUmsAm;

SEQ ID NO: 932: AmsAfsUmAmUmAfUmUmCmAmUmGfAmGfCmUfUmCmGmsUmsAm;

SEQ ID NO: 933: AmsAfsUmAmUmAfUmUmCmAmUmGfAmGfC(moe)UfUmCmGmsUmsAm;

SEQ ID NO: 934: AmsUfsUmUmAmUfUmAmCfAmGmGmUmGfAmGfUmUmGmsAmsUm;

SEQ ID NO: 935: AmsUfsUmUmAmUfUmAmCmAmGmGfUmGfAmGfUmUmGmsAmsUm;

SEQ ID NO: 936: AmsUfsUmUmAmUfUmAmCmAmGmGfUmGfA(moe)GfUmUmGmsAmsUm;

SEQ ID NO: 937: AmsUfsGmUmAmGfUmAmGfAmAmUmUmUfCmUfCmUmGmsUmsAm;

SEQ ID NO: 938: AmsUfsGmUmAmGfUmAmGmAmAmUfUmUfCmUfCmUmGmsUmsAm;

SEQ ID NO: 939: UmsAfsUmAmGmAfUmGmUfAmGmUmAmGfAmAfUmUmUmsCmsUm;

SEQ ID NO: 940: UmsUfsUmGmUmAfUmGmAfAmGmCmAmAfUmUfCmUmCmsCmsUm;

SEQ ID NO: 941: UmsUfsUmGmUmAfUmGmAmAmGmCfAmAfUmUfCmUmCmsCmsUm;

SEQ ID NO: 942: UmsUfsUmGmUmAfUmGmAfAmGmCmAmAfU(moe)UfCmUmCmsCmsUm;

and

SEQ ID NO: 943: UmsUfsUmGmUmAfUmGmAmAmGmCfAmAfU(moe)UfCmUmCmsCmsUm.

Wherein, C represents cytidine-3'-monophosphate, G represents guanosine-3'-monophosphate, U represents uridine-3'-monophosphate, A represents adenosine-3'-monophosphate, and T represents thymidine-3'-monophosphate; m indicates that a nucleotide immediately to the left of the letter m is a 2'-O-methyl-modified nucleotide; f indicates that a nucleotide immediately to the left of the letter f is a 2'-fluoro-modified nucleotide; (moe) indicates that a nucleotide immediately to the left of the combined identifier (moe) is a 2'-O-methoxyethyl-modified nucleotide; and s indicates that two adjacent nucleotides are linked by a phosphorothioate diester bond.

Wherein, the structural formula of U is

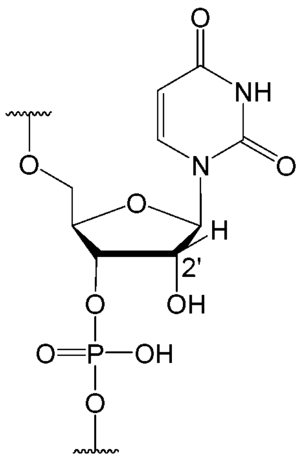

,

and the structural formula of T(moe) is

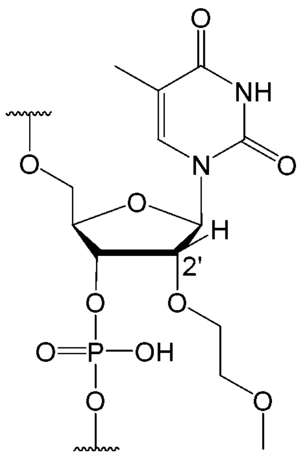

.

Herein, T(moe) is considered as a modification of U.

It is particularly noted that: in the context of the present disclosure, since sequences in which all nucleotides are modified cannot be properly validated and thus a sequence list cannot be generated, all sequences involving modifications in the present disclosure cannot be presented by a sequence list, and for all such sequences, the descriptions in the specification shall prevail.

In some specific embodiments of the present disclosure, the double-stranded oligonucleotide comprises one or more pairs of:

| Pair | Sense strand (5'-3') | | Antisense strand (5'-3') | |
|---|---|---|---|---|
| RX002 245 | CmsGmsAmAmGmCmUfCfAfUfGmAmAmUmAmUmAmUmUm | SEQ ID NO: 887 | AmsAfsUmAmUmAfUmUmCfAmUmGmAmGfC(moe)UfUmCmGmsUmsAm | SEQ ID NO: 908 |
| RX002 246 | CmsGmsAmAmGmCmUfCfAfUfGmAmAmUmAmUmAmUmUm | SEQ ID NO: 887 | AmsAfsUmAmUmAfUmUmCmAfUmGmAmGfC(moe)UfUmCmGmsUmsAm | SEQ ID NO: 909 |
| RX002 247 | CmsGmsAmAmGmCmUfCfAfUfGmAmAmUmAmUmAmUmUm | SEQ ID NO: 887 | AmsAfsUmAmUmAfUmUmCmAmUfGmAmGfC(moe)UfUmCmGmsUmsAm | SEQ ID NO: 910 |
| RX002 248 | CmsGmsAmAmG(moe)CmUfCfAfUfGmAmAmUmAmUmAmUmUm | SEQ ID NO: 888 | AmsAfsUmAmUmAfUmUmCmAfUmGmAmGfC(moe)UfUmCmGmsUmsAm | SEQ ID NO: 909 |
| RX002 249 | CmsGmsAmAmGmCmUfCfAfUfGmA(moe)AmUmAmUmAmUmUm | SEQ ID NO: 889 | AmsAfsUmAmUmAfUmUmCmAfUmGmAmGfC(moe)UfUmCmGmsUmsAm | SEQ ID NO: 909 |
| RX002 250 | CmsGmsAmAmGmCmUfCfAfUfGmAmA(moe)UmAmUmAmUmUm | SEQ ID NO: 890 | AmsAfsUmAmUmAfUmUmCmAfUmGmAmGfC(moe)UfUmCmGmsUmsAm | SEQ ID NO: 909 |
| RX002 251 | CmsGmsAmAmGmCmUfCfAfUfGmAmAmUmAmUmAmT(moe)Um | SEQ ID NO: 891 | AmsAfsUmAmUmAfUmUmCmAfUmGmAmGfC(moe)UfUmCmGmsUmsAm | SEQ ID NO: 909 |
| RX002 252 | CmsGmsAmAmG(moe)CmUfCfAfUfGmAmAmUmAmUmAmUmUm | SEQ ID NO: 888 | AmsAfsUmAmUmAfUmT(moe)CmAfUmGmAmGfCmUfUmCmGmsUmsAm | SEQ ID NO: 911 |
| RX002 253 | CmsGmsAmAmGmCmUfCfAfUfGmA(moe)AmUmAmUmAmUmUm | SEQ ID NO: 889 | AmsAfsUmAmUmAfUmT(moe)CmAfUmGmAmGfCmUfUmCmGmsUmsAm | SEQ ID NO: 911 |
| RX002 254 | CmsGmsAmAmGmCmUfCfAfUfGmAmA(moe)UmAmUmAmUmUm | SEQ ID NO: 890 | AmsAfsUmAmUmAfUmT(moe)CmAfUmGmAmGfCmUfUmCmGmsUmsAm | SEQ ID NO: 911 |
| RX002 255 | CmsGmsAmAmGmCmUfCfAfUfGmAmAmUmAmUmAmT(moe)Um | SEQ ID NO: 891 | AmsAfsUmAmUmAfUmT(moe)CmAfUmGmAmGfCmUfUmCmGmsUmsAm | SEQ ID NO: 911 |

(continued)

| Pair | Sense strand (5'-3') | | Antisense strand (5'-3') | |
|---|---|---|---|---|
| RX002 256 | CmsAmsGmAmGmAmAfAfUfUfCmUmAmCmUmAmCmAmUm | SEQ ID NO: 892 | AmsUfsGmUmAmGfUmAmGfAmAmUmUmUfC(moe)UfCmUmGmsUmsAm | SEQ ID NO: 912 |
| RX002 | CmsAmsGmAmGmAmAfAfUfUfCmU | SEQ ID | AmsUfsGmUmAmGfUmAmGmAfAmUm | SEQ ID |
| 257 | mAmCmUmAmCmAmUm | NO: 892 | UmUfC(moe)UfCmUmGmsUmsAm | NO: 913 |
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |
| RX002 259 | CmsAmsGmAmG(moe)AmAfAfUfUfCmUmAmCmUmAmCmAmUm | SEQ ID NO: 893 | AmsUfsGmUmAmGfUmAmGmAfAmUmUmUfC(moe)UfCmUmGmsUmsAm | SEQ ID NO: 913 |
| RX002 260 | CmsAmsGmAmGmAmAfAfUfUfCmT(moe)AmCmUmAmCmAmUm | SEQ ID NO: 894 | AmsUfsGmUmAmGfUmAmGmAfAmUmUmUfC(moe)UfCmUmGmsUmsAm | SEQ ID NO: 913 |
| RX002 261 | CmsAmsGmAmGmAmAfAfUfUfCmUmA(moe)CmUmAmCmAmUm | SEQ ID NO: 895 | AmsUfsGmUmAmGfUmAmGmAfAmUmUmUfC(moe)UfCmUmGmsUmsAm | SEQ ID NO: 913 |
| RX002 262 | CmsAmsGmAmGmAmAfAfUfUfCmUmAmCmUmAmCmA(moe)Um | SEQ ID NO: 896 | AmsUfsGmUmAmGfUmAmGmAfAmUmUmUfC(moe)UfCmUmGmsUmsAm | SEQ ID NO: 913 |
| RX002 263 | CmsAmsGmAmG(moe)AmAfAfUfUfCmUmAmCmUmAmCmAmUm | SEQ ID NO: 893 | AmsUfsGmUmAmGfUmA(moe)GmAfAmUmUmUfCmUfCmUmGmsUmsAm | SEQ ID NO: 915 |
| RX002 264 | CmsAmsGmAmGmAmAfAfUfUfCmT(moe)AmCmUmAmCmAmUm | SEQ ID NO: 894 | AmsUfsGmUmAmGfUmA(moe)GmAfAmUmUmUfCmUfCmUmGmsUmsAm | SEQ ID NO: 915 |
| RX002 265 | CmsAmsGmAmGmAmAfAfUfUfCmUmA(moe)CmUmAmCmAmUm | SEQ ID NO: 895 | AmsUfsGmUmAmGfUmA(moe)GmAfAmUmUmUfCmUfCmUmGmsUmsAm | SEQ ID NO: 915 |
| RX002 266 | CmsAmsGmAmGmAmAfAfUfUfCmUmAmCmUmAmCmA(moe)Um | SEQ ID NO: 896 | AmsUfsGmUmAmGfUmA(moe)GmAfAmUmUmUfCmUfCmUmGmsUmsAm | SEQ ID NO: 915 |
| RX002 267 | GmsAmsGmAmAmUmUfGfCfUfUmCmAmUmAmCmAmAmAm | SEQ ID NO: 897 | UmsUfsUmGmUmAfUmGmAfAmGmCmAmAfT(moe)UfCmUmCmsCmsUm | SEQ ID NO: 916 |

(continued)

| Pair | Sense strand (5'-3') | | Antisense strand (5'-3') | |
|---|---|---|---|---|
| RX002 268 | GmsAmsGmAmAmUmUfGfCfUfUmCmAmUmAmCmAmAmAm | SEQ ID NO: 897 | UmsUfsUmGmUmAfUmGmAmAfGmCmAmAfT(moe)UfCmUmCmsCmsUm | SEQ ID NO: 917 |
| RX002 269 | GmsAmsGmAmAmUmUfGfCfUfUmCmAmUmAmCmAmAmAm | SEQ ID NO: 897 | UmsUfsUmGmUmAfUmGmAmAmGfCmAmAfT(moe)UfCmUmCmsCmsUm | SEQ ID NO: 918 |
| RX002 270 | GmsAmsGmAmA(moe)UmUfGfCfUfUmCmAmUmAmCmAmAmAm | SEQ ID NO: 898 | UmsUfsUmGmUmAfUmGmAmAfGmCmAmAfT(moe)UfCmUmCmsCmsUm | SEQ ID NO: 917 |
| RX002 271 | GmsAmsGmAmAmUmUfGfCfUfUmC(moe)AmUmAmCmAmAmAm | SEQ ID NO: 899 | UmsUfsUmGmUmAfUmGmAmAfGmCmAmAfT(moe)UfCmUmCmsCmsUm | SEQ ID NO: 917 |
| RX002 272 | GmsAmsGmAmAmUmUfGfCfUfUmCmA(moe)UmAmCmAmAmAm | SEQ ID NO: 900 | UmsUfsUmGmUmAfUmGmAmAfGmCmAmAfT(moe)UfCmUmCmsCmsUm | SEQ ID NO: 917 |
| RX002 273 | GmsAmsGmAmAmUmUfGfCfUfUmCmAmUmAmCmAmA(moe)Am | SEQ ID NO: 901 | UmsUfsUmGmUmAfUmGmAmAfGmCmAmAfT(moe)UfCmUmCmsCmsUm | SEQ ID NO: 917 |
| RX002 274 | GmsAmsGmAmA(moe)UmUfGfCfUfUmCmAmUmAmCmAmAmAm | SEQ ID NO: 898 | UmsUfsUmGmUmAfUmG(moe)AmAfGmCmAmAfUmUfCmUmCmsCmsUm | SEQ ID NO: 919 |
| RX002 275 | GmsAmsGmAmAmUmUfGfCfUfUmC(moe)AmUmAmCmAmAmAm | SEQ ID NO: 899 | UmsUfsUmGmUmAfUmG(moe)AmAfGmCmAmAfUmUfCmUmCmsCmsUm | SEQ ID NO: 919 |
| RX002 276 | GmsAmsGmAmAmUmUfGfCfUfUmCmA(moe)UmAmCmAmAmAm | SEQ ID NO: 900 | UmsUfsUmGmUmAfUmG(moe)AmAfGmCmAmAfUmUfCmUmCmsCmsUm | SEQ ID NO: 919 |
| RX002 277 | GmsAmsGmAmAmUmUfGfCfUfUmCmAmUmAmCmAmA(moe)Am | SEQ ID NO: 901 | UmsUfsUmGmUmAfUmG(moe)AmAfGmCmAmAfUmUfCmUmCmsCmsUm | SEQ ID NO: 919 |
| RX002 278 | CmsAmsAmCmUmCmAfCfCfUfGmUmAmAmUmAmAmAmUm | SEQ ID NO: 902 | AmsUfsUmUmAmUfUmAmCfAmGmGmUmGfA(moe)GfUmUmGmsAmsUm | SEQ ID NO: 920 |
| RX002 279 | CmsAmsAmCmUmCmAfCfCfUfGmUmAmAmUmAmAmAmUm | SEQ ID NO: 902 | AmsUfsUmUmAmUfUmAmCmAfGmGmUmGfA(moe)GfUmUmGmsAmsUm | SEQ ID NO: 921 |

(continued)

| Pair | Sense strand (5'-3') | | Antisense strand (5'-3') | |
|---|---|---|---|---|
| RX002 280 | CmsAmsAmCmUmCmAfCfCfUfGmUmAmAmUmAmAmAmUm | SEQ ID NO: 902 | AmsUfsUmUmAmUfUmAmCmAmGfGmUmGfA(moe)GfUmUmGmsAmsUm | SEQ ID NO: 922 |
| RX002 281 | CmsAmsAmCmT(moe)CmAfCfCfUfGmUmAmAmUmAmAmAmUm | SEQ ID NO: 903 | AmsUfsUmUmAmUfUmAmCmAfGmGmUmGfA(moe)GfUmUmGmsAmsUm | SEQ ID NO: 921 |
| RX002 282 | CmsAmsAmCmUmCmAfCfCfUfGmT(moe)AmAmUmAmAmAmUm | SEQ ID NO: 904 | AmsUfsUmUmAmUfUmAmCmAfGmGmUmGfA(moe)GfUmUmGmsAmsUm | SEQ ID NO: 921 |
| RX002 283 | CmsAmsAmCmUmCmAfCfCfUfGmUmA(moe)AmUmAmAmAmUm | SEQ ID NO: 905 | AmsUfsUmUmAmUfUmAmCmAfGmGmUmGfA(moe)GfUmUmGmsAmsUm | SEQ ID NO: 921 |
| RX002 284 | CmsAmsAmCmUmCmAfCfCfUfGmUmAmAmUmAmAmA(moe)Um | SEQ ID NO: 906 | AmsUfsUmUmAmUfUmAmCmAfGmGmUmGfA(moe)GfUmUmGmsAmsUm | SEQ ID NO: 921 |
| RX002 285 | CmsAmsAmCmT(moe)CmAfCfCfUfGmUmAmAmUmAmAmAmUm | SEQ ID NO: 903 | AmsUfsUmUmAmUfUmA(moe)CmAfGmGmUmGfAmGfUmUmGmsAmsUm | SEQ ID NO: 923 |
| RX002 286 | CmsAmsAmCmUmCmAfCfCfUfGmT(moe)AmAmUmAmAmAmUm | SEQ ID NO: 904 | AmsUfsUmUmAmUfUmA(moe)CmAfGmGmUmGfAmGfUmUmGmsAmsUm | SEQ ID NO: 923 |
| RX002 287 | CmsAmsAmCmUmCmAfCfCfUfGmUmA(moe)AmUmAmAmAmUm | SEQ ID NO: 905 | AmsUfsUmUmAmUfUmA(moe)CmAfGmGmUmGfAmGfUmUmGmsAmsUm | SEQ ID NO: 923 |
| RX002 288 | CmsAmsAmCmUmCmAfCfCfUfGmUmAmAmUmAmAmA(moe)Um | SEQ ID NO: 906 | AmsUfsUmUmAmUfUmA(moe)CmAfGmGmUmGfAmGfUmUmGmsAmsUm | SEQ ID NO: 923 |
| RX002 289 | CmsGmsAmAmG(moe)CmUfCfAfUfGmAmAmUmAmUmAmUmUm | SEQ ID NO: 888 | AmsAfsUmAmUmAfUmUmCmAmUfGmAmGfC(moe)UfUmCmGmsUmsAm | SEQ ID NO: 910 |
| RX002 290 | CmsGmsAmAmGmCmUfCfAfUfGmAmA(moe)UmAmUmAmUmUm | SEQ ID NO: 890 | AmsAfsUmAmUmAfUmUmCmAmUfGmAmGfC(moe)UfUmCmGmsUmsAm | SEQ ID NO: 910 |
| RX002 291 | CmsGmsAmAmGmCmUfCfAfUfGmAmA(moe)UmAmUmAmUmUm | SEQ ID NO: 890 | AmsAfsUmAmUmAfUmT(moe)CmAmUfGmAmGfCmUfUmCmGmsUmsAm | SEQ ID NO: 924 |

(continued)

| Pair | Sense strand (5'-3') | | Antisense strand (5'-3') | |
|---|---|---|---|---|
| RX002 292 | CmsGmsAmAmGmCmUfCfAfUfGmAmAmUmAmUmAmT(moe)Um | SEQ ID NO: 891 | AmsAfsUmAmUmAfUmT(moe)CmAmUfGmAmGfCmUfUmCmGmsUmsAm | SEQ ID NO: 924 |
| RX002 293 | CmsAmsGmAmGmAmAfAfUfUfCmUmA(moe)CmUmAmCmAmUm | SEQ ID NO: 895 | AmsUfsGmUmAmGfUmAmGmAmAfUmUmUfC(moe)UfCmUmGmsUmsAm | SEQ ID NO: 914 |
| RX002 294 | CmsAmsGmAmGmAmAfAfUfUfCmUmAmCmUmAmCmA(moe)Um | SEQ ID NO: 896 | AmsUfsGmUmAmGfUmA(moe)GmAmAfUmUmUfCmUfCmUmGmsUmsAm | SEQ ID NO: 925 |
| RX002 295 | CmsAmsAmCmUmCmAfCfCfUfGmUmA(moe)AmUmAmAmAmUm | SEQ ID NO: 905 | AmsUfsUmUmAmUfUmAmCmAmGfGmUmGfA(moe)GfUmUmGmsAmsUm | SEQ ID NO: 922 |
| RX002 296 | CmsAmsAmCmUmCmAfCfCfUfGmUmAmAmUmAmAmA(moe)Um | SEQ ID NO: 906 | AmsUfsUmUmAmUfUmA(moe)CmAmGfGmUmGfAmGfUmUmGmsAmsUm | SEQ ID NO: 926 |
| RX002 297 | CmsAmsGmAmGmAmAfAfUfUfCmUmAmCmUmAmCmAmUm | SEQ ID NO: 892 | AmsUfsGmUmAmGfUmAmGmAmAmUfUmUfC(moe)UfCmUmGmsUmsAm | SEQ ID NO: 927 |
| RX002 298 | AmsAmsAmUmUmCmUfAfCfUfAmCmAmUmCmUmAmUmAm | SEQ ID NO: 907 | UmsAfsUmAmGmAfUmGmUmAmGmUfAmGfAmAfUmUmUmsCmsUm | SEQ ID NO: 928 |
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |

(continued)

| Pair | Sense strand (5'-3') | | Antisense strand (5'-3') | |
|---|---|---|---|---|
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |
| RX002 308 | CmsAmsGmAmGmAmAfAfUfUfCmUmAmCmUmAmCmAmUm | SEQ ID NO: 892 | AmsUfsGmUmAmGfUmAmGmAmAmUfUmUfCmUfCmUmGmsUmsAm | SEQ ID NO: 938 |
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

In some specific embodiments of the present disclosure, the sense strand is selected from any one of the sequences as set forth in SEQ ID NO: 887, 892, 897, 902, and 907; and/or

the antisense strand is selected from any one of the sequences as set forth in SEQ ID NO: 908, 912, 914, 920, and 927 to 943.

In some specific embodiments of the present disclosure, the double-stranded oligonucleotide comprises one or more pairs of:

| Pair | Sense strand (5'-3') | | Antisense strand (5'-3') | |
|---|---|---|---|---|
| RX002 245 | CmsGmsAmAmGmCmUfCfAfUfG mAmAmUmAmUmAmUmUm | SEQ ID NO: 887 | AmsAfsUmAmUmAfUmUmCfAmUmG mAmGfC(moe)UfUmCmGmsUmsAm | SEQ ID NO: 908 |
| RX002 256 | CmsAmsGmAmGmAmAfAfUfUfC mUmAmCmUmAmCmAmUm | SEQ ID NO: 892 | AmsUfsGmUmAmGfUmAmGfAmAmU mUmUfC(moe)UfCmUmGmsUmsAm | SEQ ID NO: 912 |
| RX002 258 | CmsAmsGmAmGmAmAfAfUfUfC mUmAmCmUmAmCmAmUm | SEQ ID NO: 892 | AmsUfsGmUmAmGfUmAmGmAmAfU mUmUfC(moe)UfCmUmGmsUmsAm | SEQ ID NO: 914 |
| RX002 278 | CmsAmsAmCmUmCmAfCfCfUfGm UmAmAmUmAmAmAmUm | SEQ ID NO: 902 | AmsUfsUmUmAmUfUmAmCfAmGmG mUmGfA(moe)GfUmUmGmsAmsUm | SEQ ID NO: 920 |
| RX002 297 | CmsAmsGmAmGmAmAfAfUfUfC mUmAmCmUmAmCmAmUm | SEQ ID NO: 892 | AmsUfsGmUmAmGfUmAmGmAmAmU fUmUfC(moe)UfCmUmGmsUmsAm | SEQ ID NO: 927 |
| RX002 298 | AmsAmsAmUmUmCmUfAfCfUfA mCmAmUmCmUmAmUmAm | SEQ ID NO: 907 | UmsAfsUmAmGmAfUmGmUmAmGmU fAmGfAmAfUmUmUmsCmsUm | SEQ ID NO: 928 |
| RX002 299 | AmsAmsAmUmUmCmUfAfCfUfA mCmAmUmCmUmAmUmAm | SEQ ID NO: 907 | UmsAfsUmAmGmAfUmGmUfAmGmU mAmGfA(moe)AfUmUmUmsCmsUm | SEQ ID NO: 929 |
| RX002 300 | AmsAmsAmUmUmCmUfAfCfUfA mCmAmUmCmUmAmUmAm | SEQ ID NO: 907 | UmsAfsUmAmGmAfUmGmUmAmGmU fAmGfA(moe)AfUmUmUmsCmsUm | SEQ ID NO: 930 |
| RX002 301 | CmsGmsAmAmGmCmUfCfAfUfG mAmAmUmAmUmAmUmUm | SEQ ID NO: 887 | AmsAfsUmAmUmAfUmUmCfAmUmG mAmGfCmUfUmCmGmsUmsAm | SEQ ID NO: 931 |
| RX002 302 | CmsGmsAmAmGmCmUfCfAfUfG mAmAmUmAmUmAmUmUm | SEQ ID NO: 887 | AmsAfsUmAmUmAfUmUmCmAmUmG fAmGfCmUfUmCmGmsUmsAm | SEQ ID NO: 932 |
| RX002 303 | CmsGmsAmAmGmCmUfCfAfUfG mAmAmUmAmUmAmUmUm | SEQ ID NO: 887 | AmsAfsUmAmUmAfUmUmCmAmUmG fAmGfC(moe)UfUmCmGmsUmsAm | SEQ ID NO: 933 |
| RX002 304 | CmsAmsAmCmUmCmAfCfCfUfGm UmAmAmUmAmAmAmUm | SEQ ID NO: 902 | AmsUfsUmUmAmUfUmAmCfAmGmG mUmGfAmGfUmUmGmsAmsUm | SEQ ID NO: 934 |
| RX002 305 | CmsAmsAmCmUmCmAfCfCfUfGm UmAmAmUmAmAmAmUm | SEQ ID NO: 902 | AmsUfsUmUmAmUfUmAmCmAmGmG fUmGfAmGfUmUmGmsAmsUm | SEQ ID NO: 935 |
| RX002 306 | CmsAmsAmCmUmCmAfCfCfUfGm UmAmAmUmAmAmAmUm | SEQ ID NO: 902 | AmsUfsUmUmAmUfUmAmCmAmGmG fUmGfA(moe)GfUmUmGmsAmsUm | SEQ ID NO: 936 |
| RX002 307 | CmsAmsGmAmGmAmAfAfUfUfC mUmAmCmUmAmCmAmUm | SEQ ID NO: 892 | AmsUfsGmUmAmGfUmAmGfAmAmU mUmUfCmUfCmUmGmsUmsAm | SEQ ID NO: 937 |
| RX002 308 | CmsAmsGmAmGmAmAfAfUfUfC mUmAmCmUmAmCmAmUm | SEQ ID NO: 892 | AmsUfsGmUmAmGfUmAmGmAmAmU fUmUfCmUfCmUmGmsUmsAm | SEQ ID NO: 938 |

(continued)

| Pair | Sense strand (5'-3') | | Antisense strand (5'-3') | |
|---|---|---|---|---|
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

In some specific embodiments of the present disclosure, the double-stranded oligonucleotide comprises the pair of:

| Pair | Sense strand (5'-3') | | Antisense strand (5'-3') | |
|---|---|---|---|---|
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |

In some specific embodiments of the present disclosure, the double-stranded oligonucleotide is selected from siRNA.

In a second aspect, the present disclosure provides a double-stranded oligonucleotide for inhibiting the expression of C3 gene, wherein the double-stranded oligonucleotide comprises a sense strand and an antisense strand, and each nucleotide of the double-stranded nucleotide is independently selected from a modified nucleotide;

wherein the nucleotide sequence from positions 2 to 19 of the antisense strand comprises at least 17 contiguous nucleotides from any one of the nucleotide sequences as set forth in SEQ ID NOs: 908 to 943 or a nucleotide sequence having 1, 2 or 3 nucleotide base differences from the contiguous nucleotides; and

the sense strand comprises at least 17 nucleotides, and the sense strand and the antisense strand are complementary or substantially complementary to form a duplex region, wherein being substantial complementary means that the sense strand and the antisense strand have no more than 3 nucleotide mismatches in the duplex region.

In some optional embodiments of the present disclosure, the antisense strand comprises a nucleotide sequence having 0, 1, 2 or 3 nucleotide base differences from any one of the sequences as set forth in SEQ ID NOs: 908 to 943.

In some optional embodiments of the present disclosure, the antisense strand comprises a nucleotide sequence having 0 or 1 nucleotide base difference from any one of the sequences as set forth in SEQ ID NOs: 908 to 943.

In some optional embodiments of the present disclosure, the antisense strand comprises any one of the nucleotide sequences as set forth in SEQ ID NOs: 908 to 943.

In some optional embodiments of the present disclosure, the sense strand comprises at least 17 contiguous nucleotides of any one of the nucleotide sequences as set forth in SEQ ID NOs: 887 to 907 or a nucleotide sequence having 1, 2 or 3 nucleotide base differences from the at least 17 contiguous nucleotides.

In some optional embodiments of the present disclosure, the sense strand comprises a nucleotide sequence having 0, 1, 2 or 3 nucleotide base differences from any one of the sequences as set forth in SEQ ID NOs: 887 to 907.

In some optional embodiments of the present disclosure, the sense strand comprises a nucleotide sequence having 0 or 1 nucleotide base difference from any one of the nucleotide sequences as set forth in SEQ ID NOs: 887 to 907.

In some optional embodiments of the present disclosure, the sense strand comprises any one of the nucleotide

sequences as set forth in SEQ ID NOs: 887 to 907.

In some optional embodiments of the present disclosure, the antisense strand comprises any one of the nucleotide sequences as set forth in SEQ ID NOs: 908 to 943, and the sense strand comprises any one of the nucleotide sequences as set forth in SEQ ID NOs: 887 to 907.

In some optional embodiments of the present disclosure, the double-stranded oligonucleotide comprises one or more pairs of:

an antisense strand having a nucleotide sequence with 0 or 1 nucleotide base difference from the sequence as set forth in SEQ ID NO: 908, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 888;

an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 889;

an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 890;

an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 891;

an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 888;

an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 889;

an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 890;

an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 891;

an antisense strand having a nucleotide sequence with 0 or 1 nucleotide base difference from the sequence as set forth in SEQ ID NO: 912, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 893;

an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 894;

an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 895;

an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 896;

an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 893;

an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 894;

an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 895;

an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 896;

an antisense strand having a nucleotide sequence with 0 or 1 nucleotide base difference from the sequence as set forth in SEQ ID NO: 916, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 918, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 898;

an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 899;

an antisense strand having a nucleotide sequence with 0 or 1 nucleotide base difference from the sequence as set forth in SEQ ID NO: 917, and a sense strand having a nucleotide sequence with 0 or 1 nucleotide base difference from

the sequence as set forth in SEQ ID NO: 900;

an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 901;

an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 898;

an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 899;

an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 900;

an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 901;

an antisense strand having a nucleotide sequence with 0 or 1 nucleotide base difference from the sequence as set forth in SEQ ID NO: 920, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 922, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 903;

an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 904;

an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 905;

an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 906;

an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 903;

an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 904;

an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 905;

an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 906;

an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 888;

an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 890;

an antisense strand having a nucleotide sequence with 0 or 1 nucleotide base difference from the sequence as set forth in SEQ ID NO: 924, and a sense strand having a nucleotide sequence with 0 or 1 nucleotide base difference from the sequence as set forth in SEQ ID NO: 890;

an antisense strand having a nucleotide sequence with 0 or 1 nucleotide base difference from the sequence as set forth in SEQ ID NO: 924, and a sense strand having a nucleotide sequence with 0 or 1 nucleotide base difference from the sequence as set forth in SEQ ID NO: 891;

an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 895;

an antisense strand having a nucleotide sequence with 0 or 1 nucleotide base difference from the sequence as set forth in SEQ ID NO: 925, and a sense strand having a nucleotide sequence with 0 or 1 nucleotide base difference from the sequence as set forth in SEQ ID NO: 896;

an antisense strand having a nucleotide sequence with 0 or 1 nucleotide base difference from the sequence as set forth in SEQ ID NO: 922, and a sense strand having a nucleotide sequence with 0 or 1 nucleotide base difference from the sequence as set forth in SEQ ID NO: 905;

an antisense strand having a nucleotide sequence with 0 or 1 nucleotide base difference from the sequence as set forth in SEQ ID NO: 926, and a sense strand having a nucleotide sequence with 0 or 1 nucleotide base difference from the sequence as set forth in SEQ ID NO: 906;

an antisense strand having a nucleotide sequence with 0 or 1 nucleotide base difference from the sequence as set forth in SEQ ID NO: 927, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 928, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 929, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 930, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 931, and a sense strand having a nucleotide sequence with 0 or 1 nucleotide base difference from

the sequence as set forth in SEQ ID NO: 887;

an antisense strand having a nucleotide sequence with 0 or 1 nucleotide base difference from the sequence as set forth in SEQ ID NO: 932, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 933, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 934, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 935, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 936, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 937, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 938, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 939, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 940, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 941, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 942, and a sense strand having a nucleotide sequence with 0 or 1 nucleotide base difference from the sequence as set forth in SEQ ID NO: 897; and

an antisense strand having a nucleotide sequence with 0 or 1 nucleotide base difference from the sequence as set forth in SEQ ID NO: 943, and a sense strand having a nucleotide sequence with 0 or 1 nucleotide base difference from the sequence as set forth in SEQ ID NO: 897.

In some specific embodiments of the present disclosure, the double-stranded oligonucleotide comprises one or more pairs of:

| Pair | Sense strand (5'-3') | | Antisense strand (5'-3') | |
|---|---|---|---|---|
| RX002 245 | CmsGmsAmAmGmCmUfCfAfUfGmAmAmUmAmUmAmUmUm | SEQ ID NO: 887 | AmsAfsUmAmUmAfUmUmCfAmUmGmAmGfC(moe)UfUmCmGmsUmsAm | SEQ ID NO: 908 |
| RX002 246 | CmsGmsAmAmGmCmUfCfAfUfGmAmAmUmAmUmAmUmUm | SEQ ID NO: 887 | AmsAfsUmAmUmAfUmUmCmAfUmGmAmGfC(moe)UfUmCmGmsUmsAm | SEQ ID NO: 909 |
| RX002 247 | CmsGmsAmAmGmCmUfCfAfUfGmAmAmUmAmUmAmUmUm | SEQ ID NO: 887 | AmsAfsUmAmUmAfUmUmCmAmUfGmAmGfC(moe)UfUmCmGmsUmsAm | SEQ ID NO: 910 |
| RX002 248 | CmsGmsAmAmG(moe)CmUfCfAfUfGmAmAmUmAmUmAmUmUm | SEQ ID NO: 888 | AmsAfsUmAmUmAfUmUmCmAfUmGmAmGfC(moe)UfUmCmGmsUmsAm | SEQ ID NO: 909 |
| RX002 249 | CmsGmsAmAmGmCmUfCfAfUfGmA(moe)AmUmAmUmAmUmUm | SEQ ID NO: 889 | AmsAfsUmAmUmAfUmUmCmAfUmGmAmGfC(moe)UfUmCmGmsUmsAm | SEQ ID NO: 909 |
| RX002 250 | CmsGmsAmAmGmCmUfCfAfUfGmAmA(moe)UmAmUmAmUmUm | SEQ ID NO: 890 | AmsAfsUmAmUmAfUmUmCmAfUmGmAmGfC(moe)UfUmCmGmsUmsAm | SEQ ID NO: 909 |
| RX002 251 | CmsGmsAmAmGmCmUfCfAfUfGmAmAmUmAmUmAmT(moe)Um | SEQ ID NO: 891 | AmsAfsUmAmUmAfUmUmCmAfUmGmAmGfC(moe)UfUmCmGmsUmsAm | SEQ ID NO: 909 |
| RX002 252 | CmsGmsAmAmG(moe)CmUfCfAfUfGmAmAmUmAmUmAmUmUm | SEQ ID NO: 888 | AmsAfsUmAmUmAfUmT(moe)CmAfUmGmAmGfCmUfUmCmGmsUmsAm | SEQ ID NO: 911 |
| RX002 253 | CmsGmsAmAmGmCmUfCfAfUfGmA(moe)AmUmAmUmAmUmUm | SEQ ID NO: 889 | AmsAfsUmAmUmAfUmT(moe)CmAfUmGmAmGfCmUfUmCmGmsUmsAm | SEQ ID NO: 911 |
| RX002 254 | CmsGmsAmAmGmCmUfCfAfUfGmAmA(moe)UmAmUmAmUmUm | SEQ ID NO: 890 | AmsAfsUmAmUmAfUmT(moe)CmAfUmGmAmGfCmUfUmCmGmsUmsAm | SEQ ID NO: 911 |
| RX002 255 | CmsGmsAmAmGmCmUfCfAfUfGmAmAmUmAmUmAmT(moe)Um | SEQ ID NO: 891 | AmsAfsUmAmUmAfUmT(moe)CmAfUmGmAmGfCmUfUmCmGmsUmsAm | SEQ ID NO: 911 |
| RX002 256 | CmsAmsGmAmGmAmAfAfUfUfCmUmAmCmUmAmCmAmUm | SEQ ID NO: 892 | AmsUfsGmUmAmGfUmAmGfAmAmUmUmUfC(moe)UfCmUmGmsUmsAm | SEQ ID NO: 912 |

(continued)

| Pair | Sense strand (5'-3') | | Antisense strand (5'-3') | |
|---|---|---|---|---|
| RX002 257 | CmsAmsGmAmGmAmAfAfUfUfCmUmAmCmUmAmCmAmUm | SEQ ID NO: 892 | AmsUfsGmUmAmGfUmAmGmAfAmUmUmUfC(moe)UfCmUmGmsUmsAm | SEQ ID NO: 913 |
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |
| RX002 259 | CmsAmsGmAmG(moe)AmAfAfUfUfCmUmAmCmUmAmCmAmUm | SEQ ID NO: 893 | AmsUfsGmUmAmGfUmAmGmAfAmUmUmUfC(moe)UfCmUmGmsUmsAm | SEQ ID NO: 913 |
| RX002 260 | CmsAmsGmAmGmAmAfAfUfUfCmT(moe)AmCmUmAmCmAmUm | SEQ ID NO: 894 | AmsUfsGmUmAmGfUmAmGmAfAmUmUmUfC(moe)UfCmUmGmsUmsAm | SEQ ID NO: 913 |
| RX002 261 | CmsAmsGmAmGmAmAfAfUfUfCmUmA(moe)CmUmAmCmAmUm | SEQ ID NO: 895 | AmsUfsGmUmAmGfUmAmGmAfAmUmUmUfC(moe)UfCmUmGmsUmsAm | SEQ ID NO: 913 |
| RX002 262 | CmsAmsGmAmGmAmAfAfUfUfCmUmAmCmUmAmCmA(moe)Um | SEQ ID NO: 896 | AmsUfsGmUmAmGfUmAmGmAfAmUmUmUfC(moe)UfCmUmGmsUmsAm | SEQ ID NO: 913 |
| RX002 263 | CmsAmsGmAmG(moe)AmAfAfUfUfCmUmAmCmUmAmCmAmUm | SEQ ID NO: 893 | AmsUfsGmUmAmGfUmA(moe)GmAfAmUmUmUfCmUfCmUmGmsUmsAm | SEQ ID NO: 915 |
| RX002 264 | CmsAmsGmAmGmAmAfAfUfUfCmT(moe)AmCmUmAmCmAmUm | SEQ ID NO: 894 | AmsUfsGmUmAmGfUmA(moe)GmAfAmUmUmUfCmUfCmUmGmsUmsAm | SEQ ID NO: 915 |
| RX002 265 | CmsAmsGmAmGmAmAfAfUfUfCmUmA(moe)CmUmAmCmAmUm | SEQ ID NO: 895 | AmsUfsGmUmAmGfUmA(moe)GmAfAmUmUmUfCmUfCmUmGmsUmsAm | SEQ ID NO: 915 |
| RX002 266 | CmsAmsGmAmGmAmAfAfUfUfCmUmAmCmUmAmCmA(moe)Um | SEQ ID NO: 896 | AmsUfsGmUmAmGfUmA(moe)GmAfAmUmUmUfCmUfCmUmGmsUmsAm | SEQ ID NO: 915 |
| RX002 267 | GmsAmsGmAmAmUmUfGfCfUfUmCmAmUmAmCmAmAmAm | SEQ ID NO: 897 | UmsUfsUmGmUmAfUmGmAfAmGmCmAmAfT(moe)UfCmUmCmsCmsUm | SEQ ID NO: 916 |
| RX002 268 | GmsAmsGmAmAmUmUfGfCfUfUmCmAmUmAmCmAmAmAm | SEQ ID NO: 897 | UmsUfsUmGmUmAfUmGmAmAfGmCmAmAfT(moe)UfCmUmCmsCmsUm | SEQ ID NO: 917 |

(continued)

| Pair | Sense strand (5'-3') | | Antisense strand (5'-3') | |
|---|---|---|---|---|
| RX002 269 | GmsAmsGmAmAmUmUfGfCfUfUmCmAmUmAmCmAmAmAm | SEQ ID NO: 897 | UmsUfsUmGmUmAfUmGmAmAmGfCmAmAfT(moe)UfCmUmCmsCmsUm | SEQ ID NO: 918 |
| RX002 270 | GmsAmsGmAmA(moe)UmUfGfCfUfUmCmAmUmAmCmAmAmAm | SEQ ID NO: 898 | UmsUfsUmGmUmAfUmGmAmAfGmCmAmAfT(moe)UfCmUmCmsCmsUm | SEQ ID NO: 917 |
| RX002 271 | GmsAmsGmAmAmUmUfGfCfUfUmC(moe)AmUmAmCmAmAmAm | SEQ ID NO: 899 | UmsUfsUmGmUmAfUmGmAmAfGmCmAmAfT(moe)UfCmUmCmsCmsUm | SEQ ID NO: 917 |
| RX002 272 | GmsAmsGmAmAmUmUfGfCfUfUmCmA(moe)UmAmCmAmAmAm | SEQ ID NO: 900 | UmsUfsUmGmUmAfUmGmAmAfGmCmAmAfT(moe)UfCmUmCmsCmsUm | SEQ ID NO: 917 |
| RX002 273 | GmsAmsGmAmAmUmUfGfCfUfUmCmAmUmAmCmAmA(moe)Am | SEQ ID NO: 901 | UmsUfsUmGmUmAfUmGmAmAfGmCmAmAfT(moe)UfCmUmCmsCmsUm | SEQ ID NO: 917 |
| RX002 274 | GmsAmsGmAmA(moe)UmUfGfCfUfUmCmAmUmAmCmAmAmAm | SEQ ID NO: 898 | UmsUfsUmGmUmAfUmG(moe)AmAfGmCmAmAfUmUfCmUmCmsCmsUm | SEQ ID NO: 919 |
| RX002 275 | GmsAmsGmAmAmUmUfGfCfUfUmC(moe)AmUmAmCmAmAmAm | SEQ ID NO: 899 | UmsUfsUmGmUmAfUmG(moe)AmAfGmCmAmAfUmUfCmUmCmsCmsUm | SEQ ID NO: 919 |
| RX002 276 | GmsAmsGmAmAmUmUfGfCfUfUmCmA(moe)UmAmCmAmAmAm | SEQ ID NO: 900 | UmsUfsUmGmUmAfUmG(moe)AmAfGmCmAmAfUmUfCmUmCmsCmsUm | SEQ ID NO: 919 |
| RX002 277 | GmsAmsGmAmAmUmUfGfCfUfUmCmAmUmAmCmAmA(moe)Am | SEQ ID NO: 901 | UmsUfsUmGmUmAfUmG(moe)AmAfGmCmAmAfUmUfCmUmCmsCmsUm | SEQ ID NO: 919 |
| RX002 278 | CmsAmsAmCmUmCmAfCfCfUfGmUmAmAmUmAmAmAmUm | SEQ ID NO: 902 | AmsUfsUmUmAmUfUmAmCfAmGmGmUmGfA(moe)GfUmUmGmsAmsUm | SEQ ID NO: 920 |
| RX002 279 | CmsAmsAmCmUmCmAfCfCfUfGmUmAmAmUmAmAmAmUm | SEQ ID NO: 902 | AmsUfsUmUmAmUfUmAmCmAfGmGmUmGfA(moe)GfUmUmGmsAmsUm | SEQ ID NO: 921 |
| RX002 280 | CmsAmsAmCmUmCmAfCfCfUfGmUmAmAmUmAmAmAmUm | SEQ ID NO: 902 | AmsUfsUmUmAmUfUmAmCmAmGfGmUmGfA(moe)GfUmUmGmsAmsUm | SEQ ID NO: 922 |

(continued)

| Pair | Sense strand (5'-3') | | Antisense strand (5'-3') | |
|---|---|---|---|---|
| RX002 281 | CmsAmsAmCmT(moe)CmAfCfCfUfGmUmAmAmUmAmAmAmUm | SEQ ID NO: 903 | AmsUfsUmUmAmUfUmAmCmAfGmGmUmGfA(moe)GfUmUmGmsAmsUm | SEQ ID NO: 921 |
| RX002 282 | CmsAmsAmCmUmCmAfCfCfUfGmT(moe)AmAmUmAmAmAmUm | SEQ ID NO: 904 | AmsUfsUmUmAmUfUmAmCmAfGmGmUmGfA(moe)GfUmUmGmsAmsUm | SEQ ID NO: 921 |
| RX002 283 | CmsAmsAmCmUmCmAfCfCfUfGmUmA(moe)AmUmAmAmAmUm | SEQ ID NO: 905 | AmsUfsUmUmAmUfUmAmCmAfGmGmUmGfA(moe)GfUmUmGmsAmsUm | SEQ ID NO: 921 |
| RX002 284 | CmsAmsAmCmUmCmAfCfCfUfGmUmAmAmUmAmAmA(moe)Um | SEQ ID NO: 906 | AmsUfsUmUmAmUfUmAmCmAfGmGmUmGfA(moe)GfUmUmGmsAmsUm | SEQ ID NO: 921 |
| RX002 285 | CmsAmsAmCmT(moe)CmAfCfCfUfGmUmAmAmUmAmAmAmUm | SEQ ID NO: 903 | AmsUfsUmUmAmUfUmA(moe)CmAfGmGmUmGfAmGfUmUmGmsAmsUm | SEQ ID NO: 923 |
| RX002 286 | CmsAmsAmCmUmCmAfCfCfUfGmT(moe)AmAmUmAmAmAmUm | SEQ ID NO: 904 | AmsUfsUmUmAmUfUmA(moe)CmAfGmGmUmGfAmGfUmUmGmsAmsUm | SEQ ID NO: 923 |
| RX002 287 | CmsAmsAmCmUmCmAfCfCfUfGmUmA(moe)AmUmAmAmAmUm | SEQ ID NO: 905 | AmsUfsUmUmAmUfUmA(moe)CmAfGmGmUmGfAmGfUmUmGmsAmsUm | SEQ ID NO: 923 |
| RX002 288 | CmsAmsAmCmUmCmAfCfCfUfGmUmAmAmUmAmAmA(moe)Um | SEQ ID NO: 906 | AmsUfsUmUmAmUfUmA(moe)CmAfGmGmUmGfAmGfUmUmGmsAmsUm | SEQ ID NO: 923 |
| RX002 289 | CmsGmsAmAmG(moe)CmUfCfAfUfGmAmAmUmAmUmAmUmUm | SEQ ID NO: 888 | AmsAfsUmAmUmAfUmUmCmAmUfGmAmGfC(moe)UfUmCmGmsUmsAm | SEQ ID NO: 910 |
| RX002 290 | CmsGmsAmAmGmCmUfCfAfUfGmAmA(moe)UmAmUmAmUmUm | SEQ ID NO: 890 | AmsAfsUmAmUmAfUmUmCmAmUfGmAmGfC(moe)UfUmCmGmsUmsAm | SEQ ID NO: 910 |
| RX002 291 | CmsGmsAmAmGmCmUfCfAfUfGmAmA(moe)UmAmUmAmUmUm | SEQ ID NO: 890 | AmsAfsUmAmUmAfUmT(moe)CmAmUfGmAmGfCmUfUmCmGmsUmsAm | SEQ ID NO: 924 |
| RX002 292 | CmsGmsAmAmGmCmUfCfAfUfGmAmAmUmAmUmAmT(moe)Um | SEQ ID NO: 891 | AmsAfsUmAmUmAfUmT(moe)CmAmUfGmAmGfCmUfUmCmGmsUmsAm | SEQ ID NO: 924 |

(continued)

| Pair | Sense strand (5'-3') | | Antisense strand (5'-3') | |
|---|---|---|---|---|
| RX002 293 | CmsAmsGmAmGmAmAfAfUfUfCmUmA(moe)CmUmAmCmAmUm | SEQ ID NO: 895 | AmsUfsGmUmAmGfUmAmGmAmAfUmUmUfC(moe)UfCmUmGmsUmsAm | SEQ ID NO: 914 |
| RX002 294 | CmsAmsGmAmGmAmAfAfUfUfCmUmAmCmUmAmCmA(moe)Um | SEQ ID NO: 896 | AmsUfsGmUmAmGfUmA(moe)GmAmAfUmUmUfCmUfCmUmGmsUmsAm | SEQ ID NO: 925 |
| RX002 295 | CmsAmsAmCmUmCmAfCfCfUfGmUmA(moe)AmUmAmAmAmUm | SEQ ID NO: 905 | AmsUfsUmUmAmUfUmAmCmAmGfGmUmGfA(moe)GfUmUmGmsAmsUm | SEQ ID NO: 922 |
| RX002 296 | CmsAmsAmCmUmCmAfCfCfUfGmUmAmAmUmAmAmA(moe)Um | SEQ ID NO: 906 | AmsUfsUmUmAmUfUmA(moe)CmAmGfGmUmGfAmGfUmUmGmsAmsUm | SEQ ID NO: 926 |
| RX002 297 | CmsAmsGmAmGmAmAfAfUfUfCmUmAmCmUmAmCmAmUm | SEQ ID NO: 892 | AmsUfsGmUmAmGfUmAmGmAmAmUfUmUfC(moe)UfCmUmGmsUmsAm | SEQ ID NO: 927 |
| RX002 298 | AmsAmsAmUmUmCmUfAfCfUfAmCmAmUmCmUmAmUmAm | SEQ ID NO: 907 | UmsAfsUmAmGmAfUmGmUmAmGmUfAmGfAmAfUmUmUmsCmsUm | SEQ ID NO: 928 |
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |

(continued)

| Pair | Sense strand (5'-3') | | Antisense strand (5'-3') | |
|---|---|---|---|---|
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |
| RX002 308 | CmsAmsGmAmGmAmAfAfUfUfCmUmAmCmUmAmCmAmUm | SEQ ID NO: 892 | AmsUfsGmUmAmGfUmAmGmAmAmUfUmUfCmUfCmUmGmsUmsAm | SEQ ID NO: 938 |
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

In some optional embodiments of the present disclosure, the antisense strand comprises a nucleotide sequence with 0, 1, 2 or 3 nucleotide base differences from any one of the sequences as set forth in SEQ ID NOs: 908, 912, 914, 920 and 927 to 943.

In some optional embodiments of the present disclosure, the antisense strand comprises a nucleotide sequence with 0 or 1 nucleotide base difference from any one of the sequences as set forth in SEQ ID NOs: 908, 912, 914, 920 and 927 to 943.

In some optional embodiments of the present disclosure, the antisense strand comprises any one of the sequences as set forth in SEQ ID NOs: 908, 912, 914, 920 and 927 to 943.

In some optional embodiments of the present disclosure, the sense strand comprises a nucleotide sequence with 0, 1, 2 or 3 nucleotide base differences from any one of the sequences as set forth in SEQ ID NOs: 887, 892, 897, 902 and 907.

In some optional embodiments of the present disclosure, the sense strand comprises a nucleotide sequence with 0 or 1 nucleotide base difference from any one of the sequences as set forth in SEQ ID NOs: 887, 892, 897, 902 and 907.

In some optional embodiments of the present disclosure, the sense strand comprises any one of the sequences

as set forth in SEQ ID NOs: 887, 892, 897, 902 and 907.

In some optional embodiments of the present disclosure, the antisense strand comprises any one of the sequences as set forth in SEQ ID NOs: 908, 912, 914, 920 and 927 to 943, and the sense strand comprises any one of the sequences as set forth in SEQ ID NOs: 887, 892, 897, 902 and 907.

In some optional embodiments of the present disclosure, the double-stranded oligonucleotide comprises one or more pairs of:

an antisense strand having a nucleotide sequence with 0 or 1 nucleotide base difference from the sequence as set forth in SEQ ID NO: 908, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 931, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 932, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 933, and a sense strand having a nucleotide sequence with 0 or 1 nucleotide base difference from the sequence as set forth in SEQ ID NO: 887;

an antisense strand having a nucleotide sequence with 0 or 1 nucleotide base difference from the sequence as set forth in SEQ ID NO: 912, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 927, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 937, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 938, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 940, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 941, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 942, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 943, and a sense strand having a nucleotide sequence with 0 or 1 nucleotide base difference from the sequence as set forth in SEQ ID NO: 897;

an antisense strand having a nucleotide sequence with 0 or 1 nucleotide base difference from the sequence as set forth in SEQ ID NO: 920, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 934, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 935, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 936, and a sense strand having a nucleotide sequence with 0 or 1 nucleotide base difference from the sequence as set forth in SEQ ID NO: 902;

an antisense strand having a nucleotide sequence with 0 or 1 nucleotide base difference from the sequence as set forth in SEQ ID NO: 928, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 929, and a sense strand having a nucleotide sequence with 0 or 1 nucleotide base difference from the sequence as set forth in SEQ ID NO: 907;

an antisense strand having a nucleotide sequence with 0 or 1 nucleotide base difference from the sequence as set forth in SEQ ID NO: 930, and a sense strand having a nucleotide sequence with 0 or 1 nucleotide base difference from the sequence as set forth in SEQ ID NO: 907; and

an antisense strand having a nucleotide sequence with 0 or 1 nucleotide base difference from the sequence as set forth in SEQ ID NO: 939, and a sense strand having a nucleotide sequence with 0 or 1 nucleotide base difference from the sequence as set forth in SEQ ID NO: 907.

In some specific embodiments of the present disclosure, the double-stranded oligonucleotide comprises one or more pairs of:

| Pair | Sense strand (5'-3') | | Antisense strand (5'-3') | |
|---|---|---|---|---|
| RX002 245 | CmsGmsAmAmGmCmUfCfAfUfG mAmAmUmAmUmAmUmUm | SEQ ID NO: 887 | AmsAfsUmAmUmAfUmUmCfAmUmG mAmGfC(moe)UfUmCmGmsUmsAm | SEQ ID NO: 908 |
| RX002 256 | CmsAmsGmAmGmAmAfAfUfUfC mUmAmCmUmAmCmAmUm | SEQ ID NO: 892 | AmsUfsGmUmAmGfUmAmGfAmAmU mUmUfC(moe)UfCmUmGmsUmsAm | SEQ ID NO: 912 |
| RX002 258 | CmsAmsGmAmGmAmAfAfUfUfC mUmAmCmUmAmCmAmUm | SEQ ID NO: 892 | AmsUfsGmUmAmGfUmAmGmAmAfU mUmUfC(moe)UfCmUmGmsUmsAm | SEQ ID NO: 914 |
| RX002 278 | CmsAmsAmCmUmCmAfCfCfUfGm UmAmAmUmAmAmAmUm | SEQ ID NO: 902 | AmsUfsUmUmAmUfUmAmCfAmGmG mUmGfA(moe)GfUmUmGmsAmsUm | SEQ ID NO: 920 |
| RX002 297 | CmsAmsGmAmGmAmAfAfUfUfC mUmAmCmUmAmCmAmUm | SEQ ID NO: 892 | AmsUfsGmUmAmGfUmAmGmAmAmU fUmUfC(moe)UfCmUmGmsUmsAm | SEQ ID NO: 927 |
| RX002 298 | AmsAmsAmUmUmCmUfAfCfUfA mCmAmUmCmUmAmUmAm | SEQ ID NO: 907 | UmsAfsUmAmGmAfUmGmUmAmGmU fAmGfAmAfUmUmUmsCmsUm | SEQ ID NO: 928 |

(continued)

| Pair | Sense strand (5'-3') | | Antisense strand (5'-3') | |
|---|---|---|---|---|
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |
| RX002 308 | CmsAmsGmAmGmAmAfAfUfUfCmUmAmCmUmAmCmAmUm | SEQ ID NO: 892 | AmsUfsGmUmAmGfUmAmGmAmAmUfUmUfCmUfCmUmGmsUmsAm | SEQ ID NO: 938 |
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

In some optional embodiments of the present disclosure, the antisense strand comprises a nucleotide sequence having 0, 1, 2 or 3 nucleotide base differences from the sequence as set forth in SEQ ID NO: 914.

In some optional embodiments of the present disclosure, the antisense strand comprises a nucleotide sequence

having 0 or 1 nucleotide base difference from the sequence as set forth in SEQ ID NO: 914.

In some optional embodiments of the present disclosure, the antisense strand comprises the sequence as set forth in SEQ ID NO: 914.

In some optional embodiments of the present disclosure, the sense strand comprises a nucleotide sequence having 0, 1, 2 or 3 nucleotide base differences from the sequence as set forth in SEQ ID NO: 892.

In some optional embodiments of the present disclosure, the sense strand comprises a nucleotide sequence having 0 or 1 nucleotide base difference from the sequence as set forth in SEQ ID NO: 892.

In some optional embodiments of the present disclosure, the sense strand comprises the sequence as set forth in SEQ ID NO: 892.

In some optional embodiments of the present disclosure, the double-stranded oligonucleotide comprises: an antisense strand comprising a nucleotide sequence having 0 or 1 nucleotide base difference from the sequence as set forth in SEQ ID NO: 914, and a sense strand comprising a nucleotide sequence having 0 or 1 nucleotide base difference from the sequence as set forth in SEQ ID NO: 892.

In some specific embodiments of the present disclosure, the double-stranded oligonucleotide comprises: an antisense strand having a nucleotide sequence as set forth in SEQ ID NO: 914, and a sense strand having a nucleotide sequence as set forth in SEQ ID NO: 892.

In some specific embodiments of the present disclosure, the double-stranded oligonucleotide is selected from siRNA.

In a third aspect, the present disclosure provides a conjugate comprising the double-stranded oligonucleotide of the first and/or second aspect, and one or more gene delivery carriers.

In some optional embodiments of the present disclosure, the gene delivery carrier is selected from a lipid, a nanoparticle, a polymer, and a ligand.

In some optional embodiments of the present disclosure, the lipid includes cholesterol and cholesterol derivatives; the polymer includes an amphiphilic polymer and a membrane active polymer; the ligand is selected from a ligand capable of binding to a cell surface receptor.

In some specific embodiments of the present disclosure, the gene delivery carrier is selected from a ligand capable of binding to a cell surface receptor.

In some specific embodiments of the present disclosure, the ligand specifically targets the liver tissue.

In some specific embodiments of the present disclosure, the ligand is selected from a ligand of asialoglycoprotein receptor (ASGPR). Binding between the ASGPR ligand and ASGPR can promote the specific targeting and the endocytosis of the conjugate into the hepatocytes.

In some specific embodiments of the present disclosure, the ASGPR ligand comprises galactose or a derivative thereof.

In some specific embodiments of the present disclosure, the galactose or the derivative thereof include but are not limited to galactose, galactosamine, N-formyl galactosamine, N-acetyl galactosamine, N-propionyl galactosamine, N-n-butyryl galactosamine and N-isobutyryl galactosamine.

In some specific embodiments of the present disclosure, the galactose or the derivative thereof is N-acetyl galactosamine (GalNAc).

In some specific embodiments of the present disclosure, the ASGPR ligand comprises N-acetyl galactosamine.

In some optional embodiments of the present disclosure, the ligand is conjugated to 3'-end of the sense strand, 5'-end of the sense strand, 3'-end of the antisense strand and/or 5'-end of the antisense strand.

In some specific embodiments of the present disclosure, the number of the ASGPR ligand is one, and the one ASGPR ligand is conjugated to 3'-end of the sense strand.

In some specific embodiments of the present disclosure, the double-stranded oligonucleotide is siRNA. The conjugate is a conjugate of siRNA.

In some optional embodiments of the present disclosure, the conjugate comprises a structure as shown by formula (101):

$$\left(R_{ligand}\right)_m\!-\!Nu$$

(101) ;

wherein, Nu represents the double-stranded oligonucleotide;

each $R_{ligand}$ is independently selected from an ASGPR ligand; and

m is selected from 1, 2, 3 and 4.

In some optional embodiments of the present disclosure, when m is one, the one $R_{ligand}$ is conjugated to 3'-end of the sense strand, the one $R_{ligand}$ is conjugated to 5'-end of the sense strand, the one $R_{ligand}$ is conjugated to 3'-end of the antisense strand, or the one $R_{ligand}$ is conjugated to 5'-end of the antisense strand;

when m is two, the two $R_{ligand}$ are conjugated to 3'-end of the sense strand and 5'-end of the sense strand respectively; the two $R_{ligand}$ are conjugated to 3'-end of the sense strand and 3'-end of the antisense strand respectively; the two $R_{ligand}$ are conjugated to 5'-end of the sense strand and 3'-end of the antisense strand respectively; the two $R_{ligand}$ are conjugated to 5'-end of the sense strand and 3'-end of the antisense strand respectively; the two $R_{ligand}$ are conjugated to 5'-end of the sense strand and 5'-end of the antisense strand respectively; the two $R_{ligand}$ are conjugated to 3'-end of the antisense strand and 5'-end of the antisense strand respectively;

when m is three, the three $R_{ligand}$ are conjugated to 3'-end of the sense strand, 5'-end of the sense strand, and the 3'-end of the antisense strand, respectively; the three $R_{ligand}$ are conjugated to 3'-end of the sense strand, 5'-end of the sense strand, and the 5'-end of the antisense strand, respectively; the three $R_{ligand}$ are conjugated to 3'-end of the sense strand, 3'-end of the antisense strand, and the 5'-end of the antisense strand, respectively; the three $R_{ligand}$ are conjugated to 5'-end of the sense strand, 3'-end of the antisense strand, and the 5'-end of the antisense strand, respectively;

when m is four, the four $R_{ligand}$ are conjugated to 3'-end of the sense strand, 5'-end of the sense strand, 3'-end of the antisense strand, and 5'-end of the antisense strand.

In some specific embodiments of the present disclosure, m is one.
In some specific embodiments of the present disclosure, m is one, and the $R_{ligand}$ is conjugated to 3'-end of the sense strand.
In some specific embodiments of the present disclosure, m is two.
In some specific embodiments of the present disclosure, m is two, and the two $R_{ligand}$ are conjugated to 3'-end and 5'-end of the sense strand, respectively.
In some optional embodiments of the present disclosure, each $R_{ligand}$ is independently selected from a structure as shown by formula (201), a pharmaceutically acceptable salt thereof and a steroisomer thereof:

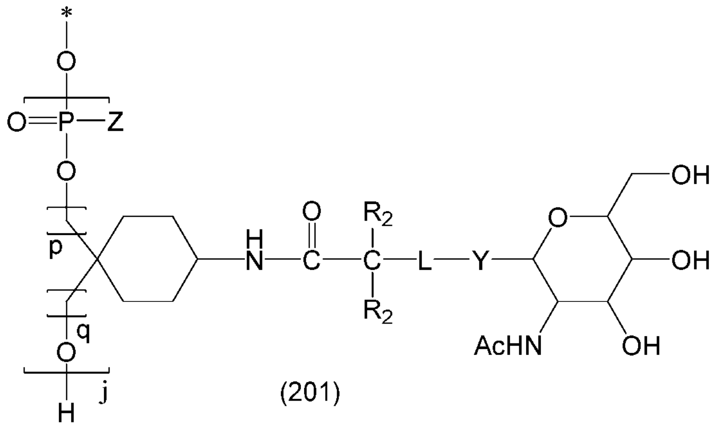
(201)
;

wherein * represents a conjugation site of the ligand on the sense strand or the antisense strand;
j is selected from 1, 2, 3 and 4;
each Z is selected from hydroxy and sulfydryl;
each p is selected from 1, 2 and 3;
each q is selected from 1, 2 and 3;
each $R_2$ is independently selected from H, any optionally substituted $C_{1-6}$ alkyl and optionally substituted $C_{1-6}$ alkoxy;
each L is selected from optionally substituted $C_{2-20}$ alkylene and

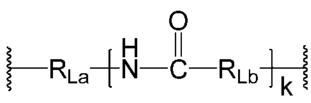
,

wherein $R_{La}$ and $R_{Lb}$ are independently selected from any optionally substituted $C_{1-10}$ alkylene, and k is selected from 1, 2, 3, 4 and 5; and
each Y is selected from O, S and NH.

In some optional embodiments of the present disclosure, m is selected from 1, 2 or 3.
In some specific embodiments of the present disclosure, m is 3.
In some specific embodiments of the present disclosure, Z is hydroxy.
In some specific embodiments of the present disclosure, p is 1.
In some specific embodiments of the present disclosure, q is 1.
In some specific embodiments of the present disclosure, $R_2$ is H.
In some optional embodiments of the present disclosure, each L is selected from optionally substituted $C_{2-10}$ alkylene and

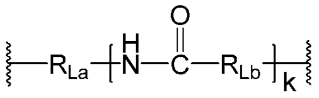,

wherein $R_{La}$ and $R_{Lb}$ are independently selected from any optionally substituted $C_{1-10}$ alkylene, and k is selected from 1, 2 and 3.
In some specific embodiments of the present disclosure, k is 1.
In some optional embodiments of the present disclosure, each L is selected from

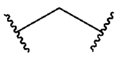, 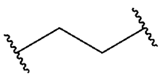, 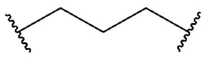, 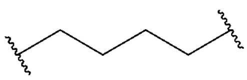, 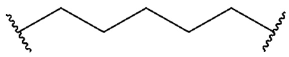,

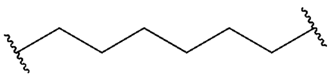, 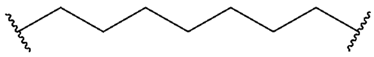, 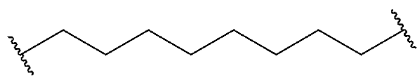,

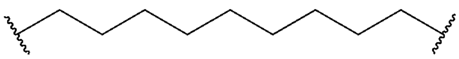, 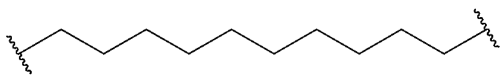,

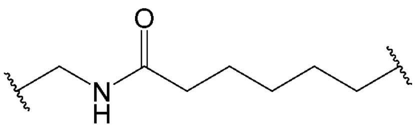, 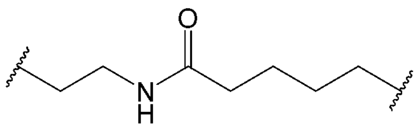,

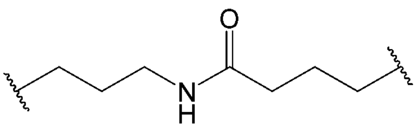, 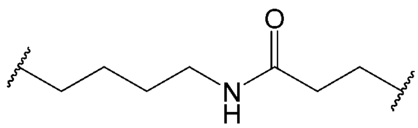

and

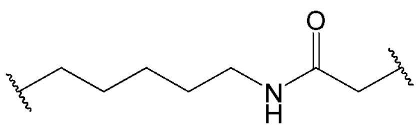.

In some optional embodiments of the present disclosure, each L is selected from

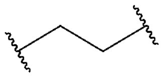

and

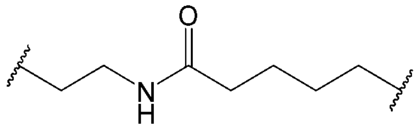

In some specific embodiments of the present disclosure, L is

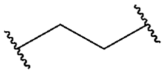

In some specific embodiments of the present disclosure, L is

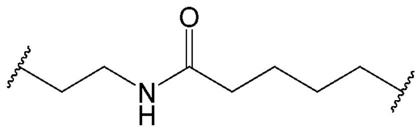

In some specific embodiments of the present disclosure, Y is O.

In some optional embodiments of the present disclosure, each $R_{ligand}$ is independently selected from a structure as shown by formula (202), a pharmaceutically acceptable salt thereof and a steroisomer thereof:

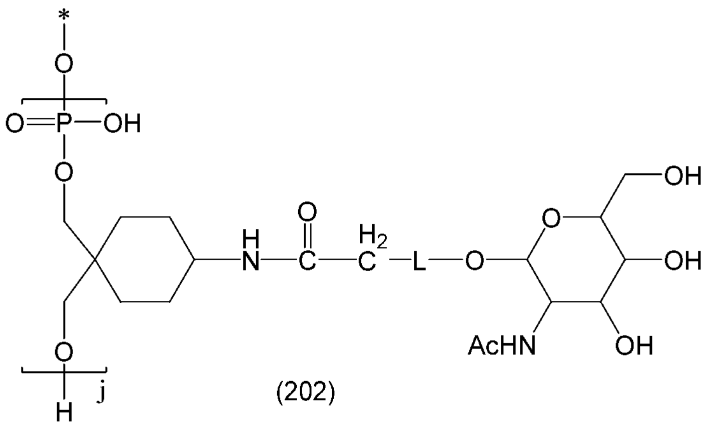

(202)

In some optional embodiments of the present disclosure, each $R_{ligand}$ is independently selected from a structure as shown by formula (CR01008×3), a pharmaceutically acceptable salt thereof and a steroisomer thereof:

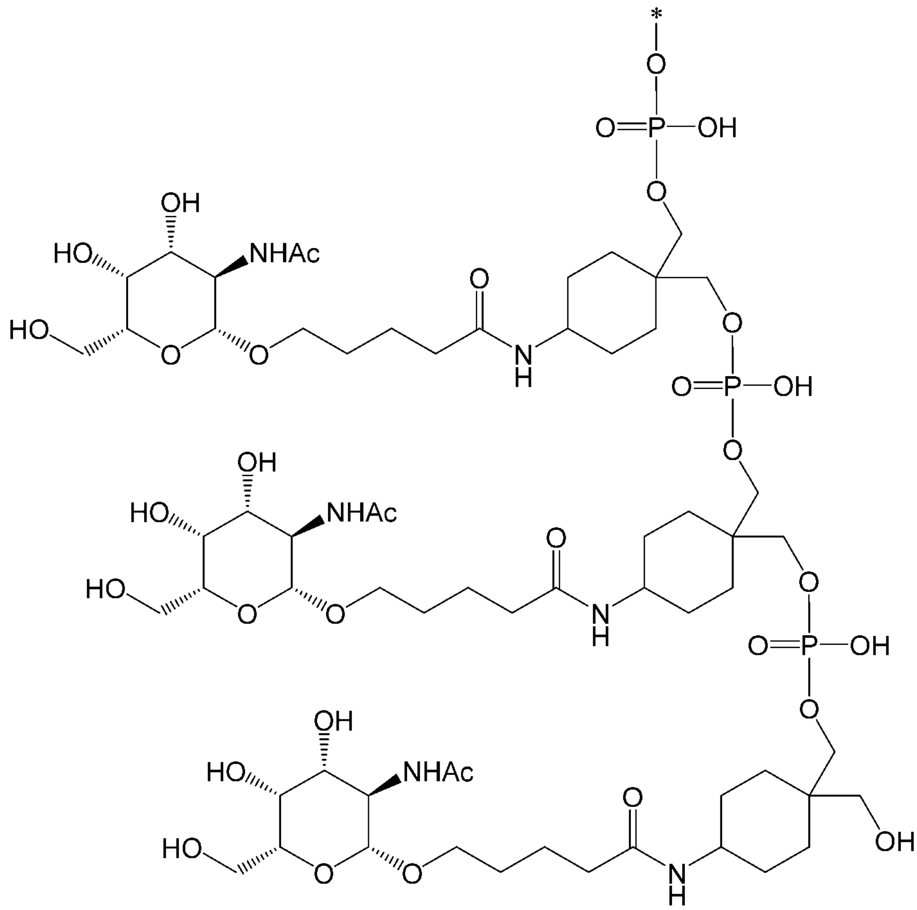

(CR01008X3)

In some optional embodiments of the present disclosure, each $R_{ligand}$ is independently selected from a structure as shown by formula (CR01013×3), a pharmaceutically acceptable salt thereof and a steroisomer thereof:

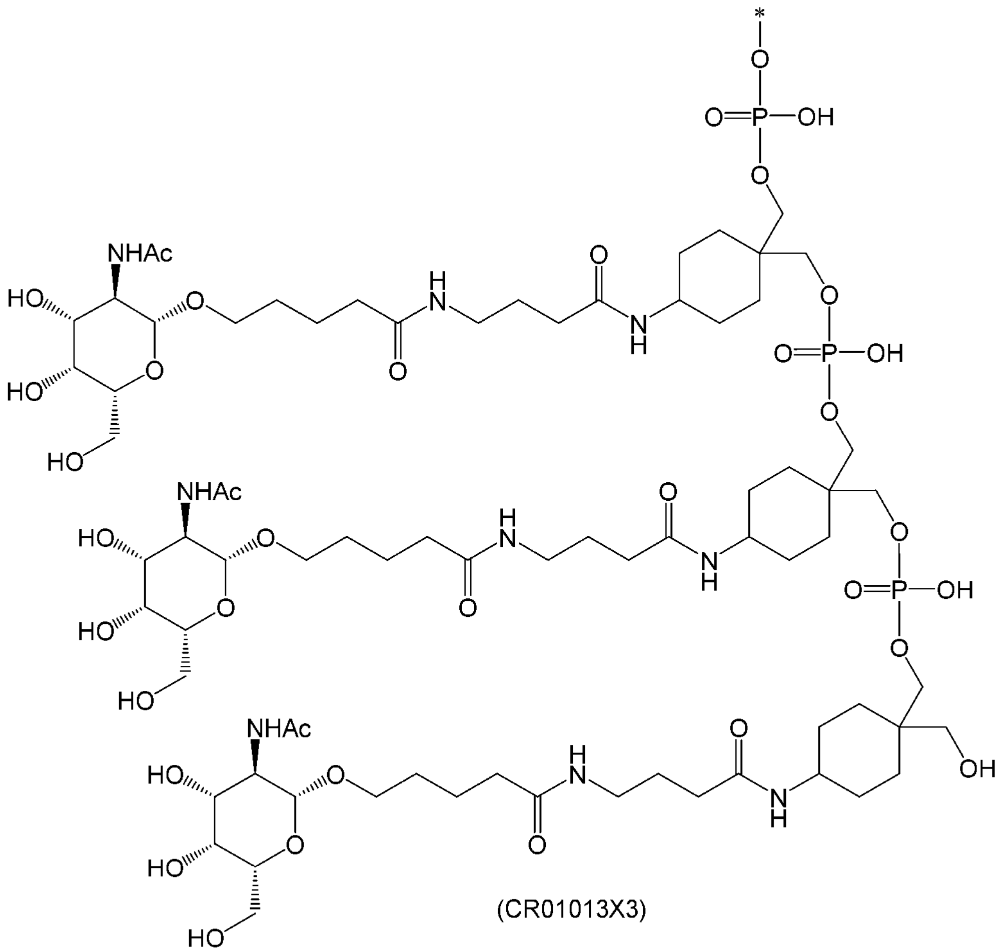

(CR01013X3)

In some optional embodiments of the present disclosure, each $R_{ligand}$ is independently selected from a structure as shown by formula L96, and a pharmaceutically acceptable salt thereof:

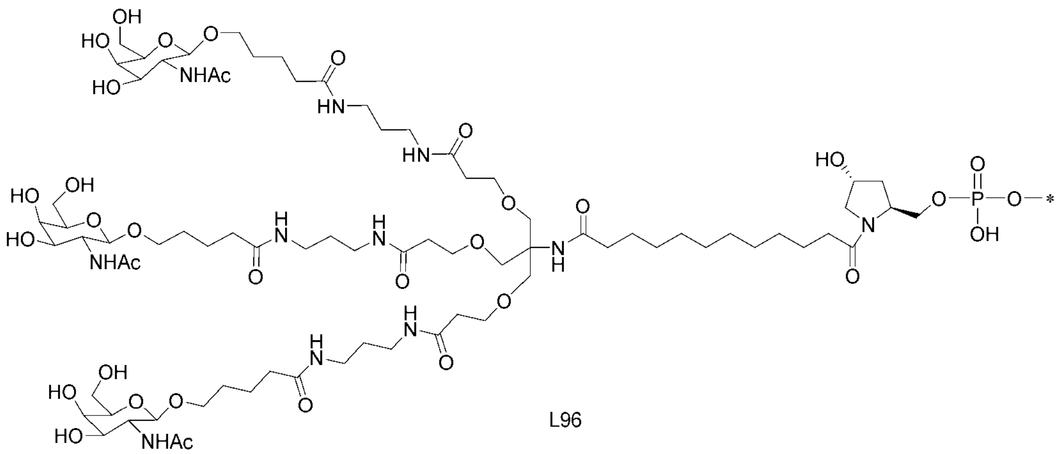

L96

In some specific embodiments of the present disclosure, the conjugate comprises one or more pairs of:

| Pair | Sense strand (5'-3') | | Antisense strand (5'-3') | |
|---|---|---|---|---|
| RZ00 2033 | CmsGmsAmAmGmCmUfCfAfUf GmAmAmUmAmUmAmUmUm_ (CR01008×3) | SEQ ID NO: 887_(CR01008× 3) | AmsAfsUmAmUmAfUmUmCfAm UmGmAmGfC(moe)UfUmCmGm sUmsAm | SEQ ID NO: 908 |

(continued)

| Pair | Sense strand (5'-3') | | Antisense strand (5'-3') | |
|---|---|---|---|---|
| RZ00 2034 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAfUmGmAmGfC(moe)UfUmCmGmsUmsAm | SEQ ID NO: 909 |
| RZ00 2036 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmUmCmAfUmGmAmGfC(moe)UfUmCmGmsUmsAm | SEQ ID NO: 909 |
| RZ00 2037 | CmsGmsAmAmGmCmUfCfAfUfGmA(moe)AmUmAmUmAmUmUm (CR01008×3) | SEQ ID NO: 889_(CR01008×3) | AmsAfsUmAmUmAfUmUmCmAfUmGmAmGfC(moe)UfUmCmGmsUmsAm | SEQ ID NO: 909 |
| RZ00 2038 | CmsGmsAmAmGmCmUfCfAfUfGmAmA(moe)UmAmUmAmUmUm (CR01008×3) | SEQ ID NO: 890(CR01008×3 ) | AmsAfsUmAmUmAfUmUmCmAfUmGmAmGfC(moe)UfUmCmGmsUmsAm | SEQ ID NO: 909 |
| RZ00 2039 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmUmCmAfUmGmAmGfC(moe)UfUmCmGmsUmsAm | SEQ ID NO: 909 |
| RZ00 2040 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmT(moe)CmAfUmGmAmGfCmUfUmCmGmsUmsAm | SEQ ID NO: 911 |
| RZ00 2041 | CmsGmsAmAmGmCmUfCfAfUfGmA(moe)AmUmAmUmAmUmUm (CR01008×3) | SEQ ID NO: 889_(CR01008×3) | AmsAfsUmAmUmAfUmT(moe)CmAfUmGmAmGfCmUfUmCmGmsUmsAm | SEQ ID NO: 911 |
| RZ00 2042 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmT(moe)CmAfUmGmAmGfCmUfUmCmGmsUmsAm | SEQ ID NO: 911 |
| RZ00 2043 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmT(moe)CmAfUmGmAmGfCmUfUmCmGmsUmsAm | SEQ ID NO: 911 |
| RZ00 2050 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfC(moe)UfCmUmGmsUmsAm | SEQ ID NO: 912 |
| RZ00 2051 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAfAmUmUmUfC(moe)UfCmUmGmsUmsAm | SEQ ID NO: 913 |
| RZ00 2053 | CmsAmsGmAmG(moe)AmAfAfUfUfCmUmAmCmUmAmCmAmUm (CR01008×3) | SEQ ID NO: 893_(CR01008×3) | AmsUfsGmUmAmGfUmAmGmAfAmUmUmUfC(moe)UfCmUmGmsUmsAm | SEQ ID NO: 913 |

(continued)

| Pair | Sense strand (5'-3') | | Antisense strand (5'-3') | |
|---|---|---|---|---|
| RZ00 2054 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmAmGmAfAmUmUmUfC(moe)UfCmUmGmsUmsAm | SEQ ID NO: 913 |
| RZ00 2055 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmAmGmAfAmUmUmUfC(moe)UfCmUmGmsUmsAm | SEQ ID NO: 913 |
| RZ00 2056 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmAmGmAfAmUmUmUfC(moe)UfCmUmGmsUmsAm | SEQ ID NO: 913 |
| RZ00 2057 | CmsAmsGmAmG(moe)AmAfAfUfUfCmUmAmCmUmAmCmAmUm (CR01008×3) | SEQ ID NO: 893_(CR01008×3) | AmsUfsGmUmAmGfUmA(moe)GmAfAmUmUmUfCmUfCmUmGmsUmsAm | SEQ ID NO: 915 |
| RZ00 2058 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmA(moe)GmAfAmUmUmUfCmUfCmUmGmsUmsAm | SEQ ID NO: 915 |
| RZ00 2059 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmA(moe)GmAfAmUmUmUfCmUfCmUmGmsUmsAm | SEQ ID NO: 915 |
| RZ00 2060 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmA(moe)GmAfAmUmUmUfCmUfCmUmGmsUmsAm | SEQ ID NO: 915 |
| RZ00 2066 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfT(moe)UfCmUmCmsCmsUm | SEQ ID NO: 916 |
| RZ00 2067 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAfGmCmAmAfT(moe)UfCmUmCmsCmsUm | SEQ ID NO: 917 |
| RZ00 2068 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGfCmAmAfT(moe)UfCmUmCmsCmsUm | SEQ ID NO: 918 |
| RZ00 2069 | GmsAmsGmAmA(moe)UmUfGfCfUfUmCmAmUmAmCmAmAmAm (CR01008×3) | SEQ ID NO: 898_(CR01008×3) | UmsUfsUmGmUmAfUmGmAmAfGmCmAmAfT(moe)UfCmUmCmsCmsUm | SEQ ID NO: 917 |
| RZ00 2070 | GmsAmsGmAmAmUmUfGfCfUfUmC(moe)AmUmAmCmAmAmAm (CR01008×3) | SEQ ID NO: 899_(CR01008×3) | UmsUfsUmGmUmAfUmGmAmAfGmCmAmAfT(moe)UfCmUmCmsCmsUm | SEQ ID NO: 917 |

(continued)

| Pair | Sense strand (5'-3') | | Antisense strand (5'-3') | |
|---|---|---|---|---|
| RZ00 2071 | GmsAmsGmAmAmUmUfGfCfUf UmCmA(moe)UmAmCmAmAm Am (CR01008×3) | SEQ ID NO: 900_(CR01008× 3) | UmsUfsUmGmUmAfUmGmAmA fGmCmAmAfT(moe)UfCmUmCm sCmsUm | SEQ ID NO: 917 |
| RZ00 2072 | GmsAmsGmAmAmUmUfGfCfUf UmCmAmUmAmCmAmA(moe) Am (CR01008×3) | SEQ ID NO: 901_(CR01008× 3) | UmsUfsUmGmUmAfUmGmAmA fGmCmAmAfT(moe)UfCmUmCm sCmsUm | SEQ ID NO: 917 |
| RZ00 2073 | GmsAmsGmAmA(moe)UmUfGf CfUfUmCmAmUmAmCmAmAm Am (CR01008×3) | SEQ ID NO: 898_(CR01008× 3) | UmsUfsUmGmUmAfUmG(moe)A mAfGmCmAmAfUmUfCmUmCm sCmsUm | SEQ ID NO: 919 |
| RZ00 2074 | GmsAmsGmAmAmUmUfGfCfUf UmC(moe)AmUmAmCmAmAm Am (CR01008×3) | SEQ ID NO: 899_(CR01008× 3) | UmsUfsUmGmUmAfUmG(moe)A mAfGmCmAmAfUmUfCmUmCm sCmsUm | SEQ ID NO: 919 |
| RZ00 2075 | GmsAmsGmAmAmUmUfGfCfUf UmCmA(moe)UmAmCmAmAm Am (CR01008×3) | SEQ ID NO: 900_(CR01008× 3) | UmsUfsUmGmUmAfUmG(moe)A mAfGmCmAmAfUmUfCmUmCm sCmsUm | SEQ ID NO: 919 |
| RZ00 2076 | GmsAmsGmAmAmUmUfGfCfUf UmCmAmUmAmCmAmA(moe) Am (CR01008×3) | SEQ ID NO: 901_(CR01008× 3) | UmsUfsUmGmUmAfUmG(moe)A mAfGmCmAmAfUmUfCmUmCm sCmsUm | SEQ ID NO: 919 |
| RZ00 2082 | CmsAmsAmCmUmCmAfCfCfUf GmUmAmAmUmAmAmAmUm_ (CR01008×3) | SEQ ID NO: 902_(CR01008× 3) | AmsUfsUmUmAmUfUmAmCfAm GmGmUmGfA(moe)GfUmUmGm sAmsUm | SEQ ID NO: 920 |
| RZ00 2083 | CmsAmsAmCmUmCmAfCfCfUf GmUmAmAmUmAmAmAmUm_ (CR01008×3) | SEQ ID NO: 902_(CR01008× 3) | AmsUfsUmUmAmUfUmAmCmAf GmGmUmGfA(moe)GfUmUmGm sAmsUm | SEQ ID NO: 921 |
| RZ00 2084 | CmsAmsAmCmUmCmAfCfCfUf GmUmAmAmUmAmAmAmUm_ (CR01008×3) | SEQ ID NO: 902_(CR01008× 3) | AmsUfsUmUmAmUfUmAmCmA mGfGmUmGfA(moe)GfUmUmG msAmsUm | SEQ ID NO: 922 |
| RZ00 2085 | CmsAmsAmCmT(moe)CmAfCfC fUfGmUmAmAmUmAmAmAm Um (CR01008×3) | SEQ ID NO: 903_(CR01008× 3) | AmsUfsUmUmAmUfUmAmCmAf GmGmUmGfA(moe)GfUmUmGm sAmsUm | SEQ ID NO: 921 |
| RZ00 2086 | CmsAmsAmCmUmCmAfCfCfUf GmT(moe)AmAmUmAmAmAm Um (CR01008×3) | SEQ ID NO: 904_(CR01008× 3) | AmsUfsUmUmAmUfUmAmCmAf GmGmUmGfA(moe)GfUmUmGm sAmsUm | SEQ ID NO: 921 |
| RZ00 2087 | CmsAmsAmCmUmCmAfCfCfUf GmUmA(moe)AmUmAmAmAm Um (CR01008×3) | SEQ ID NO: 905_(CR01008× 3) | AmsUfsUmUmAmUfUmAmCmAf GmGmUmGfA(moe)GfUmUmGm sAmsUm | SEQ ID NO: 921 |

(continued)

| Pair | Sense strand (5'-3') | | Antisense strand (5'-3') | |
|---|---|---|---|---|
| RZ00 2088 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmAmCmAfGmGmUmGfA(moe)GfUmUmGmsAmsUm | SEQ ID NO: 921 |
| RZ00 2089 | CmsAmsAmCmT(moe)CmAfCfCfUfGmUmAmAmUmAmAmAmUm (CR01008×3) | SEQ ID NO: 903_(CR01008×3) | AmsUfsUmUmAmUfUmA(moe)CmAfGmGmUmGfAmGfUmUmGmsAmsUm | SEQ ID NO: 923 |
| RZ00 2090 | CmsAmsAmCmUmCmAfCfCfUfGmT(moe)AmAmUmAmAmAmUm (CR01008×3) | SEQ ID NO: 904_(CR01008×3) | AmsUfsUmUmAmUfUmA(moe)CmAfGmGmUmGfAmGfUmUmGmsAmsUm | SEQ ID NO: 923 |
| RZ00 2091 | CmsAmsAmCmUmCmAfCfCfUfGmUmA(moe)AmUmAmAmAmUm (CR01008×3) | SEQ ID NO: 905_(CR01008×3) | AmsUfsUmUmAmUfUmA(moe)CmAfGmGmUmGfAmGfUmUmGmsAmsUm | SEQ ID NO: 923 |
| RZ00 2092 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmA(moe)CmAfGmGmUmGfAmGfUmUmGmsAmsUm | SEQ ID NO: 923 |
| RZ00 2099 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUfGmAmGfC(moe)UfUmCmGmsUmsAm | SEQ ID NO: 910 |
| RZ00 2100 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmUmCmAmUfGmAmGfC(moe)UfUmCmGmsUmsAm | SEQ ID NO: 910 |
| RZ00 2101 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmUmCmAmUfGmAmGfC(moe)UfUmCmGmsUmsAm | SEQ ID NO: 910 |
| RZ00 2102 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmT(moe)CmAmUfGmAmGfCmUfUmCmGmsUmsAm | SEQ ID NO: 924 |
| RZ00 2103 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmT(moe)CmAmUfGmAmGfCmUfUmCmGmsUmsAm | SEQ ID NO: 924 |
| RZ00 2106 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAfUmUmUfC(moe)UfCmUmGmsUmsAm | SEQ ID NO: 914 |
| RZ00 2107 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmAmGmAmAfUmUmUfC(moe)UfCmUmGmsUmsAm | SEQ ID NO: 914 |

(continued)

| Pair | Sense strand (5'-3') | | Antisense strand (5'-3') | |
|---|---|---|---|---|
| RZ00 2108 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmA(moe)GmAmAfUmUmUfCmUfCmUmGmsUmsAm | SEQ ID NO: 925 |
| RZ00 2112 | CmsAmsAmCmUmCmAfCfCfUfGmUmA(moe)AmUmAmAmAmUm (CR01008×3) | SEQ ID NO: 905_(CR01008×3) | AmsUfsUmUmAmUfUmAmCmAmGfGmUmGfA(moe)GfUmUmGmsAmsUm | SEQ ID NO: 922 |
| RZ00 2113 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmA(moe)CmAmGfGmUmGfAmGfUmUmGmsAmsUm | SEQ ID NO: 926 |
| RZ00 2115 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfC(moe)UfCmUmGmsUmsAm | SEQ ID NO: 927 |
| RZ00 2116 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfAmAfUmUmUmsCmsUm | SEQ ID NO: 928 |
| RZ00 2117 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfA(moe)AfUmUmUmsCmsUm | SEQ ID NO: 929 |
| RZ00 2118 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfA(moe)AfUmUmUmsCmsUm | SEQ ID NO: 930 |
| RZ00 2119 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfCmUfUmCmGmsUmsAm | SEQ ID NO: 931 |
| RZ00 2120 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfCmUfUmCmGmsUmsAm | SEQ ID NO: 932 |
| RZ00 2121 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfC(moe)UfUmCmGmsUmsAm | SEQ ID NO: 933 |
| RZ00 2122 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCfAmGmGmUmGfAmGfUmUmGmsAmsUm | SEQ ID NO: 934 |
| RZ00 2123 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfAmGfUmUmGmsAmsUm | SEQ ID NO: 935 |

(continued)

| Pair | Sense strand (5'-3') | | Antisense strand (5'-3') | |
|---|---|---|---|---|
| RZ00 2124 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfA(moe)GfUmUmGmsAmsUm | SEQ ID NO: 936 |
| RZ00 2125 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfCmUfCmUmGmsUmsAm | SEQ ID NO: 937 |
| RZ00 2126 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfCmUfCmUmGmsUmsAm | SEQ ID NO: 938 |
| RZ00 2130 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfAmAfUmUmUmsCmsUm | SEQ ID NO: 939 |
| RZ00 2131 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfUmUfCmUmCmsCmsUm | SEQ ID NO: 940 |
| RZ00 2132 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfUmUfCmUmCmsCmsUm | SEQ ID NO: 941 |
| RZ00 2133 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfU(moe)UfCmUmCmsCmsUm | SEQ ID NO: 942 |
| RZ00 2134 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfU(moe)UfCmUmCmsCmsUm | SEQ ID NO: 943 |

as an illustrative example, "SEQ ID NO: 892_(CR01008×3)" indicates that the ligand represented by formula (CR01008×3) is conjugated to 3'-end of the sense strand as set forth in SEQ ID NO: 892.

In some specific embodiments of the present disclosure, the conjugate comprises one or more pairs of:

| Pair | Sense strand (5'-3') | | Antisense strand (5'-3') | |
|---|---|---|---|---|
| RZ00 2033 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfC(moe)UfUmCmGmsUmsAm | SEQ ID NO: 908 |
| RZ00 2050 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfC(moe)UfCmUmGmsUmsAm | SEQ ID NO: 912 |
| RZ00 2082 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCfAmGmGmUmGfA(moe)GfUmUmGmsAmsUm | SEQ ID NO: 920 |

(continued)

| Pair | Sense strand (5'-3') | | Antisense strand (5'-3') | |
|---|---|---|---|---|
| RZ00 2106 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAfUmUmUfC(moe)UfCmUmGmsUmsAm | SEQ ID NO: 914 |
| RZ00 2115 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfC(moe)UfCmUmGmsUmsAm | SEQ ID NO: 927 |
| RZ00 2116 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfAmAfUmUmUmsCmsUm | SEQ ID NO: 928 |
| RZ00 2117 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfA(moe)AfUmUmUmsCmsUm | SEQ ID NO: 929 |
| RZ00 2118 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfA(moe)AfUmUmUmsCmsUm | SEQ ID NO: 930 |
| RZ00 2119 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfCmUfUmCmGmsUmsAm | SEQ ID NO: 931 |
| RZ00 2120 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfCmUfUmCmGmsUmsAm | SEQ ID NO: 932 |
| RZ00 2121 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfC(moe)UfUmCmGmsUmsAm | SEQ ID NO: 933 |
| RZ00 2122 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCfAmGmGmUmGfAmGfUmUmGmsAmsUm | SEQ ID NO: 934 |
| RZ00 2123 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfAmGfUmUmGmsAmsUm | SEQ ID NO: 935 |
| RZ00 2124 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfA(moe)GfUmUmGmsAmsUm | SEQ ID NO: 936 |
| RZ00 2125 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfCmUfCmUmGmsUmsAm | SEQ ID NO: 937 |

(continued)

| Pair | Sense strand (5'-3') | | Antisense strand (5'-3') | |
|---|---|---|---|---|
| RZ00 2126 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfCmUfCmUmGmsUmsAm | SEQ ID NO: 938 |
| RZ00 2130 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfAmAfUmUmUmsCmsUm | SEQ ID NO: 939 |
| RZ00 2131 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfUmUfCmUmCmsCmsUm | SEQ ID NO: 940 |
| RZ00 2132 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfUmUfCmUmCmsCmsUm | SEQ ID NO: 941 |
| RZ00 2133 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfU(moe)UfCmUmCmsCmsUm | SEQ ID NO: 942 |
| RZ00 2134 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfU(moe)UfCmUmCmsCmsUm | SEQ ID NO: 943 |

as an illustrative example, "SEQ ID NO: 892_(CR01008×3)" indicates that the ligand represented by formula (CR01008×3) is conjugated to 3'-end of the sense strand as set forth in SEQ ID NO: 892.

In some specific embodiments of the present disclosure, the conjugate comprises:

| Pair | Sense strand (5'-3') | | Antisense strand (5'-3') | |
|---|---|---|---|---|
| RZ00 2106 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAfUmUmUfC(moe)UfCmUmGmsUmsAm | SEQ ID NO: 914 |

wherein, "SEQ ID NO: 892_(CR01008×3)" indicates that the ligand represented by formula (CR01008×3) is conjugated to 3'-end of the sense strand as set forth in SEQ ID NO: 892.

In a fourth aspect, the present disclosure provides a use of the double-stranded oligonucleotide according to the first aspect, and/or the double-stranded oligonucleotide according to the second aspect, and/or the conjugate according to the third aspect, in the manufacture of a medicament for ameliorating, preventing and/or treating a disease or disorder mediated by C3 gene.

In some optional embodiments of the present disclosure, the disease or disorder mediated by C3 gene includes but is not limited to IgA nephropathy, atypical hemolytic uremic syndrome (aHUS), paroxysmal nocturnal hemoglobinuria (PNH), C3 glomerulopathy, lupus nephritis, and membranous nephropathy.

In a fifth aspect, the present disclosure provides a pharmaceutical composition comprising the double-stranded oligonucleotide according to the first aspect, and/or the double-stranded oligonucleotide according to the second aspect, and/or the conjugate according to the third aspect.

In some optional embodiments of the present disclosure, the pharmaceutical composition further comprises one or more pharmaceutically acceptable excipients or adjuvants.

In the present disclosure, a "pharmaceutical composition" may refer to one for use in the treatment of a disease, or for use in in vitro cell culture experiments. When used for the treatment of a disease, the term "pharmaceutical

composition" usually refers to a unit dosage form, and can be prepared by any of the methods well known in the pharmaceutical art. The methods include the step of bringing an active ingredient into association with an excipient which constitutes one or more accessory ingredients. Generally, compositions are prepared by uniformly and intimately bringing an active siRNA into association with a liquid excipient, a finely divided solid excipient, or both.

In the present disclosure, the term "pharmaceutically acceptable" means that a substance or composition must be chemically and/or toxicologically compatible with the other ingredients comprising a formulation and/or the mammal to be treated therewith. Preferably, "pharmaceutically acceptable" as described in the present disclosure refers to what is approved by a federal regulatory agency or a national government or listed in the U.S. Pharmacopeia or other generally recognized pharmacopeia for use in animals, and more particularly in humans.

In the present disclosure, the term "pharmaceutically acceptable excipient" include any solvents, solid excipients, diluents, or other liquid excipients, and the like, that are suitable for a particular target dosage form. The use of any conventional excipient is contemplated herein, except to the extent that such excipients are incompatible with an siRNA of the present disclosure, for example, by producing any undesirable biological effect or interacting in a deleterious manner with any other component of the pharmaceutically acceptable composition.

The use of any conventional excipient is contemplated herein, except to the extent that such excipients are incompatible with an siRNA of the present disclosure, for example, by producing any undesirable biological effect or interacting in a deleterious manner with any other component of the pharmaceutically acceptable composition.

The pharmaceutical compositions of the present disclosure include formulations suitable for parenteral administration. The formulations may conveniently be presented in unit dosage form and may be prepared by any of the methods well known in the art of pharmacy. The amount of an active ingredient that can be combined with excipient materials to produce a single dosage form will generally be that amount of the siRNA which produces a therapeutic effect.

In a sixth aspect, the present disclosure provides a medicament, wherein the medicament comprises the pharmaceutical composition according to the fifth aspect.

In a seventh aspect, the present disclosure provides a medicament combination, comprising the medicament according to the sixth aspect and any other active ingredient.

In an eighth aspect, the present disclosure provides a kit comprising the double-stranded oligonucleotide according to the first aspect, and/or the double-stranded oligonucleotide according to the second aspect, and/or the conjugate according to the third aspect, and/or the pharmaceutical composition according to the fifth aspect, and/or the medicament according to the sixth aspect, and/or the medicament combination according to the seventh aspect.

In a ninth aspect, the present disclosure provides a method for inhibiting expression of C3 gene, comprising administering to a subject in need thereof the double-stranded oligonucleotide according to the first aspect, and/or the double-stranded oligonucleotide according to the second aspect, and/or the conjugate according to the third aspect, and/or the pharmaceutical composition according to the fifth aspect, and/or the medicament according to the sixth aspect, and/or the medicament combination according to the seventh aspect.

In some specific embodiments of the present disclosure, the present disclosure provides a method for inhibiting C3 gene expression in a cell in vitro, comprising contacting the cell with the double-stranded oligonucleotide according to the first aspect, and/or the double-stranded oligonucleotide according to the second aspect, and/or the conjugate according to the third aspect, and/or the pharmaceutical composition according to the fifth aspect, and/or the medicament according to the sixth aspect, and/or the medicament combination according to the seventh aspect.

In some optional embodiments of the present disclosure, the inhibition of the expression of the C3 gene is that the cell is maintained for a time sufficient to obtain degradation of the mRNA transcript of the C3 gene.

More practically, in a tenth aspect of, the present disclosure provides a method for ameliorating, treating and/or preventing a disease or disorder mediated by C3 gene, comprising administering the double-stranded oligonucleotide according to the first aspect, and/or the double-stranded oligonucleotide according to the second aspect, and/or the conjugate according to the third aspect, and/or the pharmaceutical composition according to the fifth aspect, and/or the medicament according to the sixth aspect, and/or the medicament combination according to the seventh aspect, to a subject in need thereof.

In some specific embodiments of the present disclosure, the subject is a human.

In some specific embodiments of the present disclosure, the medicament is administered at a dose of 0.01 mg/kg to 10 mg/kg, or 0.5 mg/kg to 50 mg/kg.

In some specific embodiments of the present disclosure, the mode of administration of the medicament includes subcutaneous administration or intravenous administration.

In some specific embodiments of the present disclosure, the disease or disorder mediated by C3 gene is associated with the mRNA expression level of C3 gene.

In some specific embodiments of the present disclosure, the disease or disorder is selected from IgA nephropathy, atypical hemolytic uremic syndrome, paroxysmal nocturnal hemoglobinuria (PNH), C3 glomerulopathy, lupus nephritis and membranous nephropathy.

The double-stranded oligonucleotides or conjugates thereof provided by the present disclosure significantly

inhibit C3 gene mRNA expression in HepG2 cells, significantly inhibit C3 gene mRNA expression in animals, significantly reduce the protein level of the C3 gene in the serum of *Macaca fascicularis* and the inhibitory effect lasts until day 42, significantly inhibit the alternative complement pathway in the serum of *Macaca fascicularis* but has no effect on the classical complement pathway in the serum, reduce C3 gene mRNA and protein levels in CFA-IgA mice, reduce C3 deposition in renal tissue and improve the disease course in patients with C3 deposition in renal tissue, and no significant abnormalities are observed in animal hematology and blood biochemistry. The double-stranded oligonucleotide or a conjugate thereof provided by the present disclosure is useful for alleviating, preventing, and/or treating a disease or disorder mediated by C3 gene.

## BRIEF DESCRIPTION OF DRAWINGS

FIG. 1 shows the inhibitory activity on C3 mRNA in BALB/c-HDI mice after administration of the siRNA conjugate as described in Example 2;

FIG. 2 shows representative histopathological images (H&E staining, 200x magnification) for each experimental group in Example 3;

FIG. 3 shows the inhibitory activity on C3 mRNA in HepG2 cells after administration of the siRNA conjugate as described in Example 4;

FIG. 4 shows the inhibitory activity on C3 mRNA in the liver of *Macaca fascicularis* after administration of the siRNA conjugate as described in Example 5;

FIG. 5 shows the change in the C3 protein level in the serum of *Macaca fascicularis* after administration of the siRNA conjugate as described in Example 6;

FIG. 6 shows the change in the activity of the alternative complement pathway in the serum of *Macaca fascicularis* after administration of the siRNA conjugate as described in Example 7;

FIG. 7 shows the change in the activity of the classical complement pathway in the serum of *Macaca fascicularis* after administration of the siRNA conjugate as described in Example 7;

FIG. 8 shows the inhibitory activity on the target gene in the liver of CFA-hIgA mice after administration of the siRNA conjugate as described in Example 8;

FIG. 9 shows the change in the C3 protein level in the serum of CFA-hIgA mice after administration of the siRNA conjugate as described in Example 8; and

FIG. 10 shows the change in C3 deposition in the kidneys of CFA-hIgA mice after administration of the siRNA conjugate as described in Example 8.

## DETAILED DESCRIPTION

The present disclosure provides a double-stranded oligonucleotide for inhibiting C3 gene expression, a conjugate thereof, and uses thereof. A person skilled in the art can implement the present disclosure by referring to the content herein and appropriately modifying the process parameters. It is to be noted that all similar substitutions and modifications are obvious to a person skilled in the art and are considered to be included in the present disclosure. The methods and applications of the present disclosure have been described through preferred embodiments, and a person skilled in the art can obviously make modifications or appropriate changes and combinations to the methods and applications described herein without departing from the content, spirit, and scope of the present disclosure to realize and apply the technology of the present disclosure.

### Term Explanation

In the present disclosure, the term "comprising" or "including" is an open-ended expression, meaning that it specifies certain contents described in the present disclosure, but does not exclude the presence of other, unlisted

contents.

In the present disclosure, the terms "optionally", "optional", or "option" generally refer to an event or condition that is subsequently described that may or may not occur, and this description includes instances in which the event or condition occurs and instances in which it does not.

In the present disclosure, the term "small interfering RNA (siRNA)" is a double-stranded RNA of 17 to 25 nucleotides in length, comprising a sense strand and an antisense strand. An siRNA mediates target cleavage of an RNA transcript via the RISC pathway by forming an RNA-induced silencing complex (RISC). Specifically, an siRNA directs sequence-specific degradation of an mRNA through the known RNA interference (RNAi) process, thereby inhibiting the translation of the mRNA into amino acids and its conversion into a protein. For example, an siRNA can regulate (e.g., inhibit) the expression of C3 in a cell.

In the present disclosure, the term "antisense strand (or guide strand)" includes a region that is substantially complementary to a target sequence, such as the mRNA of C3. The "sense strand (or passenger strand)" refers to a siRNA strand that contains a sequence substantially complementary to the antisense strand. The term "substantially complementary" means fully complementary or at least partially complementary, for example, the antisense strand is fully complementary or at least partially complementary to the target sequence. In the case of partial complementarity, mismatches can be present within the internal or terminal regions of the molecule, wherein the most tolerated mismatches are present within the terminal regions, for example, within 5, 4, 3, or 2 nucleotides of the 5'-end and/or 3'-end of the siRNA.

It should be noted that an antisense strand being "at least partially substantially complementary" to an mRNA means that the antisense strand has a polynucleotide that is substantially complementary to a contiguous portion of an mRNA of interest (e.g., an mRNA encoding C3). Alternatively, the antisense strand is complementary to at least a portion of the mRNA of C3 if a polynucleotide is substantially uninterruptedly complementary to a portion of the mRNA encoding C3.

In the present disclosure, the term "target sequence" refers to a contiguous portion of the nucleotide sequence of an mRNA molecule formed during the transcription of the C3 gene, including an mRNA that is a product of RNA processing of a primary transcript. C3 can be within a cell, for example, a cell in a subject.

In the present disclosure, the term "substantially complementary" means that the sense strand and the antisense strand have no more than 3 nucleotide mismatches, no more than 2 nucleotide mismatches, or no more than 1 nucleotide mismatch in the duplex region, for example, 3 nucleotide mismatches, 2 nucleotide mismatches, 1 nucleotide mismatch, or 0 nucleotide mismatches.

In the present disclosure, the terms "nucleotide difference" and "nucleotide base difference" are used interchangeably. A "nucleotide difference" exists between one nucleotide sequence and another nucleotide sequence when the type of base of the nucleotide at the same position has changed in the former compared to the latter. For example, when a nucleotide base at a certain position in the latter is A, and the corresponding nucleotide base at the same position in the former is U, C, G, or T, it is considered that a nucleotide difference exists between the two nucleotide sequences at that position. In some embodiments, when a nucleotide at the original position is replaced by an abasic nucleotide or an equivalent thereof, a nucleotide difference can also be considered to have been created at that position.

In the present disclosure, the term "overhang" refers to at least one unpaired nucleotide that protrudes from the double-helical structure of a double-stranded oligonucleotide, and also refers to the nucleotide sequence in a siRNA structure other than the duplex region. For example, when the 3'-end of one strand in the sense strand and/or the antisense strand extends beyond the 5'-end of the other strand, or when the 5'-end of one strand in the sense strand and/or the antisense strand extends beyond the 3'-end of the other strand, a nucleotide overhang is present. The overhang may comprise at least one nucleotide, at least two nucleotides, at least three nucleotides, at least four nucleotides, at least five nucleotides, or more nucleotides. The nucleotide overhang may comprise or be composed of nucleotides or nucleoside analogs, including deoxynucleotides and deoxynucleosides. The overhang can be located on the sense strand, the antisense strand, or any combination thereof. In addition, the nucleotides of the overhang can appear at the 5'-end, the 3'-end, or both ends of the antisense or sense strand.

In the present disclosure, the term "DEPC $H_2O$" is ultrapure water (Type I water) that has been treated with diethyl pyrocarbonate (DEPC) and sterilized by high-temperature, high-pressure autoclaving.

In the present disclosure, the term "subject" refers to any animal being examined, studied, or treated, and is not intended to limit the present disclosure to any particular type of subject. In some embodiments of the present disclosure, a human is a preferred subject, while in other embodiments, a non-human animal is a preferred subject, including but not limited to a mouse, monkey, ferret, cow, sheep, goat, pig, chicken, turkey, dog, cat, horse, and reptile.

In the present disclosure, the term "inhibiting the expression of C3 gene" refers to any level of inhibition of the C3 gene expression, for example, at least partial inhibition of C3 gene expression, such as inhibition of at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99%. Herein, the expression of the C3 gene can be evaluated based on the level of any variable associated with C3 gene expression, for example, the mRNA

level or protein level of C3. Inhibition can be evaluated by a decrease in the absolute or relative level of one or more of these variables compared to a control level. A control level can be any type of control level utilized in the art, for example, a baseline level before administration, or a level determined from a similar subject, cell, or sample that was not treated or was treated with a control (e.g., a buffer-only control or an inactive agent control).

In the present disclosure, unless otherwise specified, the siRNA sequences were synthesized by Kunshan Aotai Biotechnology Co., Ltd.; the PCR primers were synthesized by Sangon Biotech (Shanghai) Co., Ltd.; the human liver cancer HepG2 cells were purchased from Wuhan Procell Life Science & Technology Co., Ltd.; the experimental animal BALB/c mice were purchased from Zhejiang Vital River Laboratory Animal Technology Co. Ltd.; the CFA-hIgA nephropathy model mice were constructed by Peking University First Hospital; and the healthy *Macaca fascicularis* were purchased from Guangzhou Landao Biotechnology Co., Ltd.

In the present disclosure, unless otherwise specified, the base compositions and modifications are represented as follows: capital letters A, U, G, C, and T represent the base composition of the nucleotides, respectively; a lowercase letter m indicates that the nucleotide immediately to the left of the letter m is a 2'-O-methyl modified (alsonamed: 2'-methoxy modified) nucleotide; a lowercase letter f indicates that the nucleotide immediately to the left of the letter f is a 2'-fluoro modified nucleotide; a lowercase letter d indicates that the nucleotide immediately to the left of the letter d is a 2'-deoxy modified ribonucleic acid (also named: deoxyribonucleic acid); (moe) indicates that the nucleotide immediately to the left of the combination identifier (moe) is a 2'-O-methoxyethyl modified nucleotide; and a lowercase letter s indicates a phosphorothioate linkage between the two nucleotides immediately adjacent to the left and right of the letter s.

In the present disclosure, unless otherwise specified, the Real-time PCR detection data of the in vivo activity experiments described herein were subjected to relative quantification of the target gene mRNA in each test group using the $\Delta\Delta$Ct method. The calculation method is summarized as follows:

$\Delta$Ct (test group) = Ct (target gene in the test group) - Ct (internal reference gene in the test group)

$\Delta$Ct (control group) = Ct (target gene in the control group) - Ct (internal reference gene in the control group)

$$\Delta\Delta\text{Ct (test group)} = \Delta\text{Ct (test group) - average } \Delta\text{Ct (control group)}$$

$$\Delta\Delta\text{Ct (control group)} = \Delta\text{Ct (control group) - average } \Delta\text{Ct (control group)}$$

The mRNA expression level of the target gene in the test group is normalized with the control group as the baseline, and the remaining mRNA expression level of the target gene in the control group is defined as 100%.

Relative remaining mRNA expression level of the target gene in the test group = $2^{-\Delta\Delta\text{Ct (test group)}} \times 100\%$

mRNA inhibition rate of the target gene in the test group = 100% - relative remaining mRNA expression level of the target gene in the test group

For the C3 protein detection data at different time points in the in vivo activity experiments of the present disclosure, the calculation formula is:

$$\text{Relative remaining expression level of C3 protein} = \frac{Dx}{Pre - dose} \times 100\%$$

wherein, "pre-dose" represents the C3 protein concentration in the serum before administration, and "Dx" represents the C3 protein concentration in the serum on day x after administration.

For the CCP and CAP activity detection data at different time points in the in vivo activity experiments of the present disclosure, the calculation formula is:

$$\text{Relative remaining activity of CAP or CCP} = \frac{Dx}{Pre - dose} \times 100\%$$

wherein, pre-dose represents the CCP activity or CAP activity in the serum before administration, and Dx represents the

CCP activity or CAP activity in the serum on day x after administration.

In the present disclosure, unless otherwise specified, the in vivo activity experimental data are expressed as mean ± SD (X ± STDEV), and the experimental data were all plotted and analyzed using GraphPad Prism 8.0 software.

In the present disclosure, unless otherwise specified, the proportions of the reagents are calculated by volume ratio (v/v).

Unless otherwise specified, the reagents used in the preparation of the compounds in the present disclosure were purchased from Beijing Ouhe Technology Co., Ltd., and information of the main reagents is shown in Table 1.

Table 1: Main reagents

| Reagent Name | Abbreviation | CAS No. |
|---|---|---|
| Lithium aluminum hydride ($LiAlH_4$) | - | 16853-85-3 |
| Wet palladium on carbon (10 wt%) | Pd/C | - |
| Palladium hydroxide on carbon (10 wt%) | $Pd(OH)_2/C$ | - |
| O-Benzotriazole-N,N,N',N'-tetramethyluronium hexafluorophosphate | HBTU | 94790-37-1 |
| O-(7-Azabenzotriazol-1-yl)-N,N,N',N'-tetramethyluronium hexafluorophosphate | HATU | 148893-10-1 |
| 4,4'-Dimethoxytrityl chloride / 4,4'-Dimethoxytriphenylchloromethane | DMTrCl | 40615-36-9 |
| bis(diisopropylamino)(2-cyanoethoxy)phosphine | - | 102691-36-1 |
| 4,5-Dicyanoimidazole | DCI | 1122-28-7 |
| 4-Dimethylaminopyridine | DMAP | 1122-58-3 |
| Amino-CPG (Aminoalkyl-CPG, Model: C3006-1000) | 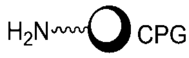 | - |
| 4M Hydrochloric acid in 1,4-dioxane solution | - | - |
| trans-4-(Boc-amino)cyclohexanecarboxaldehyde | - | 181308-57-6 |
| N-Benzyloxycarbonyl-4-aminobutanoic acid | - | 5105-78-2 |
| 5-[[(2R,3R,4R,5R,6R)-3-Acetamido-4,5-diacetoxy-6-(acetoxymethyl)2-tetrahydropyranyl]oxy]pentanoic acid | Compound 4 | 1159408-54-4 |

wherein, CPG represents a Controlled Pore Glass support.

Unless otherwise specified, the reagents, consumables, instruments, and equipment used in the biological detection experiments in the present disclosure are all commercially available products. The main reagents and consumables and their sources are shown in Table 2, and the main instruments and equipment and their sources are shown in Table 3.

Table 2: Main reagents and consumables

| Name | Manufacturer |
|---|---|
| Opti-MEM | GENOM Bio |
| MEM Medium | Gibco |
| FBS | Gibco |
| Penicilin-streptomycin | HyClone |
| Trypsin | Gibco |
| Power SYBR Green RNA-to-CT™ 1-Step | Thermo Fisher |
| 1×PBS | Zhongke Maichen (Beijing) Technology Co., Ltd. |
| DMEM Medium | Zhongke Maichen (Beijing) Technology Co., Ltd. |
| Opti-MEM™ Medium | Gibco |
| Serum | Sigma |

(continued)

| | |
|---|---|
| Trypsin | Zhongke Maichen (Beijing) Technology Co., Ltd. |
| Penicilin-streptomycin | BBI |
| Lipofectamine RNAiMax | Invitrogen |
| Nucleic Acid Extraction/Purification Kit | Zhejiang Hanwei Technology Co ltd |
| RevertAid First Strand cDNA Synthesis Kit | Thermo Fisher Scientific |
| TaqMan Fast Advanced Master Mix | Thermo Fisher Scientific |
| SYBR Select Master Mix | Thermo Fisher Scientific |
| Hanwei RNA Extraction Kit | Zhejiang Hanwei Technology Co ltd |
| RNALater™ | Thermo Fisher Scientific |
| Ambion® RNAlater® | Invitrogen |
| Human Complement C3 ELISA Kit | Hycult Biotech |
| WIESLAB® Complement System Alternative Pathway - RUO | IBL America |
| WIESLAB® Complement System Classical Pathway- RUO | IBL America |

Table 3: Main instruments and equipment

| Name | Manufacturer |
|---|---|
| Automatic Nucleic Acid Extractor | Zhejiang Hanwei Technology Co ltd |
| High-Speed Refrigerated Centrifuge | Eppendorf |
| $CO_2$ Incubator | Thermo Fisher Scientific |
| Biosafety Cabinet | Shanghai Lishen |
| Constant Temperature Water Bath | Shanghai Boxun |
| Automatic Cell Counter | Shanghai Ruiyu |
| Inverted Microscope | Olympus |
| NANODROP OneC | Thermo Fisher Scientific |
| Gradient PCR Thermal Cycler | Eppendorf |
| CFX Opus 384 | Bio-Rad |
| LightCycler 480 | Roche |
| Gel Imaging System | Shanghai Tanon Life Science Co.,Ltd. |
| Electrophoresis System | BEIJING LIUYI BIOTECHNOLOGY CO.,LTD. |
| Tissuelyser II Automatic Tissue Homogenizer | Shanghaijingxin Experimental Technology |
| Real-Time Quantitative PCR System | Roche |
| Paraffin Microtome | JINHUA YIDI MEDICAL APPLIANCE CO.,LTD. |
| Tissue Processor | Leica |
| Automatic Biological Tissue Embedding System | JINHUA YIDI MEDICAL APPLIANCE CO.,LTD. |
| Automatic Modular Hematology and Body Fluid Analyzer | SYSMEX |

The raw materials and reagents used for the double-stranded oligonucleotide for inhibiting C3 gene expression, the conjugate thereof, and the use thereof provided by the present disclosure are all commercially available.

The present disclosure is further described below in conjunction with examples:

**Preparation of compounds**

Preparation Example 1: Preparation of Compound CR01008 and Compound CR01008Z

(1.1) Synthesis of Compound CR01008

In this preparation example, the synthetic route for compound CR01008 was:

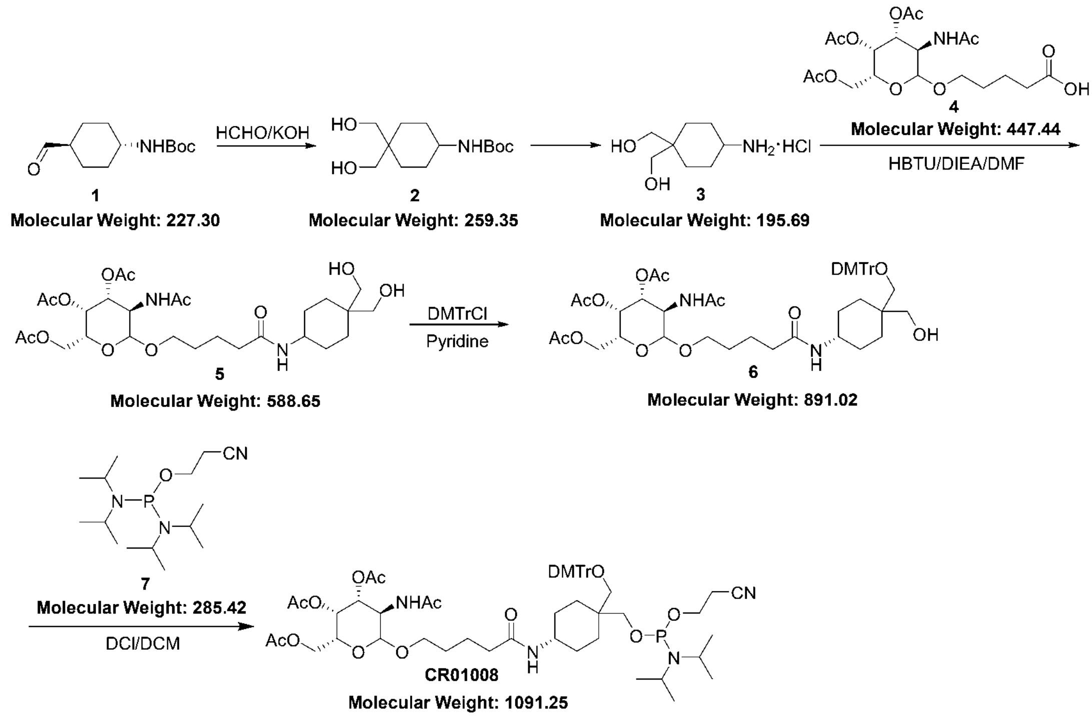


(1.1.1) Synthesis of compound 2

Compound 1 (trans-4-(Boc-amino)cyclohexanecarboxaldehyde, 10.0 g, 1.0 eq) and an aqueous formaldehyde solution (8.9 g, 37 %, 2.4 eq) were dissolved in 33 mL of methanol. 13 mL of an aqueous KOH solution with a concentration of 45.3 % was added dropwise. After the addition was completed, the mixture was stirred and reacted at 25°C for 30 min, heated to 60°C, and reacted under reflux at 60°C for 2 h. After the reaction was completed, the reaction solution was cooled to room temperature and then evaporated to dryness under reduced pressure and a white solid crude product was obtained. A small amount of water was added to the crude product to form a slurry, which was then filtered, and compound 2 (9 g, yield 78.9%) was obtained as a white solid. MS-ESI (m/z) = 260 $[M+H]^+$.

(1.1.2) Synthesis of compound 3

Compound 2 (9 g, 1 eq) prepared according to step (1.1.1) was dissolved in 70 mL of 1,4-dioxane. A solution of hydrogen chloride in 1,4-dioxane (45 mL, 4 M) was added, and the mixture was stirred and reacted at 25°C for 1 hour. After the reaction was completed, the reaction solution was evaporated to dryness under reduced pressure, and compound 3 (6.8 g, yield 100%) was obtained as a white solid.

(1.1.3) Synthesis of compound 5

Compound 3 (1.8 g, 2.0 eq) prepared according to step (1.1.2), compound 4 (5-[[(2R,3R,4R,5R,6R)-3-acetamido-4,5-diacetoxy-6-(acetoxymethyl)2-tetrahydropyranyl]oxy]pentanoic acid, 2.1 g, 1.0 eq), and DIEA (N,N-diisopropylethylamine, 3.5 g, 6.0 eq) were dissolved in 15 mL of DMF. HBTU (1.9 g, 1.1 eq) was added, and the mixture was stirred and reacted at 25°C for 3 h under an $N_2$ atmosphere. After the reaction was completed, the reaction solution was evaporated to dryness under reduced pressure and purified by reverse-phase chromatography (using a 22 vol% aqueous acetonitrile solution). Compound 5 (1.78 g, yield 64.4%) was obtained as a white solid. MS-ESI (m/z) = 589 $[M+H]^+$.

(1.1.4) Synthesis of compound 6

Compound 5 (1.54 g, 1.0 eq) prepared according to step (1.1.3) was dissolved in 15 mL of pyridine. The reaction system was cooled to 0°C with an ice-water bath, and DMTrCl (4,4'-dimethoxytrityl chloride, 1.32 g, 1.5 eq) was added at 0°C. The mixture was reacted at 25°C for 3 h, and the reaction was quenched by adding 15 mL of methanol to the reaction solution. After the reaction was completed, the reaction solution was evaporated to dryness under reduced pressure and the residue was purified by reverse-phase chromatography (using a 60 vol% aqueous solution of acetonitrile). Compound 6 (1 g, yield 42.7%) was obtained as a yellow solid. MS-ESI (m/z) = 891 $[M+H]^+$.

(1.1.5) Synthesis of compound CR01008

Compound 6 (1.08 g, 1.0 eq) prepared according to step (1.1.4) was dissolved in 20 mL of anhydrous dichloromethane. DCI (115 mg, 0.8 eq) and compound 7 (bis(diisopropylamino)(2-cyanoethoxy)phosphine, 732 mg, 2.1 eq) were added respectively. The atmosphere was purged with nitrogen 3 times, and the mixture was stirred and reacted at 25°C for 2 h. After the reaction was completed, 20 mL of a saturated aqueous sodium bicarbonate solution was added to the reaction solution, and the mixture was extracted 3 times with 20 mL of dichloromethane (3×20 mL). The organic phases were combined and evaporated to dryness under reduced pressure, and the residue was purified by reverse-phase chromatography (using a 72 vol% aqueous acetonitrile solution), and then dried under vacuum for 12 h. Compound CR01008 (1 g, yield 76.0%) was obtained as a white powder. MS-ESI (m/z) = 1091 $[M+Na]^+$.

1H NMR (400 MHz, DMSO-d6) δ 1.05 (d, J = 6.7 Hz, 6H).1.14 (d, J = 6.7 Hz, 6H), 1.37 - 1.17 (m, 5H), 1.60 - 1.40 (m, 6H),1.68 - 1.62 (m, 1H),1.80 (s, 3H),1.80 (s, 3H),1.92 (s, 3H), 2.02 (s, 5H),2.13 (s, 3H),2.71 (t, J = 5.9 Hz, 2H), 2.79 (d, J = 8.4 Hz, 1H), 2.87 (d, J = 8.4 Hz, 1H),3.36 (s, 1H), 3.58 - 3.39 (m, 3H), 3.69 - 3.60 (m, 2H), 3.75 (s, 7H), 3.90 (dt, J = 11.2, 8.8 Hz, 1H), 4.05 (s, 3H),4.51 (d, J = 8.4 Hz, 1H), 4.99 (dd, J = 11.3, 3.4 Hz, 1H), 5.24 (d, J = 3.4 Hz, 1H), 5.78 (s, 1H), 6.93 - 6.87 (m, 4H),7.35 - 7.21 (m, 7H), 7.44 - 7.37 (m, 2H), 7.66 (d, J = 7.8 Hz, 1H), 7.84 (d, J = 9.2 Hz, 1H).

(1.2) Synthesis of Compound CR01008Z

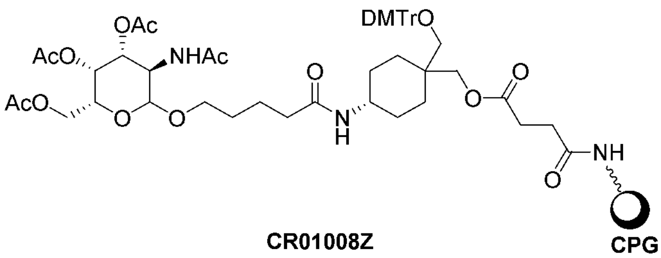

CR01008Z

In this preparation example, the synthetic route for compound CR01008Z was:

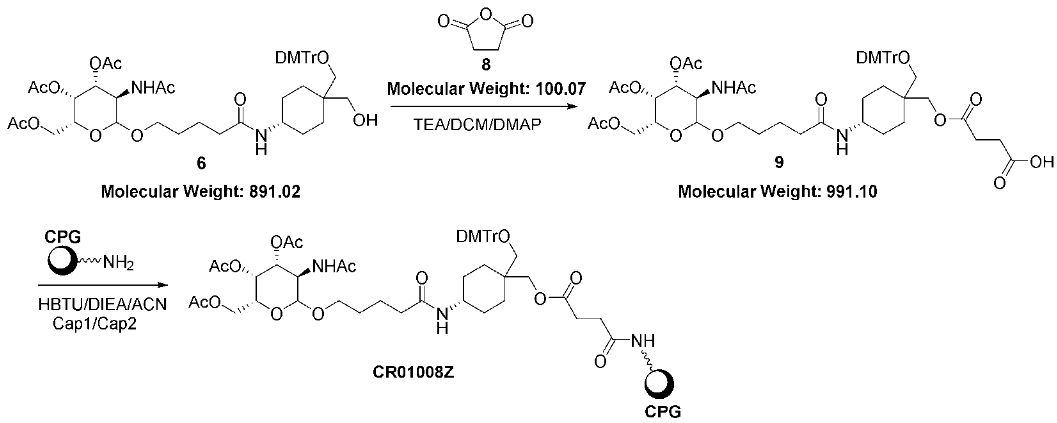


(1.2.1) Synthesis of compound 9

Compound 6 (500 mg) prepared according to step (1.1.4) was dissolved in 10 mL of dichloromethane.

Compound 8 (succinic anhydride, 112 mg), DMAP (6.8 mg), and TEA (226.2 mg) were added. The atmosphere was purged with nitrogen 3 times, and the mixture was stirred and reacted at 25°C for 16 h. The residue was purified by flash chromatography, and compound 9 (300 mg, yield 53.6%) was obtained. MS-ESI (m/z) = 1013 $[M+Na]^+$.

(1.2.2) Synthesis of compound CR01008Z

Compound 9 (50 mg) prepared according to step (1.2.1), amino-CPG (1.25 g, 80 μmol/g, 0.1 mmol), HBTU (27 mg), and DIEA (12 mg) were added to a 20 mL vial. The mixture was reacted on a shaker for 16 h. After the reaction was completed, the reaction mixture was filtered and a filter cake was obtained. The filter cake was first washed once with 10 mL of acetonitrile (1 × 10 mL) and then dried under vacuum. The dried filter cake, DMAP (3 mg), Cap1 (10 mL, 200V), and Cap2 (1 mL, 20V) were added to a 20 mL vial. The mixture was reacted on a shaker for 6 h. After the reaction was completed, the reaction mixture was filtered and a filter cake was obtained. The filter cake was first washed once with 10 mL of acetonitrile (1 × 10 mL) and then dried under vacuum, and compound CR01008Z (1.03 g, loading capacity 20 to 30 μmol/g) was obtained.

Wherein Cap1 and Cap2 are capping reagents, Cap1 is a 20 vol% solution of N-methylimidazole in a mixed solution of pyridine/acetonitrile, with a volume ratio of pyridine to acetonitrile of 3:5; and Cap2 is a 20 vol% solution of acetic anhydride in acetonitrile.

**Preparation Example 2: Preparation of Compound CR01013 and Compound CR01013Z**

(2.1) Preparation of Compound CR01013

In this preparation example, the synthetic route for compound CR01013 was:

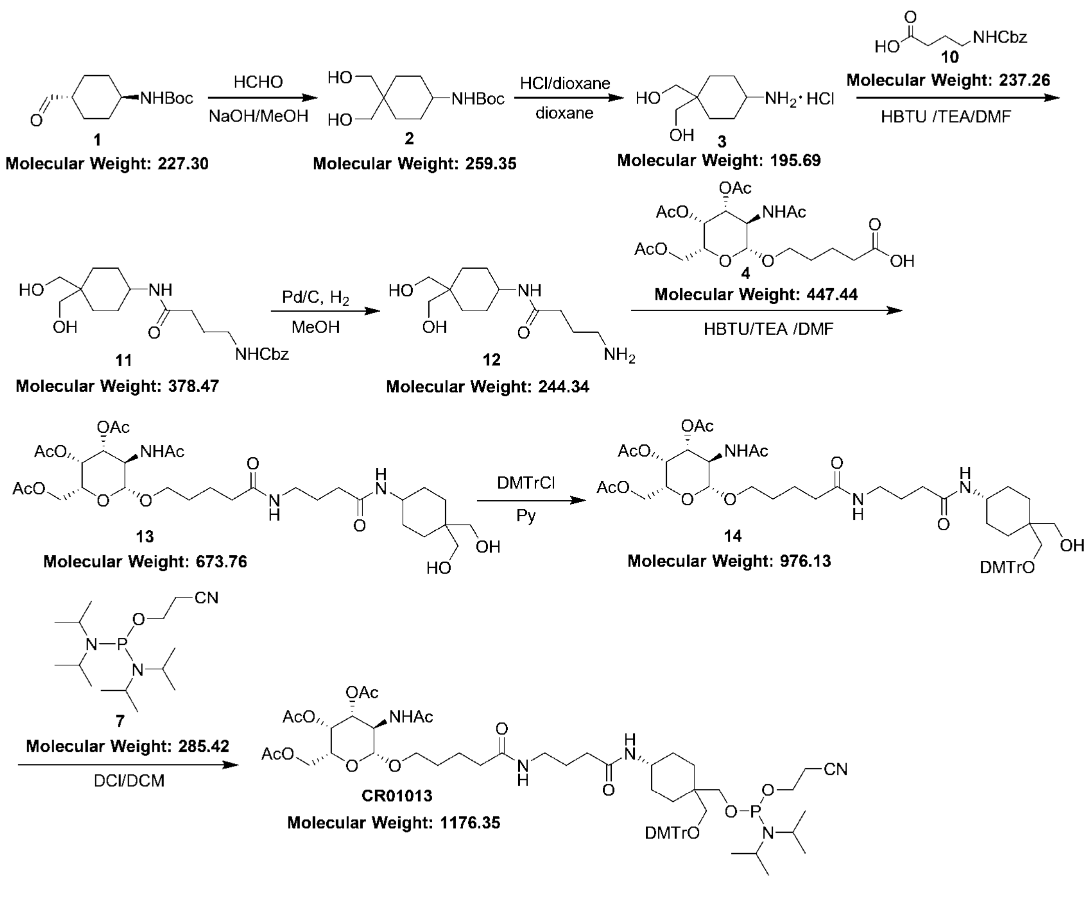


(2.1.1) Synthesis of compound 2

Compound 1 (trans-4-(Boc-amino)cyclohexanecarbaldehyde, 4.9 g) was dissolved in 17 mL of methanol. An aqueous formaldehyde solution (4.21 g, with a concentration of 37 %) and an aqueous sodium hydroxide solution (6.5 mL, with a concentration of 45.3 %) were added dropwise. After the addition was completed, the mixture was heated to 60°C

and stirred and reacted at 60°C for 2 h. After the reaction was completed, the reaction solution was cooled to 25°C and then evaporated to dryness under reduced pressure. A white solid crude product was obtained. A small amount of water was added to the crude product to form a slurry, which was then filtered and dried, and compound 2 (4.8 g, yield 85.9%) was obtained as a white solid. ESI-MS (m/z) = 260.2 $[M+H]^+$.

(2.1.2) Synthesis of compound 3

Compound 2 (4.8 g) prepared according to step (2.1.1) was dissolved in 25 mL of 1,4-dioxane. A solution of hydrochloric acid in 1,4-dioxane (25 mL, 4 M) was added, and the mixture was stirred and reacted at 25°C for 2 h. After the reaction was completed, the reaction solution was evaporated to dryness under reduced pressure and compound 3 (3.6 g, yield 99.4%) was obtained as a white solid.

(2.1.3) Synthesis of compound 11

Compound 3 (3.6 g) prepared according to step (2.1.2) was dissolved in 36 mL of DMF, and then TEA (5.62 g), compound 10 (N-benzyloxycarbonyl-4-aminobutanoic acid, 5.28 g), and HBTU (8.43 g) were added. The mixture was stirred and reacted at 25°C for 16 h. After the reaction was completed, the reaction solution was added to 200 mL of a saturated aqueous sodium bicarbonate solution and extracted three times with 100 mL of ethyl acetate (3 × 100 mL). The organic phases were combined. The combined organic phase was first washed once with 50 mL of a saturated aqueous sodium chloride solution (1 × 50 mL) and then dried over anhydrous sodium sulfate. The organic phase was evaporated to dryness under reduced pressure and the residue was purified by normal-phase column chromatography (eluent: dichloromethane/methanol = 10/1, v/v). Compound 11 (2.3 g, yield 33.0%) was obtained as a white solid. ESI-MS (m/z) = 379.5 $[M+H]^+$.

(2.1.4) Synthesis of compound 12

Compound 11 (2.3 g) prepared in step (2.1.3) was dissolved in 23 mL of methanol. Wet palladium on carbon (230 mg, 10 % loading) was added. The atmosphere was purged with hydrogen three times, and the reaction system was stirred and reacted at 25°C for 16 h under a hydrogen atmosphere (15 psi). After the reaction was completed, the reaction mixture was filtered and a filtrate was obtained. The filtrate was evaporated to dryness under reduced pressure and compound 12 (1.48 g, yield 99.8%) was obtained as a yellow oil.

(2.1.5) Synthesis of compound 13

Compound 12 (1.48 g) prepared in step (2.1.4) was dissolved in 15 mL of DMF. Triethylamine (TEA, 1.22 g), compound 4 (1.35 g), and HBTU (3.45 g) were added, and the mixture was stirred and reacted at 25°C for 16 h. After the reaction was completed, the reaction solution was added to 150 mL of a saturated aqueous sodium bicarbonate solution and extracted three times with 50 mL of ethyl acetate (3 × 50 mL). The organic phases were combined. The combined organic phase was first washed once with 30 mL of a saturated aqueous sodium chloride solution (1 × 30 mL) and then dried over anhydrous sodium sulfate. The organic phase was evaporated to dryness under reduced pressure and the residue was purified by reverse-phase column chromatography (C18 column, eluent: water/acetonitrile = 5/1, v/v). Compound 13 (1.3 g, yield 31.8%) was obtained as a white solid. ESI-MS (m/z): 674.3 [M+H]+.

(2.1.6) Synthesis of compound 14

Compound 13 (1.1 g) prepared in step (2.1.5) was dissolved in 11 mL of pyridine. The reaction system was cooled to 0°C with an ice-water bath, and DMTrCl (813 mg) was added in portions at 0°C. The reaction system was stirred at 0°C for 1 hour. After the reaction was completed, the reaction was quenched by adding methanol to the reaction solution, the solvent was evaporated, and the residue was purified by reverse-phase column chromatography (eluent: water/acetonitrile = 1/4, v/v). Compound 14 (800 mg, yield 50.3%) was obtained as a white solid. ESI-MS (m/z): 976.5 $[M+H]^+$.

(2.1.7) Synthesis of compound CR01013

At 25°C, compound 14 (550 mg) prepared according to step (2.1.6) was dissolved in 5 mL of dichloromethane (DCM). 4,5-dicyanoimidazole (DCI, 53.2 mg) and compound 7 (2-cyanoethyl N,N,N',N'-tetraisopropylphosphorodiamidite, 255.4 mg) were added. The atmosphere was purged with nitrogen three times, and the reaction system was stirred and reacted at 25°C for 1 hour under a nitrogen atmosphere. After the reaction was completed, the reaction solution was first washed twice with 5 mL of a saturated aqueous sodium bicarbonate solution (2 × 5 mL) and then washed once with 30

mL of a saturated aqueous sodium chloride solution (1 × 30 mL). The organic phase was separated, dried over anhydrous sodium sulfate, and the solvent in the organic phase was evaporated under reduced pressure. The residue was purified by normal-phase column chromatography (eluent: dichloromethane/methanol = 20/1, v/v) and compound CR01013 (532 mg, yield 80.4%) was obtained as a white solid. ESI-MS (m/z): 1176.7 $[M+H]^+$.

1H NMR (400 MHz, DMSO-d6) δ 0.95 - 1.05 (d, J = 6.7 Hz, 5H), 1.06 - 1.15 (q, J = 7.6 Hz, 8H), 1.15 - 1.21 (t, J = 7.2 Hz, 14H), 1.72 - 1.80 (s, 3H), 1.84 - 1.92 (s, 3H), 1.94 - 2.07 (d, J = 16.0 Hz, 7H), 2.07 - 2.14 (s, 3H), 2.64 - 2.72 (q, J = 5.8 Hz, 2H), 2.74 - 2.89 (d, J = 8.5 Hz, 2H), 3.35 - 3.56 (m, 4H), 3.57 - 3.70 (m, 4H), 3.71 - 3.77 (s, 6H), 3.81 - 3.93 (m, 1H), 3.96 - 4.09 (d, J = 6.4 Hz, 3H), 6.82 - 6.97 (d, J = 8.7 Hz, 4H), 7.17 - 7.27 (t, J = 8.7 Hz, 5H), 7.27 - 7.34 (t, J = 7.6 Hz, 2H), 7.34 - 7.43 (d, J = 7.5 Hz, 2H).

(2.2) Preparation of Compound CR01013Z

In this preparation example, the synthetic route for compound CR01013Z was:

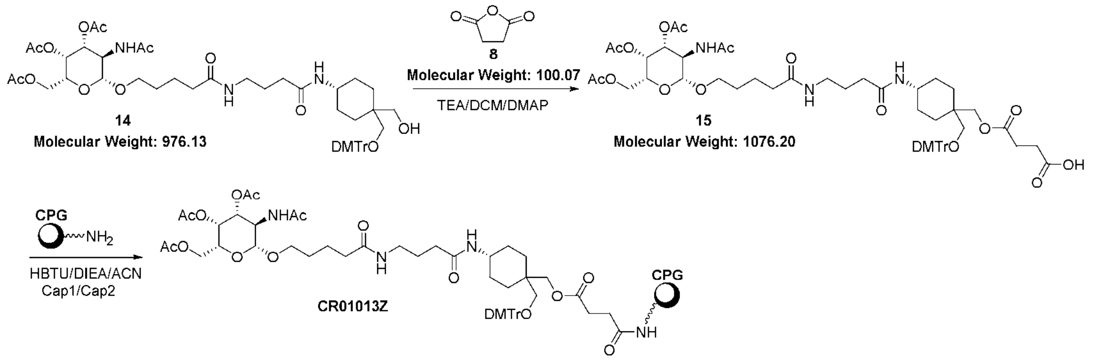


(2.2.1) Synthesis of compound 15

At 25°C, compound 14 (100 mg, 0.10 mmol) prepared according to step (2.1.6) was dissolved in 2 mL of dichloromethane. Triethylamine (25.9 mg, 0.25 mmol), DMAP (1.25 mg, 0.01 mmol), and compound 8 (succinic anhydride, 15.4 mg, 0.15 mmol) were added. The reaction system was stirred at 25°C for 16 h. After the reaction was completed, the solvent in the reaction solution was evaporated, and the residue was purified by reverse-phase column chromatography (C18 column, eluent: water/acetonitrile = 2/1, v/v). Compound 15 (110 mg, 0.10 mmol, yield 100%) was obtained as a yellow oil. ESI-MS (m/z) = 1099.3 $[M+Na]^+$.

(2.2.2) Synthesis of compound CR01013Z

Compound 15 (50 mg, 0.04 mmol) prepared according to step (2.2.1) was dissolved in 10 mL of acetonitrile. HBTU (24.2 mg, 0.06 mmol), DIEA (11.0 mg, 0.08 mmol), and amino-CPG (1.06 g, loading capacity 80 μmol/g) were added. The reaction system was stirred and reacted at 25°C for 16 h. After the reaction was completed, the reaction mixture was filtered to obtain a filter cake. The filter cake was first washed sequentially twice with 50 mL of dichloromethane (2 × 50 mL), three times with 50 mL of acetonitrile (3 × 50 mL), and once with 50 mL of ethyl acetate (1 × 50 mL), and then dried under vacuum. To the dried filter cake were added Cap1 (4.8 mL), Cap2 (0.54 mL), and DMAP (2.59 mg). The reaction system was stirred and reacted at 25°C for 5 h. After the reaction was completed, the reaction mixture was filtered and a filter cake was obtained. The filter cake was washed three times with 50 mL of acetonitrile (3 × 50 mL) and dried under vacuum. Compound CR01013Z (900 mg, with a loading capacity of 20 to 30 μmol/g) was obtained.

Wherein Cap1 and Cap2 are capping reagents, Cap1 is a 20 vol% solution of N-methylimidazole in a mixed solution of pyridine/acetonitrile with a volume ratio of pyridine to acetonitrile of 3:5; and Cap2 is a 20 vol% solution of acetic anhydride in acetonitrile.

**Preparation of siRNA/siRNA Conjugates**

**Preparation Example 3: Preparation of siRNA**

(3.1) Synthesis of sense strand (SS)

Using the phosphoramidite solid-phase nucleic acid synthesis method, nucleoside monomers were linked

sequentially in a 3' to 5' direction. Each cycle of nucleoside monomer addition included four reaction steps: deprotection, coupling, capping, and oxidation or sulfurization. The synthesis conditions were as follows.

The nucleoside monomers were prepared as 0.1 M solutions of the nucleoside monomer in acetonitrile.

The conditions for the deprotection reaction were the same for each step. The conditions for the deprotection reaction were: a temperature of 25°C, a reaction time of 70 s, and the deprotection reagent was a 3 vol% solution of dichloroacetic acid in dichloromethane, wherein the molar ratio of dichloroacetic acid to the 4,4'-dimethoxytrityl protecting group on the solid-phase support was 5:1.

The conditions for the coupling reaction were the same for each step. The conditions for the coupling reaction were: a temperature of 25°C, a molar ratio of the nucleic acid sequence linked on the solid-phase support to the nucleoside monomer of 1:10, a molar ratio of the nucleic acid sequence linked on the solid-phase support to the coupling reagent of 1:65, a reaction time of 600 s, the coupling reagent was a 0.5 M solution of 5-ethylthio-1H-tetrazole in acetonitrile, and the thiolation reagent was a 0.2 M solution of xanthane hydride in a mixed solution of acetonitrile/pyridine (the volume ratio of acetonitrile to pyridine was 1:1).

The conditions for the capping reaction were the same for each step. The conditions for the capping reaction were: a temperature of 25°C; a reaction time of 2 min; the capping reagent solution was a mixed solution of Cap1 and Cap2 in a molar ratio of 1:1, where Cap1 was a 20 vol% solution of N-methylimidazole in a mixed solution of pyridine/acetonitrile with a volume ratio of pyridine to acetonitrile of 3:5, and Cap2 was a 20 vol% solution of acetic anhydride in acetonitrile; the molar ratio of N-methylimidazole in the Cap1 capping reagent, acetic anhydride in the Cap2 capping reagent, and the nucleic acid sequence linked on the solid-phase support was 1:1:1.

The conditions for the oxidation reaction were the same for each step. The conditions for the oxidation reaction were: a temperature of 25°C; a reaction time of 3 s; the oxidizing reagent was a 0.05 M aqueous iodine solution, with a molar ratio of iodine to the nucleic acid sequence linked on the solid-phase support of 30:1; and the oxidation reaction was performed in a mixed solvent of water/pyridine (the volume ratio of water to pyridine was 1:9). The conditions for the sulfurization reaction were: a temperature of 25°C; a reaction time of 360 s; the thiolation reagent was a 0.2 M solution of xanthane hydride in pyridine, with a molar ratio of the thiolation reagent to the nucleic acid sequence linked on the solid-phase support of 4:1; and the sulfurization reaction was performed in a mixed solvent of water/pyridine (the volume ratio of water to pyridine was 1:9).

After the last nucleoside monomer was linked, the nucleic acid moleculars linked on the solid-phase support was subjected to cleavage, deprotection, purification, desalting, and lyophilization sequentially, and the sense strands were obtained.

Cleavage and deprotection conditions were as follows: the synthesized nucleotide molecular linked to the solid-phase support was added to a 25 % aqueous ammonia solution, with the amount of aqueous ammonia being 0.5 mL/μmol, and the mixture was reacted at 55°C for 16 h. The solvent was removed, and the residue was concentrated to dryness under vacuum. After the aqueous ammonia treatment, the product was dissolved using 0.4 mL of N-methylpyrrolidone per μmol of the single-stranded nucleic acid, and 0.3 mL of triethylamine per μmol and 0.6 mL of triethylamine trihydrofluoride per μmol were added to remove the 2'-O-TBDMS protecting group on the ribose.

Purification and desalting conditions: Purification of the nucleic acid was accomplished by NaCl gradient elution using a preparative ion-exchange chromatography column (Source 15Q). Specifically: Eluent 1 was 20 mM sodium phosphate (pH=8.1) in a mixed solvent of water/acetonitrile (volume ratio of water to acetonitrile was 9:1); Eluent 2 was 1.5 M sodium chloride, 20 mM sodium phosphate (pH=8.1), in a mixed solvent of water/acetonitrile (volume ratio of water to acetonitrile was 9:1); and the elution gradient was from Eluent 1:Eluent 2 = (100:0) to (50:50). The product elution fractions were collected and combined, and desalting was performed using a reverse-phase chromatography column. The desalting conditions included desalting using a dextran gel column, with the packing material being dextran gel G25, and eluting with deionized water.

Detection: Purity was detected using ion-exchange chromatography (IEX-HPLC); molecular weight was detected using liquid chromatography-mass spectrometry (LC-MS). The measured value of the molecular weight was compared with the theoretical value. If the measured value and the theoretical value were consistent, it indicated that the sense strand of the siRNA was obtained.

(3.2) Synthesis of antisense strand (AS)

A universal solid-phase support was used to synthesize the antisense strand. The conditions for deprotection, coupling, capping, and oxidation or sulfurization reactions, the conditions for cleavage and deprotection, and the processes and conditions for purification and desalting in the solid-phase synthesis method of the antisense strand were the same as those for the synthesis of the sense strand in step (3.1).

Detection: Purity was detected using ion-exchange chromatography (IEX-HPLC); molecular weight was detected using liquid chromatography-mass spectrometry (LC-MS). The measured value of the molecular weight was compared with the theoretical value. If the measured value and the theoretical value were consistent, it indicated that the

antisense strand of the siRNA was obtained.

(3.3) Synthesis of siRNA duplex

The sense strand synthesized in step (3.1) and the antisense strand synthesized in step (3.2) were mixed in an equimolar ratio, dissolved in water for injection, and heated to 95°C. The mixture was slowly cooled to room temperature and held at room temperature for 10 min. The sense and antisense strands were allowed to form a double-stranded structure through hydrogen bonds, and siRNAs having the sense and antisense strands shown in Tables 4 to 7 were obtained.

Table 4: Sequence Information of siRNAs

| siRNA No. | sense strand (5'-3') | | antisense strand (5'-3') | |
|---|---|---|---|---|
| RN002001 | GUCCACGACUUCCCAGGCA | SEQ ID NO: 1 | UGCCUGGGAAGUCGUGGACAG | SEQ ID NO: 213 |
| RN002002 | CCACGACUUCCCAGGCAAA | SEQ ID NO: 2 | UUUGCCUGGGAAGUCGUGGAC | SEQ ID NO: 214 |
| RN002003 | CACGACUUCCCAGGCAAAA | SEQ ID NO: 3 | UUUUGCCUGGGAAGUCGUGGA | SEQ ID NO: 215 |
| RN002004 | GUGCUGUCCAGUGAGAAGA | SEQ ID NO: 4 | UCUUCUCACUGGACAGCACUA | SEQ ID NO: 216 |
| RN002005 | GCUGUCCAGUGAGAAGACU | SEQ ID NO: 5 | AGUCUUCUCACUGGACAGCAC | SEQ ID NO: 217 |
| RN002006 | CAGAGCGGGUACCUCUUCA | SEQ ID NO: 6 | UGAAGAGGUACCCGCUCUGCA | SEQ ID NO: 218 |
| RN002007 | GCGGGUACCUCUUCAUCCA | SEQ ID NO: 7 | UGGAUGAAGAGGUACCCGCUC | SEQ ID NO: 219 |
| RN002008 | CAGACAGACAAGACCAUCU | SEQ ID NO: 8 | AGAUGGUCUUGUCUGUCUGGA | SEQ ID NO: 220 |
| RN002009 | GGAUCUUCACCGUCAACCA | SEQ ID NO: 9 | UGGUUGACGGUGAAGAUCCGA | SEQ ID NO: 221 |
| RN002010 | CCGUCAACCACAAGCUGCU | SEQ ID NO: 10 | AGCAGCUUGUGGUUGACGGUG | SEQ ID NO: 222 |
| RN002011 | CGUCAACCACAAGCUGCUA | SEQ ID NO: 11 | UAGCAGCUUGUGGUUGACGGU | SEQ ID NO: 223 |
| RN002012 | CGGUCAAGCAGGACUCCUU | SEQ ID NO: 12 | AAGGAGUCCUGCUUGACCGGG | SEQ ID NO: 224 |
| RN002013 | GUCAAGCAGGACUCCUUGU | SEQ ID NO: 13 | ACAAGGAGUCCUGCUUGACCG | SEQ ID NO: 225 |
| RN002014 | CAAGCAGGACUCCUUGUCU | SEQ ID NO: 14 | AGACAAGGAGUCCUGCUUGAC | SEQ ID NO: 226 |
| RN002015 | CUUGGGACAUUCCGGAACU | SEQ ID NO: 15 | AGUUCCGGAAUGUCCCAAGAC | SEQ ID NO: 227 |
| RN002016 | GGGACAUUCCGGAACUCGU | SEQ ID NO: 16 | ACGAGUUCCGGAAUGUCCCAA | SEQ ID NO: 228 |
| RN002017 | CAGUGGAAGAUCCGAGCCU | SEQ ID NO: 17 | AGGCUCGGAUCUUCCACUGGC | SEQ ID NO: 229 |
| RN002018 | AGUGGAAGAUCCGAGCCUA | SEQ ID NO: 18 | UAGGCUCGGAUCUUCCACUGG | SEQ ID NO: 230 |
| RN002019 | GGAAGAUCCGAGCCUACUA | SEQ ID NO: 19 | UAGUAGGCUCGGAUCUUCCAC | SEQ ID NO: 231 |

(continued)

| siRNA No. | sense strand (5'-3') | | antisense strand (5'-3') | |
|---|---|---|---|---|
| RN002020 | GAAGAUCCGAGCCUACUAU | SEQ ID NO: 20 | AUAGUAGGCUCGGAUCUUCCA | SEQ ID NO: 232 |
| RN002021 | GAUCCGAGCCUACUAUGAA | SEQ ID NO: 21 | UUCAUAGUAGGCUCGGAUCUU | SEQ ID NO: 233 |
| RN002022 | AGUUUGAGGUGAAGGAGUA | SEQ ID NO: 22 | UACUCCUUCACCUCAAACUCA | SEQID No.234 |
| RN002023 | GCCCAGUUUCGAGGUCAUA | SEQ ID NO: 23 | UAUGACCUCGAAACUGGGCAG | SEQ ID NO: 235 |
| RN002024 | CCAGUUUCGAGGUCAUAGU | SEQ ID NO: 24 | ACUAUGACCUCGAAACUGGGC | SEQ ID NO: 236 |
| RN002025 | CAGUUUCGAGGUCAUAGUG | SEQ ID NO: 25 | CACUAUGACCUCGAAACUGGG | SEQ ID NO: 237 |
| RN002026 | GUUUCGAGGUCAUAGUGGA | SEQ ID NO: 26 | UCCACUAUGACCUCGAAACUG | SEQ ID NO: 238 |
| RN002027 | CGAGGUCAUAGUGGAGCCU | SEQ ID NO: 27 | AGGCUCCACUAUGACCUCGAA | SEQ ID NO: 239 |
| RN002028 | AGUGGAGCCUACAGAGAAA | SEQ ID NO: 28 | UUUCUCUGUAGGCUCCACUAU | SEQ ID NO: 240 |
| RN002029 | CAGAGAAAUUCUACUACAU | SEQ ID NO: 29 | AUGUAGUAGAAUUUCUCUGUA | SEQ ID NO: 241 |
| RN002030 | AAAUUCUACUACAUCUAUA | SEQ ID NO: 30 | UAUAGAUGUAGUAGAAUUUCU | SEQ ID NO: 242 |
| RN002031 | GGAACUGCCUUUGUCAUCU | SEQ ID NO: 31 | AGAUGACAAAGGCAGUUCCCU | SEQ ID NO: 243 |
| RN002032 | GAACUGCCUUUGUCAUCUU | SEQ ID NO: 32 | AAGAUGACAAAGGCAGUUCCC | SEQ ID NO: 244 |
| RN002033 | CCUUUGUCAUCUUCGGGAU | SEQ ID NO: 33 | AUCCCGAAGAUGACAAAGGCA | SEQ ID NO: 245 |
| RN002034 | CUGAAUCCCUCAAGCGCAU | SEQ ID NO: 34 | AUGCGCUUGAGGGAUUCAGGC | SEQ ID NO: 246 |
| RN002035 | CAGGCAGUGACAUGGUGCA | SEQ ID NO: 35 | UGCACCAUGUCACUGCCUGAG | SEQ ID NO: 247 |
| RN002036 | GUGACCUCUCCCUACCAGA | SEQ ID NO: 36 | UCUGGUAGGGAGAGGUCACGA | SEQ ID NO: 248 |
| RN002037 | GAAUGCCCUUUGACCUCAU | SEQ ID NO: 37 | AUGAGGUCAAAGGGCAUUCCU | SEQ ID NO: 249 |
| RN002038 | CCUUUGACCUCAUGGUGUU | SEQ ID NO: 38 | AACACCAUGAGGUCAAAGGGC | SEQ ID NO: 250 |
| RN002039 | GACCUCAUGGUGUUCGUGA | SEQ ID NO: 39 | UCACGAACACCAUGAGGUCAA | SEQ ID NO: 251 |
| RN002040 | CUCAUGGUGUUCGUGACGA | SEQ ID NO: 40 | UCGUCACGAACACCAUGAGGU | SEQ ID NO: 252 |
| RN002041 | UCAUGGUGUUCGUGACGAA | SEQ ID NO: 41 | UUCGUCACGAACACCAUGAGG | SEQ ID NO: 253 |
| RN002042 | CUGUGCAGUCUCUAACCCA | SEQ ID NO: 42 | UGGGUUAGAGACUGCACAGUG | SEQ ID NO: 254 |

(continued)

| siRNA No. | sense strand (5'-3') | | antisense strand (5'-3') | |
|---|---|---|---|---|
| RN002043 | GCCAGAAGCCCUUGAGCAU | SEQ ID NO: 43 | AUGCUCAAGGGCUUCUGGCUG | SEQ ID NO: 255 |
| RN002044 | CAGAAGCCCUUGAGCAUCA | SEQ ID NO: 44 | UGAUGCUCAAGGGCUUCUGGC | SEQ ID NO: 256 |
| RN002045 | GCAUCACGGUGCGCACGAA | SEQ ID NO: 45 | UUCGUGCGCACCGUGAUGCUC | SEQ ID NO: 257 |
| RN002046 | CGGUGCGCACGAAGAAGCA | SEQ ID NO: 46 | UGCUUCUUCGUGCGCACCGUG | SEQ ID NO: 258 |
| RN002047 | CCGUGGGCAACUCCAACAA | SEQ ID NO: 47 | UUGUUGGAGUUGCCCACGGUG | SEQ ID NO: 259 |
| RN002048 | CGUGGGCAACUCCAACAAU | SEQ ID NO: 48 | AUUGUUGGAGUUGCCCACGGU | SEQ ID NO: 260 |
| RN002049 | GUGGGCAACUCCAACAAUU | SEQ ID NO: 49 | AAUUGUUGGAGUUGCCCACGG | SEQ ID NO: 261 |
| RN002050 | GCAACUCCAACAAUUACCU | SEQ ID NO: 50 | AGGUAAUUGUUGGAGUUGCCC | SEQ ID NO: 262 |
| RN002051 | CUCCAACAAUUACCUGCAU | SEQ ID NO: 51 | AUGCAGGUAAUUGUUGGAGUU | SEQ ID NO: 263 |
| RN002052 | GGGGAGACCCUCAACGUCA | SEQ ID NO: 52 | UGACGUUGAGGGUCUCCCCGG | SEQ ID NO: 264 |
| RN002053 | GGAGACCCUCAACGUCAA | SEQ ID NO: 53 | UUGACGUUGAGGGUCUCCCCG | SEQ ID NO: 265 |
| RN002054 | GAGACCCUCAACGUCAACU | SEQ ID NO: 54 | AGUUGACGUUGAGGGUCUCCC | SEQ ID NO: 266 |
| RN002055 | CCCUCAACGUCAACUUCCU | SEQ ID NO: 55 | AGGAAGUUGACGUUGAGGGUC | SEQ ID NO: 267 |
| RN002056 | UCAACUUCCUCCUGCGAAU | SEQ ID NO: 56 | AUUCGCAGGAGGAAGUUGACG | SEQ ID NO: 268 |
| RN002057 | GAGGCCAAGAUCCGCUACU | SEQ ID NO: 57 | AGUAGCGGAUCUUGGCCUCGU | SEQ ID NO: 269 |
| RN002058 | AGAUCCGCUACUACACCUA | SEQ ID NO: 58 | UAGGUGUAGUAGCGGAUCUUG | SEQ ID NO: 270 |
| RN002059 | CGCUACUACACCUACCUGA | SEQ ID NO: 59 | UCAGGUAGGUGUAGUAGCGGA | SEQ ID NO: 271 |
| RN002060 | GCUACUACACCUACCUGAU | SEQ ID NO: 60 | AUCAGGUAGGUGUAGUAGCGG | SEQ ID NO: 272 |
| RN002061 | CCGUGUGGGUGGACGUCAA | SEQ ID NO: 61 | UUGACGUCCACCCACACGGAG | SEQ ID NO: 273 |
| RN002062 | GGGCUCGCUGGUGGUAAAA | SEQ ID NO: 62 | UUUUACCACCAGCGAGCCCAC | SEQ ID NO: 274 |
| RN002063 | CCCUGAAGAUAGAGGGUGA | SEQ ID NO: 63 | UCACCCUCUAUCUUCAGGGUC | SEQ ID NO: 275 |
| RN002064 | CGGGAGAACCCCAUGAGGU | SEQ ID NO: 64 | ACCUCAUGGGGUUCUCCCGCA | SEQ ID NO: 276 |
| RN002065 | GGGAGAACCCCAUGAGGUU | SEQ ID NO: 65 | AACCUCAUGGGGUUCUCCCGC | SEQ ID NO: 277 |

(continued)

| siRNA No. | sense strand (5'-3') | | antisense strand (5'-3') | |
|---|---|---|---|---|
| RN002066 | GAGAACCCCAUGAGGUUCU | SEQ ID NO: 66 | AGAACCUCAUGGGGUUCUCCC | SEQ ID NO: 278 |
| RN002067 | CUGCUGCAACUACAUCACA | SEQ ID NO: 67 | UGUGAUGUAGUUGCAGCAGUC | SEQ ID NO: 279 |
| RN002068 | GGAGUAACCUGGAUGAGGA | SEQ ID NO: 68 | UCCUCAUCCAGGUUACUCCUG | SEQ ID NO: 280 |
| RN002069 | GUAACCUGGAUGAGGACAU | SEQ ID NO: 69 | AUGUCCUCAUCCAGGUUACUC | SEQ ID NO: 281 |
| RN002070 | GCAGAAGAGAACAUCGUUU | SEQ ID NO: 70 | AAACGAUGUUCUCUUCUGCAA | SEQ ID NO: 282 |
| RN002071 | GAGAACAUCGUUUCCCGAA | SEQ ID NO: 71 | UUCGGGAAACGAUGUUCUCUU | SEQ ID NO: 283 |
| RN002072 | CCCGAAGUGAGUUCCCAGA | SEQ ID NO: 72 | UCUGGGAACUCACUUCGGGAA | SEQ ID NO: 284 |
| RN002073 | CGAAGCUCAUGAAUAUAUU | SEQ ID NO: 73 | AAUAUAUUCAUGAGCUUCGUA | SEQ ID NO: 285 |
| RN002074 | GAAGCUCAUGAAUAUAUUU | SEQ ID NO: 74 | AAAUAUAUUCAUGAGCUUCGU | SEQ ID NO: 286 |
| RN002075 | CCCUUCGAGGUCACAGUAA | SEQ ID NO: 75 | UUACUGUGACCUCGAAGGGGU | SEQ ID NO: 287 |
| RN002076 | CCUUCGAGGUCACAGUAAU | SEQ ID NO: 76 | AUUACUGUGACCUCGAAGGGG | SEQ ID NO: 288 |
| RN002077 | UCACAGUAAUGCAGGACUU | SEQ ID NO: 77 | AAGUCCUGCAUUACUGUGACC | SEQ ID NO: 289 |
| RN002078 | CCUGCGGCUACCCUACUCU | SEQ ID NO: 78 | AGAGUAGGGUAGCCGCAGGUC | SEQ ID NO: 290 |
| RN002079 | GCGGCUACCCUACUCUGUU | SEQ ID NO: 79 | AACAGAGUAGGGUAGCCGCAG | SEQ ID NO: 291 |
| RN002080 | GGCUACCCUACUCUGUUGU | SEQ ID NO: 80 | ACAACAGAGUAGGGUAGCCGC | SEQ ID NO: 292 |
| RN002081 | GCUACCCUACUCUGUUGUU | SEQ ID NO: 81 | AACAACAGAGUAGGGUAGCCG | SEQ ID NO: 293 |
| RN002082 | ACCCUACUCUGUUGUUCGA | SEQ ID NO: 82 | UCGAACAACAGAGUAGGGUAG | SEQ ID NO: 294 |
| RN002083 | CCCUACUCUGUUGUUCGAA | SEQ ID NO: 83 | UUCGAACAACAGAGUAGGGUA | SEQ ID NO: 295 |
| RN002084 | CCUACUCUGUUGUUCGAAA | SEQ ID NO: 84 | UUUCGAACAACAGAGUAGGGU | SEQ ID NO: 296 |
| RN002085 | CGAAACGAGCAGGUGGAAA | SEQ ID NO: 85 | UUUCCACCUGCUCGUUUCGAA | SEQ ID NO: 297 |
| RN002086 | GAAACGAGCAGGUGGAAAU | SEQ ID NO: 86 | AUUUCCACCUGCUCGUUUCGA | SEQ ID NO: 298 |
| RN002087 | CGAGCAGGUGGAAAUCCGA | SEQ ID NO: 87 | UCGGAUUUCCACCUGCUCGUU | SEQ ID NO: 299 |
| RN002088 | CAAUUACCGGCAGAACCAA | SEQ ID NO: 88 | UUGGUUCUGCCGGUAAUUGUA | SEQ ID NO: 300 |

(continued)

| siRNA No. | sense strand (5'-3') | | antisense strand (5'-3') | |
|---|---|---|---|---|
| RN002089 | GGGUGGAACUACUCCACAA | SEQ ID NO: 89 | UUGUGGAGUAGUUCCACCCUC | SEQ ID NO: 301 |
| RN002090 | GGUGGAACUACUCCACAAU | SEQ ID NO: 90 | AUUGUGGAGUAGUUCCACCCU | SEQ ID NO: 302 |
| RN002091 | GGAACUACUCCACAAUCCA | SEQ ID NO: 91 | UGGAUUGUGGAGUAGUUCCAC | SEQ ID NO: 303 |
| RN002092 | CGUCACCAGCAGACCGUAA | SEQ ID NO: 92 | UUACGGUCUGCUGGUGACGCC | SEQ ID NO: 304 |
| RN002093 | CACCAGCAGACCGUAACCA | SEQ ID NO: 93 | UGGUUACGGUCUGCUGGUGAC | SEQ ID NO: 305 |
| RN002094 | GGAAGUCCCUGAAGGUCGU | SEQ ID NO: 94 | ACGACCUUCAGGGACUUCCUG | SEQ ID NO: 306 |
| RN002095 | GUCGUGCCGGAAGGAAUCA | SEQ ID NO: 95 | UGAUUCCUUCCGGCACGACCU | SEQ ID NO: 307 |
| RN002096 | CGUGCCGGAAGGAAUCAGA | SEQ ID NO: 96 | UCUGAUUCCUUCCGGCACGAC | SEQ ID NO: 308 |
| RN002097 | GUGCCGGAAGGAAUCAGAA | SEQ ID NO: 97 | UUCUGAUUCCUUCCGGCACGA | SEQ ID NO: 309 |
| RN002098 | CCGGAAGGAAUCAGAAUGA | SEQ ID NO: 98 | UCAUUCUGAUUCCUUCCGGCA | SEQ ID NO: 310 |
| RN002099 | CGGAAGGAAUCAGAAUGAA | SEQ ID NO: 99 | UUCAUUCUGAUUCCUUCCGGC | SEQ ID NO: 311 |
| RN002100 | UGCAGACCUCAGUGACCAA | SEQ ID NO: 100 | UUGGUCACUGAGGUCUGCAGG | SEQ ID NO: 312 |
| RN002101 | GACCAGAAUUCUCCUGCAA | SEQ ID NO: 101 | UUGCAGGAGAAUUCUGGUCUC | SEQ ID NO: 313 |
| RN002102 | GAACGGCUGAAGCACCUCA | SEQ ID NO: 102 | UGAGGUGCUUCAGCCGUUCCG | SEQ ID NO: 314 |
| RN002103 | CGCUGUGCAUUACCUGGAU | SEQ ID NO: 103 | AUCCAGGUAAUGCACAGCGAU | SEQ ID NO: 315 |
| RN002104 | GCAUUACCUGGAUGAAACG | SEQ ID NO: 104 | CGUUUCAUCCAGGUAAUGCAC | SEQ ID NO: 316 |
| RN002105 | CCUUGGAGCUCAUCAAGAA | SEQ ID NO: 105 | UUCUUGAUGAGCUCCAAGGCC | SEQ ID NO: 317 |
| RN002106 | GCAGCUGGCCUUCAGACAA | SEQ ID NO: 106 | UUGUCUGAAGGCCAGCUGCUG | SEQ ID NO: 318 |
| RN002107 | CCGCCUACGUGGUCAAGGU | SEQ ID NO: 107 | ACCUUGACCACGUAGGCGGUC | SEQ ID NO: 319 |
| RN002108 | CCUACGUGGUCAAGGUCUU | SEQ ID NO: 108 | AAGACCUUGACCACGUAGGCG | SEQ ID NO: 320 |
| RN002109 | CGUGGUCAAGGUCUUCUCU | SEQ ID NO: 109 | AGAGAAGACCUUGACCACGUA | SEQ ID NO: 321 |
| RN002110 | CUCUGGCUGUCAACCUCAU | SEQ ID NO: 110 | AUGAGGUUGACAGCCAGAGAG | SEQ ID NO: 322 |
| RN002111 | CUCUGCGGGGCUGUUAAAU | SEQ ID NO: 111 | AUUUAACAGCCCCGCAGAGGA | SEQ ID NO: 323 |

(continued)

| siRNA No. | sense strand (5'-3') | | antisense strand (5'-3') | |
|---|---|---|---|---|
| RN002112 | CUGUUAAAUGGCUGAUCCU | SEQ ID NO: 112 | AGGAUCAGCCAUUUAACAGCC | SEQ ID NO: 324 |
| RN002113 | GGAGGAUGCGCCCGUGAUA | SEQ ID NO: 113 | UAUCACGGGCGCAUCCUCCUG | SEQ ID NO: 325 |
| RN002114 | GGCCCUCACGGCCUUUGUU | SEQ ID NO: 114 | AACAAAGGCCGUGAGGGCCAU | SEQ ID NO: 326 |
| RN002115 | CUCACGGCCUUUGUUCUCA | SEQ ID NO: 115 | UGAGAACAAAGGCCGUGAGGG | SEQ ID NO: 327 |
| RN002116 | CCUUUGUUCUCAUCUCGCU | SEQ ID NO: 116 | AGCGAGAUGAGAACAAAGGCC | SEQ ID NO: 328 |
| RN002117 | CUUGAAGCCAACUACAUGA | SEQ ID NO: 117 | UCAUGUAGUUGGCUUCAAGGA | SEQ ID NO: 329 |
| RN002118 | CCAACUACAUGAACCUACA | SEQ ID NO: 118 | UGUAGGUUCAUGUAGUUGGCU | SEQ ID NO: 330 |
| RN002119 | CCUACAGAGAUCCUACACU | SEQ ID NO: 119 | AGUGUAGGAUCUCUGUAGGUU | SEQ ID NO: 331 |
| RN002120 | CUGACCACAGCCAAAGAUA | SEQ ID NO: 120 | UAUCUUUGGCUGUGGUCAGAA | SEQ ID NO: 332 |
| RN002121 | CCACAGCCAAAGAUAAGAA | SEQ ID NO: 121 | UUCUUAUCUUUGGCUGUGGUC | SEQ ID NO: 333 |
| RN002122 | GCCAAAGAUAAGAACCGCU | SEQ ID NO: 122 | AGCGGUUCUUAUCUUUGGCUG | SEQ ID NO: 334 |
| RN002123 | GCCACAUCCUAUGCCCUCU | SEQ ID NO: 123 | AGAGGGCAUAGGAUGUGGCCU | SEQ ID NO: 335 |
| RN002124 | CCACAUCCUAUGCCCUCUU | SEQ ID NO: 124 | AAGAGGGCAUAGGAUGUGGCC | SEQ ID NO: 336 |
| RN002125 | GGCCCUACUGCAGCUAAAA | SEQ ID NO: 125 | UUUUAGCUGCAGUAGGGCCAA | SEQ ID NO: 337 |
| RN002126 | CUACUGCAGCUAAAAGACU | SEQ ID NO: 126 | AGUCUUUUAGCUGCAGUAGGG | SEQ ID NO: 338 |
| RN002127 | CAGCUAAAAGACUUUGACU | SEQ ID NO: 127 | AGUCAAAGUCUUUUAGCUGCA | SEQ ID NO: 339 |
| RN002128 | GCUAAAAGACUUUGACUUU | SEQ ID NO: 128 | AAAGUCAAAGUCUUUUAGCUG | SEQ ID NO: 340 |
| RN002129 | CCGUCGUGCGUUGGCUCAA | SEQ ID NO: 129 | UUGAGCCAACGCACGACGGGA | SEQ ID NO: 341 |
| RN002130 | CGUCGUGCGUUGGCUCAAU | SEQ ID NO: 130 | AUUGAGCCAACGCACGACGGG | SEQ ID NO: 342 |
| RN002131 | CGUGCGUUGGCUCAAUGAA | SEQ ID NO: 131 | UUCAUUGAGCCAACGCACGAC | SEQ ID NO: 343 |
| RN002132 | CGUUGGCUCAAUGAACAGA | SEQ ID NO: 132 | UCUGUUCAUUGAGCCAACGCA | SEQ ID NO: 344 |
| RN002133 | GGCUCAAUGAACAGAGAUA | SEQ ID NO: 133 | UAUCUCUGUUCAUUGAGCCAA | SEQ ID NO: 345 |
| RN002134 | CUCAAUGAACAGAGAUACU | SEQ ID NO: 134 | AGUAUCUCUGUUCAUUGAGCC | SEQ ID NO: 346 |

(continued)

| siRNA No. | sense strand (5'-3') | | antisense strand (5'-3') | |
|---|---|---|---|---|
| RN002135 | CCACCUUCAUGGUGUUCCA | SEQ ID NO: 135 | UGGAACACCAUGAAGGUGGCC | SEQ ID NO: 347 |
| RN002136 | CACCUUCAUGGUGUUCCAA | SEQ ID NO: 136 | UUGGAACACCAUGAAGGUGGC | SEQ ID NO: 348 |
| RN002137 | GGUGUUCCAAGCCUUGGCU | SEQ ID NO: 137 | AGCCAAGGCUUGGAACACCAU | SEQ ID NO: 349 |
| RN002138 | CCGUAUCCACUGGGAAUCU | SEQ ID NO: 138 | AGAUUCCCAGUGGAUACGGUG | SEQ ID NO: 350 |
| RN002139 | GCCUCCUGCGAUCAGAAGA | SEQ ID NO: 139 | UCUUCUGAUCGCAGGAGGCUG | SEQ ID NO: 351 |
| RN002140 | UGCGAUCAGAAGAGACCAA | SEQ ID NO: 140 | UUGGUCUCUUCUGAUCGCAGG | SEQ ID NO: 352 |
| RN002141 | GAUCAGAAGAGACCAAGGA | SEQ ID NO: 141 | UCCUUGGUCUCUUCUGAUCGC | SEQ ID NO: 353 |
| RN002142 | GUUUCACAGUCACAGCUGA | SEQ ID NO: 142 | UCAGCUGUGACUGUGAAACCC | SEQ ID NO: 354 |
| RN002143 | UGACAAUGUACCAUGCUAA | SEQ ID NO: 143 | UUAGCAUGGUACAUUGUCACC | SEQ ID NO: 355 |
| RN002144 | GUACCAUGCUAAGGCCAAA | SEQ ID NO: 144 | UUUGGCCUUAGCAUGGUACAU | SEQ ID NO: 356 |
| RN002145 | CAACUCACCUGUAAUAAAU | SEQ ID NO: 145 | AUUUAUUACAGGUGAGUUGAU | SEQ ID NO: 357 |
| RN002146 | CCUGUAAUAAAUUCGACCU | SEQ ID NO: 146 | AGGUCGAAUUUAUUACAGGUG | SEQ ID NO: 358 |
| RN002147 | GUAAUAAAUUCGACCUCAA | SEQ ID NO: 147 | UUGAGGUCGAAUUUAUUACAG | SEQ ID NO: 359 |
| RN002148 | CGACCUCAAGGUCACCAUA | SEQ ID NO: 148 | UAUGGUGACCUUGAGGUCGAA | SEQ ID NO: 360 |
| RN002149 | GACCUCAAGGUCACCAUAA | SEQ ID NO: 149 | UUAUGGUGACCUUGAGGUCGA | SEQ ID NO: 361 |
| RN002150 | CAAGGUCACCAUAAAACCA | SEQ ID NO: 150 | UGGUUUUAUGGUGACCUUGAG | SEQ ID NO: 362 |
| RN002151 | CCAGCACCGGAAACAGAAA | SEQ ID NO: 151 | UUUCUGUUUCCGGUGCUGGUU | SEQ ID NO: 363 |
| RN002152 | GCACCGGAAACAGAAAAGA | SEQ ID NO: 152 | UCUUUUCUGUUUCCGGUGCUG | SEQ ID NO: 364 |
| RN002153 | GGCCUCAGGAUGCCAAGAA | SEQ ID NO: 153 | UUCUUGGCAUCCUGAGGCCUC | SEQ ID NO: 365 |
| RN002154 | CAGGAUGCCAAGAACACUA | SEQ ID NO: 154 | UAGUGUUCUUGGCAUCCUGAG | SEQ ID NO: 366 |
| RN002155 | GAUGCCAAGAACACUAUGA | SEQ ID NO: 155 | UCAUAGUGUUCUUGGCAUCCU | SEQ ID NO: 367 |
| RN002156 | CCAAGAACACUAUGAUCCU | SEQ ID NO: 156 | AGGAUCAUAGUGUUCUUGGCA | SEQ ID NO: 368 |
| RN002157 | AGAACACUAUGAUCCUUGA | SEQ ID NO: 157 | UCAAGGAUCAUAGUGUUCUUG | SEQ ID NO: 369 |

(continued)

| siRNA No. | sense strand (5'-3') | | antisense strand (5'-3') | |
|---|---|---|---|---|
| RN002158 | GAGACCAGGAUGCCACUAU | SEQ ID NO: 158 | AUAGUGGCAUCCUGGUCUCCC | SEQ ID NO: 370 |
| RN002159 | CCAGGAUGCCACUAUGUCU | SEQ ID NO: 159 | AGACAUAGUGGCAUCCUGGUC | SEQ ID NO: 371 |
| RN002160 | CAGGAUGCCACUAUGUCUA | SEQ ID NO: 160 | UAGACAUAGUGGCAUCCUGGU | SEQ ID NO: 372 |
| RN002161 | GGAUGCCACUAUGUCUAUA | SEQ ID NO: 161 | UAUAGACAUAGUGGCAUCCUG | SEQ ID NO: 373 |
| RN002162 | GAUGCCACUAUGUCUAUAU | SEQ ID NO: 162 | AUAUAGACAUAGUGGCAUCCU | SEQ ID NO: 374 |
| RN002163 | GUUGACAGAUACAUCUCCA | SEQ ID NO: 163 | UGGAGAUGUAUCUGUCAACAC | SEQ ID NO: 375 |
| RN002164 | GACAGAUACAUCUCCAAGU | SEQ ID NO: 164 | ACUUGGAGAUGUAUCUGUCAA | SEQ ID NO: 376 |
| RN002165 | CAGAUACAUCUCCAAGUAU | SEQ ID NO: 165 | AUACUUGGAGAUGUAUCUGUC | SEQ ID NO: 377 |
| RN002166 | AGAUACAUCUCCAAGUAUG | SEQ ID NO: 166 | CAUACUUGGAGAUGUAUCUGU | SEQ ID NO: 378 |
| RN002167 | GAUACAUCUCCAAGUAUGA | SEQ ID NO: 167 | UCAUACUUGGAGAUGUAUCUG | SEQ ID NO: 379 |
| RN002168 | CCAAGUAUGAGCUGGACAA | SEQ ID NO: 168 | UUGUCCAGCUCAUACUUGGAG | SEQ ID NO: 380 |
| RN002169 | GGACAAAGCCUUCUCCGAU | SEQ ID NO: 169 | AUCGGAGAAGGCUUUGUCCAG | SEQ ID NO: 381 |
| RN002170 | GACAAAGCCUUCUCCGAUA | SEQ ID NO: 170 | UAUCGGAGAAGGCUUUGUCCA | SEQ ID NO: 382 |
| RN002171 | GCCUUCUCCGAUAGGAACA | SEQ ID NO: 171 | UGUUCCUAUCGGAGAAGGCUU | SEQ ID NO: 383 |
| RN002172 | CCGAUAGGAACACCCUCAU | SEQ ID NO: 172 | AUGAGGGUGUUCCUAUCGGAG | SEQ ID NO: 384 |
| RN002173 | GAUAGGAACACCCUCAUCA | SEQ ID NO: 173 | UGAUGAGGGUGUUCCUAUCGG | SEQ ID NO: 385 |
| RN002174 | CUGGACAAGGUCUCACACU | SEQ ID NO: 174 | AGUGUGAGACCUUGUCCAGGU | SEQ ID NO: 386 |
| RN002175 | GCUUUCAAAGUUCACCAAU | SEQ ID NO: 175 | AUUGGUGAACUUUGAAAGCUA | SEQ ID NO: 387 |
| RN002176 | CUUUCAAAGUUCACCAAUA | SEQ ID NO: 176 | UAUUGGUGAACUUUGAAAGCU | SEQ ID NO: 388 |
| RN002177 | CAGUCAAGGUCUACGCCUA | SEQ ID NO: 177 | UAGGCGUAGACCUUGACUGCU | SEQ ID NO: 389 |
| RN002178 | GUCAAGGUCUACGCCUAUU | SEQ ID NO: 178 | AAUAGGCGUAGACCUUGACUG | SEQ ID NO: 390 |
| RN002179 | AGGUCUACGCCUAUUACAA | SEQ ID NO: 179 | UUGUAAUAGGCGUAGACCUUG | SEQ ID NO: 391 |
| RN002180 | GCUGUACCCGGUUCUACCA | SEQ ID NO: 180 | UGGUAGAACCGGGUACAGCUU | SEQ ID NO: 392 |

(continued)

| siRNA No. | sense strand (5'-3') | | antisense strand (5'-3') | |
|---|---|---|---|---|
| RN002181 | CUGUACCCGGUUCUACCAU | SEQ ID NO: 181 | AUGGUAGAACCGGGUACAGCU | SEQ ID NO: 393 |
| RN002182 | CCGGAAAAGGAGGAUGGAA | SEQ ID NO: 182 | UUCCAUCCUCCUUUUCCGGAU | SEQ ID NO: 394 |
| RN002183 | CGGAAAAGGAGGAUGGAAA | SEQ ID NO: 183 | UUUCCAUCCUCCUUUUCCGGA | SEQ ID NO: 395 |
| RN002184 | GAGGAUGGAAAGCUGAACA | SEQ ID NO: 184 | UGUUCAGCUUUCCAUCCUCCU | SEQ ID NO: 396 |
| RN002185 | GCCGCUGUGCUGAGGAGAA | SEQ ID NO: 185 | UUCUCCUCAGCACAGCGGCAC | SEQ ID NO: 397 |
| RN002186 | CCGCUGUGCUGAGGAGAAU | SEQ ID NO: 186 | AUUCUCCUCAGCACAGCGGCA | SEQ ID NO: 398 |
| RN002187 | CGCUGUGCUGAGGAGAAUU | SEQ ID NO: 187 | AAUUCUCCUCAGCACAGCGGC | SEQ ID NO: 399 |
| RN002188 | GUGCUGAGGAGAAUUGCUU | SEQ ID NO: 188 | AAGCAAUUCUCCUCAGCACAG | SEQ ID NO: 400 |
| RN002189 | GCUGAGGAGAAUUGCUUCA | SEQ ID NO: 189 | UGAAGCAAUUCUCCUCAGCAC | SEQ ID NO: 401 |
| RN002190 | GGAGAAUUGCUUCAUACAA | SEQ ID NO: 190 | UUGUAUGAAGCAAUUCUCCUC | SEQ ID NO: 402 |
| RN002191 | GAGAAUUGCUUCAUACAAA | SEQ ID NO: 191 | UUUGUAUGAAGCAAUUCUCCU | SEQ ID NO: 403 |
| RN002192 | GUGAGCCAGGAGUGGACUA | SEQ ID NO: 192 | UAGUCCACUCCUGGCUCACAG | SEQ ID NO: 404 |
| RN002193 | CCAGGAGUGGACUAUGUGU | SEQ ID NO: 193 | ACACAUAGUCCACUCCUGGCU | SEQ ID NO: 405 |
| RN002194 | CAGGAGUGGACUAUGUGUA | SEQ ID NO: 194 | UACACAUAGUCCACUCCUGGC | SEQ ID NO: 406 |
| RN002195 | GGAGUGGACUAUGUGUACA | SEQ ID NO: 195 | UGUACACAUAGUCCACUCCUG | SEQ ID NO: 407 |
| RN002196 | GAGUGGACUAUGUGUACAA | SEQ ID NO: 196 | UUGUACACAUAGUCCACUCCU | SEQ ID NO: 408 |
| RN002197 | GUGGACUAUGUGUACAAGA | SEQ ID NO: 197 | UCUUGUACACAUAGUCCACUC | SEQ ID NO: 409 |
| RN002198 | CCAAUGACUUUGACGAGUA | SEQ ID NO: 198 | UACUCGUCAAAGUCAUUGGAC | SEQ ID NO: 410 |
| RN002199 | GACUUUGACGAGUACAUCA | SEQ ID NO: 199 | UGAUGUACUCGUCAAAGUCAU | SEQ ID NO: 411 |
| RN002200 | ACUUUGACGAGUACAUCAU | SEQ ID NO: 200 | AUGAUGUACUCGUCAAAGUCA | SEQ ID NO: 412 |
| RN002201 | GACGAGUACAUCAUGGCCA | SEQ ID NO: 201 | UGGCCAUGAUGUACUCGUCAA | SEQ ID NO: 413 |
| RN002202 | CAUCAAGUCAGGCUCGGAU | SEQ ID NO: 202 | AUCCGAGCCUGACUUGAUGGU | SEQ ID NO: 414 |
| RN002203 | CAGGCUCGGAUGAGGUGCA | SEQ ID NO: 203 | UGCACCUCAUCCGAGCCUGAC | SEQ ID NO: 415 |

(continued)

| siRNA No. | sense strand (5'-3') | | antisense strand (5'-3') | |
|---|---|---|---|---|
| RN002204 | GCUCGGAUGAGGUGCAGGU | SEQ ID NO: 204 | ACCUGCACCUCAUCCGAGCCU | SEQ ID NO: 416 |
| RN002205 | CGUUCAUCAGCCCCAUCAA | SEQ ID NO: 205 | UUGAUGGGGCUGAUGAACGUG | SEQ ID NO: 417 |
| RN002206 | GGGGUCUCUCCUCCGAUUU | SEQ ID NO: 206 | AAAUCGGAGGAGAGACCCCAC | SEQ ID NO: 418 |
| RN002207 | CCGAUUUCUGGGGAGAGAA | SEQ ID NO: 207 | UUCUCUCCCCAGAAAUCGGAG | SEQ ID NO: 419 |
| RN002208 | CUCAGCUACAUCAUCGGGA | SEQ ID NO: 208 | UCCCGAUGAUGUAGCUGAGGU | SEQ ID NO: 420 |
| RN002209 | GCUACAUCAUCGGGAAGGA | SEQ ID NO: 209 | UCCUUCCCGAUGAUGUAGCUG | SEQ ID NO: 421 |
| RN002210 | CCGAGGAGGACGAAUGCCA | SEQ ID NO: 210 | UGGCAUUCGUCCUCCUCGGGC | SEQ ID NO: 422 |
| RN002211 | CGAGGAGGACGAAUGCCAA | SEQ ID NO: 211 | UUGGCAUUCGUCCUCCUCGGG | SEQ ID NO: 423 |
| RN002212 | CUUUGGGUGCCCCAACUGA | SEQ ID NO: 212 | UCAGUUGGGGCACCCAAAGAC | SEQ ID NO: 424 |

The software for preparing the sequence listing requires that U be represented as T when generating RNA sequences. Therefore, the siRNA sequences of the present disclosure cannot be correctly represented in the sequence listing. All sequences are as set forth in the description.

Table 5: Sequence Information of siRNAs

| siRNA No. | sense strand (5'-3') | | antisense strand (5'-3') | |
|---|---|---|---|---|
| RX002 001 | GmsUmsCmCmAmCmGfAfCfUmUm CmCmCmAmGmGmCmAm | SEQ ID NO: 425 | UmsGfsCmCmUmGfGmGmAmAmGm UmCmGfUmGfGmAmCmsAmsGm | SEQ ID NO: 637 |
| RX002 002 | CmsCmsAmCmGmAmCfUfUfCmCm CmAmGmGmCmAmAmAm | SEQ ID NO: 426 | UmsUfsUmGmCmCfUmGmGmGmAm AmGmUfCmGfUmGmGmsAmsCm | SEQ ID NO: 638 |
| RX002 003 | CmsAmsCmGmAmCmUfUfCfCmCm AmGmGmCmAmAmAmAm | SEQ ID NO: 427 | UmsUfsUmUmGmCfCmUmGmGmGm AmAmGfUmCfGmUmGmsGmsAm | SEQ ID NO: 639 |
| RX002 004 | GmsUmsGmCmUmGmUfCfCfAmGm UmGmAmGmAmAmGmAm | SEQ ID NO: 428 | UmsCfsUmUmCmUfCmAmCmUmGm GmAmCfAmGfCmAmCmsUmsAm | SEQ ID NO: 640 |
| RX002 005 | GmsCmsUmGmUmCmCfAfGfUmGm AmGmAmAmGmAmCmUm | SEQ ID NO: 429 | AmsGfsUmCmUmUfCmUmCmAmCm UmGmGfAmCfAmGmCmsAmsCm | SEQ ID NO: 641 |
| RX002 006 | CmsAmsGmAmGmCmGfGfGfUmAm CmCmUmCmUmUmCmAm | SEQ ID NO: 430 | UmsGfsAmAmGmAfGmGmUmAmCm CmCmGfCmUfCmUmGmsCmsAm | SEQ ID NO: 642 |
| RX002 007 | GmsCmsGmGmGmUmAfCfCfUmCm UmUmCmAmUmCmCmAm | SEQ ID NO: 431 | UmsGfsGmAmUmGfAmAmGmAmGm GmUmAfCmCfCmGmCmsUmsCm | SEQ ID NO: 643 |

(continued)

| siRNA No. | sense strand (5'-3') | | antisense strand (5'-3') | |
|---|---|---|---|---|
| RX002 008 | CmsAmsGmAmCmAmGfAfCfAmAm GmAmCmCmAmUmCmUm | SEQ ID NO: 432 | AmsGfsAmUmGmGfUmCmUmUmGm UmCmUfGmUfCmUmGmsGmsAm | SEQ ID NO: 644 |
| RX002 009 | GmsGmsAmUmCmUmUfCfAfCmCm GmUmCmAmAmCmCmAm | SEQ ID NO: 433 | UmsGfsGmUmUmGfAmCmGmGmUm GmAmAfGmAfUmCmCmsGmsAm | SEQ ID NO: 645 |
| RX002 010 | CmsCmsGmUmCmAmAfCfCfAmCm AmAmGmCmUmGmCmUm | SEQ ID NO: 434 | AmsGfsCmAmGmCfUmUmGmUmGm GmUmUfGmAfCmGmGmsUmsGm | SEQ ID NO: 646 |
| RX002 011 | CmsGmsUmCmAmAmCfCfAfCmAm AmGmCmUmGmCmUmAm | SEQ ID NO: 435 | UmsAfsGmCmAmGfCmUmUmGmUm GmGmUfUmGfAmCmGmsGmsUm | SEQ ID NO: 647 |
| RX002 012 | CmsGmsGmUmCmAmAfGfCfAmGm GmAmCmUmCmCmUmUm | SEQ ID NO: 436 | AmsAfsGmGmAmGfUmCmCmUmGm CmUmUfGmAfCmCmGmsGmsGm | SEQ ID NO: 648 |
| RX002 013 | GmsUmsCmAmAmGmCfAfGfGmAm CmUmCmCmUmUmGmUm | SEQ ID NO: 437 | AmsCfsAmAmGmGfAmGmUmCmCm UmGmCfUmUfGmAmCmsCmsGm | SEQ ID NO: 649 |
| RX002 014 | CmsAmsAmGmCmAmGfGfAfCmUm CmCmUmUmGmUmCmUm | SEQ ID NO: 438 | AmsGfsAmCmAmAfGmGmAmGmUm CmCmUfGmCfUmUmGmsAmsCm | SEQ ID NO: 650 |
| RX002 015 | CmsUmsUmGmGmGmAfCfAfUmUm CmCmGmGmAmAmCmUm | SEQ ID NO: 439 | AmsGfsUmUmCmCfGmGmAmAmUm GmUmCfCmCfAmAmGmsAmsCm | SEQ ID NO: 651 |
| RX002 016 | GmsGmsGmAmCmAmUfUfCfCmGm GmAmAmCmUmCmGmUm | SEQ ID NO: 440 | AmsCfsGmAmGmUfUmCmCmGmGm AmAmUfGmUfCmCmCmsAmsAm | SEQ ID NO: 652 |
| RX002 017 | CmsAmsGmUmGmGmAfAfGfAmUm CmCmGmAmGmCmCmUm | SEQ ID NO: 441 | AmsGfsGmCmUmCfGmGmAmUmCm UmUmCfCmAfCmUmGmsGmsCm | SEQ ID NO: 653 |
| RX002 018 | AmsGmsUmGmGmAmAfGfAfUmCm CmGmAmGmCmCmUmAm | SEQ ID NO: 442 | UmsAfsGmGmCmUfCmGmGmAmUm CmUmUfCmCfAmCmUmsGmsGm | SEQ ID NO: 654 |
| RX002 019 | GmsGmsAmAmGmAmUfCfCfGmAm GmCmCmUmAmCmUmAm | SEQ ID NO: 443 | UmsAfsGmUmAmGfGmCmUmCmGm GmAmUfCmUfUmCmCmsAmsCm | SEQ ID NO: 655 |
| RX002 020 | GmsAmsAmGmAmUmCfCfGfAmGm CmCmUmAmCmUmAmUm | SEQ ID NO: 444 | AmsUfsAmGmUmAfGmGmCmUmCm GmGmAfUmCfUmUmCmsCmsAm | SEQ ID NO: 656 |
| RX002 021 | GmsAmsUmCmCmGmAfGfCfCmUm AmCmUmAmUmGmAmAm | SEQ ID NO: 445 | UmsUfsCmAmUmAfGmUmAmGmGm CmUmCfGmGfAmUmCmsUmsUm | SEQ ID NO: 657 |
| RX002 022 | AmsGmsUmUmUmGmAfGfGfUmGm AmAmGmGmAmGmUmAm | SEQ ID NO: 446 | UmsAfsCmUmCmCfUmUmCmAmCm CmUmCfAmAfAmCmUmsCmsAm | SEQ ID NO: 658 |
| RX002 023 | GmsCmsCmCmAmGmUfUfUfCmGm AmGmGmUmCmAmUmAm | SEQ ID NO: 447 | UmsAfsUmGmAmCfCmUmCmGmAm AmAmCfUmGfGmGmCmsAmsGm | SEQ ID NO: 659 |

(continued)

| siRNA No. | sense strand (5'-3') | | antisense strand (5'-3') | |
|---|---|---|---|---|
| RX002 024 | CmsCmsAmGmUmUmUfCfGfAmGm GmUmCmAmUmAmGmUm | SEQ ID NO: 448 | AmsCfsUmAmUmGfAmCmCmUmCm GmAmAfAmCfUmGmGmsGmsCm | SEQ ID NO: 660 |
| RX002 025 | CmsAmsGmUmUmUmCfGfAfGmGm UmCmAmUmAmGmUmGm | SEQ ID NO: 449 | CmsAfsCmUmAmUfGmAmCmCmUm CmGmAfAmAfCmUmGmsGmsGm | SEQ ID NO: 661 |
| RX002 026 | GmsUmsUmUmCmGmAfGfGfUmCm AmUmAmGmUmGmGmAm | SEQ ID NO: 450 | UmsCfsCmAmCmUfAmUmGmAmCm CmUmCfGmAfAmAmCmsUmsGm | SEQ ID NO: 662 |
| RX002 027 | CmsGmsAmGmGmUmCfAfUfAmGm UmGmGmAmGmCmCmUm | SEQ ID NO: 451 | AmsGfsGmCmUmCfCmAmCmUmAm UmGmAfCmCfUmCmGmsAmsAm | SEQ ID NO: 663 |
| RX002 028 | AmsGmsUmGmGmAmGfCfCfUmAm CmAmGmAmGmAmAmAm | SEQ ID NO: 452 | UmsUfsUmCmUmCfUmGmUmAmGm GmCmUfCmCfAmCmUmsAmsUm | SEQ ID NO: 664 |
| RX002 029 | CmsAmsGmAmGmAmAfAfUfUmCm UmAmCmUmAmCmAmUm | SEQ ID NO: 453 | AmsUfsGmUmAmGfUmAmGmAmAm UmUmUfCmUfCmUmGmsUmsAm | SEQ ID NO: 665 |
| RX002 030 | AmsAmsAmUmUmCmUfAfCfUmAm CmAmUmCmUmAmUmAm | SEQ ID NO: 454 | UmsAfsUmAmGmAfUmGmUmAmGm UmAmGfAmAfUmUmUmsCmsUm | SEQ ID NO: 666 |
| RX002 031 | GmsGmsAmAmCmUmGfCfCfUmUm UmGmUmCmAmUmCmUm | SEQ ID NO: 455 | AmsGfsAmUmGmAfCmAmAmAmGm GmCmAfGmUfUmCmCmsCmsUm | SEQ ID NO: 667 |
| RX002 032 | GmsAmsAmCmUmGmCfCfUfUmUm GmUmCmAmUmCmUmUm | SEQ ID NO: 456 | AmsAfsGmAmUmGfAmCmAmAmAm GmGmCfAmGfUmUmCmsCmsCm | SEQ ID NO: 668 |
| RX002 033 | CmsCmsUmUmUmGmUfCfAfUmCm UmUmCmGmGmGmAmUm | SEQ ID NO: 457 | AmsUfsCmCmCmGfAmAmGmAmUm GmAmCfAmAfAmGmGmsCmsAm | SEQ ID NO: 669 |
| RX002 034 | CmsUmsGmAmAmUmCfCfCfUmCm AmAmGmCmGmCmAmUm | SEQ ID NO: 458 | AmsUfsGmCmGmCfUmUmGmAmGm GmGmAfUmUfCmAmGmsGmsCm | SEQ ID NO: 670 |
| RX002 035 | CmsAmsGmGmCmAmGfUfGfAmCm AmUmGmGmUmGmCmAm | SEQ ID NO: 459 | UmsGfsCmAmCmCfAmUmGmUmCm AmCmUfGmCfCmUmGmsAmsGm | SEQ ID NO: 671 |
| RX002 036 | GmsUmsGmAmCmCmUfCfUfCmCm CmUmAmCmCmAmGmAm | SEQ ID NO: 460 | UmsCfsUmGmGmUfAmGmGmGmAm GmAmGfGmUfCmAmCmsGmsAm | SEQ ID NO: 672 |
| RX002 037 | GmsAmsAmUmGmCmCfCfUfUmUm GmAmCmCmUmCmAmUm | SEQ ID NO: 461 | AmsUfsGmAmGmGfUmCmAmAmAm GmGmGfCmAfUmUmCmsCmsUm | SEQ ID NO: 673 |
| RX002 038 | CmsCmsUmUmUmGmAfCfCfUmCm AmUmGmGmUmGmUmUm | SEQ ID NO: 462 | AmsAfsCmAmCmCfAmUmGmAmGm GmUmCfAmAfAmGmGmsGmsCm | SEQ ID NO: 674 |
| RX002 039 | GmsAmsCmCmUmCmAfUfGfGmUm GmUmUmCmGmUmGmAm | SEQ ID NO: 463 | UmsCfsAmCmGmAfAmCmAmCmCm AmUmGfAmGfGmUmCmsAmsAm | SEQ ID NO: 675 |

(continued)

| siRNA No. | sense strand (5'-3') | | antisense strand (5'-3') | |
|---|---|---|---|---|
| RX002 040 | CmsUmsCmAmUmGmGfUfGfUmUmCmGmUmGmAmCmGmAm | SEQ ID NO: 464 | UmsCfsGmUmCmAfCmGmAmAmCmAmCmCfAmUfGmAmGmsGmsUm | SEQ ID NO: 676 |
| RX002 041 | UmsCmsAmUmGmGmUfGfUfUmCmGmUmGmAmCmGmAmAm | SEQ ID NO: 465 | UmsUfsCmGmUmCfAmCmGmAmAmCmAmCfCmAfUmGmAmsGmsGm | SEQ ID NO: 677 |
| RX002 042 | CmsUmsGmUmGmCmAfGfUfCmUmCmUmAmAmCmCmCmAm | SEQ ID NO: 466 | UmsGfsGmGmUmUfAmGmAmGmAmCmUmGfCmAfCmAmGmsUmsGm | SEQ ID NO: 678 |
| RX002 043 | GmsCmsCmAmGmAmAfGfCfCmCmUmUmGmAmGmCmAmUm | SEQ ID NO: 467 | AmsUfsGmCmUmCfAmAmGmGmGmCmUmUfCmUfGmGmCmsUmsGm | SEQ ID NO: 679 |
| RX002 044 | CmsAmsGmAmAmGmCfCfCfUmUmGmAmGmCmAmUmCmAm | SEQ ID NO: 468 | UmsGfsAmUmGmCfUmCmAmAmGmGmGmCfUmUfCmUmGmsGmsCm | SEQ ID NO: 680 |
| RX002 045 | GmsCmsAmUmCmAmCfGfGfUmGmCmGmCmAmCmGmAmAm | SEQ ID NO: 469 | UmsUfsCmGmUmGfCmGmCmAmCmCmGmUfGmAfUmGmCmsUmsCm | SEQ ID NO: 681 |
| RX002 046 | CmsGmsGmUmGmCmGfCfAfCmGmAmAmGmAmAmGmCmAm | SEQ ID NO: 470 | UmsGfsCmUmUmCfUmUmCmGmUmGmCmGfCmAfCmCmGmsUmsGm | SEQ ID NO: 682 |
| RX002 047 | CmsCmsGmUmGmGmGfCfAfAmCmUmCmCmAmAmCmAmAm | SEQ ID NO: 471 | UmsUfsGmUmUmGfGmAmGmUmUmGmCmCfCmAfCmGmGmsUmsGm | SEQ ID NO: 683 |
| RX002 048 | CmsGmsUmGmGmGmCfAfAfCmUmCmCmAmAmCmAmAmUm | SEQ ID NO: 472 | AmsUfsUmGmUmUfGmGmAmGmUmUmGmCfCmCfAmCmGmsGmsUm | SEQ ID NO: 684 |
| RX002 049 | GmsUmsGmGmGmCmAfAfCfUmCmCmAmAmCmAmAmUmUm | SEQ ID NO: 473 | AmsAfsUmUmGmUfUmGmGmAmGmUmUmGfCmCfCmAmCmsGmsGm | SEQ ID NO: 685 |
| RX002 050 | GmsCmsAmAmCmUmCfCfAfAmCmAmAmUmUmAmCmCmUm | SEQ ID NO: 474 | AmsGfsGmUmAmAfUmUmGmUmUmGmGmAfGmUfUmGmCmsCmsCm | SEQ ID NO: 686 |
| RX002 051 | CmsUmsCmCmAmAmCfAfAfUmUmAmCmCmUmGmCmAmUm | SEQ ID NO: 475 | AmsUfsGmCmAmGfGmUmAmAmUmUmGmUfUmGfGmAmGmsUmsUm | SEQ ID NO: 687 |
| RX002 052 | GmsGmsGmGmAmGmAfCfCfCmUmCmAmAmCmGmUmCmAm | SEQ ID NO: 476 | UmsGfsAmCmGmUfUmGmAmGmGmGmUmCfUmCfCmCmCmsGmsGm | SEQ ID NO: 688 |
| RX002 053 | GmsGmsGmAmGmAmCfCfCfUmCmAmAmCmGmUmCmAmAm | SEQ ID NO: 477 | UmsUfsGmAmCmGfUmUmGmAmGmGmGmUfCmUfCmCmCmsCmsGm | SEQ ID NO: 689 |
| RX002 054 | GmsAmsGmAmCmCmCfUfCfAmAmCmGmUmCmAmAmCmUm | SEQ ID NO: 478 | AmsGfsUmUmGmAfCmGmUmUmGmAmGmGfGmUfCmUmCmsCmsCm | SEQ ID NO: 690 |
| RX002 055 | CmsCmsCmUmCmAmAfCfGfUmCmAmAmCmUmUmCmCmUm | SEQ ID NO: 479 | AmsGfsGmAmAmGfUmUmGmAmCmGmUmUfGmAfGmGmGmsUmsCm | SEQ ID NO: 691 |

(continued)

| siRNA No. | sense strand (5'-3') | | antisense strand (5'-3') | |
|---|---|---|---|---|
| RX002 056 | UmsCmsAmAmCmUmUfCfCfUmCm CmUmGmCmGmAmAmUm | SEQ ID NO: 480 | AmsUfsUmCmGmCfAmGmGmAmGm GmAmAfGmUfUmGmAmsCmsGm | SEQ ID NO: 692 |
| RX002 057 | GmsAmsGmGmCmCmAfAfGfAmUm CmCmGmCmUmAmCmUm | SEQ ID NO: 481 | AmsGfsUmAmGmCfGmGmAmUmCm UmUmGfGmCfCmUmCmsGmsUm | SEQ ID NO: 693 |
| RX002 058 | AmsGmsAmUmCmCmGfCfUfAmCm UmAmCmAmCmCmUmAm | SEQ ID NO: 482 | UmsAfsGmGmUmGfUmAmGmUmAm GmCmGfGmAfUmCmUmsUmsGm | SEQ ID NO: 694 |
| RX002 059 | CmsGmsCmUmAmCmUfAfCfAmCm CmUmAmCmCmUmGmAm | SEQ ID NO: 483 | UmsCfsAmGmGmUfAmGmGmUmGm UmAmGfUmAfGmCmGmsGmsAm | SEQ ID NO: 695 |
| RX002 060 | GmsCmsUmAmCmUmAfCfAfCmCm UmAmCmCmUmGmAmUm | SEQ ID NO: 484 | AmsUfsCmAmGmGfUmAmGmGmUm GmUmAfGmUfAmGmCmsGmsGm | SEQ ID NO: 696 |
| RX002 061 | CmsCmsGmUmGmUmGfGfGfUmGm GmAmCmGmUmCmAmAm | SEQ ID NO: 485 | UmsUfsGmAmCmGfUmCmCmAmCm CmCmAfCmAfCmGmGmsAmsGm | SEQ ID NO: 697 |
| RX002 062 | GmsGmsGmCmUmCmGfCfUfGmGm UmGmGmUmAmAmAmAm | SEQ ID NO: 486 | UmsUfsUmUmAmCfCmAmCmCmAm GmCmGfAmGfCmCmCmsAmsCm | SEQ ID NO: 698 |
| RX002 063 | CmsCmsCmUmGmAmAfGfAfUmAm GmAmGmGmGmUmGmAm | SEQ ID NO: 487 | UmsCfsAmCmCmCfUmCmUmAmUm CmUmUfCmAfGmGmGmsUmsCm | SEQ ID NO: 699 |
| RX002 064 | CmsGmsGmGmAmGmAfAfCfCmCm CmAmUmGmAmGmGmUm | SEQ ID NO: 488 | AmsCfsCmUmCmAfUmGmGmGmGm UmUmCfUmCfCmCmGmsCmsAm | SEQ ID NO: 700 |
| RX002 065 | GmsGmsGmAmGmAmAfCfCfCmCm AmUmGmAmGmGmUmUm | SEQ ID NO: 489 | AmsAfsCmCmUmCfAmUmGmGmGm GmUmUfCmUfCmCmCmsGmsCm | SEQ ID NO: 701 |
| RX002 066 | GmsAmsGmAmAmCmCfCfCfAmUm GmAmGmGmUmUmCmUm | SEQ ID NO: 490 | AmsGfsAmAmCmCfUmCmAmUmGm GmGmGfUmUfCmUmCmsCmsCm | SEQ ID NO: 702 |
| RX002 067 | CmsUmsGmCmUmGmCfAfAfCmUm AmCmAmUmCmAmCmAm | SEQ ID NO: 491 | UmsGfsUmGmAmUfGmUmAmGmUm UmGmCfAmGfCmAmGmsUmsCm | SEQ ID NO: 703 |
| RX002 068 | GmsGmsAmGmUmAmAfCfCfUmGm GmAmUmGmAmGmGmAm | SEQ ID NO: 492 | UmsCfsCmUmCmAfUmCmCmAmGm GmUmUfAmCfUmCmCmsUmsGm | SEQ ID NO: 704 |
| RX002 069 | GmsUmsAmAmCmCmUfGfGfAmUm GmAmGmGmAmCmAmUm | SEQ ID NO: 493 | AmsUfsGmUmCmCfUmCmAmUmCm CmAmGfGmUfUmAmCmsUmsCm | SEQ ID NO: 705 |
| RX002 070 | GmsCmsAmGmAmAmGfAfGfAmAm CmAmUmCmGmUmUmUm | SEQ ID NO: 494 | AmsAfsAmCmGmAfUmGmUmUmCm UmCmUfUmCfUmGmCmsAmsAm | SEQ ID NO: 706 |
| RX002 071 | GmsAmsGmAmAmCmAfUfCfGmUm UmUmCmCmCmGmAmAm | SEQ ID NO: 495 | UmsUfsCmGmGmGfAmAmAmCmGm AmUmGfUmUfCmUmCmsUmsUm | SEQ ID NO: 707 |

(continued)

| siRNA No. | sense strand (5'-3') | | antisense strand (5'-3') | |
|---|---|---|---|---|
| RX002 072 | CmsCmsCmGmAmAmGfUfGfAmGm UmUmCmCmCmAmGmAm | SEQ ID NO: 496 | UmsCfsUmGmGmGfAmAmCmUmCm AmCmUfUmCfGmGmGmsAmsAm | SEQ ID NO: 708 |
| RX002 073 | CmsGmsAmAmGmCmUfCfAfUmGm AmAmUmAmUmAmUmUm | SEQ ID NO: 497 | AmsAfsUmAmUmAfUmUmCmAmUm GmAmGfCmUfUmCmGmsUmsAm | SEQ ID NO: 709 |
| RX002 074 | GmsAmsAmGmCmUmCfAfUfGmAm AmUmAmUmAmUmUmUm | SEQ ID NO: 498 | AmsAfsAmUmAmUfAmUmUmCmAm UmGmAfGmCfUmUmCmsGmsUm | SEQ ID NO: 710 |
| RX002 075 | CmsCmsCmUmUmCmGfAfGfGmUm CmAmCmAmGmUmAmAm | SEQ ID NO: 499 | UmsUfsAmCmUmGfUmGmAmCmCm UmCmGfAmAfGmGmGmsGmsUm | SEQ ID NO: 711 |
| RX002 076 | CmsCmsUmUmCmGmAfGfGfUmCm AmCmAmGmUmAmAmUm | SEQ ID NO: 500 | AmsUfsUmAmCmUfGmUmGmAmCm CmUmCfGmAfAmGmGmsGmsGm | SEQ ID NO: 712 |
| RX002 077 | UmsCmsAmCmAmGmUfAfAfUmGm CmAmGmGmAmCmUmUm | SEQ ID NO: 501 | AmsAfsGmUmCmCfUmGmCmAmUm UmAmCfUmGfUmGmAmsCmsCm | SEQ ID NO: 713 |
| RX002 078 | CmsCmsUmGmCmGmGfCfUfAmCm CmCmUmAmCmUmCmUm | SEQ ID NO: 502 | AmsGfsAmGmUmAfGmGmGmUmAm GmCmCfGmCfAmGmGmsUmsCm | SEQ ID NO: 714 |
| RX002 079 | GmsCmsGmGmCmUmAfCfCfCmUm AmCmUmCmUmGmUmUm | SEQ ID NO: 503 | AmsAfsCmAmGmAfGmUmAmGmGm GmUmAfGmCfCmGmCmsAmsGm | SEQ ID NO: 715 |
| RX002 080 | GmsGmsCmUmAmCmCfCfUfAmCm UmCmUmGmUmUmGmUm | SEQ ID NO: 504 | AmsCfsAmAmCmAfGmAmGmUmAm GmGmGfUmAfGmCmCmsGmsCm | SEQ ID NO: 716 |
| RX002 081 | GmsCmsUmAmCmCmCfUfAfCmUm CmUmGmUmUmGmUmUm | SEQ ID NO: 505 | AmsAfsCmAmAmCfAmGmAmGmUm AmGmGfGmUfAmGmCmsCmsGm | SEQ ID NO: 717 |
| RX002 082 | AmsCmsCmCmUmAmCfUfCfUmGm UmUmGmUmUmCmGmAm | SEQ ID NO: 506 | UmsCfsGmAmAmCfAmAmCmAmGm AmGmUfAmGfGmGmUmsAmsGm | SEQ ID NO: 718 |
| RX002 083 | CmsCmsCmUmAmCmUfCfUfGmUm UmGmUmUmCmGmAmAm | SEQ ID NO: 507 | UmsUfsCmGmAmAfCmAmAmCmAm GmAmGfUmAfGmGmGmsUmsAm | SEQ ID NO: 719 |
| RX002 084 | CmsCmsUmAmCmUmCfUfGfUmUm GmUmUmCmGmAmAmAm | SEQ ID NO: 508 | UmsUfsUmCmGmAfAmCmAmAmCm AmGmAfGmUfAmGmGmsGmsUm | SEQ ID NO: 720 |
| RX002 085 | CmsGmsAmAmAmCmGfAfGfCmAm GmGmUmGmGmAmAmAm | SEQ ID NO: 509 | UmsUfsUmCmCmAfCmCmUmGmCm UmCmGfUmUfUmCmGmsAmsAm | SEQ ID NO: 721 |
| RX002 086 | GmsAmsAmAmCmGmAfGfCfAmGm GmUmGmGmAmAmAmUm | SEQ ID NO: 510 | AmsUfsUmUmCmCfAmCmCmUmGm CmUmCfGmUfUmUmCmsGmsAm | SEQ ID NO: 722 |
| RX002 087 | CmsGmsAmGmCmAmGfGfUfGmGm AmAmAmUmCmCmGmAm | SEQ ID NO: 511 | UmsCfsGmGmAmUfUmUmCmCmAm CmCmUfGmCfUmCmGmsUmsUm | SEQ ID NO: 723 |

(continued)

| siRNA No. | sense strand (5'-3') | | antisense strand (5'-3') | |
|---|---|---|---|---|
| RX002 088 | CmsAmsAmUmUmAmCfCfGfGmCm AmGmAmAmCmCmAmAm | SEQ ID NO: 512 | UmsUfsGmGmUmUfCmUmGmCmCm GmGmUfAmAfUmUmGmsUmsAm | SEQ ID NO: 724 |
| RX002 089 | GmsGmsGmUmGmGmAfAfCfUmAm CmUmCmCmAmCmAmAm | SEQ ID NO: 513 | UmsUfsGmUmGmGfAmGmUmAmGm UmUmCfCmAfCmCmCmsUmsCm | SEQ ID NO: 725 |
| RX002 090 | GmsGmsUmGmGmAmAfCfUfAmCm UmCmCmAmCmAmAmUm | SEQ ID NO: 514 | AmsUfsUmGmUmGfGmAmGmUmAm GmUmUfCmCfAmCmCmsCmsUm | SEQ ID NO: 726 |
| RX002 091 | GmsGmsAmAmCmUmAfCfUfCmCm AmCmAmAmUmCmCmAm | SEQ ID NO: 515 | UmsGfsGmAmUmUfGmUmGmGmAm GmUmAfGmUfUmCmCmsAmsCm | SEQ ID NO: 727 |
| RX002 092 | CmsGmsUmCmAmCmCfAfGfCmAm GmAmCmCmGmUmAmAm | SEQ ID NO: 516 | UmsUfsAmCmGmGfUmCmUmGmCm UmGmGfUmGfAmCmGmsCmsCm | SEQ ID NO: 728 |
| RX002 093 | CmsAmsCmCmAmGmCfAfGfAmCm CmGmUmAmAmCmCmAm | SEQ ID NO: 517 | UmsGfsGmUmUmAfCmGmGmUmCm UmGmCfUmGfGmUmGmsAmsCm | SEQ ID NO: 729 |
| RX002 094 | GmsGmsAmAmGmUmCfCfCfUmGm AmAmGmGmUmCmGmUm | SEQ ID NO: 518 | AmsCfsGmAmCmCfUmUmCmAmGm GmGmAfCmUfUmCmCmsUmsGm | SEQ ID NO: 730 |
| RX002 095 | GmsUmsCmGmUmGmCfCfGfGmAm AmGmGmAmAmUmCmAm | SEQ ID NO: 519 | UmsGfsAmUmUmCfCmUmUmCmCm GmGmCfAmCfGmAmCmsCmsUm | SEQ ID NO: 731 |
| RX002 096 | CmsGmsUmGmCmCmGfGfAfAmGm GmAmAmUmCmAmGmAm | SEQ ID NO: 520 | UmsCfsUmGmAmUfUmCmCmUmUm CmCmGfGmCfAmCmGmsAmsCm | SEQ ID NO: 732 |
| RX002 097 | GmsUmsGmCmCmGmGfAfAfGmGm AmAmUmCmAmGmAmAm | SEQ ID NO: 521 | UmsUfsCmUmGmAfUmUmCmCmUm UmCmCfGmGfCmAmCmsGmsAm | SEQ ID NO: 733 |
| RX002 098 | CmsCmsGmGmAmAmGfGfAfAmUm CmAmGmAmAmUmGmAm | SEQ ID NO: 522 | UmsCfsAmUmUmCfUmGmAmUmUm CmCmUfUmCfCmGmGmsCmsAm | SEQ ID NO: 734 |
| RX002 099 | CmsGmsGmAmAmGmGfAfAfUmCm AmGmAmAmUmGmAmAm | SEQ ID NO: 523 | UmsUfsCmAmUmUfCmUmGmAmUm UmCmCfUmUfCmCmGmsGmsCm | SEQ ID NO: 735 |
| RX002 100 | UmsGmsCmAmGmAmCfCfUfCmAm GmUmGmAmCmCmAmAm | SEQ ID NO: 524 | UmsUfsGmGmUmCfAmCmUmGmAm GmGmUfCmUfGmCmAmsGmsGm | SEQ ID NO: 736 |
| RX002 101 | GmsAmsCmCmAmGmAfAfUfUmCm UmCmCmUmGmCmAmAm | SEQ ID NO: 525 | UmsUfsGmCmAmGfGmAmGmAmAm UmUmCfUmGfGmUmCmsUmsCm | SEQ ID NO: 737 |
| RX002 102 | GmsAmsAmCmGmGmCfUfGfAmAm GmCmAmCmCmUmCmAm | SEQ ID NO: 526 | UmsGfsAmGmGmUfGmCmUmUmCm AmGmCfCmGfUmUmCmsCmsGm | SEQ ID NO: 738 |
| RX002 103 | CmsGmsCmUmGmUmGfCfAfUmUm AmCmCmUmGmGmAmUm | SEQ ID NO: 527 | AmsUfsCmCmAmGfGmUmAmAmUm GmCmAfCmAfGmCmGmsAmsUm | SEQ ID NO: 739 |

(continued)

| siRNA No. | sense strand (5'-3') | | antisense strand (5'-3') | |
|---|---|---|---|---|
| RX002 104 | GmsCmsAmUmUmAmCfCfUfGmGm AmUmGmAmAmAmCmGm | SEQ ID NO: 528 | CmsGfsUmUmUmCfAmUmCmCmAm GmGmUfAmAfUmGmCmsAmsCm | SEQ ID NO: 740 |
| RX002 105 | CmsCmsUmUmGmGmAfGfCfUmCm AmUmCmAmAmGmAmAm | SEQ ID NO: 529 | UmsUfsCmUmUmGfAmUmGmAmGm CmUmCfCmAfAmGmGmsCmsCm | SEQ ID NO: 741 |
| RX002 106 | GmsCmsAmGmCmUmGfGfCfCmUm UmCmAmGmAmCmAmAm | SEQ ID NO: 530 | UmsUfsGmUmCmUfGmAmAmGmGm CmCmAfGmCfUmGmCmsUmsGm | SEQ ID NO: 742 |
| RX002 107 | CmsCmsGmCmCmUmAfCfGfUmGm GmUmCmAmAmGmGmUm | SEQ ID NO: 531 | AmsCfsCmUmUmGfAmCmCmAmCm GmUmAfGmGfCmGmGmsUmsCm | SEQ ID NO: 743 |
| RX002 108 | CmsCmsUmAmCmGmUfGfGfUmCm AmAmGmGmUmCmUmUm | SEQ ID NO: 532 | AmsAfsGmAmCmCfUmUmGmAmCm CmAmCfGmUfAmGmGmsCmsGm | SEQ ID NO: 744 |
| RX002 109 | CmsGmsUmGmGmUmCfAfAfGmGm UmCmUmUmCmUmCmUm | SEQ ID NO: 533 | AmsGfsAmGmAmAfGmAmCmCmUm UmGmAfCmCfAmCmGmsUmsAm | SEQ ID NO: 745 |
| RX002 110 | CmsUmsCmUmGmGmCfUfGfUmCm AmAmCmCmUmCmAmUm | SEQ ID NO: 534 | AmsUfsGmAmGmGfUmUmGmAmCm AmGmCfCmAfGmAmGmsAmsGm | SEQ ID NO: 746 |
| RX002 111 | CmsUmsCmUmGmCmGfGfGfGmCm UmGmUmUmAmAmAmUm | SEQ ID NO: 535 | AmsUfsUmUmAmAfCmAmGmCmCm CmCmGfCmAfGmAmGmsGmsAm | SEQ ID NO: 747 |
| RX002 112 | CmsUmsGmUmUmAmAfAfUfGmGm CmUmGmAmUmCmCmUm | SEQ ID NO: 536 | AmsGfsGmAmUmCfAmGmCmCmAm UmUmUfAmAfCmAmGmsCmsCm | SEQ ID NO: 748 |
| RX002 113 | GmsGmsAmGmGmAmUfGfCfGmCm CmCmGmUmGmAmUmAm | SEQ ID NO: 537 | UmsAfsUmCmAmCfGmGmGmCmGm CmAmUfCmCfUmCmCmsUmsGm | SEQ ID NO: 749 |
| RX002 114 | GmsGmsCmCmCmUmCfAfCfGmGm CmCmUmUmUmGmUmUm | SEQ ID NO: 538 | AmsAfsCmAmAmAfGmGmCmCmGm UmGmAfGmGfGmCmCmsAmsUm | SEQ ID NO: 750 |
| RX002 115 | CmsUmsCmAmCmGmGfCfCfUmUm UmGmUmUmCmUmCmAm | SEQ ID NO: 539 | UmsGfsAmGmAmAfCmAmAmAmGm GmCmCfGmUfGmAmGmsGmsGm | SEQ ID NO: 751 |
| RX002 116 | CmsCmsUmUmUmGmUfUfCfUmCm AmUmCmUmCmGmCmUm | SEQ ID NO: 540 | AmsGfsCmGmAmGfAmUmGmAmGm AmAmCfAmAfAmGmGmsCmsCm | SEQ ID NO: 752 |
| RX002 117 | CmsUmsUmGmAmAmGfCfCfAmAm CmUmAmCmAmUmGmAm | SEQ ID NO: 541 | UmsCfsAmUmGmUfAmGmUmUmGm GmCmUfUmCfAmAmGmsGmsAm | SEQ ID NO: 753 |
| RX002 118 | CmsCmsAmAmCmUmAfCfAfUmGm AmAmCmCmUmAmCmAm | SEQ ID NO: 542 | UmsGfsUmAmGmGfUmUmCmAmUm GmUmAfGmUfUmGmGmsCmsUm | SEQ ID NO: 754 |
| RX002 119 | CmsCmsUmAmCmAmGfAfGfAmUm CmCmUmAmCmAmCmUm | SEQ ID NO: 543 | AmsGfsUmGmUmAfGmGmAmUmCm UmCmUfGmUfAmGmGmsUmsUm | SEQ ID NO: 755 |

(continued)

| siRNA No. | sense strand (5'-3') | | antisense strand (5'-3') | |
|---|---|---|---|---|
| RX002 120 | CmsUmsGmAmCmCmAfCfAfGmCm CmAmAmAmGmAmUmAm | SEQ ID NO: 544 | UmsAfsUmCmUmUfUmGmGmCmUm GmUmGfGmUfCmAmGmsAmsAm | SEQ ID NO: 756 |
| RX002 121 | CmsCmsAmCmAmGmCfCfAfAmAm GmAmUmAmAmGmAmAm | SEQ ID NO: 545 | UmsUfsCmUmUmAfUmCmUmUmUm GmGmCfUmGfUmGmGmsUmsCm | SEQ ID NO: 757 |
| RX002 122 | GmsCmsCmAmAmAmGfAfUfAmAm GmAmAmCmCmGmCmUm | SEQ ID NO: 546 | AmsGfsCmGmGmUfUmCmUmUmAm UmCmUfUmUfGmGmCmsUmsGm | SEQ ID NO: 758 |
| RX002 123 | GmsCmsCmAmCmAmUfCfCfUmAm UmGmCmCmCmUmCmUm | SEQ ID NO: 547 | AmsGfsAmGmGmGfCmAmUmAmGm GmAmUfGmUfGmGmCmsCmsUm | SEQ ID NO: 759 |
| RX002 124 | CmsCmsAmCmAmUmCfCfUfAmUm GmCmCmCmUmCmUmUm | SEQ ID NO: 548 | AmsAfsGmAmGmGfGmCmAmUmAm GmGmAfUmGfUmGmGmsCmsCm | SEQ ID NO: 760 |
| RX002 125 | GmsGmsCmCmCmUmAfCfUfGmCm AmGmCmUmAmAmAmAm | SEQ ID NO: 549 | UmsUfsUmUmAmGfCmUmGmCmAm GmUmAfGmGfGmCmCmsAmsAm | SEQ ID NO: 761 |
| RX002 126 | CmsUmsAmCmUmGmCfAfGfCmUm AmAmAmAmGmAmCmUm | SEQ ID NO: 550 | AmsGfsUmCmUmUfUmUmAmGmCm UmGmCfAmGfUmAmGmsGmsGm | SEQ ID NO: 762 |
| RX002 127 | CmsAmsGmCmUmAmAfAfAfGmAm CmUmUmUmGmAmCmUm | SEQ ID NO: 551 | AmsGfsUmCmAmAfAmGmUmCmUm UmUmUfAmGfCmUmGmsCmsAm | SEQ ID NO: 763 |
| RX002 128 | GmsCmsUmAmAmAmAfGfAfCmUm UmUmGmAmCmUmUmUm | SEQ ID NO: 552 | AmsAfsAmGmUmCfAmAmAmGmUm CmUmUfUmUfAmGmCmsUmsGm | SEQ ID NO: 764 |
| RX002 129 | CmsCmsGmUmCmGmUfGfCfGmUm UmGmGmCmUmCmAmAm | SEQ ID NO: 553 | UmsUfsGmAmGmCfCmAmAmCmGm CmAmCfGmAfCmGmGmsGmsAm | SEQ ID NO: 765 |
| RX002 130 | CmsGmsUmCmGmUmGfCfGfUmUm GmGmCmUmCmAmAmUm | SEQ ID NO: 554 | AmsUfsUmGmAmGfCmCmAmAmCm GmCmAfCmGfAmCmGmsGmsGm | SEQ ID NO: 766 |
| RX002 131 | CmsGmsUmGmCmGmUfUfGfGmCm UmCmAmAmUmGmAmAm | SEQ ID NO: 555 | UmsUfsCmAmUmUfGmAmGmCmCm AmAmCfGmCfAmCmGmsAmsCm | SEQ ID NO: 767 |
| RX002 132 | CmsGmsUmUmGmGmCfUfCfAmAm UmGmAmAmCmAmGmAm | SEQ ID NO: 556 | UmsCfsUmGmUmUfCmAmUmUmGm AmGmCfCmAfAmCmGmsCmsAm | SEQ ID NO: 768 |
| RX002 133 | GmsGmsCmUmCmAmAfUfGfAmAm CmAmGmAmGmAmUmAm | SEQ ID NO: 557 | UmsAfsUmCmUmCfUmGmUmUmCm AmUmUfGmAfGmCmCmsAmsAm | SEQ ID NO: 769 |
| RX002 134 | CmsUmsCmAmAmUmGfAfAfCmAm GmAmGmAmUmAmCmUm | SEQ ID NO: 558 | AmsGfsUmAmUmCfUmCmUmGmUm UmCmAfUmUfGmAmGmsCmsCm | SEQ ID NO: 770 |
| RX002 135 | CmsCmsAmCmCmUmUfCfAfUmGm GmUmGmUmUmCmCmAm | SEQ ID NO: 559 | UmsGfsGmAmAmCfAmCmCmAmUm GmAmAfGmGfUmGmGmsCmsCm | SEQ ID NO: 771 |

(continued)

| siRNA No. | sense strand (5'-3') | | antisense strand (5'-3') | |
|---|---|---|---|---|
| RX002 136 | CmsAmsCmCmUmUmCfAfUfGmGmUmGmUmUmCmCmAmAm | SEQ ID NO: 560 | UmsUfsGmGmAmAfCmAmCmCmAmUmGmAfAmGfGmUmGmsGmsCm | SEQ ID NO: 772 |
| RX002 137 | GmsGmsUmGmUmUmCfCfAfAmGmCmCmUmUmGmGmCmUm | SEQ ID NO: 561 | AmsGfsCmCmAmAfGmGmCmUmUmGmGmAfAmCfAmCmCmsAmsUm | SEQ ID NO: 773 |
| RX002 138 | CmsCmsGmUmAmUmCfCfAfCmUmGmGmGmAmAmUmCmUm | SEQ ID NO: 562 | AmsGfsAmUmUmCfCmCmAmGmUmGmGmAfUmAfCmGmGmsUmsGm | SEQ ID NO: 774 |
| RX002 139 | GmsCmsCmUmCmCmUfGfCfGmAmUmCmAmGmAmAmGmAm | SEQ ID NO: 563 | UmsCfsUmUmCmUfGmAmUmCmGmCmAmGfGmAfGmGmCmsUmsGm | SEQ ID NO: 775 |
| RX002 140 | UmsGmsCmGmAmUmCfAfGfAmAmGmAmGmAmCmCmAmAm | SEQ ID NO: 564 | UmsUfsGmGmUmCfUmCmUmUmCmUmGmAfUmCfGmCmAmsGmsGm | SEQ ID NO: 776 |
| RX002 141 | GmsAmsUmCmAmGmAfAfGfAmGmAmCmCmAmAmGmGmAm | SEQ ID NO: 565 | UmsCfsCmUmUmGfGmUmCmUmCmUmUmCfUmGfAmUmCmsGmsCm | SEQ ID NO: 777 |
| RX002 142 | GmsUmsUmUmCmAmCfAfGfUmCmAmCmAmGmCmUmGmAm | SEQ ID NO: 566 | UmsCfsAmGmCmUfGmUmGmAmCmUmGmUfGmAfAmAmCmsCmsCm | SEQ ID NO: 778 |
| RX002 143 | UmsGmsAmCmAmAmUfGfUfAmCmCmAmUmGmCmUmAmAm | SEQ ID NO: 567 | UmsUfsAmGmCmAfUmGmGmUmAmCmAmUfUmGfUmCmAmsCmsCm | SEQ ID NO: 779 |
| RX002 144 | GmsUmsAmCmCmAmUfGfCfUmAmAmGmGmCmCmAmAmAm | SEQ ID NO: 568 | UmsUfsUmGmGmCfCmUmUmAmGmCmAmUfGmGfUmAmCmsAmsUm | SEQ ID NO: 780 |
| RX002 145 | CmsAmsAmCmUmCmAfCfCfUmGmUmAmAmUmAmAmAmUm | SEQ ID NO: 569 | AmsUfsUmUmAmUfUmAmCmAmGmGmUmGfAmGfUmUmGmsAmsUm | SEQ ID NO: 781 |
| RX002 146 | CmsCmsUmGmUmAmAfUfAfAmAmUmUmCmGmAmCmCmUm | SEQ ID NO: 570 | AmsGfsGmUmCmGfAmAmUmUmUmAmUmUfAmCfAmGmGmsUmsGm | SEQ ID NO: 782 |
| RX002 147 | GmsUmsAmAmUmAmAfAfUfUmCmGmAmCmCmUmCmAmAm | SEQ ID NO: 571 | UmsUfsGmAmGmGfUmCmGmAmAmUmUmUfAmUfUmAmCmsAmsGm | SEQ ID NO: 783 |
| RX002 148 | CmsGmsAmCmCmUmCfAfAfGmGmUmCmAmCmCmAmUmAm | SEQ ID NO: 572 | UmsAfsUmGmGmUfGmAmCmCmUmUmGmAfGmGfUmCmGmsAmsAm | SEQ ID NO: 784 |
| RX002 149 | GmsAmsCmCmUmCmAfAfGfGmUmCmAmCmCmAmUmAmAm | SEQ ID NO: 573 | UmsUfsAmUmGmGfUmGmAmCmCmUmUmGfAmGfGmUmCmsGmsAm | SEQ ID NO: 785 |
| RX002 150 | CmsAmsAmGmGmUmCfAfCfCmAmUmAmAmAmAmCmCmAm | SEQ ID NO: 574 | UmsGfsGmUmUmUfUmAmUmGmGmUmGmAfCmCfUmUmGmsAmsGm | SEQ ID NO: 786 |
| RX002 151 | CmsCmsAmGmCmAmCfCfGfGmAmAmAmCmAmGmAmAmAm | SEQ ID NO: 575 | UmsUfsUmCmUmGfUmUmUmCmCmGmGmUfGmCfUmGmGmsUmsUm | SEQ ID NO: 787 |

(continued)

| siRNA No. | sense strand (5'-3') | | antisense strand (5'-3') | |
|---|---|---|---|---|
| RX002 152 | GmsCmsAmCmCmGmGfAfAfAmCm AmGmAmAmAmAmGmAm | SEQ ID NO: 576 | UmsCfsUmUmUmUfCmUmGmUmUm UmCmCfGmGfUmGmCmsUmsGm | SEQ ID NO: 788 |
| RX002 153 | GmsGmsCmCmUmCmAfGfGfAmUm GmCmCmAmAmGmAmAm | SEQ ID NO: 577 | UmsUfsCmUmUmGfGmCmAmUmCm CmUmGfAmGfGmCmCmsUmsCm | SEQ ID NO: 789 |
| RX002 154 | CmsAmsGmGmAmUmGfCfCfAmAm GmAmAmCmAmCmUmAm | SEQ ID NO: 578 | UmsAfsGmUmGmUfUmCmUmUmGm GmCmAfUmCfCmUmGmsAmsGm | SEQ ID NO: 790 |
| RX002 155 | GmsAmsUmGmCmCmAfAfGfAmAm CmAmCmUmAmUmGmAm | SEQ ID NO: 579 | UmsCfsAmUmAmGfUmGmUmUmCm UmUmGfGmCfAmUmCmsCmsUm | SEQ ID NO: 791 |
| RX002 156 | CmsCmsAmAmGmAmAfCfAfCmUm AmUmGmAmUmCmCmUm | SEQ ID NO: 580 | AmsGfsGmAmUmCfAmUmAmGmUm GmUmUfCmUfUmGmGmsCmsAm | SEQ ID NO: 792 |
| RX002 157 | AmsGmsAmAmCmAmCfUfAfUmGm AmUmCmCmUmUmGmAm | SEQ ID NO: 581 | UmsCfsAmAmGmGfAmUmCmAmUm AmGmUfGmUfUmCmUmsUmsGm | SEQ ID NO: 793 |
| RX002 158 | GmsAmsGmAmCmCmAfGfGfAmUm GmCmCmAmCmUmAmUm | SEQ ID NO: 582 | AmsUfsAmGmUmGfGmCmAmUmCm CmUmGfGmUfCmUmCmsCmsCm | SEQ ID NO: 794 |
| RX002 159 | CmsCmsAmGmGmAmUfGfCfCmAm CmUmAmUmGmUmCmUm | SEQ ID NO: 583 | AmsGfsAmCmAmUfAmGmUmGmGm CmAmUfCmCfUmGmGmsUmsCm | SEQ ID NO: 795 |
| RX002 160 | CmsAmsGmGmAmUmGfCfCfAmCm UmAmUmGmUmCmUmAm | SEQ ID NO: 584 | UmsAfsGmAmCmAfUmAmGmUmGm GmCmAfUmCfCmUmGmsGmsUm | SEQ ID NO: 796 |
| RX002 161 | GmsGmsAmUmGmCmCfAfCfUmAm UmGmUmCmUmAmUmAm | SEQ ID NO: 585 | UmsAfsUmAmGmAfCmAmUmAmGm UmGmGfCmAfUmCmCmsUmsGm | SEQ ID NO: 797 |
| RX002 162 | GmsAmsUmGmCmCmAfCfUfAmUm GmUmCmUmAmUmAmUm | SEQ ID NO: 586 | AmsUfsAmUmAmGfAmCmAmUmAm GmUmGfGmCfAmUmCmsCmsUm | SEQ ID NO: 798 |
| RX002 163 | GmsUmsUmGmAmCmAfGfAfUmAm CmAmUmCmUmCmCmAm | SEQ ID NO: 587 | UmsGfsGmAmGmAfUmGmUmAmUm CmUmGfUmCfAmAmCmsAmsCm | SEQ ID NO: 799 |
| RX002 164 | GmsAmsCmAmGmAmUfAfCfAmUm CmUmCmCmAmAmGmUm | SEQ ID NO: 588 | AmsCfsUmUmGmGfAmGmAmUmGm UmAmUfCmUfGmUmCmsAmsAm | SEQ ID NO: 780 |
| RX002 165 | CmsAmsGmAmUmAmCfAfUfCmUm CmCmAmAmGmUmAmUm | SEQ ID NO: 589 | AmsUfsAmCmUmUfGmGmAmGmAm UmGmUfAmUfCmUmGmsUmsCm | SEQ ID NO: 801 |
| RX002 166 | AmsGmsAmUmAmCmAfUfCfUmCm CmAmAmGmUmAmUmGm | SEQ ID NO: 590 | CmsAfsUmAmCmUfUmGmGmAmGm AmUmGfUmAfUmCmUmsGmsUm | SEQ ID NO: 802 |
| RX002 167 | GmsAmsUmAmCmAmUfCfUfCmCm AmAmGmUmAmUmGmAm | SEQ ID NO: 591 | UmsCfsAmUmAmCfUmUmGmGmAm GmAmUfGmUfAmUmCmsUmsGm | SEQ ID NO: 803 |

(continued)

| siRNA No. | sense strand (5'-3') | | antisense strand (5'-3') | |
|---|---|---|---|---|
| RX002 168 | CmsCmsAmAmGmUmAfUfGfAmGm CmUmGmGmAmCmAmAm | SEQ ID NO: 592 | UmsUfsGmUmCmCfAmGmCmUmCm AmUmAfCmUfUmGmGmsAmsGm | SEQ ID NO: 804 |
| RX002 169 | GmsGmsAmCmAmAmAfGfCfCmUm UmCmUmCmCmGmAmUm | SEQ ID NO: 593 | AmsUfsCmGmGmAfGmAmAmGmGm CmUmUfUmGfUmCmCmsAmsGm | SEQ ID NO: 805 |
| RX002 170 | GmsAmsCmAmAmAmGfCfCfUmUm CmUmCmCmGmAmUmAm | SEQ ID NO: 594 | UmsAfsUmCmGmGfAmGmAmAmGm GmCmUfUmUfGmUmCmsCmsAm | SEQ ID NO: 806 |
| RX002 171 | GmsCmsCmUmUmCmUfCfCfGmAm UmAmGmGmAmAmCmAm | SEQ ID NO: 595 | UmsGfsUmUmCmCfUmAmUmCmGm GmAmGfAmAfGmGmCmsUmsUm | SEQ ID NO: 807 |
| RX002 172 | CmsCmsGmAmUmAmGfGfAfAmCm AmCmCmCmUmCmAmUm | SEQ ID NO: 596 | AmsUfsGmAmGmGfGmUmGmUmUm CmCmUfAmUfCmGmGmsAmsGm | SEQ ID NO: 808 |
| RX002 173 | GmsAmsUmAmGmGmAfAfCfAmCm CmCmUmCmAmUmCmAm | SEQ ID NO: 597 | UmsGfsAmUmGmAfGmGmGmUmGm UmUmCfCmUfAmUmCmsGmsGm | SEQ ID NO: 809 |
| RX002 174 | CmsUmsGmGmAmCmAfAfGfGmUm CmUmCmAmCmAmCmUm | SEQ ID NO: 598 | AmsGfsUmGmUmGfAmGmAmCmCm UmUmGfUmCfCmAmGmsGmsUm | SEQ ID NO: 810 |
| RX002 175 | GmsCmsUmUmUmCmAfAfAfGmUm UmCmAmCmCmAmAmUm | SEQ ID NO: 599 | AmsUfsUmGmGmUfGmAmAmCmUm UmUmGfAmAfAmGmCmsUmsAm | SEQ ID NO: 811 |
| RX002 176 | CmsUmsUmUmCmAmAfAfGfUmUm CmAmCmCmAmAmUmAm | SEQ ID NO: 600 | UmsAfsUmUmGmGfUmGmAmAmCm UmUmUfGmAfAmAmGmsCmsUm | SEQ ID NO: 812 |
| RX002 177 | CmsAmsGmUmCmAmAfGfGfUmCm UmAmCmGmCmCmUmAm | SEQ ID NO: 601 | UmsAfsGmGmCmGfUmAmGmAmCm CmUmUfGmAfCmUmGmsCmsUm | SEQ ID NO: 813 |
| RX002 178 | GmsUmsCmAmAmGmGfUfCfUmAm CmGmCmCmUmAmUmUm | SEQ ID NO: 602 | AmsAfsUmAmGmGfCmGmUmAmGm AmCmCfUmUfGmAmCmsUmsGm | SEQ ID NO: 814 |
| RX002 179 | AmsGmsGmUmCmUmAfCfGfCmCm UmAmUmUmAmCmAmAm | SEQ ID NO: 603 | UmsUfsGmUmAmAfUmAmGmGmCm GmUmAfGmAfCmCmUmsUmsGm | SEQ ID NO: 815 |
| RX002 180 | GmsCmsUmGmUmAmCfCfCfGmGm UmUmCmUmAmCmCmAm | SEQ ID NO: 604 | UmsGfsGmUmAmGfAmAmCmCmGm GmGmUfAmCfAmGmCmsUmsUm | SEQ ID NO: 816 |
| RX002 181 | CmsUmsGmUmAmCmCfCfGfGmUm UmCmUmAmCmCmAmUm | SEQ ID NO: 605 | AmsUfsGmGmUmAfGmAmAmCmCm GmGmGfUmAfCmAmGmsCmsUm | SEQ ID NO: 817 |
| RX002 182 | CmsCmsGmGmAmAmAfAfGfGmAm GmGmAmUmGmGmAmAm | SEQ ID NO: 606 | UmsUfsCmCmAmUfCmCmUmCmCm UmUmUfUmCfCmGmGmsAmsUm | SEQ ID NO: 818 |
| RX002 183 | CmsGmsGmAmAmAmAfGfGfAmGm GmAmUmGmGmAmAmAm | SEQ ID NO: 607 | UmsUfsUmCmCmAfUmCmCmUmCm CmUmUfUmUfCmCmGmsGmsAm | SEQ ID NO: 819 |

(continued)

| siRNA No. | sense strand (5'-3') | | antisense strand (5'-3') | |
|---|---|---|---|---|
| RX002 184 | GmsAmsGmGmAmUmGfGfAfAmAm GmCmUmGmAmAmCmAm | SEQ ID NO: 608 | UmsGfsUmUmCmAfGmCmUmUmUm CmCmAfUmCfCmUmCmsCmsUm | SEQ ID NO: 820 |
| RX002 185 | GmsCmsCmGmCmUmGfUfGfCmUm GmAmGmGmAmGmAmAm | SEQ ID NO: 609 | UmsUfsCmUmCmCfUmCmAmGmCm AmCmAfGmCfGmGmCmsAmsCm | SEQ ID NO: 821 |
| RX002 186 | CmsCmsGmCmUmGmUfGfCfUmGm AmGmGmAmGmAmAmUm | SEQ ID NO: 610 | AmsUfsUmCmUmCfCmUmCmAmGm CmAmCfAmGfCmGmGmsCmsAm | SEQ ID NO: 822 |
| RX002 187 | CmsGmsCmUmGmUmGfCfUfGmAm GmGmAmGmAmAmUmUm | SEQ ID NO: 611 | AmsAfsUmUmCmUfCmCmUmCmAm GmCmAfCmAfGmCmGmsGmsCm | SEQ ID NO: 823 |
| RX002 188 | GmsUmsGmCmUmGmAfGfGfAmGm AmAmUmUmGmCmUmUm | SEQ ID NO: 612 | AmsAfsGmCmAmAfUmUmCmUmCm CmUmCfAmGfCmAmCmsAmsGm | SEQ ID NO: 824 |
| RX002 189 | GmsCmsUmGmAmGmGfAfGfAmAm UmUmGmCmUmUmCmAm | SEQ ID NO: 613 | UmsGfsAmAmGmCfAmAmUmUmCm UmCmCfUmCfAmGmCmsAmsCm | SEQ ID NO: 825 |
| RX002 190 | GmsGmsAmGmAmAmUfUfGfCmUm UmCmAmUmAmCmAmAm | SEQ ID NO: 614 | UmsUfsGmUmAmUfGmAmAmGmCm AmAmUfUmCfUmCmCmsUmsCm | SEQ ID NO: 826 |
| RX002 191 | GmsAmsGmAmAmUmUfGfCfUmUm CmAmUmAmCmAmAmAm | SEQ ID NO: 615 | UmsUfsUmGmUmAfUmGmAmAmGm CmAmAfUmUfCmUmCmsCmsUm | SEQ ID NO: 827 |
| RX002 192 | GmsUmsGmAmGmCmCfAfGfGmAm GmUmGmGmAmCmUmAm | SEQ ID NO: 616 | UmsAfsGmUmCmCfAmCmUmCmCm UmGmGfCmUfCmAmCmsAmsGm | SEQ ID NO: 828 |
| RX002 193 | CmsCmsAmGmGmAmGfUfGfGmAm CmUmAmUmGmUmGmUm | SEQ ID NO: 617 | AmsCfsAmCmAmUfAmGmUmCmCm AmCmUfCmCfUmGmGmsCmsUm | SEQ ID NO: 829 |
| RX002 194 | CmsAmsGmGmAmGmUfGfGfAmCm UmAmUmGmUmGmUmAm | SEQ ID NO: 618 | UmsAfsCmAmCmAfUmAmGmUmCm CmAmCfUmCfCmUmGmsGmsCm | SEQ ID NO: 830 |
| RX002 195 | GmsGmsAmGmUmGmGfAfCfUmAm UmGmUmGmUmAmCmAm | SEQ ID NO: 619 | UmsGfsUmAmCmAfCmAmUmAmGm UmCmCfAmCfUmCmCmsUmsGm | SEQ ID NO: 831 |
| RX002 196 | GmsAmsGmUmGmGmAfCfUfAmUm GmUmGmUmAmCmAmAm | SEQ ID NO: 620 | UmsUfsGmUmAmCfAmCmAmUmAm GmUmCfCmAfCmUmCmsCmsUm | SEQ ID NO: 832 |
| RX002 197 | GmsUmsGmGmAmCmUfAfUfGmUm GmUmAmCmAmAmGmAm | SEQ ID NO: 621 | UmsCfsUmUmGmUfAmCmAmCmAm UmAmGfUmCfCmAmCmsUmsCm | SEQ ID NO: 833 |
| RX002 198 | CmsCmsAmAmUmGmAfCfUfUmUm GmAmCmGmAmGmUmAm | SEQ ID NO: 622 | UmsAfsCmUmCmGfUmCmAmAmAm GmUmCfAmUfUmGmGmsAmsCm | SEQ ID NO: 834 |
| RX002 199 | GmsAmsCmUmUmUmGfAfCfGmAm GmUmAmCmAmUmCmAm | SEQ ID NO: 623 | UmsGfsAmUmGmUfAmCmUmCmGm UmCmAfAmAfGmUmCmsAmsUm | SEQ ID NO: 835 |

(continued)

| siRNA No. | sense strand (5'-3') | | antisense strand (5'-3') | |
|---|---|---|---|---|
| RX002 200 | AmsCmsUmUmUmGmAfCfGfAmGm UmAmCmAmUmCmAmUm | SEQ ID NO: 624 | AmsUfsGmAmUmGfUmAmCmUmCm GmUmCfAmAfAmGmUmsCmsAm | SEQ ID NO: 836 |
| RX002 201 | GmsAmsCmGmAmGmUfAfCfAmUm CmAmUmGmGmCmCmAm | SEQ ID NO: 625 | UmsGfsGmCmCmAfUmGmAmUmGm UmAmCfUmCfGmUmCmsAmsAm | SEQ ID NO: 837 |
| RX002 202 | CmsAmsUmCmAmAmGfUfCfAmGm GmCmUmCmGmGmAmUm | SEQ ID NO: 626 | AmsUfsCmCmGmAfGmCmCmUmGm AmCmUfUmGfAmUmGmsGmsUm | SEQ ID NO: 838 |
| RX002 203 | CmsAmsGmGmCmUmCfGfGfAmUm GmAmGmGmUmGmCmAm | SEQ ID NO: 627 | UmsGfsCmAmCmCfUmCmAmUmCm CmGmAfGmCfCmUmGmsAmsCm | SEQ ID NO: 839 |
| RX002 204 | GmsCmsUmCmGmGmAfUfGfAmGm GmUmGmCmAmGmGmUm | SEQ ID NO: 628 | AmsCfsCmUmGmCfAmCmCmUmCm AmUmCfCmGfAmGmCmsCmsUm | SEQ ID NO: 840 |
| RX002 205 | CmsGmsUmUmCmAmUfCfAfGmCm CmCmCmAmUmCmAmAm | SEQ ID NO: 629 | UmsUfsGmAmUmGfGmGmGmCmUm GmAmUfGmAfAmCmGmsUmsGm | SEQ ID NO: 841 |
| RX002 206 | GmsGmsGmGmUmCmUfCfUfCmCm UmCmCmGmAmUmUmUm | SEQ ID NO: 630 | AmsAfsAmUmCmGfGmAmGmGmAm GmAmGfAmCfCmCmCmsAmsCm | SEQ ID NO: 842 |
| RX002 207 | CmsCmsGmAmUmUmUfCfUfGmGm GmGmAmGmAmGmAmAm | SEQ ID NO: 631 | UmsUfsCmUmCmUfCmCmCmCmAm GmAmAfAmUfCmGmGmsAmsGm | SEQ ID NO: 843 |
| RX002 208 | CmsUmsCmAmGmCmUfAfCfAmUm CmAmUmCmGmGmGmAm | SEQ ID NO: 632 | UmsCfsCmCmGmAfUmGmAmUmGm UmAmGfCmUfGmAmGmsGmsUm | SEQ ID NO: 844 |
| RX002 209 | GmsCmsUmAmCmAmUfCfAfUmCm GmGmGmAmAmGmGmAm | SEQ ID NO: 633 | UmsCfsCmUmUmCfCmCmGmAmUm GmAmUfGmUfAmGmCmsUmsGm | SEQ ID NO: 845 |
| RX002 210 | CmsCmsGmAmGmGmAfGfGfAmCm GmAmAmUmGmCmCmAm | SEQ ID NO: 634 | UmsGfsGmCmAmUfUmCmGmUmCm CmUmCfCmUfCmGmGmsGmsCm | SEQ ID NO: 846 |
| RX002 211 | CmsGmsAmGmGmAmGfGfAfCmGm AmAmUmGmCmCmAmAm | SEQ ID NO: 635 | UmsUfsGmGmCmAfUmUmCmGmUm CmCmUfCmCfUmCmGmsGmsGm | SEQ ID NO: 847 |
| RX002 212 | CmsUmsUmUmGmGmGfUfGfCmCm CmCmAmAmCmUmGmAm | SEQ ID NO: 636 | UmsCfsAmGmUmUfGmGmGmGmCm AmCmCfCmAfAmAmGmsAmsCm | SEQ ID NO: 848 |

Table 6: Sequence Information of siRNAs

| siRNA No. | sense strand (5'-3') | | antisense strand (5'-3') | |
|---|---|---|---|---|
| RX002213 | CmsAmsGmAmCmAmGfAdCfAmAm GmAmCmCmAmUmCmUm | SEQ ID NO: 849 | AmsGfsAmUmGmGfUmCmUmUmGm UmCmUfGmUfCmUmGmsGmsAm | SEQ ID NO: 644 |
| RX002214 | CmsAmsAmGmCmAmGfGdAfCmUm CmCmUmUmGmUmCmUm | SEQ ID NO: 850 | AmsGfsAmCmAmAfGmGmAmGmUm CmCmUfGmCfUmUmGmsAmsCm | SEQ ID NO: 650 |
| RX002215 | CmsAmsGmAmGmAmAfAdUfUmCm UmAmCmUmAmCmAmUm | SEQ ID NO: 851 | AmsUfsGmUmAmGfUmAmGmAmAm UmUmUfCmUfCmUmGmsUmsAm | SEQ ID NO: 665 |
| RX002216 | UmsCmsAmAmCmUmUfCdCfUmCm CmUmGmCmGmAmAmUm | SEQ ID NO: 852 | AmsUfsUmCmGmCfAmGmGmAmGm GmAmAfGmUfUmGmAmsCmsGm | SEQ ID NO: 692 |
| RX002217 | GmsCmsAmGmAmAmGfAdGfAmAm CmAmUmCmGmUmUmUm | SEQ ID NO: 853 | AmsAfsAmCmGmAfUmGmUmUmCm UmCmUfUmCfUmGmCmsAmsAm | SEQ ID NO: 706 |
| RX002218 | CmsGmsAmAmGmCmUfCdAfUmGm AmAmUmAmUmAmUmUm | SEQ ID NO: 854 | AmsAfsUmAmUmAfUmUmCmAmUm GmAmGfCmUfUmCmGmsUmsAm | SEQ ID NO: 709 |
| RX002219 | CmsCmsUmAmCmUmCfTdGfUmUm GmUmUmCmGmAmAmAm | SEQ ID NO: 855 | UmsUfsUmCmGmAfAmCmAmAmCmA mGmAfGmUfAmGmGmsGmsUm | SEQ ID NO: 720 |
| RX002220 | CmsCmsAmAmCmUmAfCdAfUmGm AmAmCmCmUmAmCmAm | SEQ ID NO: 856 | UmsGfsUmAmGmGfUmUmCmAmUm GmUmAfGmUfUmGmGmsCmsUm | SEQ ID NO: 754 |
| RX002221 | CmsAmsGmCmUmAmAfAdAfGmAm CmUmUmUmGmAmCmUm | SEQ ID NO: 857 | AmsGfsUmCmAmAfAmGmUmCmUm UmUmUfAmGfCmUmGmsCmsAm | SEQ ID NO: 763 |
| RX002222 | CmsGmsUmCmGmUmGfCdGfUmUm GmGmCmUmCmAmAmUm | SEQ ID NO: 858 | AmsUfsUmGmAmGfCmCmAmAmCmG mCmAfCmGfAmCmGmsGmsGm | SEQ ID NO: 766 |
| RX002223 | CmsAmsAmCmUmCmAfCdCfUmGm UmAmAmUmAmAmAmUm | SEQ ID NO: 859 | AmsUfsUmUmAmUfUmAmCmAmGm GmUmGfAmGfUmUmGmsAmsUm | SEQ ID NO: 781 |
| RX002224 | CmsAmsGmAmUmAmCfAdUfCmUm CmCmAmAmGmUmAmUm | SEQ ID NO: 860 | AmsUfsAmCmUmUfGmGmAmGmAm UmGmUfAmUfCmUmGmsUmsCm | SEQ ID NO: 801 |
| RX002225 | CmsCmsGmAmUmAmGfGdAfAmCm AmCmCmCmUmCmAmUm | SEQ ID NO: 861 | AmsUfsGmAmGmGfGmUmGmUmUm CmCmUfAmUfCmGmGmsAmsGm | SEQ ID NO: 808 |
| RX002226( AD570714 ) | GmsAmsGmCmCmGmUfUmCfUfCfU mAmCmAmAmUmUmAmCmUm | SEQ ID NO: 862 | AmsGfsUmAmAmUfUmGfUfAmGmA mGmAfAmCfGmGmCmUmCmsGmsG m | SEQ ID NO: 881 |

(continued)

| siRNA No. | sense strand (5'-3') | | antisense strand (5'-3') | |
|---|---|---|---|---|
| RX002227 | AmsAmsAmUmUmCmUfAdCfUmAm CmAmUmCmUmAmUmAm | SEQ ID NO: 863 | UmsAfsUmAmGmAfUmGmUmAmGm UmAmGfAmAfUmUmUmsCmsUm | SEQ ID NO: 666 |
| RX002228 | CmsUmsCmCmAmAmCfAdAfUmUm AmCmCmUmGmCmAmUm | SEQ ID NO: 864 | AmsUfsGmCmAmGfGmUmAmAmUm UmGmUfUmGfGmAmGmsUmsUm | SEQ ID NO: 687 |
| RX002229 | CmsUmsGmCmUmGmCfAdAfCmUm AmCmAmUmCmAmCmAm | SEQ ID NO: 865 | UmsGfsUmGmAmUfGmUmAmGmUm UmGmCfAmGfCmAmGmsUmsCm | SEQ ID NO: 703 |
| RX002230 | GmsAmsAmGmCmUmCfAdUfGmAm AmUmAmUmAmUmUmUm | SEQ ID NO: 866 | AmsAfsAmUmAmUfAmUmUmCmAm UmGmAfGmCfUmUmCmsGmsUm | SEQ ID NO: 710 |
| RX002231 | CmsCmsUmUmCmGmAfGdGfUmCm AmCmAmGmUmAmAmUm | SEQ ID NO: 867 | AmsUfsUmAmCmUfGmUmGmAmCmC mUmCfGmAfAmGmGmsGmsGm | SEQ ID NO: 712 |
| RX002232 | CmsCmsGmGmAmAmGfGdAfAmUm CmAmGmAmAmUmGmAm | SEQ ID NO: 868 | UmsCfsAmUmUmCfUmGmAmUmUmC mCmUfUmCfCmGmGmsCmsAm | SEQ ID NO: 734 |
| RX002233 | CmsGmsCmUmGmUmGfCdAfUmUm AmCmCmUmGmGmAmUm | SEQ ID NO: 869 | AmsUfsCmCmAmGfGmUmAmAmUm GmCmAfCmAfGmCmGmsAmsUm | SEQ ID NO: 739 |
| RX002234 | CmsUmsUmGmAmAmGfCdCfAmAm CmUmAmCmAmUmGmAm | SEQ ID NO: 870 | UmsCfsAmUmGmUfAmGmUmUmGm GmCmUfUmCfAmAmGmsGmsAm | SEQ ID NO: 753 |
| RX002235 | CmsUmsAmCmUmGmCfAdGfCmUm AmAmAmAmGmAmCmUm | SEQ ID NO: 871 | AmsGfsUmCmUmUfUmUmAmGmCm UmGmCfAmGfUmAmGmsGmsGm | SEQ ID NO: 762 |
| RX002236 | GmsCmsUmAmAmAmAfGdAfCmUm UmUmGmAmCmUmUmUm | SEQ ID NO: 872 | AmsAfsAmGmUmCfAmAmAmGmUm CmUmUfUmUfAmGmCmsUmsGm | SEQ ID NO: 764 |
| RX002237 | AmsGmsAmAmCmAmCfUdAfUmGm AmUmCmCmUmUmGmAm | SEQ ID NO: 873 | UmsCfsAmAmGmGfAmUmCmAmUm AmGmUfGmUfUmCmUmsUmsGm | SEQ ID NO: 793 |
| RX002238 | GmsAmsUmAmCmAmUfCdUfCmCm AmAmGmUmAmUmGmAm | SEQ ID NO: 874 | UmsCfsAmUmAmCfUmUmGmGmAm GmAmUfGmUfAmUmCmsUmsGm | SEQ ID NO: 803 |
| RX002239 | GmsGmsAmCmAmAmAfGdCfCmUm UmCmUmCmCmGmAmUm | SEQ ID NO: 875 | AmsUfsCmGmGmAfGmAmAmGmGm CmUmUfUmGfUmCmCmsAmsGm | SEQ ID NO: 805 |
| RX002240 | CmsUmsUmUmCmAmAfAdGfUmUm CmAmCmCmAmAmUmAm | SEQ ID NO: 876 | UmsAfsUmUmGmGfUmGmAmAmCm UmUmUfGmAfAmAmGmsCmsUm | SEQ ID NO: 812 |

(continued)

| siRNA No. | sense strand (5'-3') | | antisense strand (5'-3') | |
|---|---|---|---|---|
| RX002241 | CmsAmsGmUmCmAmAfGdGfUmCmUmAmCmGmCmCmUmAm | SEQ ID NO: 877 | UmsAfsGmGmCmGfUmAmGmAmCmCmUmUfGmAfCmUmGmsCmsUm | SEQ ID NO: 813 |
| RX002242 | GmsUmsCmAmAmGmGfUdCfUmAmCmGmCmCmUmAmUmUm | SEQ ID NO: 878 | AmsAfsUmAmGmGfCmGmUmAmGmAmCmCfUmUfGmAmCmsUmsGm | SEQ ID NO: 814 |
| RX002243 | GmsAmsGmAmAmUmUfGdCfUmUmCmAmUmAmCmAmAmAm | SEQ ID NO: 879 | UmsUfsUmGmUmAfUmGmAmAmGmCmAmAfUmUfCmUmCmsCmsUm | SEQ ID NO: 827 |
| RX002244 | CmsGmsAmGmGmAmGfGdAfCmGmAmAmUmGmCmCmAmAm | SEQ ID NO: 880 | UmsUfsGmGmCmAfUmUmCmGmUmCmCmUfCmCfUmCmGmsGmsGm | SEQ ID NO: 847 |
| RXM02003 | AmsGmsAmAmGmAmAfGfAfUfAmUmUmAmUmCmUmCmUm | SEQ ID NO: 882 | AmsGfsAmGmAmUfAmAmUmAmUmCmUmUfCmUfUmCmUmsGmsGm | SEQ ID NO: 883 |
| RX000001 | UmsUmsCmUmCmCmGfAfAfCmGmUmGmUmCmAmCmGmUm | SEQ ID NO: 884 | AmsCfsGmUmGmAfCmAmCmGmUmUmCmGfGmAfGmAmAmsCmsUm | SEQ ID NO: 886 |
| RX000002 | UmsUmsCmUmCmCmGfAdAfCmGmUmGmUmCmAmCmGmUm | SEQ ID NO: 885 | AmsCfsGmUmGmAfCmAmCmGmUmUmCmGfGmAfGmAmAmsCmsUm | SEQ ID NO: 886 |

Table 7: Sequence Information of siRNAs

| siRNA No. | sense strand (5'-3') | | antisense strand (5'-3') | |
|---|---|---|---|---|
| RX002 245 | CmsGmsAmAmGmCmUfCfAfUfGmAmAmUmAmUmAmUmUm | SEQ ID NO: 887 | AmsAfsUmAmUmAfUmUmCfAmUmGmAmGfC(moe)UfUmCmGmsUmsAm | SEQ ID NO: 908 |
| RX002 246 | CmsGmsAmAmGmCmUfCfAfUfGmAmAmUmAmUmAmUmUm | SEQ ID NO: 887 | AmsAfsUmAmUmAfUmUmCmAfUmGmAmGfC(moe)UfUmCmGmsUmsAm | SEQ ID NO: 909 |
| RX002 247 | CmsGmsAmAmGmCmUfCfAfUfGmAmAmUmAmUmAmUmUm | SEQ ID NO: 887 | AmsAfsUmAmUmAfUmUmCmAmUfGmAmGfC(moe)UfUmCmGmsUmsAm | SEQ ID NO: 910 |
| RX002 248 | CmsGmsAmAmG(moe)CmUfCfAfUfGmAmAmUmAmUmAmUmUm | SEQ ID NO: 888 | AmsAfsUmAmUmAfUmUmCmAfUmGmAmGfC(moe)UfUmCmGmsUmsAm | SEQ ID NO: 909 |
| RX002 249 | CmsGmsAmAmGmCmUfCfAfUfGmA(moe)AmUmAmUmAmUmUm | SEQ ID NO: 889 | AmsAfsUmAmUmAfUmUmCmAfUmGmAmGfC(moe)UfUmCmGmsUmsAm | SEQ ID NO: 909 |
| RX002 250 | CmsGmsAmAmGmCmUfCfAfUfGmAmA(moe)UmAmUmAmUmUm | SEQ ID NO: 890 | AmsAfsUmAmUmAfUmUmCmAfUmGmAmGfC(moe)UfUmCmGmsUmsAm | SEQ ID NO: 909 |
| RX002 251 | CmsGmsAmAmGmCmUfCfAfUfGmAmAmUmAmUmAmT(moe)Um | SEQ ID NO: 891 | AmsAfsUmAmUmAfUmUmCmAfUmGmAmGfC(moe)UfUmCmGmsUmsAm | SEQ ID NO: 909 |
| RX002 252 | CmsGmsAmAmG(moe)CmUfCfAfUfGmAmAmUmAmUmAmUmUm | SEQ ID NO: 888 | AmsAfsUmAmUmAfUmT(moe)CmAfUmGmAmGfCmUfUmCmGmsUmsAm | SEQ ID NO: 911 |
| RX002 253 | CmsGmsAmAmGmCmUfCfAfUfGmA(moe)AmUmAmUmAmUmUm | SEQ ID NO: 889 | AmsAfsUmAmUmAfUmT(moe)CmAfUmGmAmGfCmUfUmCmGmsUmsAm | SEQ ID NO: 911 |
| RX002 254 | CmsGmsAmAmGmCmUfCfAfUfGmAmA(moe)UmAmUmAmUmUm | SEQ ID NO: 890 | AmsAfsUmAmUmAfUmT(moe)CmAfUmGmAmGfCmUfUmCmGmsUmsAm | SEQ ID NO: 911 |
| RX002 255 | CmsGmsAmAmGmCmUfCfAfUfGmAmAmUmAmUmAmT(moe)Um | SEQ ID NO: 891 | AmsAfsUmAmUmAfUmT(moe)CmAfUmGmAmGfCmUfUmCmGmsUmsAm | SEQ ID NO: 911 |
| RX002 256 | CmsAmsGmAmGmAmAfAfUfUfCmUmAmCmUmAmCmAmUm | SEQ ID NO: 892 | AmsUfsGmUmAmGfUmAmGfAmAmUmUmUfC(moe)UfCmUmGmsUmsAm | SEQ ID NO: 912 |

(continued)

| siRNA No. | sense strand (5'-3') | | antisense strand (5'-3') | |
|---|---|---|---|---|
| RX002 257 | CmsAmsGmAmGmAmAfAfUfUfCmUmAmCmUmAmCmAmUm | SEQ ID NO: 892 | AmsUfsGmUmAmGfUmAmGmAfAmUmUmUfC(moe)UfCmUmGmsUmsAm | SEQ ID NO: 913 |
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |
| RX002 259 | CmsAmsGmAmG(moe)AmAfAfUfUfCmUmAmCmUmAmCmAmUm | SEQ ID NO: 893 | AmsUfsGmUmAmGfUmAmGmAfAmUmUmUfC(moe)UfCmUmGmsUmsAm | SEQ ID NO: 913 |
| RX002 260 | CmsAmsGmAmGmAmAfAfUfUfCmT(moe)AmCmUmAmCmAmUm | SEQ ID NO: 894 | AmsUfsGmUmAmGfUmAmGmAfAmUmUmUfC(moe)UfCmUmGmsUmsAm | SEQ ID NO: 913 |
| RX002 261 | CmsAmsGmAmGmAmAfAfUfUfCmUmA(moe)CmUmAmCmAmUm | SEQ ID NO: 895 | AmsUfsGmUmAmGfUmAmGmAfAmUmUmUfC(moe)UfCmUmGmsUmsAm | SEQ ID NO: 913 |
| RX002 262 | CmsAmsGmAmGmAmAfAfUfUfCmUmAmCmUmAmCmA(moe)Um | SEQ ID NO: 896 | AmsUfsGmUmAmGfUmAmGmAfAmUmUmUfC(moe)UfCmUmGmsUmsAm | SEQ ID NO: 913 |
| RX002 263 | CmsAmsGmAmG(moe)AmAfAfUfUfCmUmAmCmUmAmCmAmUm | SEQ ID NO: 893 | AmsUfsGmUmAmGfUmA(moe)GmAfAmUmUmUfCmUfCmUmGmsUmsAm | SEQ ID NO: 915 |
| RX002 264 | CmsAmsGmAmGmAmAfAfUfUfCmT(moe)AmCmUmAmCmAmUm | SEQ ID NO: 894 | AmsUfsGmUmAmGfUmA(moe)GmAfAmUmUmUfCmUfCmUmGmsUmsAm | SEQ ID NO: 915 |
| RX002 265 | CmsAmsGmAmGmAmAfAfUfUfCmUmA(moe)CmUmAmCmAmUm | SEQ ID NO: 895 | AmsUfsGmUmAmGfUmA(moe)GmAfAmUmUmUfCmUfCmUmGmsUmsAm | SEQ ID NO: 915 |
| RX002 266 | CmsAmsGmAmGmAmAfAfUfUfCmUmAmCmUmAmCmA(moe)Um | SEQ ID NO: 896 | AmsUfsGmUmAmGfUmA(moe)GmAfAmUmUmUfCmUfCmUmGmsUmsAm | SEQ ID NO: 915 |
| RX002 267 | GmsAmsGmAmAmUmUfGfCfUfUmCmAmUmAmCmAmAmAm | SEQ ID NO: 897 | UmsUfsUmGmUmAfUmGmAfAmGmCmAmAfT(moe)UfCmUmCmsCmsUm | SEQ ID NO: 916 |
| RX002 268 | GmsAmsGmAmAmUmUfGfCfUfUmCmAmUmAmCmAmAmAm | SEQ ID NO: 897 | UmsUfsUmGmUmAfUmGmAmAfGmCmAmAfT(moe)UfCmUmCmsCmsUm | SEQ ID NO: 917 |

(continued)

| siRNA No. | sense strand (5'-3') | | antisense strand (5'-3') | |
|---|---|---|---|---|
| RX002 269 | GmsAmsGmAmAmUmUfGfCfUfUmCmAmUmAmCmAmAmAm | SEQ ID NO: 897 | UmsUfsUmGmUmAfUmGmAmAmGfCmAmAfT(moe)UfCmUmCmsCmsUm | SEQ ID NO: 918 |
| RX002 270 | GmsAmsGmAmA(moe)UmUfGfCfUfUmCmAmUmAmCmAmAmAm | SEQ ID NO: 898 | UmsUfsUmGmUmAfUmGmAmAfGmCmAmAfT(moe)UfCmUmCmsCmsUm | SEQ ID NO: 917 |
| RX002 271 | GmsAmsGmAmAmUmUfGfCfUfUmC(moe)AmUmAmCmAmAmAm | SEQ ID NO: 899 | UmsUfsUmGmUmAfUmGmAmAfGmCmAmAfT(moe)UfCmUmCmsCmsUm | SEQ ID NO: 917 |
| RX002 272 | GmsAmsGmAmAmUmUfGfCfUfUmCmA(moe)UmAmCmAmAmAm | SEQ ID NO: 900 | UmsUfsUmGmUmAfUmGmAmAfGmCmAmAfT(moe)UfCmUmCmsCmsUm | SEQ ID NO: 917 |
| RX002 273 | GmsAmsGmAmAmUmUfGfCfUfUmCmAmUmAmCmAmA(moe)Am | SEQ ID NO: 901 | UmsUfsUmGmUmAfUmGmAmAfGmCmAmAfT(moe)UfCmUmCmsCmsUm | SEQ ID NO: 917 |
| RX002 274 | GmsAmsGmAmA(moe)UmUfGfCfUfUmCmAmUmAmCmAmAmAm | SEQ ID NO: 898 | UmsUfsUmGmUmAfUmG(moe)AmAfGmCmAmAfUmUfCmUmCmsCmsUm | SEQ ID NO: 919 |
| RX002 275 | GmsAmsGmAmAmUmUfGfCfUfUmC(moe)AmUmAmCmAmAmAm | SEQ ID NO: 899 | UmsUfsUmGmUmAfUmG(moe)AmAfGmCmAmAfUmUfCmUmCmsCmsUm | SEQ ID NO: 919 |
| RX002 276 | GmsAmsGmAmAmUmUfGfCfUfUmCmA(moe)UmAmCmAmAmAm | SEQ ID NO: 900 | UmsUfsUmGmUmAfUmG(moe)AmAfGmCmAmAfUmUfCmUmCmsCmsUm | SEQ ID NO: 919 |
| RX002 277 | GmsAmsGmAmAmUmUfGfCfUfUmCmAmUmAmCmAmA(moe)Am | SEQ ID NO: 901 | UmsUfsUmGmUmAfUmG(moe)AmAfGmCmAmAfUmUfCmUmCmsCmsUm | SEQ ID NO: 919 |
| RX002 278 | CmsAmsAmCmUmCmAfCfCfUfGmUmAmAmUmAmAmAmUm | SEQ ID NO: 902 | AmsUfsUmUmAmUfUmAmCfAmGmGmUmGfA(moe)GfUmUmGmsAmsUm | SEQ ID NO: 920 |
| RX002 279 | CmsAmsAmCmUmCmAfCfCfUfGmUmAmAmUmAmAmAmUm | SEQ ID NO: 902 | AmsUfsUmUmAmUfUmAmCmAfGmGmUmGfA(moe)GfUmUmGmsAmsUm | SEQ ID NO: 921 |
| RX002 280 | CmsAmsAmCmUmCmAfCfCfUfGmUmAmAmUmAmAmAmUm | SEQ ID NO: 902 | AmsUfsUmUmAmUfUmAmCmAmGfGmUmGfA(moe)GfUmUmGmsAmsUm | SEQ ID NO: 922 |

(continued)

| siRNA No. | sense strand (5'-3') | | antisense strand (5'-3') | |
|---|---|---|---|---|
| RX002 281 | CmsAmsAmCmT(moe)CmAfCfCfUfGmUmAmAmUmAmAmAmUm | SEQ ID NO: 903 | AmsUfsUmUmAmUfUmAmCmAfGmGmUmGfA(moe)GfUmUmGmsAmsUm | SEQ ID NO: 921 |
| RX002 282 | CmsAmsAmCmUmCmAfCfCfUfGmT(moe)AmAmUmAmAmAmUm | SEQ ID NO: 904 | AmsUfsUmUmAmUfUmAmCmAfGmGmUmGfA(moe)GfUmUmGmsAmsUm | SEQ ID NO: 921 |
| RX002 283 | CmsAmsAmCmUmCmAfCfCfUfGmUmA(moe)AmUmAmAmAmUm | SEQ ID NO: 905 | AmsUfsUmUmAmUfUmAmCmAfGmGmUmGfA(moe)GfUmUmGmsAmsUm | SEQ ID NO: 921 |
| RX002 284 | CmsAmsAmCmUmCmAfCfCfUfGmUmAmAmUmAmAmA(moe)Um | SEQ ID NO: 906 | AmsUfsUmUmAmUfUmAmCmAfGmGmUmGfA(moe)GfUmUmGmsAmsUm | SEQ ID NO: 921 |
| RX002 285 | CmsAmsAmCmT(moe)CmAfCfCfUfGmUmAmAmUmAmAmAmUm | SEQ ID NO: 903 | AmsUfsUmUmAmUfUmA(moe)CmAfGmGmUmGfAmGfUmUmGmsAmsUm | SEQ ID NO: 923 |
| RX002 286 | CmsAmsAmCmUmCmAfCfCfUfGmT(moe)AmAmUmAmAmAmUm | SEQ ID NO: 904 | AmsUfsUmUmAmUfUmA(moe)CmAfGmGmUmGfAmGfUmUmGmsAmsUm | SEQ ID NO: 923 |
| RX002 287 | CmsAmsAmCmUmCmAfCfCfUfGmUmA(moe)AmUmAmAmAmUm | SEQ ID NO: 905 | AmsUfsUmUmAmUfUmA(moe)CmAfGmGmUmGfAmGfUmUmGmsAmsUm | SEQ ID NO: 923 |
| RX002 288 | CmsAmsAmCmUmCmAfCfCfUfGmUmAmAmUmAmAmA(moe)Um | SEQ ID NO: 906 | AmsUfsUmUmAmUfUmA(moe)CmAfGmGmUmGfAmGfUmUmGmsAmsUm | SEQ ID NO: 923 |
| RX002 289 | CmsGmsAmAmG(moe)CmUfCfAfUfGmAmAmUmAmUmAmUmUm | SEQ ID NO: 888 | AmsAfsUmAmUmAfUmUmCmAmUfGmAmGfC(moe)UfUmCmGmsUmsAm | SEQ ID NO: 910 |
| RX002 290 | CmsGmsAmAmGmCmUfCfAfUfGmAmA(moe)UmAmUmAmUmUm | SEQ ID NO: 890 | AmsAfsUmAmUmAfUmUmCmAmUfGmAmGfC(moe)UfUmCmGmsUmsAm | SEQ ID NO: 910 |
| RX002 291 | CmsGmsAmAmGmCmUfCfAfUfGmAmA(moe)UmAmUmAmUmUm | SEQ ID NO: 890 | AmsAfsUmAmUmAfUmT(moe)CmAmUfGmAmGfCmUfUmCmGmsUmsAm | SEQ ID NO: 924 |
| RX002 292 | CmsGmsAmAmGmCmUfCfAfUfGmAmAmUmAmUmAmT(moe)Um | SEQ ID NO: 891 | AmsAfsUmAmUmAfUmT(moe)CmAmUfGmAmGfCmUfUmCmGmsUmsAm | SEQ ID NO: 924 |

(continued)

| siRNA No. | sense strand (5'-3') | | antisense strand (5'-3') | |
|---|---|---|---|---|
| RX002 293 | CmsAmsGmAmGmAmAfAfUfUfCmUmA(moe)CmUmAmCmAmUm | SEQ ID NO: 895 | AmsUfsGmUmAmGfUmAmGmAmAfUmUmUfC(moe)UfCmUmGmsUmsAm | SEQ ID NO: 914 |
| RX002 294 | CmsAmsGmAmGmAmAfAfUfUfCmUmAmCmUmAmCmA(moe)Um | SEQ ID NO: 896 | AmsUfsGmUmAmGfUmA(moe)GmAmAfUmUmUfCmUfCmUmGmsUmsAm | SEQ ID NO: 925 |
| RX002 295 | CmsAmsAmCmUmCmAfCfCfUfGmUmA(moe)AmUmAmAmAmUm | SEQ ID NO: 905 | AmsUfsUmUmAmUfUmAmCmAmGfGmUmGfA(moe)GfUmUmGmsAmsUm | SEQ ID NO: 922 |
| RX002 296 | CmsAmsAmCmUmCmAfCfCfUfGmUmAmAmUmAmAmA(moe)Um | SEQ ID NO: 906 | AmsUfsUmUmAmUfUmA(moe)CmAmGfGmUmGfAmGfUmUmGmsAmsUm | SEQ ID NO: 926 |
| RX002 297 | CmsAmsGmAmGmAmAfAfUfUfCmUmAmCmUmAmCmAmUm | SEQ ID NO: 892 | AmsUfsGmUmAmGfUmAmGmAmAmUfUmUfC(moe)UfCmUmGmsUmsAm | SEQ ID NO: 927 |
| RX002 298 | AmsAmsAmUmUmCmUfAfCfUfAmCmAmUmCmUmAmUmAm | SEQ ID NO: 907 | UmsAfsUmAmGmAfUmGmUmAmGmUfAmGfAmAfUmUmUmsCmsUm | SEQ ID NO: 928 |
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |

(continued)

| siRNA No. | sense strand (5'-3') | | antisense strand (5'-3') | |
|---|---|---|---|---|
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |
| RX002 308 | CmsAmsGmAmGmAmAfAfUfUfCmUmAmCmUmAmCmAmUm | SEQ ID NO: 892 | AmsUfsGmUmAmGfUmAmGmAmAmUfUmUfCmUfCmUmGmsUmsAm | SEQ ID NO: 938 |
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

**Preparation Example 4: Preparation of siRNA Conjugate with L96 Moiety Conjugated to 3'-end of Sense Strand**

Wherein, compound L96-PS was purchased from Asymchem Laboratories (Tianjin) Co., Ltd., with a loading capacity of 120 ± 12 μmol/g (detection method: UV/HPLC). The structural formula of compound L96-PS is:

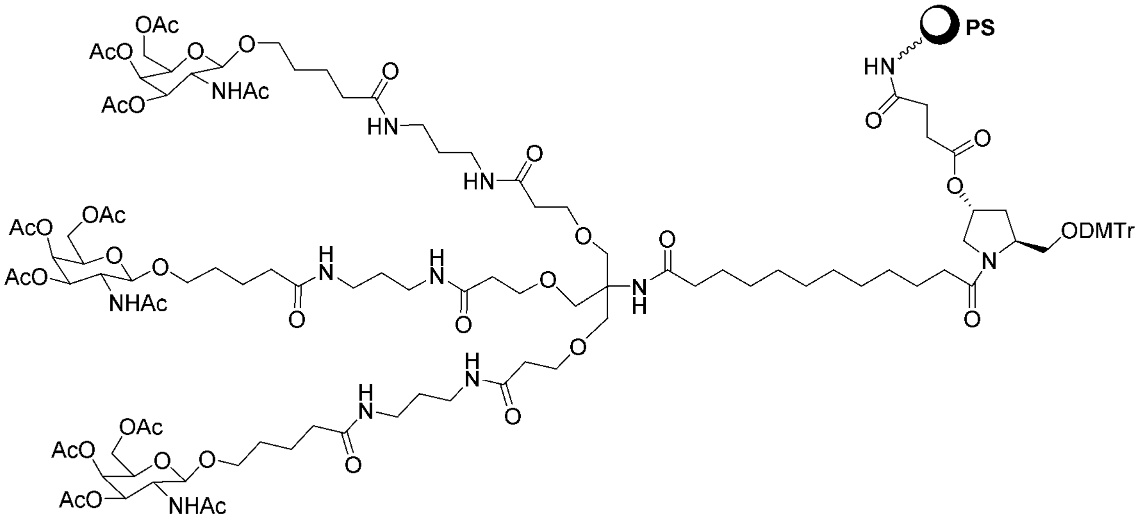

Wherein, PS represents a polystyrene resin solid-phase support.

(4.1) Synthesis of sense strand

Using the phosphoramidite solid-phase nucleic acid synthesis method, reaction cycles were started with the aforementioned L96-PS compound linked to a solid-phase support, and nucleoside monomers were linked sequentially in a 3' to 5' direction according to the nucleotide sequence. Each cycle of nucleoside monomer addition included four reaction steps: deprotection, coupling, capping, and oxidation or sulfurization. The synthesis method and conditions followed the sense strand synthesis method shown in step (3.1) of Preparation Example 3.

(4.2) Synthesis of antisense strand

The siRNA antisense strands in this preparation example were synthesized according to the antisense strand synthesis method shown in step (3.2) of Preparation Example 3.

(4.3) Synthesis of siRNA conjugate

The siRNA conjugates of this preparation example were synthesized according to the method shown in step (3.3) of Preparation Example 3.

Wherein, the structural formula of the siRNA conjugate with the L96 moiety conjugated to the 3'-end of the sense strand is as follows:

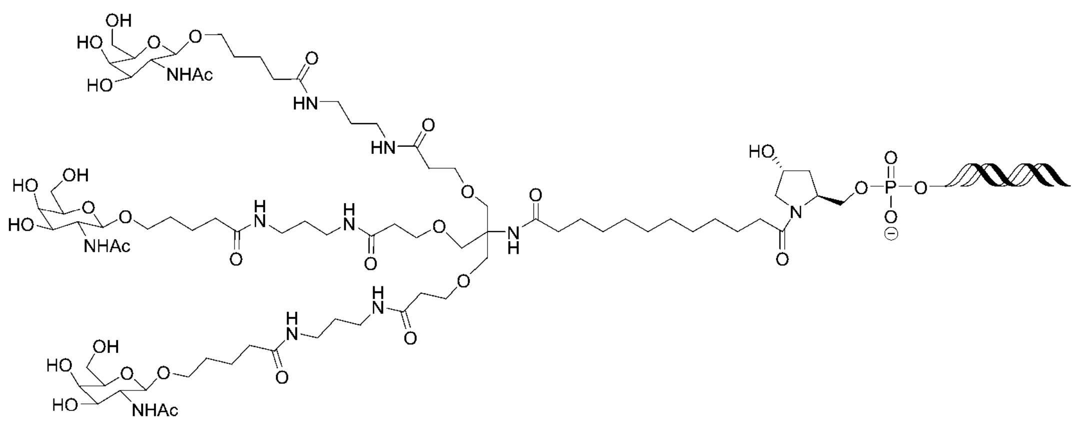

Wherein,

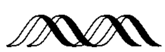

represents siRNA. L96 was conjugated to the 3'-end of the sense strand of the siRNA via a phosphodiester bond.

Table 8: Sequence Information of siRNA Conjugates with an L96 Moiety Conjugated to the 3'-end of the Sense Strand

| siRNA conjugate No. | sense strand (5'-3') | | antisense strand (5'-3') | |
|---|---|---|---|---|
| RZ002001 | CmsAmsGmAmCmAmGfAdCfAmAmGmAmCmCmAmUmCmUm_L96 | SEQ ID NO: 849_L96 | AmsGfsAmUmGmGfUmCmUmUmGmUmCmUfGmUfCmUmGmsGmsAm | SEQ ID NO: 644 |
| RZ002002 | CmsAmsAmGmCmAmGfGdAfCmUmCmCmUmUmGmUmCmUm_L96 | SEQ ID NO: 850_L96 | AmsGfsAmCmAmAfGmGmAmGmUmCmCmUfGmCfUmUmGmsAmsCm | SEQ ID NO: 650 |
| RZ002003 | CmsAmsGmAmGmAmAfAdUfUmCmUmAmCmUmAmCmAmUm_L96 | SEQ ID NO: 851_L96 | AmsUfsGmUmAmGfUmAmGmAmAmUmUmUfCmUfCmUmGmsUmsAm | SEQ ID NO: 665 |
| RZ002004 | UmsCmsAmAmCmUmUfCdCfUmCmCmUmGmCmGmAmAmUm_L96 | SEQ ID NO: 852_L96 | AmsUfsUmCmGmCfAmGmGmAmGmGmAmAfGmUfUmGmAmsCmsGm | SEQ ID NO: 692 |
| RZ002005 | GmsCmsAmGmAmAmGfAdGfAmAmCmAmUmCmGmUmUmUm_L96 | SEQ ID NO: 853_L96 | AmsAfsAmCmGmAfUmGmUmUmCmUmCmUfUmCfUmGmCmsAmsAm | SEQ ID NO: 706 |
| RZ002006 | CmsGmsAmAmGmCmUfCdAfUmGmAmAmUmAmUmAmUmUm_L96 | SEQ ID NO: 854_L96 | AmsAfsUmAmUmAfUmUmCmAmUmGmAmGfCmUfUmCmGmsUmsAm | SEQ ID NO: 709 |
| RZ002007 | CmsCmsUmAmCmUmCfTdGfUmUmGmUmUmCmGmAmAmAm_L96 | SEQ ID NO: 855_L96 | UmsUfsUmCmGmAfAmCmAmAmCmAmGmAfGmUfAmGmGmsGmsUm | SEQ ID NO: 720 |
| RZ002008 | CmsCmsAmAmCmUmAfCdAfUmGmAmAmCmCmUmAmCmAm_L96 | SEQ ID NO: 856_L96 | UmsGfsUmAmGmGfUmUmCmAmUmGmUmAfGmUfUmGmGmsCmsUm | SEQ ID NO: 754 |
| RZ002009 | CmsAmsGmCmUmAmAfAdAfGmAmCmUmUmUmGmAmCmUm_L96 | SEQ ID NO: 857_L96 | AmsGfsUmCmAmAfAmGmUmCmUmUmUmUfAmGfCmUmGmsCmsAm | SEQ ID NO: 763 |
| RZ002010 | CmsGmsUmCmGmUmGfCdGfUmUmGmGmCmUmCmAmAmUm_L96 | SEQ ID NO: 858_L96 | AmsUfsUmGmAmGfCmCmAmAmCmGmCmAfCmGfAmCmGmsGmsGm | SEQ ID NO: 766 |
| RZ002011 | CmsAmsAmCmUmCmAfCdCfUmGmUmAmAmUmAmAmAmUm L96 | SEQ ID NO: 859_L96 | AmsUfsUmUmAmUfUmAmCmAmGmGmUmGfAmGfUmUmGmsAmsUm | SEQ ID NO: 781 |
| RZ002012 | CmsAmsGmAmUmAmCfAdUfCmUmCmCmAmAmGmUmAmUm_L96 | SEQ ID NO: 860_L96 | AmsUfsAmCmUmUfGmGmAmGmAmUmGmUfAmUfCmUmGmsUmsCm | SEQ ID NO: 801 |

(continued)

| siRNA conjugate No. | sense strand (5'-3') | | antisense strand (5'-3') | |
|---|---|---|---|---|
| RZ002013 | CmsCmsGmAmUmAmGfGdAfAmCmAmCmCmCmUmCmAmUm_L96 | SEQ ID NO: 861_L96 | AmsUfsGmAmGmGfGmUmGmUmUmCmCmUfAmUfCmGmGmsAmsGm | SEQ ID NO: 808 |
| RZ002014(AD570714) | GmsAmsGmCmCmGmUfUmCfUfCfUmAmCmAmAmUmUmAmCmUm_L96 | SEQ ID NO: 862_L96 | AmsGfsUmAmAmUfUmGfUfAmGmAmGmAfAmCfGmGmCmUmCmsGmsGm | SEQ ID NO: 881 |
| RZ002015 | AmsAmsAmUmUmCmUfAdCfUmAmCmAmUmCmUmAmUmAm_L96 | SEQ ID NO: 863_L96 | UmsAfsUmAmGmAfUmGmUmAmGmUmAmGfAmAfUmUmUmsCmsUm | SEQ ID NO: 666 |
| RZ002016 | CmsUmsCmCmAmAmCfAdAfUmUmAmCmCmUmGmCmAmUm_L96 | SEQ ID NO: 864_L96 | AmsUfsGmCmAmGfGmUmAmAmUmUmGmUfUmGfGmAmGmsUmsUm | SEQ ID NO: 687 |
| RZ002017 | CmsUmsGmCmUmGmCfAdAfCmUmAmCmAmUmCmAmCmAm_L96 | SEQ ID NO: 865_L96 | UmsGfsUmGmAmUfGmUmAmGmUmUmGmCfAmGfCmAmGmsUmsCm | SEQ ID NO: 703 |
| RZ002018 | GmsAmsAmGmCmUmCfAdUfGmAmAmUmAmUmAmUmUmUm_L96 | SEQ ID NO: 866_L96 | AmsAfsAmUmAmUfAmUmUmCmAmUmGmAfGmCfUmUmCmsGmsUm | SEQ ID NO: 710 |
| RZ002019 | CmsCmsUmUmCmGmAfGdGfUmCmAmCmAmGmUmAmAmUm_L96 | SEQ ID NO: 867_L96 | AmsUfsUmAmCmUfGmUmGmAmCmCmUmCfGmAfAmGmGmsGmsGm | SEQ ID NO: 712 |
| RZ002020 | CmsCmsGmGmAmAmGfGdAfAmUmCmAmGmAmAmUmGmAm_L96 | SEQ ID NO: 868_L96 | UmsCfsAmUmUmCfUmGmAmUmUmCmCmUfUmCfCmGmGmsCmsAm | SEQ ID NO: 734 |
| RZ002021 | CmsGmsCmUmGmUmGfCdAfUmUmAmCmCmUmGmGmAmUm_L96 | SEQ ID NO: 869_L96 | AmsUfsCmCmAmGfGmUmAmAmUmGmCmAfCmAfGmCmGmsAmsUm | SEQ ID NO: 739 |
| RZ002022 | CmsUmsUmGmAmAmGfCdCfAmAmCmUmAmCmAmUmGmAm_L96 | SEQ ID NO: 870_L96 | UmsCfsAmUmGmUfAmGmUmUmGmGmCmUfUmCfAmAmGmsGmsAm | SEQ ID NO: 753 |
| RZ002023 | CmsUmsAmCmUmGmCfAdGfCmUmAmAmAmAmGmAmCmUm_L96 | SEQ ID NO: 871_L96 | AmsGfsUmCmUmUfUmUmAmGmCmUmGmCfAmGfUmAmGmsGmsGm | SEQ ID NO: 762 |
| RZ002024 | GmsCmsUmAmAmAmAfGdAfCmUmUmUmGmAmCmUmUmUm_L96 | SEQ ID NO: 872_L96 | AmsAfsAmGmUmCfAmAmAmGmUmCmUmUfUmUfAmGmCmsUmsGm | SEQ ID NO: 764 |

(continued)

| siRNA conjugate No. | sense strand (5'-3') | | antisense strand (5'-3') | |
|---|---|---|---|---|
| RZ002025 | AmsGmsAmAmCmAmCfUdAfUmGmAmUmCmCmUmUmGmAm_L96 | SEQ ID NO: 873_L96 | UmsCfsAmAmGmGfAmUmCmAmUmAmGmUfGmUfUmCmUmsUmsGm | SEQ ID NO: 793 |
| RZ002026 | GmsAmsUmAmCmAmUfCdUfCmCmAmAmGmUmAmUmGmAm_L96 | SEQ ID NO: 874_L96 | UmsCfsAmUmAmCfUmUmGmGmAmGmAmUfGmUfAmUmCmsUmsGm | SEQ ID NO: 803 |
| RZ002027 | GmsGmsAmCmAmAmAfGdCfCmUmUmCmUmCmCmGmAmUm_L96 | SEQ ID NO: 875_L96 | AmsUfsCmGmGmAfGmAmAmGmGmCmUmUfUmGfUmCmCmsAmsGm | SEQ ID NO: 805 |
| RZ002028 | CmsUmsUmUmCmAmAfAdGfUmUmCmAmCmCmAmAmUmAm_L96 | SEQ ID NO: 876_L96 | UmsAfsUmUmGmGfUmGmAmAmCmUmUmUfGmAfAmAmGmsCmsUm | SEQ ID NO: 812 |
| RZ002029 | CmsAmsGmUmCmAmAfGdGfUmCmUmAmCmGmCmCmUmAm_L96 | SEQ ID NO: 877_L96 | UmsAfsGmGmCmGfUmAmGmAmCmCmUmUfGmAfCmUmGmsCmsUm | SEQ ID NO: 813 |
| RZ002030 | GmsUmsCmAmAmGmGfUdCfUmAmCmGmCmCmUmAmUmUm_L96 | SEQ ID NO: 878_L96 | AmsAfsUmAmGmGfCmGmUmAmGmAmCmCfUmUfGmAmCmsUmsGm | SEQ ID NO: 814 |
| RZ002031 | GmsAmsGmAmAmUmUfGdCfUmUmCmAmUmAmCmAmAmAm L96 | SEQ ID NO: 879_L96 | UmsUfsUmGmUmAfUmGmAmAmGmCmAmAfUmUfCmUmCmsCmsUm | SEQ ID NO: 827 |
| RZ002032 | CmsGmsAmGmGmAmGfGdAfCmGmAmAmUmGmCmCmAmAm_L96 | SEQ ID NO: 880_L96 | UmsUfsGmGmCmAfUmUmCmGmUmCmCmUfCmCfUmCmGmsGmsGm | SEQ ID NO: 847 |

As an exemplary explanation, "SEQ ID NO: 849_L96" indicates that the L96 moiety is conjugated to the 3'-end of the sense strand as shown in SEQ ID NO: 849 via a phosphodiester bond.

Table 9: Sequence Information of Murine siRNA Conjugates with an L96 Moiety Conjugated to the 3'-end of the Sense Strand

| siRNA conjugate No. | sense strand (5'-3') | | antisense strand (5'-3') | |
|---|---|---|---|---|
| RZM02003 | AmsGmsAmAmGmAmAfGfAfUfAmUmUmAmUmCmUmCmUm_L96 | SEQ ID NO: 882_L96 | AmsGfsAmGmAmUfAmAmUmAmUmCmUmUfCmUfUmCmUmsGmsGm | SEQ ID NO: 883 |

Table 10: Sequence Information of siRNA Conjugates with an L96 Moiety Conjugated to the 3'-end of the Sense Strand

| siRNA conjugate No. | sense strand (5'-3') | | antisense strand (5'-3') | |
|---|---|---|---|---|
| RZ000001 | UmsUmsCmUmCmCmGfAfAfCmGmUmGmUmCmAmCmGmUm_L96 | SEQ ID NO: 884_L96 | AmsCfsGmUmGmAfCmAmCmGmUmUmCmGfGmAfGmAmAmsCmsUm | SEQ ID NO: 886 |
| RZ000002 | UmsUmsCmUmCmCmGfAdAfCmGmUmGmUmCmAmCmGmUm_L96 | SEQ ID NO: 885_L96 | AmsCfsGmUmGmAfCmAmCmGmUmUmCmGfGmAfGmAmAmsCmsUm | SEQ ID NO: 886 |

**Preparation Example 5: Synthesis of siRNA Conjugate with (CR01008×3) Moiety Conjugated to 3'-end of Sense Strand**

(5.1) Synthesis of sense strand

Using the phosphoramidite solid-phase nucleic acid synthesis method, starting with the aforementioned compound CR01008Z linked to a solid-phase support, nucleoside monomers were linked sequentially in cycles in a 3' to 5' direction according to the nucleotide sequence (compound CR01008 was regarded as a nucleoside monomer).

Each cycle of nucleoside monomer addition included four reaction steps: deprotection, coupling, capping, and oxidation or sulfurization. In the synthesis of the sense strand of this preparation example, the conditions for deprotection, coupling, capping, and oxidation or sulfurization reactions, the conditions for cleavage and deprotection, and the conditions for purification and desalting were the same as those for the synthesis of the sense strand in step (3.1) of Preparation Example 3.

In this step, three clusters of CR01008 were synthesized during the synthesis of the sense strand, denoted as (CR01008)×3 or (CR01008×3).

The structural formula of the three clusters of CR01008 is:

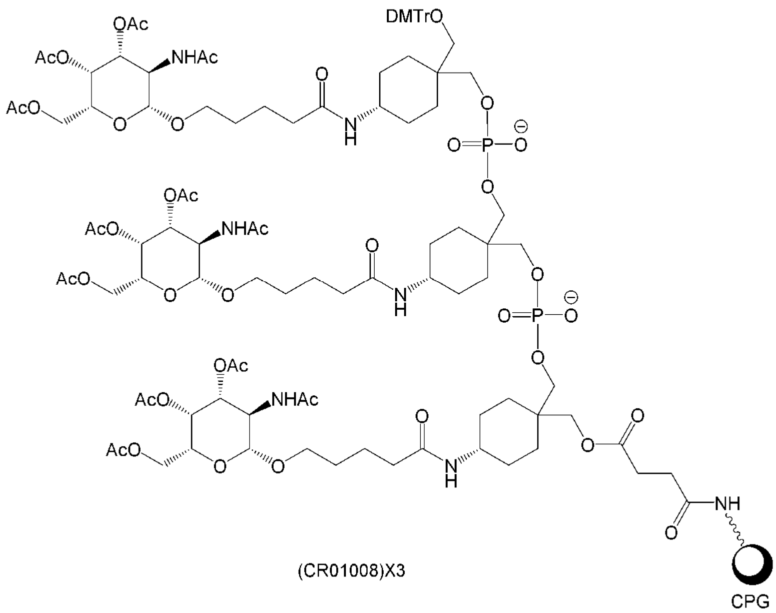

(CR01008)X3

(5.2) Synthesis of antisense strand:

The antisense strands of this preparation example were synthesized according to the antisense strand synthesis method shown in step (3.2) of Preparation Example 3.

(5.3) Synthesis of siRNA conjugate

The siRNA conjugates of this preparation example were synthesized according to the method shown in step (3.3) of Preparation Example 3.

Wherein, when the ligand is the three clusters of CR01008, the structural formula of the siRNA conjugate is:

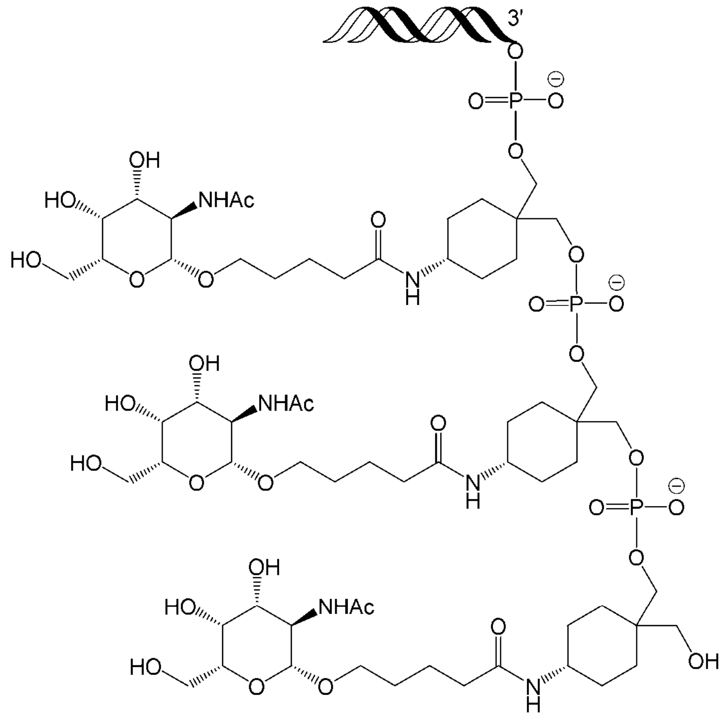

Wherein,

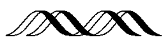

represents siRNA. The (CR01018×3) moiety was conjugated to the 3'-end of the sense strand of the siRNA.

Table 11: Sequence Information of siRNA Conjugates with a (CR01008×3) Moiety Conjugated to the 3'-end of the Sense Strand

| siRNA conjugate No. | sense strand (5'-3') | | antisense strand (5'-3') | |
|---|---|---|---|---|
| RZ002033 | CmsGmsAmAmGmCmUfCfAfUfGmAmAmUmAmUmAmUmUm (CR01008×3) | SEQ ID NO: 887_(CR01008×3) | AmsAfsUmAmUmAfUmUmCfAmUmGmAmGfC(moe)UfUmCmGmsUmsAm | SEQ ID NO: 908 |
| RZ002034 | CmsGmsAmAmGmCmUfCfAfUfGmAmAmUmAmUmAmUmUm (CR01008×3) | SEQ ID NO: 887_(CR01008×3) | AmsAfsUmAmUmAfUmUmCmAfUmGmAmGfC(moe)UfUmCmGmsUmsAm | SEQ ID NO: 909 |
| RZ002036 | CmsGmsAmAmG(moe)CmUfCfAfUfGmAmAmUmAmUmAmUmUm_(CR01008×3) | SEQ ID NO: 888_(CR01008×3) | AmsAfsUmAmUmAfUmUmCmAfUmGmAmGfC(moe)UfUmCmGmsUmsAm | SEQ ID NO: 909 |
| RZ002037 | CmsGmsAmAmGmCmUfCfAfUfGmA(moe)AmUmAmUmAmUmUm_(CR01008×3) | SEQ ID NO: 889_(CR01008×3) | AmsAfsUmAmUmAfUmUmCmAfUmGmAmGfC(moe)UfUmCmGmsUmsAm | SEQ ID NO: 909 |

(continued)

| siRNA conjugate No. | sense strand (5'-3') | | antisense strand (5'-3') | |
|---|---|---|---|---|
| RZ002038 | CmsGmsAmAmGmCmUfCfAfUfGmAmA(moe)UmAmUmAmUmUm_(CR01008×3) | SEQ ID NO: 890(CR01008×3) | AmsAfsUmAmUmAfUmUmCmAfUmGmAmGfC(moe)UfUmCmGmsUmsAm | SEQ ID NO: 909 |
| RZ002039 | CmsGmsAmAmGmCmUfCfAfUfGmAmAmUmAmUmAmT(moe)Um_(CR01008×3) | SEQ ID NO: 891_(CR01008×3) | AmsAfsUmAmUmAfUmUmCmAfUmGmAmGfC(moe)UfUmCmGmsUmsAm | SEQ ID NO: 909 |
| RZ002040 | CmsGmsAmAmG(moe)CmUfCfAfUfGmAmAmUmAmUmAmUmUm_(CR01008×3) | SEQ ID NO: 888_(CR01008×3) | AmsAfsUmAmUmAfUmT(moe)CmAfUmGmAmGfCmUfUmCmGmsUmsAm | SEQ ID NO: 911 |
| RZ002041 | CmsGmsAmAmGmCmUfCfAfUfGmA(moe)AmUmAmUmAmUmUm_(CR01008×3) | SEQ ID NO: 889_(CR01008×3) | AmsAfsUmAmUmAfUmT(moe)CmAfUmGmAmGfCmUfUmCmGmsUmsAm | SEQ ID NO: 911 |
| RZ002042 | CmsGmsAmAmGmCmUfCfAfUfGmAmA(moe)UmAmUmAmUmUm_(CR01008×3) | SEQ ID NO: 890_(CR01008×3) | AmsAfsUmAmUmAfUmT(moe)CmAfUmGmAmGfCmUfUmCmGmsUmsAm | SEQ ID NO: 911 |
| RZ002043 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmT(moe)CmAfUmGmAmGfCmUfUmCmGmsUmsAm | SEQ ID NO: 911 |
| RZ002050 | CmsAmsGmAmGmAmAfAfUfUfCmUmAmCmUmAmCmAmUm (CR01008×3) | SEQ ID NO: 892_(CR01008×3) | AmsUfsGmUmAmGfUmAmGfAmAmUmUmUfC(moe)UfCmUmGmsUmsAm | SEQ ID NO: 912 |
| RZ002051 | CmsAmsGmAmGmAmAfAfUfUfCmUmAmCmUmAmCmAmUm (CR01008×3) | SEQ ID NO: 892_(CR01008×3) | AmsUfsGmUmAmGfUmAmGmAfAmUmUmUfC(moe)UfCmUmGmsUmsAm | SEQ ID NO: 913 |
| RZ002053 | CmsAmsGmAmG(moe)AmAfAfUfUfCmUmAmCmUmAmCmAmUm_(CR01008×3) | SEQ ID NO: 893_(CR01008×3) | AmsUfsGmUmAmGfUmAmGmAfAmUmUmUfC(moe)UfCmUmGmsUmsAm | SEQ ID NO: 913 |
| RZ002054 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmAmGmAfAmUmUmUfC(moe)UfCmUmGmsUmsAm | SEQ ID NO: 913 |
| RZ002055 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmAmGmAfAmUmUmUfC(moe)UfCmUmGmsUmsAm | SEQ ID NO: 913 |
| RZ002056 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmAmGmAfAmUmUmUfC(moe)UfCmUmGmsUmsAm | SEQ ID NO: 913 |
| RZ002057 | CmsAmsGmAmG(moe)AmAfAfUfUfCmUmAmCmUmAmCmAmUm_(CR01008×3) | SEQ ID NO: 893_(CR01008×3) | AmsUfsGmUmAmGfUmA(moe)GmAfAmUmUmUfCmUfCmUmGmsUmsAm | SEQ ID NO: 915 |

(continued)

| siRNA conjugate No. | sense strand (5'-3') | | antisense strand (5'-3') | |
|---|---|---|---|---|
| RZ002058 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmA(moe)GmAfAmUmUmUfCmUfCmUmGmsUmsAm | SEQ ID NO: 915 |
| RZ002059 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmA(moe)GmAfAmUmUmUfCmUfCmUmGmsUmsAm | SEQ ID NO: 915 |
| RZ002060 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmA(moe)GmAfAmUmUmUfCmUfCmUmGmsUmsAm | SEQ ID NO: 915 |
| RZ002066 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAfAmGmCmAmAfT(moe)UfCmUmCmsCmsUm | SEQ ID NO: 916 |
| RZ002067 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAmAfGmCmAmAfT(moe)UfCmUmCmsCmsUm | SEQ ID NO: 917 |
| RZ002068 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAmAmGfCmAmAfT(moe)UfCmUmCmsCmsUm | SEQ ID NO: 918 |
| RZ002069 | GmsAmsGmAmA(moe)UmUfGfCfUfUmCmAmUmAmCmAmAmAm_(CR01008×3) | SEQ ID NO: 898_(CR01008×3) | UmsUfsUmGmUmAfUmGmAmAfGmCmAmAfT(moe)UfCmUmCmsCmsUm | SEQ ID NO: 917 |
| RZ002070 | GmsAmsGmAmAmUmUfGfCfUfUmC(moe)AmUmAmCmAmAmAm_(CR01008×3) | SEQ ID NO: 899_(CR01008×3) | UmsUfsUmGmUmAfUmGmAmAfGmCmAmAfT(moe)UfCmUmCmsCmsUm | SEQ ID NO: 917 |
| RZ002071 | GmsAmsGmAmAmUmUfGfCfUfUmCmA(moe)UmAmCmAmAmAm_(CR01008×3) | SEQ ID NO: 900_(CR01008×3) | UmsUfsUmGmUmAfUmGmAmAfGmCmAmAfT(moe)UfCmUmCmsCmsUm | SEQ ID NO: 917 |
| RZ002072 | GmsAmsGmAmAmUmUfGfCfUfUmCmAmUmAmCmAmA(moe)Am_(CR01008×3) | SEQ ID NO: 901_(CR01008×3) | UmsUfsUmGmUmAfUmGmAmAfGmCmAmAfT(moe)UfCmUmCmsCmsUm | SEQ ID NO: 917 |
| RZ002073 | GmsAmsGmAmA(moe)UmUfGfCfUfUmCmAmUmAmCmAmAmAm_(CR01008×3) | SEQ ID NO: 898_(CR01008×3) | UmsUfsUmGmUmAfUmG(moe)AmAfGmCmAmAfUmUfCmUmCmsCmsUm | SEQ ID NO: 919 |
| RZ002074 | GmsAmsGmAmAmUmUfGfCfUfUmC(moe)AmUmAmCmAmAmAm_(CR01008×3) | SEQ ID NO: 899_(CR01008×3) | UmsUfsUmGmUmAfUmG(moe)AmAfGmCmAmAfUmUfCmUmCmsCmsUm | SEQ ID NO: 919 |
| RZ002075 | GmsAmsGmAmAmUmUfGfCfUfUmCmA(moe)UmAmCmAmAmAm_(CR01008×3) | SEQ ID NO: 900_(CR01008×3) | UmsUfsUmGmUmAfUmG(moe)AmAfGmCmAmAfUmUfCmUmCmsCmsUm | SEQ ID NO: 919 |

(continued)

| siRNA conjugate No. | sense strand (5'-3') | | antisense strand (5'-3') | |
|---|---|---|---|---|
| RZ002076 | GmsAmsGmAmAmUmUfGfCfUfUmCmAmUmAmCmAmA(moe)Am_(CR01008×3) | SEQ ID NO: 901_(CR01008×3) | UmsUfsUmGmUmAfUmG(moe)AmAfGmCmAmAfUmUfCmUmCmsCmsUm | SEQ ID NO: 919 |
| RZ002082 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCfAmGmGmUmGfA(moe)GfUmUmGmsAmsUm | SEQ ID NO: 920 |
| RZ002083 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCmAfGmGmUmGfA(moe)GfUmUmGmsAmsUm | SEQ ID NO: 921 |
| RZ002084 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCmAmGfGmUmGfA(moe)GfUmUmGmsAmsUm | SEQ ID NO: 922 |
| RZ002085 | CmsAmsAmCmT(moe)CmAfCfCfUfGmUmAmAmUmAmAmAmUm (CR01008×3) | SEQ ID NO: 903_(CR01008×3) | AmsUfsUmUmAmUfUmAmCmAfGmGmUmGfA(moe)GfUmUmGmsAmsUm | SEQ ID NO: 921 |
| RZ002086 | CmsAmsAmCmUmCmAfCfCfUfGmT(moe)AmAmUmAmAmAmUm (CR01008×3) | SEQ ID NO: 904_(CR01008×3) | AmsUfsUmUmAmUfUmAmCmAfGmGmUmGfA(moe)GfUmUmGmsAmsUm | SEQ ID NO: 921 |
| RZ002087 | CmsAmsAmCmUmCmAfCfCfUfGmUmA(moe)AmUmAmAmAmUm_(CR01008×3) | SEQ ID NO: 905_(CR01008×3) | AmsUfsUmUmAmUfUmAmCmAfGmGmUmGfA(moe)GfUmUmGmsAmsUm | SEQ ID NO: 921 |
| RZ002088 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmAmCmAfGmGmUmGfA(moe)GfUmUmGmsAmsUm | SEQ ID NO: 921 |
| RZ002089 | CmsAmsAmCmT(moe)CmAfCfCfUfGmUmAmAmUmAmAmAmUm (CR01008×3) | SEQ ID NO: 903_(CR01008×3) | AmsUfsUmUmAmUfUmA(moe)CmAfGmGmUmGfAmGfUmUmGmsAmsUm | SEQ ID NO: 923 |
| RZ002090 | CmsAmsAmCmUmCmAfCfCfUfGmT(moe)AmAmUmAmAmAmUm (CR01008×3) | SEQ ID NO: 904_(CR01008×3) | AmsUfsUmUmAmUfUmA(moe)CmAfGmGmUmGfAmGfUmUmGmsAmsUm | SEQ ID NO: 923 |
| RZ002091 | CmsAmsAmCmUmCmAfCfCfUfGmUmA(moe)AmUmAmAmAmUm_(CR01008×3) | SEQ ID NO: 905_(CR01008×3) | AmsUfsUmUmAmUfUmA(moe)CmAfGmGmUmGfAmGfUmUmGmsAmsUm | SEQ ID NO: 923 |
| RZ002092 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmA(moe)CmAfGmGmUmGfAmGfUmUmGmsAmsUm | SEQ ID NO: 923 |
| RZ002099 | CmsGmsAmAmGmCmUfCfAfUfGmAmAmUmAmUmAmUmUm (CR01008×3) | SEQ ID NO: 887_(CR01008×3) | AmsAfsUmAmUmAfUmUmCmAmUfGmAmGfC(moe)UfUmCmGmsUmsAm | SEQ ID NO: 910 |
| RZ002100 | CmsGmsAmAmG(moe)CmUfCfAfUfGmAmAmUmAmUmAmUmUm_(CR01008×3) | SEQ ID NO: 888_(CR01008×3) | AmsAfsUmAmUmAfUmUmCmAmUfGmAmGfC(moe)UfUmCmGmsUmsAm | SEQ ID NO: 910 |

(continued)

| siRNA conjugate No. | sense strand (5'-3') | | antisense strand (5'-3') | |
|---|---|---|---|---|
| RZ002101 | CmsGmsAmAmGmCmUfCfAfUfGmAmA(moe)UmAmUmAmUmUm_(CR01008×3) | SEQ ID NO: 890_(CR01008×3) | AmsAfsUmAmUmAfUmUmCmAmUfGmAmGfC(moe)UfUmCmGmsUmsAm | SEQ ID NO: 910 |
| RZ002102 | CmsGmsAmAmGmCmUfCfAfUfGmAmA(moe)UmAmUmAmUmUm_(CR01008×3) | SEQ ID NO: 890_(CR01008×3) | AmsAfsUmAmUmAfUmT(moe)CmAmUfGmAmGfCmUfUmCmGmsUmsAm | SEQ ID NO: 924 |
| RZ002103 | CmsGmsAmAmGmCmUfCfAfUfGmAmAmUmAmUmAmT(moe)Um_(CR01008×3) | SEQ ID NO: 891_(CR01008×3) | AmsAfsUmAmUmAfUmT(moe)CmAmUfGmAmGfCmUfUmCmGmsUmsAm | SEQ ID NO: 924 |
| RZ002106 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAfUmUmUfC(moe)UfCmUmGmsUmsAm | SEQ ID NO: 914 |
| RZ002107 | CmsAmsGmAmGmAmAfAfUfUfCmUmA(moe)CmUmAmCmAmUm_(CR01008×3) | SEQ ID NO: 895_(CR01008×3) | AmsUfsGmUmAmGfUmAmGmAmAfUmUmUfC(moe)UfCmUmGmsUmsAm | SEQ ID NO: 914 |
| RZ002108 | CmsAmsGmAmGmAmAfAfUfUfCmUmAmCmUmAmCmA(moe)Um_(CR01008×3) | SEQ ID NO: 896_(CR01008×3) | AmsUfsGmUmAmGfUmA(moe)GmAmAfUmUmUfCmUfCmUmGmsUmsAm | SEQ ID NO: 925 |
| RZ002112 | CmsAmsAmCmUmCmAfCfCfUfGmUmA(moe)AmUmAmAmAmUm_(CR01008×3) | SEQ ID NO: 905_(CR01008×3) | AmsUfsUmUmAmUfUmAmCmAmGfGmUmGfA(moe)GfUmUmGmsAmsUm | SEQ ID NO: 922 |
| RZ002113 | CmsAmsAmCmUmCmAfCfCfUfGmUmAmAmUmAmAmA(moe)Um_(CR01008×3) | SEQ ID NO: 906_(CR01008×3) | AmsUfsUmUmAmUfUmA(moe)CmAmGfGmUmGfAmGfUmUmGmsAmsUm | SEQ ID NO: 926 |
| RZ002115 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfC(moe)UfCmUmGmsUmsAm | SEQ ID NO: 927 |
| RZ002116 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfAmAfUmUmUmsCmsUm | SEQ ID NO: 928 |
| RZ002117 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfA(moe)AfUmUmUmsCmsUm | SEQ ID NO: 929 |
| RZ002118 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfA(moe)AfUmUmUmsCmsUm | SEQ ID NO: 930 |
| RZ002119 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfCmUfUmCmGmsUmsAm | SEQ ID NO: 931 |
| RZ002120 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfCmUfUmCmGmsUmsAm | SEQ ID NO: 932 |

(continued)

| siRNA conjugate No. | sense strand (5'-3') | | antisense strand (5'-3') | |
|---|---|---|---|---|
| RZ002121 | CmsGmsAmAmGmCmUfCfAfUfGmAmAmUmAmUmAmUmUm (CR01008×3) | SEQ ID NO: 887_(CR01008×3) | AmsAfsUmAmUmAfUmUmCmAmUmGfAmGfC(moe)UfUmCmGmsUmsAm | SEQ ID NO: 933 |
| RZ002122 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCfAmGmGmUmGfAmGfUmUmGmsAmsUm | SEQ ID NO: 934 |
| RZ002123 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCmAmGmGfUmGfAmGfUmUmGmsAmsUm | SEQ ID NO: 935 |
| RZ002124 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCmAmGmGfUmGfA(moe)GfUmUmGmsAmsUm | SEQ ID NO: 936 |
| RZ002125 | CmsAmsGmAmGmAmAfAfUfUfCmUmAmCmUmAmCmAmUm (CR01008×3) | SEQ ID NO: 892_(CR01008×3) | AmsUfsGmUmAmGfUmAmGfAmAmUmUmUfCmUfCmUmGmsUmsAm | SEQ ID NO: 937 |
| RZ002126 | CmsAmsGmAmGmAmAfAfUfUfCmUmAmCmUmAmCmAmUm (CR01008×3) | SEQ ID NO: 892_(CR01008×3) | AmsUfsGmUmAmGfUmAmGmAmAmUfUmUfCmUfCmUmGmsUmsAm | SEQ ID NO: 938 |
| RZ002130 | AmsAmsAmUmUmCmUfAfCfUfAmCmAmUmCmUmAmUmAm (CR01008×3) | SEQ ID NO: 907_(CR01008×3) | UmsAfsUmAmGmAfUmGmUfAmGmUmAmGfAmAfUmUmUmsCmsUm | SEQ ID NO: 939 |
| RZ002131 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAfAmGmCmAmAfUmUfCmUmCmsCmsUm | SEQ ID NO: 940 |
| RZ002132 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAmAmGmCfAmAfUmUfCmUmCmsCmsUm | SEQ ID NO: 941 |
| RZ002133 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAfAmGmCmAmAfU(moe)UfCmUmCmsCmsUm | SEQ ID NO: 942 |
| RZ002134 | GmsAmsGmAmAmUmUfGfCfUfUmCmAmUmAmCmAmAmAm (CR01008×3) | SEQ ID NO: 897_(CR01008×3) | UmsUfsUmGmUmAfUmGmAmAmGmCfAmAfU(moe)UfCmUmCmsCmsUm | SEQ ID NO: 943 |

For the table above, as an exemplary explanation, "SEQ ID NO: 858_(CR01008×3)" indicates that the (CR01008×3) moiety is conjugated to the 3'-end of the sense strand as shown in SEQ ID NO: 858.

Biological Assays

Example 1: *In vitro* Activity Assessment of Complement C3 (C3) siRNA

In this example, the inhibitory activity of RX002001 to RX002212 targeting the same C3 target region on the C3 mRNA (Accession No. NM_000064.4) in cells, was assessed using a target gene inhibition activity assay in the liver cancer HepG2 cell line.

Preparation of test sample

After the above siRNA test samples were centrifuged, an appropriate amount of DEPC $H_2O$ was added to each

tube to dissolve the siRNA, and 20 μM stock solutions was prepared. The stock solutions were then further serially diluted with DEPC $H_2O$ into 0.04 μM and 0.004 μM working solutions. Dose screening experiments were performed at final duplex concentrations of 1 nM and 0.1 nM.

96-well transfection and detection:

HepG2 cells grown to near confluence were digested with trypsin, the cells were washed, and a cell suspension was prepared for plating in 96-well plates.

5 μL of 0.4 μM and 0.04 μM working solutions were dispersed in 20 μL of Opti-MEM to form siRNA mixtures. 0.2 μL of RNAiMAX was dispersed in 24.8 μL of Opti-MEM, and after incubating for 5 min, was mixed with each siRNA mixture and incubated for 10 min to form transfection complexes. 150 μL of cell suspension (12,000 cells) was added to each well of a 96-well plate containing the transfection complex, and the 96-well plate was placed in an incubator for continued culture for 24 h.

For the mock control group, 0.2 μL of RNAiMAX was dispersed in 24.8 μL of Opti-MEM, and after incubating for 5 min, 25 μL of Opti-MEM was added, followed by incubation for 10 min to form the transfection complex. 150 μL of cell suspension (12,000 cells) was added to each well of a 96-well plate containing the transfection complex, and the 96-well plate was placed in an incubator for continued culture for 24 h.

The 96-well plate was taken out, the cells were lysed, and the lysate was diluted. A one-step qPCR kit (Thermo Fisher, Power SYBR Green RNA-to-CT$^{TM}$ 1-Step, 4389986) was used to detect the expression level of the target gene mRNA in HepG2 cells on a real-time quantitative PCR system (Roche, LightCycler 480). In this real-time quantitative PCR method, the TATA-box binding protein (TBP) gene was used as an internal reference gene, and primers for the target gene and primers for the TBP internal reference gene were used to detect the target gene and the TBP internal reference gene, respectively. The sequences of the detection primers are shown in Table 12.

Table 12: Sequences of Detection Primers

| Gene | | Primer type | Primer sequence |
|---|---|---|---|
| Target gene | C3 | Forward primer | 5'-AAGCGCATTCCGATTGAGGA-3' (SEQ ID NO: 944) |
| | | Reverse primer | 5'-CCTGAGTGCAAGATGACGGT-3' (SEQ ID NO: 945) |
| Reference gene | TBP | Forward primer | 5'-TGCTCACCCACCAACAATTTAG-3' (SEQ ID NO: 946) |
| | | Reverse primer | 5'-TCTGCTCTGACTTTAGCACCTG-3' (SEQ ID NO: 947) |

In the real-time quantitative PCR method, the ΔΔCt method was used to perform relative quantitative calculations on the expression level and inhibition rate of the target gene mRNA in each test group according to the technical method described above in DETAILED DESCRIPTION.

Table 13: Inhibitory Activity of siRNAs on the Target Gene in HepG2 Cells after Administration of the siRNAs

| Group | 10 nM | | 1 nM | |
|---|---|---|---|---|
| | Relative remaining expression level (%) | STDEV | Relative remaining expression level (%) | STDEV |
| Mock | 100.00 | 6.32 | 100.00 | 6.36 |
| RX002001 | 88.92 | 8.70 | 91.03 | 7.28 |
| RX002002 | 32.08 | 2.72 | 78.55 | 4.62 |
| RX002003 | 97.20 | 2.38 | 96.63 | 0.32 |
| RX002004 | 59.07 | 5.59 | 83.87 | 8.89 |
| RX002005 | 21.55 | 0.46 | 74.79 | 5.74 |
| RX002006 | 21.20 | 0.45 | 70.17 | 0.80 |

(continued)

| Group | 10 nM | | 1 nM | |
|---|---|---|---|---|
| | Relative remaining expression level (%) | STDEV | Relative remaining expression level (%) | STDEV |
| RX002007 | 53.48 | 0.17 | 87.91 | 2.58 |
| RX002008 | 16.50 | 4.86 | 66.24 | 7.99 |
| RX002009 | 47.33 | 4.79 | 85.36 | 4.74 |
| RX002010 | 33.17 | 9.10 | 80.28 | 8.77 |
| RX002011 | 19.57 | 4.56 | 68.58 | 5.15 |
| RX002012 | 36.59 | 10.84 | 83.70 | 7.37 |
| RX002013 | 69.76 | 4.67 | 106.55 | 5.57 |
| RX002014 | 13.88 | 0.59 | 59.50 | 4.56 |
| RX002015 | 59.80 | 15.93 | 96.89 | 4.11 |
| RX002016 | 34.05 | 6.58 | 66.32 | 11.21 |
| RX002017 | 71.24 | 1.86 | 94.47 | 4.32 |
| RX002018 | 40.33 | 8.05 | 77.44 | 3.29 |
| RX002019 | 60.78 | 10.67 | 84.84 | 3.46 |
| RX002020 | 21.11 | 4.79 | 64.86 | 0.11 |
| RX002021 | 17.12 | 5.23 | 73.89 | 12.49 |
| RX002022 | 53.64 | 6.29 | 115.88 | 7.00 |
| RX002023 | 72.78 | 15.69 | 131.80 | 4.09 |
| RX002024 | 16.74 | 1.82 | 90.86 | 16.02 |
| RX002025 | 28.19 | 1.01 | 88.20 | 4.18 |
| RX002026 | 35.77 | 11.82 | 108.83 | 23.81 |
| RX002027 | 85.79 | 13.68 | 131.81 | 29.89 |
| RX002028 | 14.71 | 0.96 | 97.87 | 10.69 |
| RX002029 | 5.33 | 1.17 | 47.14 | 6.11 |
| RX002030 | 8.26 | 3.42 | 42.37 | 9.47 |
| RX002031 | 28.29 | 17.54 | 81.26 | 22.92 |
| RX002032 | 47.25 | 23.91 | 99.86 | 0.49 |
| RX002033 | 40.09 | 2.62 | 99.54 | 10.07 |
| RX002034 | 24.25 | 2.73 | 77.37 | 2.02 |
| RX002035 | 79.47 | 6.23 | 97.35 | 6.36 |
| RX002036 | 86.71 | 7.78 | 104.42 | 36.74 |
| RX002037 | 27.42 | 11.56 | 60.53 | 15.07 |
| RX002038 | 35.63 | 0.17 | 86.48 | 7.20 |
| RX002039 | 109.95 | 4.31 | 126.48 | 6.40 |
| RX002040 | 90.28 | 7.95 | 121.25 | 8.31 |
| RX002041 | 19.33 | 0.38 | 70.01 | 7.48 |
| RX002042 | 63.06 | 8.01 | 95.41 | 11.04 |
| RX002043 | 32.88 | 3.00 | 68.85 | 5.84 |
| RX002044 | 18.42 | 0.42 | 64.59 | 4.74 |

(continued)

| Group | 10 nM | | 1 nM | |
|---|---|---|---|---|
| | Relative remaining expression level (%) | STDEV | Relative remaining expression level (%) | STDEV |
| RX002045 | 15.61 | 5.58 | 68.67 | 12.50 |
| RX002046 | 71.98 | 10.89 | 79.60 | 1.69 |
| RX002047 | 71.96 | 19.85 | 80.73 | 6.32 |
| RX002048 | 28.40 | 6.35 | 70.45 | 8.72 |
| RX002049 | 15.93 | 1.55 | 80.72 | 23.46 |
| RX002050 | 21.72 | 1.08 | 79.15 | 2.59 |
| RX002051 | 9.52 | 4.02 | 47.97 | 19.35 |
| RX002052 | 63.67 | 4.94 | 85.52 | 2.65 |
| RX002053 | 30.14 | 12.56 | 78.96 | 7.73 |
| RX002054 | 81.67 | 20.07 | 100.44 | 8.03 |
| RX002055 | 21.17 | 9.45 | 73.13 | 13.78 |
| RX002056 | 10.46 | 3.33 | 13.82 | 3.77 |
| RX002057 | 71.21 | 7.89 | 76.89 | 1.38 |
| RX002058 | 79.01 | 5.74 | 93.15 | 2.59 |
| RX002059 | 92.56 | 2.80 | 82.44 | 3.64 |
| RX002060 | 26.23 | 0.94 | 72.29 | 6.37 |
| RX002061 | 21.32 | 0.38 | 70.21 | 9.66 |
| RX002062 | 73.05 | 10.94 | 91.82 | 15.08 |
| RX002063 | 43.75 | 1.22 | 85.30 | 1.25 |
| RX002064 | 95.49 | 8.57 | 86.94 | 9.50 |
| RX002065 | 74.28 | 3.03 | 96.80 | 4.74 |
| RX002066 | 70.54 | 6.10 | 86.38 | 13.07 |
| RX002067 | 18.88 | 13.32 | 58.14 | 14.29 |
| RX002068 | 24.77 | 5.04 | 78.19 | 8.80 |
| RX002069 | 64.37 | 5.25 | 98.77 | 10.55 |
| RX002070 | 6.53 | 1.20 | 33.94 | 10.05 |
| RX002071 | 74.34 | 5.22 | 81.50 | 14.17 |
| RX002072 | 83.25 | 13.81 | 83.11 | 1.43 |
| RX002073 | 5.62 | 2.25 | 20.36 | 8.12 |
| RX002074 | 7.28 | 3.89 | 29.56 | 5.24 |
| RX002075 | 86.15 | 4.64 | 93.48 | 3.66 |
| RX002076 | 10.73 | 0.28 | 37.74 | 3.88 |
| RX002077 | 29.35 | 2.49 | 65.25 | 4.69 |
| RX002078 | 83.30 | 4.35 | 91.22 | 2.53 |
| RX002079 | 25.47 | 3.44 | 86.01 | 22.64 |
| RX002080 | 86.76 | 18.29 | 82.78 | 2.03 |
| RX002081 | 29.17 | 6.20 | 66.34 | 0.65 |
| RX002082 | 37.01 | 12.34 | 79.57 | 3.51 |

(continued)

| Group | 10 nM | | 1 nM | |
|---|---|---|---|---|
| | Relative remaining expression level (%) | STDEV | Relative remaining expression level (%) | STDEV |
| RX002083 | 40.56 | 0.53 | 111.30 | 38.65 |
| RX002084 | 7.50 | 2.02 | 29.94 | 1.22 |
| RX002085 | 78.59 | 11.51 | 104.16 | 4.76 |
| RX002086 | 11.51 | 0.11 | 59.01 | 22.83 |
| RX002087 | 26.78 | 5.00 | 72.22 | 5.89 |
| RX002088 | 59.94 | 4.40 | 98.15 | 0.80 |
| RX002089 | 95.37 | 7.01 | 94.88 | 9.52 |
| RX002090 | 92.87 | 2.88 | 94.77 | 3.17 |
| RX002091 | 73.63 | 10.31 | 102.08 | 1.17 |
| RX002092 | 27.46 | 7.01 | 75.38 | 6.52 |
| RX002093 | 82.63 | 0.81 | 99.27 | 6.16 |
| RX002094 | 43.09 | 10.08 | 95.26 | 11.49 |
| RX002095 | 91.65 | 3.22 | 111.33 | 2.91 |
| RX002096 | 61.23 | 3.40 | 89.27 | 5.83 |
| RX002097 | 41.10 | 0.77 | 82.72 | 9.71 |
| RX002098 | 12.61 | 2.35 | 32.95 | 1.29 |
| RX002099 | 43.88 | 11.55 | 72.18 | 1.06 |
| RX002100 | 42.55 | 5.41 | 82.51 | 5.12 |
| RX002101 | 50.21 | 0.16 | 68.39 | 5.58 |
| RX002102 | 40.12 | 3.67 | 88.41 | 11.52 |
| RX002103 | 11.74 | 2.96 | 65.23 | 1.12 |
| RX002104 | 62.66 | 5.52 | 93.37 | 10.96 |
| RX002105 | 23.86 | 1.68 | 89.66 | 5.71 |
| RX002106 | 21.00 | 2.60 | 57.57 | 2.44 |
| RX002107 | 73.21 | 12.09 | 88.31 | 2.67 |
| RX002108 | 16.40 | 0.42 | 79.88 | 14.15 |
| RX002109 | 18.45 | 1.10 | 63.84 | 10.85 |
| RX002110 | 18.44 | 7.13 | 51.16 | 1.34 |
| RX002111 | 77.15 | 4.66 | 68.32 | 3.57 |
| RX002112 | 56.80 | 1.62 | 90.03 | 7.35 |
| RX002113 | 68.05 | 1.89 | 89.83 | 16.63 |
| RX002114 | 90.74 | 12.71 | 92.74 | 6.66 |
| RX002115 | 41.90 | 9.50 | 90.01 | 3.09 |
| RX002116 | 33.60 | 10.84 | 67.39 | 10.85 |
| RX002117 | 14.28 | 1.21 | 45.74 | 1.42 |
| RX002118 | 7.93 | 1.94 | 27.30 | 2.94 |
| RX002119 | 23.35 | 10.29 | 70.12 | 12.14 |
| RX002120 | 65.79 | 5.21 | 75.26 | 5.77 |

(continued)

| Group | 10 nM | | 1 nM | |
|---|---|---|---|---|
| | Relative remaining expression level (%) | STDEV | Relative remaining expression level (%) | STDEV |
| RX002121 | 24.86 | 2.43 | 84.69 | 40.29 |
| RX002122 | 20.41 | 0.37 | 87.12 | 9.23 |
| RX002123 | 14.56 | 1.61 | 83.55 | 33.96 |
| RX002124 | 35.75 | 4.49 | 86.63 | 18.12 |
| RX002125 | 19.33 | 4.28 | 61.15 | 10.24 |
| RX002126 | 14.48 | 1.51 | 37.15 | 21.28 |
| RX002127 | 15.10 | 5.36 | 38.05 | 6.98 |
| RX002128 | 13.02 | 4.30 | 54.98 | 15.68 |
| RX002129 | 72.17 | 14.29 | 95.39 | 20.41 |
| RX002130 | 12.03 | 4.38 | 43.21 | 3.60 |
| RX002131 | 84.28 | 21.12 | 89.93 | 8.66 |
| RX002132 | 77.35 | 16.18 | 94.03 | 24.15 |
| RX002133 | 43.46 | 13.55 | 89.19 | 24.95 |
| RX002134 | 27.71 | 3.21 | 61.83 | 6.85 |
| RX002135 | 37.92 | 5.37 | 83.08 | 15.25 |
| RX002136 | 60.48 | 2.86 | 88.08 | 10.34 |
| RX002137 | 39.71 | 1.88 | 77.74 | 16.51 |
| RX002138 | 20.58 | 1.78 | 52.71 | 6.78 |
| RX002139 | 92.08 | 31.70 | 104.80 | 27.75 |
| RX002140 | 50.22 | 7.36 | 72.04 | 4.70 |
| RX002141 | 72.00 | 8.92 | 112.40 | 10.45 |
| RX002142 | 22.40 | 4.18 | 75.67 | 11.94 |
| RX002143 | 17.15 | 0.13 | 67.91 | 9.95 |
| RX002144 | 18.54 | 3.25 | 65.58 | 3.21 |
| RX002145 | 7.54 | 1.73 | 17.61 | 0.86 |
| RX002146 | 57.03 | 14.65 | 92.90 | 0.30 |
| RX002147 | 66.15 | 12.67 | 99.91 | 6.52 |
| RX002148 | 18.72 | 7.22 | 79.84 | 16.51 |
| RX002149 | 108.94 | 9.95 | 96.51 | 0.47 |
| RX002150 | 15.27 | 0.02 | 52.75 | 13.30 |
| RX002151 | 41.59 | 17.34 | 84.58 | 21.86 |
| RX002152 | 79.40 | 26.60 | 96.65 | 16.34 |
| RX002153 | 108.89 | 22.61 | 117.10 | 4.59 |
| RX002154 | 15.25 | 2.92 | 46.49 | 2.73 |
| RX002155 | 59.28 | 5.90 | 99.20 | 8.90 |
| RX002156 | 11.88 | 4.97 | 41.07 | 3.35 |
| RX002157 | 10.00 | 6.13 | 29.95 | 7.55 |
| RX002158 | 31.99 | 3.18 | 63.43 | 12.66 |

(continued)

| Group | 10 nM | | 1 nM | |
|---|---|---|---|---|
| | Relative remaining expression level (%) | STDEV | Relative remaining expression level (%) | STDEV |
| RX002159 | 15.88 | 2.97 | 48.71 | 1.03 |
| RX002160 | 35.89 | 3.10 | 75.98 | 17.71 |
| RX002161 | 25.16 | 3.56 | 82.42 | 3.23 |
| RX002162 | 27.58 | 6.60 | 68.42 | 1.90 |
| RX002163 | 40.79 | 4.46 | 102.03 | 1.83 |
| RX002164 | 21.86 | 4.29 | 76.61 | 1.13 |
| RX002165 | 10.83 | 2.25 | 37.09 | 2.66 |
| RX002166 | 49.92 | 13.14 | 93.55 | 0.31 |
| RX002167 | 13.42 | 5.35 | 55.49 | 14.43 |
| RX002168 | 71.33 | 2.68 | 93.66 | 0.46 |
| RX002169 | 12.19 | 2.98 | 45.76 | 15.61 |
| RX002170 | 14.00 | 0.75 | 51.64 | 5.64 |
| RX002171 | 92.05 | 0.00 | 95.86 | 9.54 |
| RX002172 | 10.02 | 2.64 | 36.70 | 0.84 |
| RX002173 | 33.36 | 1.25 | 80.30 | 9.03 |
| RX002174 | 69.36 | 12.40 | 92.65 | 21.30 |
| RX002175 | 16.98 | 2.49 | 74.89 | 6.11 |
| RX002176 | 10.83 | 0.88 | 20.50 | 1.94 |
| RX002177 | 9.00 | 0.25 | 31.74 | 2.98 |
| RX002178 | 14.11 | 1.31 | 45.92 | 3.07 |
| RX002179 | 18.55 | 2.96 | 55.38 | 4.07 |
| RX002180 | 102.34 | 16.65 | 106.61 | 7.66 |
| RX002181 | 89.85 | 14.69 | 90.17 | 3.68 |
| RX002182 | 100.82 | 0.99 | 105.62 | 4.14 |
| RX002183 | 65.10 | 2.87 | 90.89 | 12.73 |
| RX002184 | 38.38 | 0.88 | 93.89 | 3.99 |
| RX002185 | 86.78 | 2.84 | 100.14 | 2.62 |
| RX002186 | 25.42 | 8.44 | 62.34 | 5.90 |
| RX002187 | 22.25 | 0.15 | 72.57 | 14.25 |
| RX002188 | 30.98 | 6.83 | 94.67 | 13.72 |
| RX002189 | 72.25 | 3.89 | 109.49 | 8.93 |
| RX002190 | 13.96 | 2.92 | 56.74 | 9.37 |
| RX002191 | 7.54 | 2.97 | 30.24 | 10.27 |
| RX002192 | 101.65 | 12.26 | 105.80 | 8.03 |
| RX002193 | 67.68 | 0.33 | 98.30 | 2.25 |
| RX002194 | 76.28 | 15.10 | 105.19 | 6.18 |
| RX002195 | 36.19 | 7.80 | 96.71 | 0.32 |
| RX002196 | 32.28 | 10.34 | 64.29 | 5.56 |

(continued)

| Group | 10 nM | | 1 nM | |
|---|---|---|---|---|
| | Relative remaining expression level (%) | STDEV | Relative remaining expression level (%) | STDEV |
| RX002197 | 52.12 | 4.85 | 81.45 | 3.06 |
| RX002198 | 47.23 | 8.21 | 74.57 | 8.75 |
| RX002199 | 57.00 | 2.61 | 84.64 | 4.42 |
| RX002200 | 46.22 | 12.82 | 80.84 | 7.52 |
| RX002201 | 87.15 | 9.24 | 104.87 | 5.65 |
| RX002202 | 19.06 | 7.03 | 83.61 | 7.10 |
| RX002203 | 68.75 | 6.73 | 93.46 | 12.48 |
| RX002204 | 78.25 | 2.30 | 107.51 | 7.72 |
| RX002205 | 41.98 | 14.13 | 81.37 | 0.27 |
| RX002206 | 101.99 | 8.65 | 108.83 | 9.59 |
| RX002207 | 92.20 | 6.77 | 113.75 | 7.61 |
| RX002208 | 78.89 | 2.19 | 106.14 | 1.04 |
| RX002209 | 60.79 | 3.28 | 105.51 | 17.33 |
| RX002210 | 52.75 | 11.21 | 101.34 | 0.33 |
| RX002211 | 14.45 | 0.09 | 36.90 | 7.25 |
| RX002212 | 109.84 | 14.32 | 107.61 | 7.20 |

**Example 2: *In vivo* Activity Assessment of siRNA Conjugates with L96 Moiety Conjugated to 3'-end of Sense Strand in BALB/c-HDI Mice**

In this example, a BALB/c mouse hydrodynamic tail vein injection model was used to assess the inhibitory activity of siRNA conjugates RZ002001-RZ0020032, with an L96 moiety conjugated to the 3'-end of the sense strand targeting the same C3 target region on the C3 mRNA, with RZ000001 as a negative control.

Plasmid construction

A pcDNA-CMV-RG002 plasmid (ID: NM_000064.4) was constructed by Sangon Biotech (Shanghai) Co., Ltd.

Mouse model construction

The BALB/c mouse hydrodynamic injection model was established by rapidly injecting a pcDNA-CMV-RG002 plasmid solution into the mice via tail vein under high pressure. On day 3 of the experiment, mice were injected with 10 μg of pcDNA-CMV-RG002 via hydrodynamic tail vein injection within 5 s, with an injection volume equivalent to 8% of the mouse's body weight. The plasmid DNA for injection was diluted with PBS. The solution was prepared before injection and stored at 4°C.

Animal grouping, administration, and tissue sample collection

6 to 7-week-old female BALB/c mice were randomly grouped by body weight, with 5 mice per group. Each test group was administered a predetermined dose of the drug conjugate, and a PBS control group was included. The administration dose for all mice was calculated based on their body weight. A single dose was administered via subcutaneous injection in the abdomen. Each drug conjugate was administered as a 0.1 mg/mL (calculated as siRNA) solution in PBS, with a dosing volume of 10 mL/kg (mouse body weight). That is, the administration dose of each drug conjugate was 1 mg/kg (siRNA/mouse body weight). The PBS control group was administered 10 mL/kg (mouse body weight) of a PBS solution without the siRNA conjugate. The day of administration was recorded as day 0 (D0). Plasmid injection was performed on day 3 (D3) after administration, and all 5 mice in each group were sacrificed on day 4 (D4). Dissection was performed on the sacrificed mice, and the liver tissue from each sacrificed mouse was collected. The liver

tissue was cut into approximately 2 $mm^3$ small pieces and preserved in RNAlater™ solution.

For each mouse, an appropriate amount of liver tissue sample was taken out from RNAlater™ solution. The liver tissue sample was disrupted for 60 s in a Tissuelyser II automatic tissue homogenizer. Then, total RNA was extracted using an automatic nucleic acid extractor (purchased from Zhejiang Hanwei Technology Co., Ltd.) and a nucleic acid extraction kit (purchased from Zhejiang Hanwei Technology Co., Ltd., GO-MNTR-100) according to the standard operating procedure for total RNA extraction.

For each mouse, 1 μg of total RNA was used for reverse-transcription using a reverse transcription kit (Thermo Fisher Scientific, RevertAid First Strand cDNA Synthesis Kit, K1622) with Oligo(dT)18 as the reverse transcription primer. A 20 μL reverse transcription system was prepared, and the reverse transcription reaction was completed according to the instruction manual. After the reaction, 60 μL of RNase-Free water was added to the reverse transcription system to obtain a cDNA solution. Next, the expression level of the target gene mRNA in the animals was detected on a real-time quantitative PCR system (Bio-Rad, CFX Opus 384) using a real-time quantitative PCR kit (Thermo Fisher Scientific, TaqMan Fast Advanced Master Mix, 4444557). In this real-time quantitative PCR method, the Nero gene on the plasmid backbone was used as an internal reference gene, and primers for the target gene and primers for the Nero internal reference gene were used to detect the target gene and the Nero internal reference gene, respectively. The sequences of the detection primers are shown in Table 14.

Table 14: Sequences of Detection Primers

| Gene | | Primer type | Primer sequence | Fluorescen t group |
|---|---|---|---|---|
| Target gene | C3 | Forward primer | 5'-AGTCTCCTGCTTTAGTGATGC-3' (SEQ ID NO: 948) | / |
| | | Reverse primer | 5'-GCCTTTGTTCTCATCTCGCT-3' (SEQ ID NO: 949) | / |
| | | Probe primer | 5'-CTGTTGACCTGCTCCTCGCAAATATCT-3' (SEQ ID NO: 950) | 5'FAM; 3'MGB |
| Reference gene | Nero | Forward primer | 5'-CGTTGGCTACCCGTGATATT-3' (SEQ ID NO: 951) | / |
| | | Reverse primer | 5'-CTCGTCAAGAAGGCGATAGAAG-3' (SEQ ID NO: 952) | / |
| | | Probe primer | 5'-CCGCTTCCTCGTGCTTTACGGTAT-3' (SEQ ID NO: 953) | 5'VIC; 3'MGB |

A 10 μL Real-time PCR reaction system was prepared for each PCR detection well according to the method described in the instruction manual of the real-time quantitative PCR kit. Each reaction system contained 4 μL of the cDNA solution obtained from the reverse transcription reaction described above, 5 μL of TaqMan™ Fast Advanced Master Mix (2×), 0.15 μL of 10 μM forward primer, 0.15 μL of 10 μM reverse primer (for primer information, see Table 8), 0.15 μL of 10 μM probe primer, and 0.55 μL of RNase-Free $H_2O$. The prepared reaction system was placed on a real-time quantitative PCR system (Bio-Rad, CFX Opus 384), and Real-time PCR amplification was performed using a two-step method. The amplification program was: 50°C for 2 min, pre-denaturation at 95°C for 20 s, and then 40 cycles of denaturation at 95°C for 3 s and annealing and extension at 60°C for 30 s. In this real-time quantitative PCR method, the ΔΔCt method was used to perform relative quantitative calculations on the expression level and inhibition rate of the target gene mRNA in each test group according to the technical method described above in DETAILED DESCRIPTION.

The results of Example 2 showed that at a dose of 1 mg/kg, RZ002003, RZ002006, RZ002011, RZ002015, and RZ002031 significantly inhibited the expression of C3 gene mRNA in mouse liver tissue (FIG. 1, Table 15).

Table 15: Inhibitory Activity of siRNA Conjugates on C3 Gene mRNA in Liver Tissue of BALB/c-HDI Mice after Administration of the siRNA Conjugates

| Group | 1 mg/kg | |
|---|---|---|
| | Relative remaining expression level (%) | STDEV |
| PBS | 100.00 | 21.64 |
| RZ000001 | 107.29 | 21.82 |
| RZ002001 | 71.27 | 40.59 |
| RZ002002 | 49.64 | 13.24 |
| RZ002003 | 27.97 | 15.06 |
| RZ002004 | 47.73 | 9.78 |
| RZ002005 | 60.75 | 11.91 |
| RZ002006 | 23.39 | 4.50 |
| RZ002007 | 40.26 | 10.97 |
| RZ002008 | 49.83 | 7.77 |
| RZ002009 | 43.55 | 9.71 |
| RZ002010 | 57.71 | 14.87 |
| RZ002011 | 39.12 | 16.78 |
| RZ002012 | 109.83 | 39.57 |
| RZ002013 | 90.25 | 15.70 |
| RZ002015 | 12.45 | 3.11 |
| RZ002016 | 39.26 | 6.37 |
| RZ002017 | 75.85 | 16.85 |
| RZ002018 | 42.00 | 16.33 |
| RZ002019 | 66.95 | 11.04 |
| RZ002020 | 91.29 | 24.69 |
| RZ002021 | 55.92 | 17.25 |
| RZ002022 | 117.15 | 74.63 |
| RZ002023 | 33.31 | 27.44 |
| RZ002024 | 45.34 | 13.76 |
| RZ002025 | 74.24 | 33.05 |
| RZ002026 | 68.33 | 25.03 |
| RZ002027 | 53.43 | 13.26 |
| RZ002028 | 63.89 | 28.42 |
| RZ002029 | 58.55 | 20.98 |
| RZ002030 | 63.63 | 19.14 |
| RZ002031 | 17.91 | 7.95 |
| RZ002032 | 67.48 | 23.43 |

**Example 3: Toxicological Assessment of siRNA Conjugates with L96 Moiety Conjugated to 3'-end of Sense Strand in ICR Mice**

This example assessed the nature and extent of toxic reactions of L96 moiety conjugates RZ002003, RZ002006, RZ002011, RZ002015, and RZ002031 in ICR mice via a single subcutaneous injection at 300 mg/kg.

Animal grouping, administration, and tissue sample collection

6 to 8-week-old ICR mice (purchased from Beijing Vital River Laboratory Animal Technology Co., Ltd.) were randomly grouped by body weight, with 10 mice per group, half male and half female. Each test group was administered a predetermined dose of the drug conjugate, and a PBS control group was included. The administration dose for all mice was calculated based on their body weight. A single dose was administered via subcutaneous injection in the abdomen. Each drug conjugate was administered as a 30 mg/mL (calculated as siRNA) solution in PBS, with a dosing volume of 10 mL/kg (mouse body weight). That is, the administration dose of each drug conjugate was 300 mg/kg (siRNA/mouse body weight). The PBS control group was administered 10 mL/kg (mouse body weight) of a PBS solution without the siRNA conjugate and observed for 14 days. The day of administration was recorded as day 0 (D0). During the experiment, clinical observations were performed at least once daily after the first administration, and body weight and food consumption were measured twice a week. On day 14 (D14) of the experiment, blood was collected from all animals (mice were fasted for no less than 12 h before sampling and detection, with free access to water) for hematology and blood biochemistry tests. Collected blood was anticoagulated with EDTA-K2, and a complete blood count was performed using an automatic modular hematology and body fluid analyzer (Sysmex XN-10B1). Blood samples (without anticoagulant) were collected and left at room temperature for about 30 min. After blood coagulation, the samples were centrifuged at 2000 g for 10 min at 4°C, and blood biochemistry was tested using a fully automatic biochemical analyzer (Sysmex BX-4000). On day 14 (D14) of the experiment, all animals were desected. Before desection, the mice to be desected were fasted for at least 12 h with free access to water, anesthetized with Zoletil 50 (Virbac, France, 8G4VA), and then euthanized by exsanguination from the abdominal aorta after blood collection, followed by gross necropsy observation. Histopathological examinations were performed on the livers and kidneys of all animals, as shown in FIG. 2 (using hematoxylin-eosin staining). The results showed that each test sample had no significant effect on the general condition, body weight, food consumption, and gross necropsy observations of the ICR mice. Compared to the PBS group, at a dose of 300 mg/kg (siRNA/mouse body weight), no significant abnormalities were observed in the hematology and blood biochemistry of animals in the RZ002003, RZ002006, RZ002011, and RZ002015 groups; no significant abnormalities were observed in the hematology of animals in the RZ002031 group, with slight elevations in ALT and AST.

### Example 4: *In vitro* Activity Assessment of siRNA Conjugates with (CR01008)×3 Moiety Conjugated to 3'-end of Sense Strand

In this example, the inhibitory activity of (CR01008)×3 moiety siRNA conjugates on the target gene C3 mRNA in cells was assessed using a target gene inhibition activity assay in the human liver cancer HepG2 cell line.

Preparation of test samples

After each of the above siRNA test samples was centrifuged, an appropriate amount of PBS was added to each tube to dissolve the siRNA, and 20 μM stock solutions were prepared. The stock solutions were then further serially diluted with PBS into 0.5 μM and 0.05 μM working solutions. Dose experiments were performed at final duplex concentrations of 5 nM and 0.5 nM.

96-well transfection and detection

HepG2 cells grown to near confluence were digested with trypsin, the cells were washed to prepare a cell suspension, and 100 μL of the cell suspension, containing 12,000 cells, was added to each well of a 96-well plate and cultured in an incubator at 37°C with 5% $CO_2$. After the cells had adhered for 24 h, the DMEM medium in the 96-well plate was aspirated, 80 μL of Opti-MEM™ medium was added to each well, and the 96-well plate was then placed back in the incubator for continued culture. 1 μL of 0.1 μM and 0.01 μM working solutions were dispersed in 9 μL of Opti-MEM to form siRNA mixtures, and 0.3 μL of RNAiMAX was dispersed in 9.7 μL of Opti-MEM and mixed with each siRNA mixture to form transfection complexes. The transfection complexes were incubated at room temperature for 10 min and then added to the 96-well plate at 20 μL/well. After culturing for 4 h, 100 μL of DMEM medium containing 20% FBS was added to each well, and the 96-well plate was placed in the incubator for continued culture for 24 h. For the Mock control group: 0.3 μL of RNAiMAX was dispersed in 9.7 μL of Opti-MEM, and then 10 μL of Opti-MEM was added, followed by incubation at room temperature for 10 min; it was then added to the 96-well plate at 20 μL/well. After culturing for 4 h, 100 μL of DMEM medium containing 20% FBS was added to each well, and the 96-well plate was placed in the incubator for continued culture for 24 h.

The 96-well plate was taken out, and total RNA was extracted using an automatic nucleic acid extractor (purchased from Zhejiang Hanwei Technology Co., Ltd.) and a nucleic acid extraction kit (purchased from Zhejiang Hanwei Technology Co., Ltd., GO-MNTR-100) according to the standard operating procedure for total RNA extraction.

A reverse transcription kit (Thermo Fisher Scientific, RevertAid First Strand cDNA Synthesis Kit, K1622) was used with Oligo(dT)18 as the reverse transcription primer. A 20 μL reverse transcription system was prepared, and the reverse transcription reaction was completed according to the method described in the instruction manual. Next, a real-time quantitative PCR kit (Thermo Fisher Scientific, TaqMan Fast Advanced Master Mix, 4444557) was used to detect the expression level of the target gene mRNA in HepG2 cells on a real-time quantitative PCR system (Bio-Rad, CFX Opus 384). In this real-time quantitative PCR method, the glyceraldehyde-3-phosphate dehydrogenase (GAPDH) gene was used as an internal reference gene, and primers for the target gene and primers for the GAPDH internal reference gene were used to detect the target gene and the GAPDH internal reference gene, respectively. The sequences of the detection primers are shown in Table 16.

Table 16: Sequences of Detection Primers

| Gene | | Primer type | Primer sequence | Fluorescent group |
|---|---|---|---|---|
| Target gene | C3 | Forward primer | 5'-AGTCTCCTGCTTTAGTGATGC-3' (SEQ ID NO: 948) | / |
| | | Reverse primer | 5'-GCCTTTGTTCTCATCTCGCT-3' (SEQ ID NO: 949) | / |
| | | Probe primer | 5'-CTGTTGACCTGCTCCTCGCAAATATCT-3' (SEQ ID NO: 951) | 5'FAM; 3'MGB |
| Reference gene | GAPDH | Forward primer | 5'-AAGAAGGTGGTGAAGCAGG-3' (SEQ ID NO: 954) | / |
| | | Reverse primer | 5'-CAAAGTGGTCGTTGAGGG-3' (SEQ ID NO: 955) | / |
| | | Probe primer | 5'-CAACAGCGACACCCACTC-3' (SEQ ID NO: 956) | 5'VIC; 3'MGB |

A 10 μL Real-time PCR reaction system was prepared for each PCR detection well according to the method described in the instruction manual of the real-time quantitative PCR kit. Each reaction system contained 4 μL of the cDNA solution obtained from the reverse transcription reaction described above, 5 μL of TaqMan™ Fast Advanced Master Mix (2×), 0.15 μL of 10 μM forward primer, 0.15 μL of 10 μM reverse primer (for primer information, see Table 14), 0.15 μL of 10 μM probe primer, and 0.55 μL of RNase-Free $H_2O$. The prepared reaction system was placed on a real-time quantitative PCR system (Bio-Rad, CFX Opus 384), and Real-time PCR amplification was performed using a two-step method. The amplification program was: 50°C for 2 min, followed by pre-denaturation at 95°C for 20 s, and then 40 cycles of denaturation at 95°C for 3 s and annealing and extension at 60°C for 30 s. In this real-time quantitative PCR method, the ΔΔCt method was used to perform relative quantitative calculations on the expression level and inhibition rate of the target gene mRNA in each test group according to the technical method described above in DETAILED DESCRIPTION.

Table 17: Inhibitory Activity of siRNA Conjugates on the Target Gene C3 mRNA in HepG2 Cells After Administration of the siRNA Conjugates

| Name | 5 nM | | 0.5 nM | |
|---|---|---|---|---|
| | Relative remaining expression level (%) | STDEV | Relative remaining expression level (%) | STDEV |
| Mock | 100.00 | 3.12 | 100.00 | 3.12 |

(continued)

| Name | 5 nM | | 0.5 nM | |
|---|---|---|---|---|
| | Relative remaining expression level (%) | STDEV | Relative remaining expression level (%) | STDEV |
| RZ002033 | 9.16 | 0.94 | 31.05 | 0.71 |
| RZ002034 | 8.80 | 0.76 | 24.48 | 6.22 |
| RZ002099 | 7.65 | 1.92 | 32.10 | 1.97 |
| RZ002036 | 11.98 | 0.18 | 24.00 | 1.03 |
| RZ002037 | 11.26 | 0.61 | 27.13 | 0.60 |
| RZ002038 | 9.04 | 1.21 | 30.71 | 0.51 |
| RZ002039 | 10.98 | 0.37 | 36.07 | 1.88 |
| RZ002040 | 12.62 | 0.59 | 31.73 | 1.40 |
| RZ002041 | 10.48 | 1.47 | 27.68 | 6.13 |
| RZ002042 | 9.07 | 0.71 | 26.13 | 1.61 |
| RZ002043 | 8.04 | 0.71 | 30.68 | 0.16 |
| RZ002050 | 17.65 | 2.29 | 36.54 | 2.38 |
| RZ002051 | 16.27 | 2.17 | 27.43 | 6.32 |
| RZ002106 | 13.92 | 1.18 | 30.03 | 0.72 |
| RZ002053 | 14.88 | 4.01 | 26.00 | 0.90 |
| RZ002054 | 17.8 | 10.42 | 28.19 | 2.16 |
| RZ002055 | 15.57 | 2.71 | 24.53 | 2.19 |
| RZ002056 | 16.48 | 2.71 | 43.19 | 1.83 |
| RZ002057 | 17.52 | 3.31 | 40.75 | 5.49 |
| RZ002058 | 14.75 | 0.11 | 31.72 | 1.35 |
| RZ002059 | 11.28 | 1.47 | 26.63 | 4.85 |
| RZ002060 | 10.28 | 1.48 | 25.93 | 0.70 |
| RZ002066 | 20.09 | 9.16 | 42.86 | 15.61 |
| RZ002067 | 16.89 | 4.88 | 27.44 | 1.50 |
| RZ002068 | 15.99 | 9.04 | 38.27 | 2.66 |
| RZ002069 | 18.44 | 5.73 | 31.22 | 2.43 |
| RZ002070 | 16.77 | 8.10 | 34.34 | 1.93 |
| RZ002071 | 16.24 | 5.08 | 32.74 | 1.91 |
| RZ002072 | 24.64 | 4.76 | 53.87 | 6.33 |
| RZ002073 | 34.06 | 3.25 | 51.24 | 0.29 |
| RZ002074 | 14.91 | 0.16 | 44.37 | 1.31 |
| RZ002075 | 10.69 | 0.15 | 41.05 | 4.82 |
| RZ002076 | 10.76 | 1.08 | 41.10 | 1.94 |
| RZ002082 | 21.93 | 5.23 | 28.44 | 2.52 |
| RZ002083 | 19.48 | 8.68 | 25.39 | 1.18 |
| RZ002084 | 15.71 | 8.17 | 27.85 | 2.26 |
| RZ002085 | 23.27 | 5.75 | 33.20 | 2.55 |
| RZ002086 | 21.12 | 8.65 | 34.10 | 6.23 |

(continued)

| Name | 5 nM | | 0.5 nM | |
|---|---|---|---|---|
| | Relative remaining expression level (%) | STDEV | Relative remaining expression level (%) | STDEV |
| RZ002087 | 21.77 | 0.85 | 36.53 | 3.15 |
| RZ002088 | 34.48 | 0.91 | 69.65 | 6.92 |
| RZ002089 | 31.49 | 5.73 | 54.13 | 8.64 |
| RZ002090 | 14.91 | 0.03 | 51.77 | 15.52 |
| RZ002091 | 11.73 | 0.65 | 36.02 | 0.00 |
| RZ002092 | 11.67 | 1.28 | 37.72 | 0.36 |

The results of Example 4 showed that at doses of 5 nM and 0.5 nM, different siRNA conjugates modified with CR01008 significantly inhibited C3 gene mRNA expression in HepG2 cells (Table 17).

**Example 5: Assessment of mRNA Knockdown Effect of siRNA Conjugates with (CR01008)×3 Moiety Conjugated to 3'-end of Sense Strand in Normal *Macaca fascicularis***

This example assessed the inhibitory activity of RZ002099, RZ002101, RZ002106, and RZ002113 on the target gene C3 mRNA in *Macaca fascicularis*.

Animal grouping, administration, and tissue sample collection

Healthy *Macaca fascicularis* weighing 3 to 5 kg were grouped, with 4 *Macaca fascicularis* per group, half male and half female. Each test group was administered a predetermined dose of the drug conjugate, and a PBS control group was included. The administration dose for all animals was calculated based on their body weight. A single dose was administered via subcutaneous injection in the abdomen. Each drug conjugate was administered as a 9 mg/mL (calculated as siRNA) solution in PBS, with a dosing volume of 1 mL/kg (*Macaca fascicularis* body weight). That is, the administration dose of each drug conjugate was 9 mg/kg (siRNA/*Macaca fascicularis* body weight). The PBS control group was administered 1 mL/kg (*Macaca fascicularis* body weight) of a PBS solution without the siRNA conjugate. The day of administration was recorded as day 0 (D0). On day 14 (D14) after administration, liver biopsies were performed on the *Macaca fascicularis* in each group, and the liver biopsy tissues were immediately preserved in RNAlater$^{TM}$ solution.

For each *Macaca fascicularis*, a liver tissue sample was taken from RNAlater$^{TM}$ solution. The liver tissue sample was disrupted for 60 s in a Tissuelyser II automatic tissue homogenizer. Then, total RNA was extracted using an automatic nucleic acid extractor (purchased from Zhejiang Hanwei Technology Co., Ltd.) and a nucleic acid extraction kit (purchased from Zhejiang Hanwei Technology Co., Ltd., GO-MNTR-100) according to the standard operating procedure for total RNA extraction.

For each *Macaca fascicularis*, 1 μg of total RNA was taken for reverse-transcription using a reverse transcription kit (Thermo Fisher Scientific, RevertAid First Strand cDNA Synthesis Kit, K1622) with Oligo(dT)18 as the reverse transcription primer. A 20 μL reverse transcription system was prepared, and the reverse transcription reaction was completed according to the method described in the instruction manual of the reverse transcription kit. After the reaction, 60 μL of RNase-Free water was added to the reverse transcription system to obtain a cDNA solution. Next, the expression level of the target gene mRNA in the animals was detected on a real-time quantitative PCR system (Roche, Lightcycler 480II) using a real-time quantitative PCR kit (Thermo Fisher Scientific, TaqMan Fast Advanced Master Mix, 4444557). In this real-time quantitative PCR method, the glyceraldehyde-3-phosphate dehydrogenase (GAPDH) gene was used as an internal reference gene, and primers for the target gene and primers for the GAPDH internal reference gene were used to detect the target gene and the GAPDH internal reference gene, respectively. The sequences of the detection primers are shown in Table 18.

Table 18: Sequences of Detection Primers

| Gene | | Primer type | Primer sequence | Fluorescent group |
|---|---|---|---|---|
| Target gene | C3 | Forward primer | 5'-TCCTGCGATCAGAAGAGACC-3' (SEQ ID NO: 957) | / |
| | | Reverse primer | 5'-GACCTTTGGCCTTAGCATGG-3' (SEQ ID NO: 958) | / |
| | | Probe primer | 5'-ACCGACAAGGTGCCTTGGCCT-3' (SEQ ID NO: 959) | 5'FAM; 3'MGB |
| Reference gene | GAPDH | Forward primer | 5'-TCGTGGAAGGACTCATGACC-3' (SEQ ID NO: 960) | / |
| | | Reverse primer | 5'-GCAGGGATGATGTTCTGGAG-3' (SEQ ID NO: 961) | / |
| | | Probe primer | 5'-CTCCGCGGCCATCACGCCAC-3' (SEQ ID NO: 962) | 5'VIC; 3'MGB |

A 10 μL Real-time PCR reaction system was prepared for each PCR detection well according to the method described in the instruction manual of the real-time quantitative PCR kit. Each reaction system contained 4 μL of the cDNA solution obtained from the reverse transcription reaction described above, 5 μL of TaqMan$^{TM}$ Fast Advanced Master Mix (2×), 0.15 μL of 10 μM forward primer, 0.15 μL of 10 μM reverse primer (for primer information, see Table 16), 0.15 μL of 10 μM probe primer, and 0.55 μL of RNase-Free $H_2O$. The prepared reaction system was placed on a real-time quantitative PCR system (Bio-Rad, CFX Opus 384), and Real-time PCR amplification was performed using a two-step method. The amplification program was: 50°C for 2 min, followed by pre-denaturation at 95°C for 20 s, and then 40 cycles of denaturation at 95°C for 3 s and annealing and extension at 60°C for 30 s. In this real-time quantitative PCR method, the ΔΔCt method was used to perform relative quantitative calculations on the expression level and inhibition rate of the target gene mRNA in each test group according to the technical method described above in DETAILED DESCRIPTION.

The results of Example 5 showed that at a single dose of 9 mg/kg, RZ002099, RZ002101, RZ002106, and RZ002113 significantly inhibited C3 gene mRNA expression (Table 19, FIG. 4).

Table 19: Change of C3 mRNA Level in *Macaca fascicularis* Liver After Administration of the siRNA Conjugates

| Group | C3 mRNA level (9 mg/kg, D14) | |
|---|---|---|
| | Relative remaining expression level (%) | STDEV |
| PBS | 100.00 | 28.24 |
| RZ002099 | 15.51 | 5.60 |
| RZ002101 | 22.29 | 12.07 |
| RZ002106 | 9.86 | 1.44 |
| RZ002113 | 12.96 | 6.94 |

**Example 6: Assessment of C3 Protein Reduction Caused by siRNA Conjugates with (CR01008)×3 Moiety at 3'-end of Sense Strand in Normal *Macaca fascicularis***

This example assessed the expression of C3 protein in the serum of *Macaca fascicularis* at different time points after a single administration of (CR01008)×3 moiety conjugates RZ002099, RZ002101, RZ002106, and RZ002113 using an ELISA method.

Animal grouping, administration, and tissue sample collection

Healthy *Macaca fascicularis* weighing 3 to 5 kg were grouped according to serum C3 protein levels, with 4 *Macaca fascicularis* per group, half male and half female. Each test group was administered a predetermined dose of the drug conjugate, and a PBS control group was included. The administration dose for all animals was calculated based on their body weight. A single dose was administered via subcutaneous injection in the abdomen. Each drug conjugate was administered as a 9 mg/mL (calculated as siRNA) solution in PBS, with a dosing volume of 1 mL/kg (*Macaca fascicularis* body weight). That is, the administration dose of each drug conjugate was 9 mg/kg (siRNA/*Macaca fascicularis* body weight). The PBS control group was administered 1 mL/kg (*Macaca fascicularis* body weight) of a PBS solution without the siRNA conjugate. *Macaca fascicularis* serum was collected before administration (recorded as pre-dose), on the day of administration recorded as day 0 (D0), and on day 7 (D7), day 14 (D14), day 21 (D21), day 28 (D28), day 35 (D35), day 42 (D42), day 49 (D49), day 56 (D56), day 63 (D63), day 70 (D70), day 77 (D77), day 84 (D84), day 91 (D91), day 98 (D98), and day 105 (D105) after administration. C3 protein expression was measured using a human complement C3 ELISA kit (Hycult Biotech, HK366-01).

The results of Example 6 showed that at a single dose of 9 mg/kg, RZ002099, RZ002101, RZ002106, and RZ002113 all significantly reduced the C3 protein level in *Macaca fascicularis* serum. Among them, the C3 protein reduction caused by RZ002106 reached up to 90%, with an 80% protein reduction still observed at D70, and the protein level had not returned to the pre-dose level by D105 (FIG. 5, Table 20).

Table 20: Change of C3 Protein Level in *Macaca fascicularis* Serum after Administration of the siRNA Conjugates

| Group | pre-dose | | D7 | | D14 | | D21 | |
|---|---|---|---|---|---|---|---|---|
| | Relative remaining expression level (%) | STDEV | Relative remaining expression level (%) | STDEV | Relative remaining expression level (%) | STDEV | Relative remaining expression level (%) | STDEV |
| PBS | 100.00 | 0.00 | 100.20 | 6.80 | 95.70 | 14.00 | 104.10 | 18.10 |
| RZ002099 | 100.00 | 0.00 | 48.57 | 12.93 | 27.63 | 11.82 | 30.95 | 14.81 |
| RZ002101 | 100.00 | 0.00 | 47.87 | 15.67 | 26.08 | 8.71 | 22.75 | 10.48 |
| RZ002106 | 100.00 | 0.00 | 44.31 | 6.21 | 21.03 | 5.24 | 11.88 | 3.48 |
| RZ002113 | 100.00 | 0.00 | 41.55 | 13.82 | 21.54 | 9.85 | 17.12 | 8.27 |

| Group | D28 | | D35 | | D42 | | D49 | |
|---|---|---|---|---|---|---|---|---|
| | Relative remaining expression level (%) | STDEV | Relative remaining expression level (%) | STDEV | Relative remaining expression level (%) | STDEV | Relative remaining expression level (%) | STDEV |
| PBS | 82.90 | 10.00 | 79.30 | 7.90 | 80.60 | 4.50 | 88.10 | 8.40 |
| RZ002099 | 24.25 | 11.70 | 26.75 | 8.80 | 30.29 | 15.16 | 37.84 | 24.22 |
| RZ002101 | 19.11 | 6.12 | 20.99 | 4.49 | 23.77 | 6.44 | 21.76 | 4.33 |
| RZ002106 | 13.34 | 6.63 | 12.15 | 4.03 | 11.87 | 4.67 | 9.01 | 1.95 |
| RZ002113 | 16.91 | 5.24 | 15.70 | 5.49 | 15.73 | 5.48 | 14.45 | 5.65 |

(continued)

| Group | D56 | | D63 | | D70 | | D77 | |
|---|---|---|---|---|---|---|---|---|
| | Relative remaining expression level (%) | STDEV | Relative remaining expression level (%) | STDEV | Relative remaining expression level (%) | STDEV | Relative remaining expression level (%) | STDEV |
| PBS | 79.41 | 7.84 | 84.87 | 17.19 | 87.71 | 13.88 | 103.71 | 10.45 |
| RZ002099 | 34.34 | 26.33 | / | / | / | / | / | / |
| RZ002101 | 25.18 | 3.66 | / | / | / | / | / | / |
| RZ002106 | 9.51 | 2.59 | 15.41 | 5.99 | 15.17 | 4.63 | 24.45 | 8.90 |
| RZ002113 | 19.25 | 8.15 | / | / | / | / | / | / |
| | | | | | | | | |
| Group | D84 | | D91 | | D98 | | D105 | |
| | Relative remaining expression level (%) | STDEV | Relative remaining expression level (%) | STDEV | Relative remaining expression level (%) | STDEV | Relative remaining expression level (%) | STDEV |
| PBS | 99.52 | 15.57 | 91.57 | 3.00 | 95.00 | 1.80 | 117.08 | 9.46 |
| RZ002099 | / | / | / | / | / | / | / | / |
| RZ002101 | / | / | / | / | / | / | / | / |
| RZ002106 | 38.93 | 16.88 | 34.23 | 13.01 | 36.06 | 14.85 | 36.61 | 13.59 |
| RZ002113 | / | / | / | / | / | / | / | / |

**Example 7: Effect of siRNA Conjugates with (CR01008)×3 Conjugated to 3'-end of Sense Strand on Complement Pathway Activity in Normal *Macaca fascicularis***

This example evaluated the complement activity in the serum of *Macaca fascicularis* at different time points after a single administration of (CR01008)×3 (i.e., (CR01008×3) ligand) conjugates RZ002099, RZ002101, RZ002106, and RZ002113.

Animal grouping, administration, and tissue sample collection

Healthy *Macaca fascicularis* weighing 3 to 5 kg were grouped, with 4 *Macaca fascicularis* per group, half male and half female. Each test group was administered a predetermined dose of the drug conjugate, and a PBS control group was included. The administration dose for all animals was calculated based on their body weight. A single dose was administered via subcutaneous injection in the abdomen. Each drug conjugate was administered as a 9 mg/mL (calculated as siRNA) solution in PBS, with a dosing volume of 1 mL/kg (*Macaca fascicularis* body weight). That is, the administration dose of each drug conjugate was 9 mg/kg (siRNA/*Macaca fascicularis* body weight). The PBS control group was administered 1 mL/kg (*Macaca fascicularis* body weight) of a PBS solution without the siRNA conjugate. *Macaca fascicularis* serum in all groups was collected before administration (recorded as pre-dose), on the day of administration recorded as day 0 (D0), and on day 7 (D7), day 14 (D14), day 21 (D21), day 28 (D28), day 35 (D35), day 42 (D42), day 49 (D49), day 56 (D56), day 63 (D63), day 70 (D70), day 77 (D77), day 84 (D84), day 91 (D91), day 98 (D98), and day 105 (D105) after administration. The activities of the complement system alternative pathway (CAP) and the complement system classical pathway (CCP) were determined using the WIESLAB® Complement System Alternative Pathway Kit (COMPL AP330 RUO, IBL America) and the Complement System Classical Pathway Kit (COMPL CP310 RUO, IBL America).

The results of Example 7 showed that at a single dose of 9 mg/kg, RZ002099, RZ002101, RZ002106, and RZ002113 all significantly inhibited the complement alternative pathway in the serum of *Macaca fascicularis*, but had no significant effect on the complement classical pathway in the serum (FIG. 6, FIG. 7, Table 21, Table 22).

Table 21: Change of Serum CAP Activity in *Macaca fascicularis* after Administration of the siRNA Conjugates

| Group | pre-dose | | D7 | | D14 | | D21 | |
|---|---|---|---|---|---|---|---|---|
| | Relative remaining activity (%) | STDEV | Relative remaining activity (%) | STDEV | Relative remaining activity (%) | STDEV | Relative remaining activity (%) | STDEV |
| PBS | 100.00 | 0.00 | 88.36 | 21.09 | 101.24 | 11.59 | 92.70 | 4.88 |
| RZ002099 | 100.00 | 0.00 | 50.06 | 38.25 | 37.05 | 44.75 | 53.62 | 38.25 |
| RZ002101 | 100.00 | 0.00 | 70.48 | 21.06 | 48.03 | 13.21 | 35.31 | 13.64 |
| RZ002106 | 100.00 | 0.00 | 70.73 | 32.47 | 20.20 | 13.96 | 9.87 | 8.83 |
| RZ002113 | 100.00 | 0.00 | 74.86 | 27.76 | 32.66 | 34.63 | 27.77 | 27.59 |
| | | | | | | | | |
| Group | D28 | | D35 | | D42 | | D49 | |
| | Relative remaining activity (%) | STDEV | Relative remaining activity (%) | STDEV | Relative remaining activity (%) | STDEV | Relative remaining activity (%) | STDEV |
| PBS | 83.52 | 15.70 | 88.55 | 8.63 | 93.52 | 6.11 | 85.74 | 6.29 |
| RZ002099 | 33.90 | 48.51 | 65.90 | 33.90 | 44.33 | 42.25 | 53.30 | 42.55 |
| RZ002101 | 21.65 | 17.68 | 38.20 | 22.71 | 35.92 | 17.57 | 46.36 | 15.37 |
| RZ002106 | 10.69 | 11.51 | 4.69 | 4.04 | 7.55 | 10.01 | 9.04 | 10.40 |
| RZ002113 | 22.33 | 26.74 | 21.20 | 26.92 | 18.09 | 22.65 | 37.16 | 37.55 |
| | | | | | | | | |
| Group | D56 | | D63 | | D70 | | D77 | |
| | Relative remaining activity (%) | STDEV | Relative remaining activity (%) | STDEV | Relative remaining activity (%) | STDEV | Relative remaining activity (%) | STDEV |
| PBS | 87.26 | 12.82 | 84.05 | 13.23 | 85.94 | 19.26 | 90.42 | 10.01 |
| RZ002099 | 65.11 | 27.63 | / | / | / | / | / | / |
| RZ002101 | 58.12 | 17.72 | / | / | / | / | / | / |
| RZ002106 | 8.63 | 10.62 | 30.58 | 30.75 | 31.67 | 29.63 | 66.68 | 38.17 |
| RZ002113 | 48.77 | 33.16 | / | / | / | / | / | / |
| | | | | | | | | |
| Group | D84 | | D91 | | D98 | | D105 | |
| | Relative remaining activity (%) | STDEV | Relative remaining activity (%) | STDEV | Relative remaining activity (%) | STDEV | Relative remaining activity (%) | STDEV |
| PBS | 82.42 | 17.05 | 77.47 | 18.50 | 82.48 | 12.97 | 91.80 | 10.47 |
| RZ002099 | / | / | / | / | / | / | / | / |
| RZ002101 | / | / | / | / | / | / | / | / |
| RZ002106 | 59.91 | 37.62 | 63.49 | 32.68 | 74.24 | 36.30 | 72.33 | 31.73 |
| RZ002113 | / | / | / | / | / | / | / | / |

Table 22: Change of Serum CCP Activity in *Macaca fascicularis* after Administration of the siRNA Conjugates

| Group | pre-dose | | D7 | | D21 | | D28 | | D35 | | D42 | |
|---|---|---|---|---|---|---|---|---|---|---|---|---|
| | Relati ve remain ing activit y (%) | STD EV | Relati ve remain ing activit y (%) | STD EV | Relati ve remain ing activit y (%) | STD EV | Relati ve remain ing activit y (%) | STD EV | Relati ve remain ing activit y (%) | STD EV | Relati ve remain ing activit y (%) | STD EV |
| PBS | 100.00 | 0.00 | 104.01 | 11.16 | 99.79 | 7.36 | 104.11 | 16.2 3 | 97.08 | 6.62 | 91.57 | 5.24 |
| RZ002 099 | 100.00 | 0.00 | 113.28 | 16.7 0 | 108.76 | 10.8 0 | 109.49 | 21.9 2 | 103.35 | 3.85 | 91.43 | 13.7 6 |
| RZ002 101 | 100.00 | 0.00 | 112.86 | 9.29 | 93.61 | 6.07 | 108.28 | 8.23 | 87.59 | 6.20 | 86.39 | 2.74 |
| RZ002 106 | 100.00 | 0.00 | 102.62 | 8.50 | 89.24 | 6.88 | 91.60 | 5.93 | 81.16 | 3.79 | 79.86 | 4.17 |
| RZ002 113 | 100.00 | 0.00 | 107.08 | 6.66 | 92.17 | 7.22 | 104.28 | 9.25 | 101.74 | 11.14 | 98.14 | 3.99 |

### Example 8: *In Vivo* Efficacy Evaluation of siRNA Conjugates with L96 Ligand Conjugated to 3'-end of the Sense Strand in CFA-hIgA Mice

In this embodiment, an IgA nephropathy mouse model was constructed by inducing IgA-humanized mice with Complete Freund's Adjuvant (CFA). The inhibitory effect of RZM02003 on mouse C3 mRNA in CFA-IgA mice was detected by a QPCR method; the expression level of mouse C3 protein in serum at different time points was determined by an enzyme-linked immunosorbent assay (ELISA); the deposition of mouse C3 protein and human IgA immune protein in kidney tissue was observed by an immunofluorescence method; and kidney tissue lesions were observed by a Periodic Acid-Schiff (PAS) staining method.

Animal grouping, administration, and tissue sample collection

The CFA-hIgA model mice were randomly grouped, with 3 mice in each group. Each test group was administered a predetermined dose of a drug conjugate, and a PBS control group was included. For all mice, the drug dose was calculated based on body weight, and the administration volume was 10 mL/kg (mouse body weight). A single dose of RZM02003 was administered via subcutaneous injection in the abdomen. Each drug conjugate was administered as a 0.9 mg/mL (calculated as siRNA) solution in PBS. That is, the administration dose of the drug conjugate was 9 mg/kg (siRNA/mouse body weight). The PBS control group (Model PBS Control Group) was administered with a PBS solution without an siRNA conjugate at 10 mL/kg (mouse body weight).

Before administration, the upper pole of kidney tissue of each mouse after modeling was collected by an open surgical method. A portion of the kidney tissue was fixed in a 10% formalin solution, embedded in paraffin, and sectioned for PAS staining to observe pre-administration kidney tissue lesions; another portion of the kidney tissue was embedded and fixed with OCT (optimal cutting temperature compound) and then cryosectioned for immunofluorescence staining of kidney tissue to observe pre-administration mouse C3 deposition and human IgA deposition in the kidney. The day of administration was recorded as day 0 (D0). On day 14 (D14) after administration, 3 mice from all groups were euthanized, and on day 28 (D28) after administration, 3 mice from all groups were euthanized. Serum from all euthanized mice was collected, and the C3 protein expression in the serum was detected using a mouse complement C3 ELISA kit (abcam, 157711). Dissection was performed on the euthanized mice, respectively. The liver tissue of each euthanized mouse was collected and cut into small pieces of about 2 $mm^3$ and stored in RNAlater. The kidney tissue of each euthanized mouse was collected. A portion of the kidney tissue was fixed in a 10% formalin solution, embedded in paraffin, and sectioned for PAS staining to observe kidney tissue lesions on D14 and D28 after administration; another portion of the kidney tissue was embedded and fixed with OCT, and then cryosectioned for immunofluorescence staining of kidney tissue to observe mouse C3 deposition and human IgA deposition in the kidney on D14 and D28 after administration.

The results of Example 8 showed that at a single dose of 9 mg/kg, RZM02003 significantly reduced the C3 mRNA and protein levels in CFA-IgA mice. On day 14 after administration, the reduction of C3 mRNA and protein reached 98% (FIG. 8, Table 23) and 78% (FIG. 9, Table 24), respectively. On day 28 after administration, the reduction of C3 mRNA was still greater than 98% (FIG. 8, Table 23), and the reduction of C3 protein was about 70% (FIG. 9, Table 24).

The results of Example 8 showed that at a single dose of 9 mg/kg, RZM02003 could reduce C3 deposition in kidney tissue, and a significant reduction in C3 fluorescence staining in kidney tissue was observed on both day 14 and day 28 after administration (FIG. 10).

Table 23: Inhibitory Activity of siRNA Conjugates on C3 mRNA in the Liver of CFA-hIgA Mice after Administration of the siRNA Conjugates

| Group | C3 mRNA | |
|---|---|---|
| | Relative remaining expression level (%) | STDEV |
| Model PBS (D14) | 100.00 | 15.63 |
| RZM02003 (D14) | 1.11 | 1.05 |
| Model PBS (D28) | 100.00 | 8.55 |
| RZM02003 (D28) | 1.67 | 0.38 |

Table 24: Change of Serum C3 Protein Level in CFA-hIgA Mice after Administration of the siRNA Conjugate

| Group | Serum C3 protein | |
|---|---|---|
| | Relative remaining expression level (%) | STDEV |
| Model PBS (D14) | 100.00 | 22.24 |
| RZM02003 (D14) | 21.80 | 4.17 |
| Model PBS (D28) | 100.00 | 29.70 |
| RZM02003 (D28) | 30.14 | 10.12 |

The double-stranded oligonucleotide for inhibiting C3 gene expression, the conjugate thereof, and uses thereof provided by the present disclosure have been described in detail above. Specific examples have been used herein to illustrate the principles and embodiments of the present disclosure, and the description of the above examples is only for the purpose of helping to understand the method of the present disclosure and its core idea. It should be pointed out that, for a person skilled in the art, several improvements and modifications can be made to the present disclosure without departing from the principles of the present disclosure, and these improvements and modifications also fall within the protection scope of the claims of the present disclosure.

## Claims

**1.** A double-stranded oligonucleotide for inhibiting expression of C3 gene, comprising a sense strand and an antisense strand;

from 5'-end to 3'-end, nucleotides of positions 2 to 19 of the antisense strand comprise a complementary region to a C3 RNA transcript, wherein the complementary region comprises at least 17 contiguous nucleotides of any one of the nucleotide sequences as set forth in SEQ ID NOs: 213 to 424 or a nucleotide sequence having 1, 2 or 3 nucleotide differences from the at least 17 contiguous nucleotides; and

the sense strand comprises at least 17 nucleotides, and the sense strand and the antisense strand are complementary or substantially complementary to form a duplex region, wherein being substantial complementary means that the sense strand and the antisense strand have no more than 3 nucleotide mismatches in the duplex region.

**2.** The double-stranded oligonucleotide according to claim 1, wherein the antisense strand comprises a nucleotide sequence having 0, 1, 2 or 3 nucleotide differences from any one of the sequences as set forth in SEQ ID NOs: 213 to 424;

optionally, the antisense strand comprises a nucleotide sequence having 0 or 1 nucleotide difference from any one of the sequences as set forth in SEQ ID NOs: 213 to 424; and

optionally, the antisense strand is selected from or comprises any one of the nucleotide sequences as set forth in SEQ ID NOs: 213 to 424.

**3.** The double-stranded oligonucleotide according to claim 1, wherein the sense strand comprises at least 17 contiguous nucleotides of any one of the nucleotide sequences as set forth in SEQ ID NOs: 1 to 212 or a nucleotide sequence having 1, 2 or 3 nucleotide differences from the at least 17 contiguous nucleotides;

optionally, the sense strand comprises a nucleotide sequence having 0, 1, 2 or 3 nucleotide differences from any one of the sequences as set forth in SEQ ID NOs: 1 to 212;

optionally, the sense strand comprises a nucleotide sequence having 0 or 1 nucleotide difference from any one of the nucleotide sequences as set forth in SEQ ID NOs: 1 to 212; and

optionally, the sense strand is selected from or comprises any one of the nucleotide sequences as set forth in SEQ ID NOs: 1 to 212.

**4.** The double-stranded oligonucleotide according to claim 3, wherein the antisense strand comprises a nucleotide sequence having 0 or 1 nucleotide difference from any one of the nucleotide sequences as set forth in SEQ ID NOs: 241, 242, 285, 357 or 403;

optionally, the antisense strand comprises any one of the nucleotide sequences as set forth in SEQ ID NOs: 241, 242, 285, 357 or 403;
optionally, the sense strand comprises a nucleotide sequence having 0 or 1 nucleotide difference from any one of the nucleotide sequences as set forth in SEQ ID NOs: 29, 30, 73, 145 or 191;
optionally, the sense strand comprises any one of the nucleotide sequences as set forth in SEQ ID NOs: 29, 30, 73, 145 or 191;
optionally, the double-stranded oligonucleotide comprises one or more pairs of:

an antisense strand having a nucleotide sequence as set forth in SEQ ID NO: 241 or a nucleotide sequence having 0 or 1 nucleotide difference therefrom, and a sense strand having a nucleotide sequence as set forth in SEQ ID NO: 29 or a nucleotide sequence having 0 or 1 nucleotide difference therefrom;
an antisense strand having a nucleotide sequence as set forth in SEQ ID NO: 242 or a nucleotide sequence having 0 or 1 nucleotide difference therefrom, and a sense strand having a nucleotide sequence as set forth in SEQ ID NO: 30 or a nucleotide sequence having 0 or 1 nucleotide difference therefrom;
an antisense strand having a nucleotide sequence as set forth in SEQ ID NO: 285 or a nucleotide sequence having 0 or 1 nucleotide difference therefrom, and a sense strand having a nucleotide sequence as set forth in SEQ ID NO: 73 or a nucleotide sequence having 0 or 1 nucleotide difference therefrom;
an antisense strand having a nucleotide sequence as set forth in SEQ ID NO: 357 or a nucleotide sequence having 0 or 1 nucleotide difference therefrom, and a sense strand having a nucleotide sequence as set forth in SEQ ID NO: 145 or a nucleotide sequence having 0 or 1 nucleotide difference therefrom; and
an antisense strand having a nucleotide sequence as set forth in SEQ ID NO: 403 or a nucleotide sequence having 0 or 1 nucleotide difference therefrom, and a sense strand having a nucleotide sequence as set forth in SEQ ID NO: 191 or a nucleotide sequence having 0 or 1 nucleotide difference therefrom; and
optionally, the double-stranded oligonucleotide comprises:
an antisense strand having a nucleotide sequence as set forth in SEQ ID NO: 241 or a nucleotide sequence having 0 or 1 nucleotide difference therefrom, and a sense strand having a nucleotide sequence as set forth in SEQ ID NO: 29 or a nucleotide sequence having 0 or 1 nucleotide difference therefrom.

**5.** The double-stranded oligonucleotide according to any one of claims 1 to 4, wherein each nucleotide of the double-stranded oligonucleotide is independently selected from a modified or unmodified nucleotide;

optionally, substantially all nucleotides of the double-stranded oligonucleotide are independently selected from modified nucleotides;
optionally, all nucleotides of the double-stranded oligonucleotide are independently selected from modified nucleotides;
optionally, each nucleotide of the double-stranded oligonucleotide is independently selected from the group consisting of:
a 2'-halogen-modified nucleotide, a 2'-deoxy-modified nucleotide, a 2'-O-optionally substituted $C_{1-6}$ alkyl-modified nucleotide, a 2'-O-$(CH_2)_n$-O-$R_1$-modified nucleotide, a 2'-amino-modified nucleotide, an abasic nucleotide or a nucleotide analog, wherein the nucleotide analog is one or more selected from PNA, MNA, BNA, LNA, GNA, TNA and UNA;
wherein n is selected from 1 or 2, $R_1$ is selected from an optionally substituted $C_{1-6}$ alkyl or an optionally substituted $C_{1-6}$ alkoxy; when $R_1$ is substituted, the substituent is selected from a halogen, a $C_{1-6}$ alkoxy, a hydroxyl and an amino;
optionally, the 2'-halogen-modified nucleotide is a 2'-fluoro-modified nucleotide;
optionally, the 2'-O-optionally substituted $C_{1-6}$ alkyl-modified nucleotide is a 2'-O-methyl-modified nucleotide;
optionally, the 2'-O-$(CH_2)_n$-O-$R_1$-modified nucleotide is selected from a 2'-O-methoxyethyl-modified nucleotide, a 2'-O-ethoxymethyl-modified nucleotide and a 2'-O-2,2,2-trifluoroethoxymethyl-modified nucleotide;
optionally, the 2'-O-$(CH_2)_n$-O-$R_1$-modified nucleotide is selected from a 2'-O-methoxyethyl-modified nucleotide;
optionally, each nucleotide of the double-stranded oligonucleotide is independently selected from the group consisting of:

a 2'-fluoro-modified nucleotide, a 2'-deoxy-modified nucleotide, a 2'-O-methyl-modified nucleotide and a 2'-O-methoxyethyl-modified nucleotide; and
optionally, each nucleotide of the double-stranded oligonucleotide is independently selected from the group consisting of:
a 2'-fluoro-modified nucleotide, a 2'-O-methyl-modified nucleotide and a 2'-O-methoxyethyl-modified nucleotide.

**6.** The double-stranded oligonucleotide according to claim 5, wherein the double-stranded oligonucleotide comprises at least one 2'-O-methoxyethyl-modified nucleotide;

optionally, the antisense strand comprises at least one 2'-O-methoxyethyl-modified nucleotide; and the sense strand comprises no more than one 2'-O-methoxyethyl-modified nucleotide;
optionally, from 5'-end to 3'-end, in the nucleotide sequence of the sense strand, at least 3 nucleotides at positions 7 to 10 are selected from a 2'-fluoro-modified nucleotide, no more than one nucleotide at positions 5, 12, 13, and 18 is selected from a 2'-O-methoxyethyl-modified nucleotide, and the remaining nucleotides are independently selected from a 2'-O-methyl-modified nucleotide; in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, at least one nucleotide at positions 8 and 15 is selected from a 2'-O-methoxyethyl-modified nucleotide, and the remaining nucleotides are independently selected from a 2'-O-methyl-modified nucleotide;
optionally, from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, at least one nucleotide at positions 8 and 15 is selected from a 2'-O-methoxyethyl-modified nucleotide, and the remaining nucleotides are independently selected from a 2'-O-methyl-modified nucleotide;
optionally, from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and in the nucleotide sequence of the antisense strand, at least 4 nucleotides at positions 2, 6, 14, and 16 are selected from a 2'-fluoro-modified nucleotide, any one of the nucleotides at positions 9 to 12 is selected from a 2'-fluoro-modified nucleotide, the nucleotide at position 8 or 15 is selected from a 2'-O-methoxyethyl-modified nucleotide, and the remaining nucleotides are independently selected from a 2'-O-methyl-modified nucleotide;
optionally, the sense strand and the antisense strand of the double-stranded oligonucleotide have a modification pattern selected from (ds-1) to (ds-8):

(ds-1) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 9, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;
(ds-2) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 10, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;
(ds-3) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 11, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;
(ds-4) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 12, 14, and 16 are selected from a 2'-fluoro-modified nucleotide, the nucleotide at position 8 is selected from a 2'-O-methoxyethyl-modified nucleotide, and the remaining nucleotides are selected from a 2'-O-methyl-modified nucleotide;

(ds-5) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 9, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;
(ds-6) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 10, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide;
(ds-7) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 11, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide; and
(ds-8) from 5'-end to 3'-end, in the nucleotide sequence of the sense strand, the nucleotides at positions 7 to 10 are selected from a 2'-fluoro-modified nucleotide, and the remaining nucleotides are selected from a 2'-O-methyl-modified nucleotide; and
in the nucleotide sequence of the antisense strand, the nucleotides at positions 2, 6, 12, 14, and 16 are selected from a 2'-fluoro-modified nucleotide, the nucleotide at position 15 is selected from a 2'-O-methoxyethyl-modified nucleotide, and the remaining nucleotides are selected from a 2'-O-methyl-modified nucleotide; and
optionally, the sense strand comprises two contiguous internucleotide phosphorothioate linkages at 5'-end; and
the antisense strand comprises two internucleotide phosphorothioate linkages at 5'-end, and two contiguous internucleotide phosphorothioate linkages at 3'-end.

**7.** The double-stranded oligonucleotide according to claim 5, wherein each nucleotide of the double-stranded oligonucleotide is independently selected from a modified nucleotide;
wherein the sense strand is selected from any one of the sequences as set forth in SEQ ID NOs: 887 to 907; and/or the antisense strand is selected from any one of the sequences as set forth in SEQ ID NOs: 908 to 943.

**8.** The double-stranded oligonucleotide according to claim 7, wherein the double-stranded oligonucleotide comprises one or more pairs of:

➽Too large Table

| Pair | Sense strand (5'-3') | | Antisense strand (5'-3') | |
|---|---|---|---|---|
| RX002 245 | CmsGmsAmAmGmCmUfCfAfUfGmAmAmUmAmUmAmUmUm | SEQ ID NO: 887 | AmsAfsUmAmUmAfUmUmCfAmUmGmAmGfC(moe)UfUmCmGmsUmsAm | SEQ ID NO: 908 |
| RX002 246 | CmsGmsAmAmGmCmUfCfAfUfGmAmAmUmAmUmAmUmUm | SEQ ID NO: 887 | AmsAfsUmAmUmAfUmUmCmAfUmGmAmGfC(moe)UfUmCmGmsUmsAm | SEQ ID NO: 909 |
| RX002 247 | CmsGmsAmAmGmCmUfCfAfUfGmAmAmUmAmUmAmUmUm | SEQ ID NO: 887 | AmsAfsUmAmUmAfUmUmCmAmUfGmAmGfC(moe)UfUmCmGmsUmsAm | SEQ ID NO: 910 |

(continued)

| Pair | Sense strand (5'-3') | | Antisense strand (5'-3') | |
|---|---|---|---|---|
| RX002 248 | CmsGmsAmAmG(moe)CmUfCfAfUfGmAmAmUmAmUmAmUmUm | SEQ ID NO: 888 | AmsAfsUmAmUmAfUmUmCmAfUmGmAmGfC(moe)UfUmCmGmsUmsAm | SEQ ID NO: 909 |
| RX002 249 | CmsGmsAmAmGmCmUfCfAfUfGmA(moe)AmUmAmUmAmUmUm | SEQ ID NO: 889 | AmsAfsUmAmUmAfUmUmCmAfUmGmAmGfC(moe)UfUmCmGmsUmsAm | SEQ ID NO: 909 |
| RX002 250 | CmsGmsAmAmGmCmUfCfAfUfGmAmA(moe)UmAmUmAmUmUm | SEQ ID NO: 890 | AmsAfsUmAmUmAfUmUmCmAfUmGmAmGfC(moe)UfUmCmGmsUmsAm | SEQ ID NO: 909 |
| RX002 251 | CmsGmsAmAmGmCmUfCfAfUfGmAmAmUmAmUmAmT(moe)Um | SEQ ID NO: 891 | AmsAfsUmAmUmAfUmUmCmAfUmGmAmGfC(moe)UfUmCmGmsUmsAm | SEQ ID NO: 909 |
| RX002 252 | CmsGmsAmAmG(moe)CmUfCfAfUfGmAmAmUmAmUmAmUmUm | SEQ ID NO: 888 | AmsAfsUmAmUmAfUmT(moe)CmAfUmGmAmGfCmUfUmCmGmsUmsAm | SEQ ID NO: 911 |
| RX002 253 | CmsGmsAmAmGmCmUfCfAfUfGmA(moe)AmUmAmUmAmUmUm | SEQ ID NO: 889 | AmsAfsUmAmUmAfUmT(moe)CmAfUmGmAmGfCmUfUmCmGmsUmsAm | SEQ ID NO: 911 |
| RX002 254 | CmsGmsAmAmGmCmUfCfAfUfGmAmA(moe)UmAmUmAmUmUm | SEQ ID NO: 890 | AmsAfsUmAmUmAfUmT(moe)CmAfUmGmAmGfCmUfUmCmGmsUmsAm | SEQ ID NO: 911 |
| RX002 255 | CmsGmsAmAmGmCmUfCfAfUfGmAmAmUmAmUmAmT(moe)Um | SEQ ID NO: 891 | AmsAfsUmAmUmAfUmT(moe)CmAfUmGmAmGfCmUfUmCmGmsUmsAm | SEQ ID NO: 911 |
| RX002 256 | CmsAmsGmAmGmAmAfAfUfUfCmUmAmCmUmAmCmAmUm | SEQ ID NO: 892 | AmsUfsGmUmAmGfUmAmGfAmAmUmUmUfC(moe)UfCmUmGmsUmsAm | SEQ ID NO: 912 |
| RX002 257 | CmsAmsGmAmGmAmAfAfUfUfCmUmAmCmUmAmCmAmUm | SEQ ID NO: 892 | AmsUfsGmUmAmGfUmAmGmAfAmUmUmUfC(moe)UfCmUmGmsUmsAm | SEQ ID NO: 913 |
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |
| RX002 259 | CmsAmsGmAmG(moe)AmAfAfUfUfCmUmAmCmUmAmCmAmUm | SEQ ID NO: 893 | AmsUfsGmUmAmGfUmAmGmAfAmUmUmUfC(moe)UfCmUmGmsUmsAm | SEQ ID NO: 913 |

(continued)

| Pair | Sense strand (5'-3') | | Antisense strand (5'-3') | |
|---|---|---|---|---|
| RX002 260 | CmsAmsGmAmGmAmAfAfUfUfCmT(moe)AmCmUmAmCmAmUm | SEQ ID NO: 894 | AmsUfsGmUmAmGfUmAmGmAfAmUmUmUfC(moe)UfCmUmGmsUmsAm | SEQ ID NO: 913 |
| RX002 261 | CmsAmsGmAmGmAmAfAfUfUfCmUmA(moe)CmUmAmCmAmUm | SEQ ID NO: 895 | AmsUfsGmUmAmGfUmAmGmAfAmUmUmUfC(moe)UfCmUmGmsUmsAm | SEQ ID NO: 913 |
| RX002 262 | CmsAmsGmAmGmAmAfAfUfUfCmUmAmCmUmAmCmA(moe)Um | SEQ ID NO: 896 | AmsUfsGmUmAmGfUmAmGmAfAmUmUmUfC(moe)UfCmUmGmsUmsAm | SEQ ID NO: 913 |
| RX002 263 | CmsAmsGmAmG(moe)AmAfAfUfUfCmUmAmCmUmAmCmAmUm | SEQ ID NO: 893 | AmsUfsGmUmAmGfUmA(moe)GmAfAmUmUmUfCmUfCmUmGmsUmsAm | SEQ ID NO: 915 |
| RX002 264 | CmsAmsGmAmGmAmAfAfUfUfCmT(moe)AmCmUmAmCmAmUm | SEQ ID NO: 894 | AmsUfsGmUmAmGfUmA(moe)GmAfAmUmUmUfCmUfCmUmGmsUmsAm | SEQ ID NO: 915 |
| RX002 265 | CmsAmsGmAmGmAmAfAfUfUfCmUmA(moe)CmUmAmCmAmUm | SEQ ID NO: 895 | AmsUfsGmUmAmGfUmA(moe)GmAfAmUmUmUfCmUfCmUmGmsUmsAm | SEQ ID NO: 915 |
| RX002 266 | CmsAmsGmAmGmAmAfAfUfUfCmUmAmCmUmAmCmA(moe)Um | SEQ ID NO: 896 | AmsUfsGmUmAmGfUmA(moe)GmAfAmUmUmUfCmUfCmUmGmsUmsAm | SEQ ID NO: 915 |
| RX002 267 | GmsAmsGmAmAmUmUfGfCfUfUmCmAmUmAmCmAmAmAm | SEQ ID NO: 897 | UmsUfsUmGmUmAfUmGmAfAmGmCmAmAfT(moe)UfCmUmCmsCmsUm | SEQ ID NO: 916 |
| RX002 268 | GmsAmsGmAmAmUmUfGfCfUfUmCmAmUmAmCmAmAmAm | SEQ ID NO: 897 | UmsUfsUmGmUmAfUmGmAmAfGmCmAmAfT(moe)UfCmUmCmsCmsUm | SEQ ID NO: 917 |
| RX002 269 | GmsAmsGmAmAmUmUfGfCfUfUmCmAmUmAmCmAmAmAm | SEQ ID NO: 897 | UmsUfsUmGmUmAfUmGmAmAmGfCmAmAfT(moe)UfCmUmCmsCmsUm | SEQ ID NO: 918 |
| RX002 270 | GmsAmsGmAmA(moe)UmUfGfCfUfUmCmAmUmAmCmAmAmAm | SEQ ID NO: 898 | UmsUfsUmGmUmAfUmGmAmAfGmCmAmAfT(moe)UfCmUmCmsCmsUm | SEQ ID NO: 917 |
| RX002 271 | GmsAmsGmAmAmUmUfGfCfUfUmC(moe)AmUmAmCmAmAmAm | SEQ ID NO: 899 | UmsUfsUmGmUmAfUmGmAmAfGmCmAmAfT(moe)UfCmUmCmsCmsUm | SEQ ID NO: 917 |

(continued)

| Pair | Sense strand (5'-3') | | Antisense strand (5'-3') | |
|---|---|---|---|---|
| RX002 272 | GmsAmsGmAmAmUmUfGfCfUfUmCmA(moe)UmAmCmAmAmAm | SEQ ID NO: 900 | UmsUfsUmGmUmAfUmGmAmAfGmCmAmAfT(moe)UfCmUmCmsCmsUm | SEQ ID NO: 917 |
| RX002 273 | GmsAmsGmAmAmUmUfGfCfUfUmCmAmUmAmCmAmA(moe)Am | SEQ ID NO: 901 | UmsUfsUmGmUmAfUmGmAmAfGmCmAmAfT(moe)UfCmUmCmsCmsUm | SEQ ID NO: 917 |
| RX002 274 | GmsAmsGmAmA(moe)UmUfGfCfUfUmCmAmUmAmCmAmAmAm | SEQ ID NO: 898 | UmsUfsUmGmUmAfUmG(moe)AmAfGmCmAmAfUmUfCmUmCmsCmsUm | SEQ ID NO: 919 |
| RX002 275 | GmsAmsGmAmAmUmUfGfCfUfUmC(moe)AmUmAmCmAmAmAm | SEQ ID NO: 899 | UmsUfsUmGmUmAfUmG(moe)AmAfGmCmAmAfUmUfCmUmCmsCmsUm | SEQ ID NO: 919 |
| RX002 276 | GmsAmsGmAmAmUmUfGfCfUfUmCmA(moe)UmAmCmAmAmAm | SEQ ID NO: 900 | UmsUfsUmGmUmAfUmG(moe)AmAfGmCmAmAfUmUfCmUmCmsCmsUm | SEQ ID NO: 919 |
| RX002 277 | GmsAmsGmAmAmUmUfGfCfUfUmCmAmUmAmCmAmA(moe)Am | SEQ ID NO: 901 | UmsUfsUmGmUmAfUmG(moe)AmAfGmCmAmAfUmUfCmUmCmsCmsUm | SEQ ID NO: 919 |
| RX002 278 | CmsAmsAmCmUmCmAfCfCfUfGmUmAmAmUmAmAmAmUm | SEQ ID NO: 902 | AmsUfsUmUmAmUfUmAmCfAmGmGmUmGfA(moe)GfUmUmGmsAmsUm | SEQ ID NO: 920 |
| RX002 279 | CmsAmsAmCmUmCmAfCfCfUfGmUmAmAmUmAmAmAmUm | SEQ ID NO: 902 | AmsUfsUmUmAmUfUmAmCmAfGmGmUmGfA(moe)GfUmUmGmsAmsUm | SEQ ID NO: 921 |
| RX002 280 | CmsAmsAmCmUmCmAfCfCfUfGmUmAmAmUmAmAmAmUm | SEQ ID NO: 902 | AmsUfsUmUmAmUfUmAmCmAmGfGmUmGfA(moe)GfUmUmGmsAmsUm | SEQ ID NO: 922 |
| RX002 281 | CmsAmsAmCmT(moe)CmAfCfCfUfGmUmAmAmUmAmAmAmUm | SEQ ID NO: 903 | AmsUfsUmUmAmUfUmAmCmAfGmGmUmGfA(moe)GfUmUmGmsAmsUm | SEQ ID NO: 921 |
| RX002 282 | CmsAmsAmCmUmCmAfCfCfUfGmT(moe)AmAmUmAmAmAmUm | SEQ ID NO: 904 | AmsUfsUmUmAmUfUmAmCmAfGmGmUmGfA(moe)GfUmUmGmsAmsUm | SEQ ID NO: 921 |
| RX002 283 | CmsAmsAmCmUmCmAfCfCfUfGmUmA(moe)AmUmAmAmAmUm | SEQ ID NO: 905 | AmsUfsUmUmAmUfUmAmCmAfGmGmUmGfA(moe)GfUmUmGmsAmsUm | SEQ ID NO: 921 |

(continued)

| Pair | Sense strand (5'-3') | | Antisense strand (5'-3') | |
|---|---|---|---|---|
| RX002 284 | CmsAmsAmCmUmCmAfCfCfUfGmUmAmAmUmAmAmA(moe)Um | SEQ ID NO: 906 | AmsUfsUmUmAmUfUmAmCmAfGmGmUmGfA(moe)GfUmUmGmsAmsUm | SEQ ID NO: 921 |
| RX002 285 | CmsAmsAmCmT(moe)CmAfCfCfUfGmUmAmAmUmAmAmAmUm | SEQ ID NO: 903 | AmsUfsUmUmAmUfUmA(moe)CmAfGmGmUmGfAmGfUmUmGmsAmsUm | SEQ ID NO: 923 |
| RX002 286 | CmsAmsAmCmUmCmAfCfCfUfGmT(moe)AmAmUmAmAmAmUm | SEQ ID NO: 904 | AmsUfsUmUmAmUfUmA(moe)CmAfGmGmUmGfAmGfUmUmGmsAmsUm | SEQ ID NO: 923 |
| RX002 287 | CmsAmsAmCmUmCmAfCfCfUfGmUmA(moe)AmUmAmAmAmUm | SEQ ID NO: 905 | AmsUfsUmUmAmUfUmA(moe)CmAfGmGmUmGfAmGfUmUmGmsAmsUm | SEQ ID NO: 923 |
| RX002 288 | CmsAmsAmCmUmCmAfCfCfUfGmUmAmAmUmAmAmA(moe)Um | SEQ ID NO: 906 | AmsUfsUmUmAmUfUmA(moe)CmAfGmGmUmGfAmGfUmUmGmsAmsUm | SEQ ID NO: 923 |
| RX002 289 | CmsGmsAmAmG(moe)CmUfCfAfUfGmAmAmUmAmUmAmUmUm | SEQ ID NO: 888 | AmsAfsUmAmUmAfUmUmCmAmUfGmAmGfC(moe)UfUmCmGmsUmsAm | SEQ ID NO: 910 |
| RX002 290 | CmsGmsAmAmGmCmUfCfAfUfGmAmA(moe)UmAmUmAmUmUm | SEQ ID NO: 890 | AmsAfsUmAmUmAfUmUmCmAmUfGmAmGfC(moe)UfUmCmGmsUmsAm | SEQ ID NO: 910 |
| RX002 291 | CmsGmsAmAmGmCmUfCfAfUfGmAmA(moe)UmAmUmAmUmUm | SEQ ID NO: 890 | AmsAfsUmAmUmAfUmT(moe)CmAmUfGmAmGfCmUfUmCmGmsUmsAm | SEQ ID NO: 924 |
| RX002 292 | CmsGmsAmAmGmCmUfCfAfUfGmAmAmUmAmUmAmT(moe)Um | SEQ ID NO: 891 | AmsAfsUmAmUmAfUmT(moe)CmAmUfGmAmGfCmUfUmCmGmsUmsAm | SEQ ID NO: 924 |
| RX002 293 | CmsAmsGmAmGmAmAfAfUfUfCmUmA(moe)CmUmAmCmAmUm | SEQ ID NO: 895 | AmsUfsGmUmAmGfUmAmGmAmAfUmUmUfC(moe)UfCmUmGmsUmsAm | SEQ ID NO: 914 |
| RX002 294 | CmsAmsGmAmGmAmAfAfUfUfCmUmAmCmUmAmCmA(moe)Um | SEQ ID NO: 896 | AmsUfsGmUmAmGfUmA(moe)GmAmAfUmUmUfCmUfCmUmGmsUmsAm | SEQ ID NO: 925 |
| RX002 295 | CmsAmsAmCmUmCmAfCfCfUfGmUmA(moe)AmUmAmAmAmUm | SEQ ID NO: 905 | AmsUfsUmUmAmUfUmAmCmAmGfGmUmGfA(moe)GfUmUmGmsAmsUm | SEQ ID NO: 922 |

(continued)

| Pair | Sense strand (5'-3') | | Antisense strand (5'-3') | |
|---|---|---|---|---|
| RX002 296 | CmsAmsAmCmUmCmAfCfCfUfGmUmAmAmUmAmAmA(moe)Um | SEQ ID NO: 906 | AmsUfsUmUmAmUfUmA(moe)CmAmGfGmUmGfAmGfUmUmGmsAmsUm | SEQ ID NO: 926 |
| RX002 297 | CmsAmsGmAmGmAmAfAfUfUfCmUmAmCmUmAmCmAmUm | SEQ ID NO: 892 | AmsUfsGmUmAmGfUmAmGmAmAmUfUmUfC(moe)UfCmUmGmsUmsAm | SEQ ID NO: 927 |
| RX002 298 | AmsAmsAmUmUmCmUfAfCfUfAmCmAmUmCmUmAmUmAm | SEQ ID NO: 907 | UmsAfsUmAmGmAfUmGmUmAmGmUfAmGfAmAfUmUmUmsCmsUm | SEQ ID NO: 928 |
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |

(continued)

| Pair | Sense strand (5'-3') | | Antisense strand (5'-3') | |
|---|---|---|---|---|
| RX002 308 | CmsAmsGmAmGmAmAfAfUfUfCmUmAmCmUmAmCmAmUm | SEQ ID NO: 892 | AmsUfsGmUmAmGfUmAmGmAmAmUfUmUfCmUfCmUmGmsUmsAm | SEQ ID NO: 938 |
| RX002 309 | AmsAmsAmUmUmCmUfAfCfUfAmCmAmUmCmUmAmUmAm | SEQ ID NO: 907 | UmsAfsUmAmGmAfUmGmUfAmGmUmAmGfAmAfUmUmUmsCmsUm | SEQ ID NO: 939 |
| RX002 310 | GmsAmsGmAmAmUmUfGfCfUfUmCmAmUmAmCmAmAmAm | SEQ ID NO: 897 | UmsUfsUmGmUmAfUmGmAfAmGmCmAmAfUmUfCmUmCmsCmsUm | SEQ ID NO: 940 |
| RX002 311 | GmsAmsGmAmAmUmUfGfCfUfUmCmAmUmAmCmAmAmAm | SEQ ID NO: 897 | UmsUfsUmGmUmAfUmGmAmAmGmCfAmAfUmUfCmUmCmsCmsUm | SEQ ID NO: 941 |
| RX002 312 | GmsAmsGmAmAmUmUfGfCfUfUmCmAmUmAmCmAmAmAm | SEQ ID NO: 897 | UmsUfsUmGmUmAfUmGmAfAmGmCmAmAfU(moe)UfCmUmCmsCmsUm | SEQ ID NO: 942 |
| RX002 313 | GmsAmsGmAmAmUmUfGfCfUfUmCmAmUmAmCmAmAmAm | SEQ ID NO: 897 | UmsUfsUmGmUmAfUmGmAmAmGmCfAmAfU(moe)UfCmUmCmsCmsUm | SEQ ID NO: 943 |

**9.** The double-stranded oligonucleotide according to claim 7, wherein the sense strand is selected from any one of the sequences as set forth in SEQ ID NOs: 887, 892, 897, 902, and 907; and/or
the antisense strand is selected from any one of the sequences as set forth in SEQ ID NOs: 908, 912, 914, 920, and 927 to 943.

**10.** The double-stranded oligonucleotide according to claim 9, wherein the double-stranded oligonucleotide comprises one or more pairs of:

| Pair | Sense strand (5'-3') | | Antisense strand (5'-3') | |
|---|---|---|---|---|
| RX002 245 | CmsGmsAmAmGmCmUfCfAfUfGmAmAmUmAmUmAmUmUm | SEQ ID NO: 887 | AmsAfsUmAmUmAfUmUmCfAmUmGmAmGfC(moe)UfUmCmGmsUmsAm | SEQ ID NO: 908 |
| RX002 256 | CmsAmsGmAmGmAmAfAfUfUfCmUmAmCmUmAmCmAmUm | SEQ ID NO: 892 | AmsUfsGmUmAmGfUmAmGfAmAmUmUmUfC(moe)UfCmUmGmsUmsAm | SEQ ID NO: 912 |
| RX002 258 | CmsAmsGmAmGmAmAfAfUfUfCmUmAmCmUmAmCmAmUm | SEQ ID NO: 892 | AmsUfsGmUmAmGfUmAmGmAmAfUmUmUfC(moe)UfCmUmGmsUmsAm | SEQ ID NO: 914 |

(continued)

| Pair | Sense strand (5'-3') | | Antisense strand (5'-3') | |
|---|---|---|---|---|
| RX002 278 | CmsAmsAmCmUmCmAfCfCfUfGmUmAmAmUmAmAmAmUm | SEQ ID NO: 902 | AmsUfsUmUmAmUfUmAmCfAmGmGmUmGfA(moe)GfUmUmGmsAmsUm | SEQ ID NO: 920 |
| RX002 297 | CmsAmsGmAmGmAmAfAfUfUfCmUmAmCmUmAmCmAmUm | SEQ ID NO: 892 | AmsUfsGmUmAmGfUmAmGmAmAmUfUmUfC(moe)UfCmUmGmsUmsAm | SEQ ID NO: 927 |
| RX002 298 | AmsAmsAmUmUmCmUfAfCfUfAmCmAmUmCmUmAmUmAm | SEQ ID NO: 907 | UmsAfsUmAmGmAfUmGmUmAmGmUfAmGfAmAfUmUmUmsCmsUm | SEQ ID NO: 928 |
| RX002 299 | AmsAmsAmUmUmCmUfAfCfUfAmCmAmUmCmUmAmUmAm | SEQ ID NO: 907 | UmsAfsUmAmGmAfUmGmUfAmGmUmAmGfA(moe)AfUmUmUmsCmsUm | SEQ ID NO: 929 |
| RX002 300 | AmsAmsAmUmUmCmUfAfCfUfAmCmAmUmCmUmAmUmAm | SEQ ID NO: 907 | UmsAfsUmAmGmAfUmGmUmAmGmUfAmGfA(moe)AfUmUmUmsCmsUm | SEQ ID NO: 930 |
| RX002 301 | CmsGmsAmAmGmCmUfCfAfUfGmAmAmUmAmUmAmUmUm | SEQ ID NO: 887 | AmsAfsUmAmUmAfUmUmCfAmUmGmAmGfCmUfUmCmGmsUmsAm | SEQ ID NO: 931 |
| RX002 302 | CmsGmsAmAmGmCmUfCfAfUfGmAmAmUmAmUmAmUmUm | SEQ ID NO: 887 | AmsAfsUmAmUmAfUmUmCmAmUmGfAmGfCmUfUmCmGmsUmsAm | SEQ ID NO: 932 |
| RX002 303 | CmsGmsAmAmGmCmUfCfAfUfGmAmAmUmAmUmAmUmUm | SEQ ID NO: 887 | AmsAfsUmAmUmAfUmUmCmAmUmGfAmGfC(moe)UfUmCmGmsUmsAm | SEQ ID NO: 933 |
| RX002 304 | CmsAmsAmCmUmCmAfCfCfUfGmUmAmAmUmAmAmAmUm | SEQ ID NO: 902 | AmsUfsUmUmAmUfUmAmCfAmGmGmUmGfAmGfUmUmGmsAmsUm | SEQ ID NO: 934 |
| RX002 305 | CmsAmsAmCmUmCmAfCfCfUfGmUmAmAmUmAmAmAmUm | SEQ ID NO: 902 | AmsUfsUmUmAmUfUmAmCmAmGmGfUmGfAmGfUmUmGmsAmsUm | SEQ ID NO: 935 |
| RX002 306 | CmsAmsAmCmUmCmAfCfCfUfGmUmAmAmUmAmAmAmUm | SEQ ID NO: 902 | AmsUfsUmUmAmUfUmAmCmAmGmGfUmGfA(moe)GfUmUmGmsAmsUm | SEQ ID NO: 936 |
| RX002 307 | CmsAmsGmAmGmAmAfAfUfUfCmUmAmCmUmAmCmAmUm | SEQ ID NO: 892 | AmsUfsGmUmAmGfUmAmGfAmAmUmUmUfCmUfCmUmGmsUmsAm | SEQ ID NO: 937 |

(continued)

| Pair | Sense strand (5'-3') | | Antisense strand (5'-3') | |
|---|---|---|---|---|
| RX002 308 | CmsAmsGmAmGmAmAfAfUfUfC mUmAmCmUmAmCmAmUm | SEQ ID NO: 892 | AmsUfsGmUmAmGfUmAmGmAmAmU fUmUfCmUfCmUmGmsUmsAm | SEQ ID NO: 938 |
| RX002 309 | AmsAmsAmUmUmCmUfAfCfUfA mCmAmUmCmUmAmUmAm | SEQ ID NO: 907 | UmsAfsUmAmGmAfUmGmUfAmGmU mAmGfAmAfUmUmUmsCmsUm | SEQ ID NO: 939 |
| RX002 310 | GmsAmsGmAmAmUmUfGfCfUfU mCmAmUmAmCmAmAmAm | SEQ ID NO: 897 | UmsUfsUmGmUmAfUmGmAfAmGmC mAmAfUmUfCmUmCmsCmsUm | SEQ ID NO: 940 |
| RX002 311 | GmsAmsGmAmAmUmUfGfCfUfU mCmAmUmAmCmAmAmAm | SEQ ID NO: 897 | UmsUfsUmGmUmAfUmGmAmAmGmC fAmAfUmUfCmUmCmsCmsUm | SEQ ID NO: 941 |
| RX002 312 | GmsAmsGmAmAmUmUfGfCfUfU mCmAmUmAmCmAmAmAm | SEQ ID NO: 897 | UmsUfsUmGmUmAfUmGmAfAmGmC mAmAfU(moe)UfCmUmCmsCmsUm | SEQ ID NO: 942 |
| RX002 313 | GmsAmsGmAmAmUmUfGfCfUfU mCmAmUmAmCmAmAmAm | SEQ ID NO: 897 | UmsUfsUmGmUmAfUmGmAmAmGmC fAmAfU(moe)UfCmUmCmsCmsUm | SEQ ID NO: 943 |

.

**11.** The double-stranded oligonucleotide according to claim 10, wherein the double-stranded oligonucleotide is selected from:

| Pair | Sense strand (5'-3') | | Antisense strand (5'-3') | |
|---|---|---|---|---|
| RX002 258 | CmsAmsGmAmGmAmAfAfUfUfC mUmAmCmUmAmCmAmUm | SEQ ID NO: 892 | AmsUfsGmUmAmGfUmAmGmAmAfU mUmUfC(moe)UfCmUmGmsUmsAm | SEQ ID NO: 914 |

**12.** A double-stranded oligonucleotide for inhibiting the expression of C3 gene, wherein the double-stranded oligonucleotide comprises a sense strand and an antisense strand, and each nucleotide of the double-stranded nucleotide is independently selected from a modified nucleotide;

wherein the nucleotide sequence from positions 2 to 19 of the antisense strand comprises at least 17 contiguous nucleotides from any one of the nucleotide sequences as set forth in SEQ ID NOs: 908 to 943 or a nucleotide sequence having 1, 2 or 3 nucleotide base differences from the contiguous nucleotides; and
the sense strand comprises at least 17 nucleotides, and the sense strand and the antisense strand are complementary or substantially complementary to form a duplex region, wherein being substantial complementary means that the sense strand and the antisense strand have no more than 3 nucleotide mismatches in the duplex region.

**13.** The double-stranded oligonucleotide according to claim 12, wherein the antisense strand comprises a nucleotide sequence having 0, 1, 2 or 3 nucleotide base differences from any one of the sequences as set forth in SEQ ID NOs: 908 to 943;

optionally, the antisense strand comprises a nucleotide sequence having 0 or 1 nucleotide base difference from any one of the sequences as set forth in SEQ ID NOs: 908 to 943; and
optionally, the antisense strand comprises any one of the nucleotide sequences as set forth in SEQ ID NOs: 908 to 943.

**14.** The double-stranded oligonucleotide according to claim 12, wherein the sense strand comprises at least 17 contiguous nucleotides of any one of the nucleotide sequences as set forth in SEQ ID NOs: 887 to 907 or a nucleotide sequence having 1, 2 or 3 nucleotide base differences from the at least 17 contiguous nucleotides;

optionally, the sense strand comprises a nucleotide sequence having 0, 1, 2 or 3 nucleotide base differences from any one of the sequences as set forth in SEQ ID NOs: 887 to 907;
optionally, the sense strand comprises a nucleotide sequence having 0 or 1 nucleotide base difference from any one of the nucleotide sequences as set forth in SEQ ID NOs: 887 to 907; and
optionally, the sense strand comprises any one of the nucleotide sequences as set forth in SEQ ID NOs: 887 to 907.

**15.** The double-stranded oligonucleotide according to any one of claims 12 to 14, wherein the double-stranded oligonucleotide comprises one or more pairs of:

an antisense strand having a nucleotide sequence with 0 or 1 nucleotide base difference from the sequence as set forth in SEQ ID NO: 908, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 888;
an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 889;
an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 890;
an antisense strand having a nucleotide sequence with 0 or 1 nucleotide base difference from the sequence as set forth in SEQ ID NO: 909, and a sense strand having a nucleotide sequence with 0 or 1 nucleotide base difference from the sequence as set forth in SEQ ID NO: 891;
an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 888;
an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 889;
an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 890;
an antisense strand having a nucleotide sequence with 0 or 1 nucleotide base difference from the sequence as set forth in SEQ ID NO: 911, and a sense strand having a nucleotide sequence with 0 or 1 nucleotide base difference from the sequence as set forth in SEQ ID NO: 891;
an antisense strand having a nucleotide sequence with 0 or 1 nucleotide base difference from the sequence as set forth in SEQ ID NO: 912, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 893;
an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 894;
an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 895;
an antisense strand having a nucleotide sequence with 0 or 1 nucleotide base difference from the sequence as set forth in SEQ ID NO: 913, and a sense strand having a nucleotide sequence with 0 or 1 nucleotide base difference from the sequence as set forth in SEQ ID NO: 896;
an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 893;
an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 894;
an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 895;
an antisense strand having a nucleotide sequence with 0 or 1 nucleotide base difference from the sequence as set forth in SEQ ID NO: 915, and a sense strand having a nucleotide sequence with 0 or 1 nucleotide base difference from the sequence as set forth in SEQ ID NO: 896;
an antisense strand having a nucleotide sequence with 0 or 1 nucleotide base difference from the sequence as set forth in SEQ ID NO: 916, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 918, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 898;
an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 899;
an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 900;
an antisense strand having a nucleotide sequence with 0 or 1 nucleotide base difference from the sequence as set forth in SEQ ID NO: 917, and a sense strand having a nucleotide sequence with 0 or 1 nucleotide base difference from the sequence as set forth in SEQ ID NO: 901;
an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 898;
an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 899;
an antisense strand having a nucleotide sequence with 0 or 1 nucleotide base difference from the sequence as set forth in SEQ ID NO: 919, and a sense strand having a nucleotide sequence with 0 or 1 nucleotide base difference from the sequence as set forth in SEQ ID NO: 900;
an antisense strand having a nucleotide sequence with 0 or 1 nucleotide base difference from the sequence as set

forth in SEQ ID NO: 919, and a sense strand having a nucleotide sequence with 0 or 1 nucleotide base difference from the sequence as set forth in SEQ ID NO: 901;
an antisense strand having a nucleotide sequence with 0 or 1 nucleotide base difference from the sequence as set forth in SEQ ID NO: 920, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 922, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 903;
an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 904;
an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 905;
an antisense strand having a nucleotide sequence with 0 or 1 nucleotide base difference from the sequence as set forth in SEQ ID NO: 921, and a sense strand having a nucleotide sequence with 0 or 1 nucleotide base difference from the sequence as set forth in SEQ ID NO: 906;
an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 903;
an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 904;
an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 905;
an antisense strand having a nucleotide sequence with 0 or 1 nucleotide base difference from the sequence as set forth in SEQ ID NO: 923, and a sense strand having a nucleotide sequence with 0 or 1 nucleotide base difference from the sequence as set forth in SEQ ID NO: 906;
an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 888;
an antisense strand having a nucleotide sequence with 0 or 1 nucleotide base difference from the sequence as set forth in SEQ ID NO: 910, and a sense strand having a nucleotide sequence with 0 or 1 nucleotide base difference from the sequence as set forth in SEQ ID NO: 890;
an antisense strand having a nucleotide sequence with 0 or 1 nucleotide base difference from the sequence as set forth in SEQ ID NO: 924, and a sense strand having a nucleotide sequence with 0 or 1 nucleotide base difference from the sequence as set forth in SEQ ID NO: 890;
an antisense strand having a nucleotide sequence with 0 or 1 nucleotide base difference from the sequence as set forth in SEQ ID NO: 924, and a sense strand having a nucleotide sequence with 0 or 1 nucleotide base difference from the sequence as set forth in SEQ ID NO: 891;
an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 895;
an antisense strand having a nucleotide sequence with 0 or 1 nucleotide base difference from the sequence as set forth in SEQ ID NO: 925, and a sense strand having a nucleotide sequence with 0 or 1 nucleotide base difference from the sequence as set forth in SEQ ID NO: 896;
an antisense strand having a nucleotide sequence with 0 or 1 nucleotide base difference from the sequence as set forth in SEQ ID NO: 922, and a sense strand having a nucleotide sequence with 0 or 1 nucleotide base difference from the sequence as set forth in SEQ ID NO: 905;
an antisense strand having a nucleotide sequence with 0 or 1 nucleotide base difference from the sequence as set forth in SEQ ID NO: 926, and a sense strand having a nucleotide sequence with 0 or 1 nucleotide base difference

from the sequence as set forth in SEQ ID NO: 906;
an antisense strand having a nucleotide sequence with 0 or 1 nucleotide base difference from the sequence as set forth in SEQ ID NO: 927, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 928, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 929, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 930, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 931, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 932, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 933, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 934, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 935, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 936, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 937, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 938, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 939, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 940, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 941, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 942, and a sense strand having a nucleotide sequence with 0 or 1 nucleotide base difference from the sequence as set forth in SEQ ID NO: 897; and
an antisense strand having a nucleotide sequence with 0 or 1 nucleotide base difference from the sequence as set forth in SEQ ID NO: 943, and a sense strand having a nucleotide sequence with 0 or 1 nucleotide base difference from the sequence as set forth in SEQ ID NO: 897;
optionally, the double-stranded oligonucleotide comprises one or more pairs of:

an antisense strand having a nucleotide sequence as set forth in SEQ ID NO: 908 and a sense strand having a nucleotide sequence as set forth in SEQ ID NO: 887;
an antisense strand having a nucleotide sequence as set forth in SEQ ID NO: 909 and a sense strand having a nucleotide sequence as set forth in SEQ ID NO: 887;

an antisense strand having a nucleotide sequence as set forth in SEQ ID NO: 910 and a sense strand having a nucleotide sequence as set forth in SEQ ID NO: 887;
an antisense strand having a nucleotide sequence as set forth in SEQ ID NO: 909 and a sense strand having a nucleotide sequence as set forth in SEQ ID NO: 888;
an antisense strand having a nucleotide sequence as set forth in SEQ ID NO: 909 and a sense strand having a nucleotide sequence as set forth in SEQ ID NO: 889;
an antisense strand having a nucleotide sequence as set forth in SEQ ID NO: 909 and a sense strand having a nucleotide sequence as set forth in SEQ ID NO: 890;
an antisense strand having a nucleotide sequence as set forth in SEQ ID NO: 909 and a sense strand having a nucleotide sequence as set forth in SEQ ID NO: 891;
an antisense strand having a nucleotide sequence as set forth in SEQ ID NO: 911 and a sense strand having a nucleotide sequence as set forth in SEQ ID NO: 888;
an antisense strand having a nucleotide sequence as set forth in SEQ ID NO: 911 and a sense strand having a nucleotide sequence as set forth in SEQ ID NO: 889;
an antisense strand having a nucleotide sequence as set forth in SEQ ID NO: 911 and a sense strand having a nucleotide sequence as set forth in SEQ ID NO: 890;
an antisense strand having a nucleotide sequence as set forth in SEQ ID NO: 911 and a sense strand having a nucleotide sequence as set forth in SEQ ID NO: 891;
an antisense strand having a nucleotide sequence as set forth in SEQ ID NO: 912 and a sense strand having a nucleotide sequence as set forth in SEQ ID NO: 892;
an antisense strand having a nucleotide sequence as set forth in SEQ ID NO: 913 and a sense strand having a nucleotide sequence as set forth in SEQ ID NO: 892;
an antisense strand having a nucleotide sequence as set forth in SEQ ID NO: 914, and a sense strand having a nucleotide sequence as set forth in SEQ ID NO: 892;
an antisense strand having a nucleotide sequence as set forth in SEQ ID NO: 913 and a sense strand having a nucleotide sequence as set forth in SEQ ID NO: 893;
an antisense strand having a nucleotide sequence as set forth in SEQ ID NO: 913 and a sense strand having a nucleotide sequence as set forth in SEQ ID NO: 894;
an antisense strand having a nucleotide sequence as set forth in SEQ ID NO: 913 and a sense strand having a nucleotide sequence as set forth in SEQ ID NO: 895;
an antisense strand having a nucleotide sequence as set forth in SEQ ID NO: 913 and a sense strand having a nucleotide sequence as set forth in SEQ ID NO: 896;
an antisense strand having a nucleotide sequence as set forth in SEQ ID NO: 915 and a sense strand having a nucleotide sequence as set forth in SEQ ID NO: 893;
an antisense strand having a nucleotide sequence as set forth in SEQ ID NO: 915 and a sense strand having a nucleotide sequence as set forth in SEQ ID NO: 894;
an antisense strand having a nucleotide sequence as set forth in SEQ ID NO: 915 and a sense strand having a nucleotide sequence as set forth in SEQ ID NO: 895;
an antisense strand having a nucleotide sequence as set forth in SEQ ID NO: 915 and a sense strand having a nucleotide sequence as set forth in SEQ ID NO: 896;
an antisense strand having a nucleotide sequence as set forth in SEQ ID NO: 916 and a sense strand having a nucleotide sequence as set forth in SEQ ID NO: 897;
an antisense strand having a nucleotide sequence as set forth in SEQ ID NO: 917 and a sense strand having a nucleotide sequence as set forth in SEQ ID NO: 897;
an antisense strand having a nucleotide sequence as set forth in SEQ ID NO: 918 and a sense strand having a nucleotide sequence as set forth in SEQ ID NO: 897;
an antisense strand having a nucleotide sequence as set forth in SEQ ID NO: 917 and a sense strand having a nucleotide sequence as set forth in SEQ ID NO: 898;
an antisense strand having a nucleotide sequence as set forth in SEQ ID NO: 917 and a sense strand having a nucleotide sequence as set forth in SEQ ID NO: 899;
an antisense strand having a nucleotide sequence as set forth in SEQ ID NO: 917 and a sense strand having a nucleotide sequence as set forth in SEQ ID NO: 900;
an antisense strand having a nucleotide sequence as set forth in SEQ ID NO: 917 and a sense strand having a nucleotide sequence as set forth in SEQ ID NO: 901;
an antisense strand having a nucleotide sequence as set forth in SEQ ID NO: 919 and a sense strand having a nucleotide sequence as set forth in SEQ ID NO: 898;
an antisense strand having a nucleotide sequence as set forth in SEQ ID NO: 919 and a sense strand having a nucleotide sequence as set forth in SEQ ID NO: 899;

an antisense strand having a nucleotide sequence as set forth in SEQ ID NO: 919 and a sense strand having a nucleotide sequence as set forth in SEQ ID NO: 900;
an antisense strand having a nucleotide sequence as set forth in SEQ ID NO: 919 and a sense strand having a nucleotide sequence as set forth in SEQ ID NO: 901;
an antisense strand having a nucleotide sequence as set forth in SEQ ID NO: 920 and a sense strand having a nucleotide sequence as set forth in SEQ ID NO: 902;
an antisense strand having a nucleotide sequence as set forth in SEQ ID NO: 921 and a sense strand having a nucleotide sequence as set forth in SEQ ID NO: 902;
an antisense strand having a nucleotide sequence as set forth in SEQ ID NO: 922 and a sense strand having a nucleotide sequence as set forth in SEQ ID NO: 902;
an antisense strand having a nucleotide sequence as set forth in SEQ ID NO: 921 and a sense strand having a nucleotide sequence as set forth in SEQ ID NO: 903;
an antisense strand having a nucleotide sequence as set forth in SEQ ID NO: 921 and a sense strand having a nucleotide sequence as set forth in SEQ ID NO: 904;
an antisense strand having a nucleotide sequence as set forth in SEQ ID NO: 921 and a sense strand having a nucleotide sequence as set forth in SEQ ID NO: 905;
an antisense strand having a nucleotide sequence as set forth in SEQ ID NO: 921 and a sense strand having a nucleotide sequence as set forth in SEQ ID NO: 906;
an antisense strand having a nucleotide sequence as set forth in SEQ ID NO: 923 and a sense strand having a nucleotide sequence as set forth in SEQ ID NO: 903;
an antisense strand having a nucleotide sequence as set forth in SEQ ID NO: 923 and a sense strand having a nucleotide sequence as set forth in SEQ ID NO: 904;
an antisense strand having a nucleotide sequence as set forth in SEQ ID NO: 923 and a sense strand having a nucleotide sequence as set forth in SEQ ID NO: 905;
an antisense strand having a nucleotide sequence as set forth in SEQ ID NO: 923 and a sense strand having a nucleotide sequence as set forth in SEQ ID NO: 906;
an antisense strand having a nucleotide sequence as set forth in SEQ ID NO: 910 and a sense strand having a nucleotide sequence as set forth in SEQ ID NO: 888;
an antisense strand having a nucleotide sequence as set forth in SEQ ID NO: 910 and a sense strand having a nucleotide sequence as set forth in SEQ ID NO: 890;
an antisense strand having a nucleotide sequence as set forth in SEQ ID NO: 924 and a sense strand having a nucleotide sequence as set forth in SEQ ID NO: 890;
an antisense strand having a nucleotide sequence as set forth in SEQ ID NO: 924 and a sense strand having a nucleotide sequence as set forth in SEQ ID NO: 891;
an antisense strand having a nucleotide sequence as set forth in SEQ ID NO: 914 and a sense strand having a nucleotide sequence as set forth in SEQ ID NO: 895;
an antisense strand having a nucleotide sequence as set forth in SEQ ID NO: 925 and a sense strand having a nucleotide sequence as set forth in SEQ ID NO: 896;
an antisense strand having a nucleotide sequence as set forth in SEQ ID NO: 922 and a sense strand having a nucleotide sequence as set forth in SEQ ID NO: 905;
an antisense strand having a nucleotide sequence as set forth in SEQ ID NO: 926 and a sense strand having a nucleotide sequence as set forth in SEQ ID NO: 906;
an antisense strand having a nucleotide sequence as set forth in SEQ ID NO: 927 and a sense strand having a nucleotide sequence as set forth in SEQ ID NO: 892;
an antisense strand having a nucleotide sequence as set forth in SEQ ID NO: 928 and a sense strand having a nucleotide sequence as set forth in SEQ ID NO: 907;
an antisense strand having a nucleotide sequence as set forth in SEQ ID NO: 929 and a sense strand having a nucleotide sequence as set forth in SEQ ID NO: 907;
an antisense strand having a nucleotide sequence as set forth in SEQ ID NO: 930 and a sense strand having a nucleotide sequence as set forth in SEQ ID NO: 907;
an antisense strand having a nucleotide sequence as set forth in SEQ ID NO: 931 and a sense strand having a nucleotide sequence as set forth in SEQ ID NO: 887;
an antisense strand having a nucleotide sequence as set forth in SEQ ID NO: 932 and a sense strand having a nucleotide sequence as set forth in SEQ ID NO: 887;
an antisense strand having a nucleotide sequence as set forth in SEQ ID NO: 933 and a sense strand having a nucleotide sequence as set forth in SEQ ID NO: 887;
an antisense strand having a nucleotide sequence as set forth in SEQ ID NO: 934 and a sense strand having a nucleotide sequence as set forth in SEQ ID NO: 902;

an antisense strand having a nucleotide sequence as set forth in SEQ ID NO: 935 and a sense strand having a nucleotide sequence as set forth in SEQ ID NO: 902;
an antisense strand having a nucleotide sequence as set forth in SEQ ID NO: 936 and a sense strand having a nucleotide sequence as set forth in SEQ ID NO: 902;
an antisense strand having a nucleotide sequence as set forth in SEQ ID NO: 937 and a sense strand having a nucleotide sequence as set forth in SEQ ID NO: 892;
an antisense strand having a nucleotide sequence as set forth in SEQ ID NO: 938 and a sense strand having a nucleotide sequence as set forth in SEQ ID NO: 892;
an antisense strand having a nucleotide sequence as set forth in SEQ ID NO: 939 and a sense strand having a nucleotide sequence as set forth in SEQ ID NO: 907;
an antisense strand having a nucleotide sequence as set forth in SEQ ID NO: 940 and a sense strand having a nucleotide sequence as set forth in SEQ ID NO: 897;
an antisense strand having a nucleotide sequence as set forth in SEQ ID NO: 941 and a sense strand having a nucleotide sequence as set forth in SEQ ID NO: 897;
an antisense strand having a nucleotide sequence as set forth in SEQ ID NO: 942 and a sense strand having a nucleotide sequence as set forth in SEQ ID NO: 897; and
an antisense strand having a nucleotide sequence as set forth in SEQ ID NO: 943 and a sense strand having a nucleotide sequence as set forth in SEQ ID NO: 897.

**16.** The double-stranded oligonucleotide according to any one of claims 12 to 14, wherein the antisense strand comprises a nucleotide sequence having 0, 1, 2 or 3 nucleotide base differences from any one of the sequences as set forth in SEQ ID NO: 908, 912, 914, 920, and 927 to 943;

optionally, the antisense strand comprises a nucleotide sequence having 0 or 1 nucleotide base difference from any one of the sequences as set forth in SEQ ID NO: 908, 912, 914, 920, and 927 to 943; and
optionally, the antisense strand comprises any one of the sequences as set forth in SEQ ID NO: 908, 912, 914, 920, and 927 to 943.

**17.** The double-stranded oligonucleotide according to any one of claims 12 to 14, wherein the sense strand comprises a nucleotide sequence having 0, 1, 2 or 3 nucleotide base differences from any one of the sequences as set forth in SEQ ID NO: 887, 892, 897, 902, and 907;

optionally, the sense strand comprises a nucleotide sequence having 0 or 1 nucleotide base difference from any one of the sequences as set forth in SEQ ID NO: 887, 892, 897, 902, and 907; and
optionally, the sense strand comprises any one of the sequences as set forth in SEQ ID NO: 887, 892, 897, 902, and 907.

**18.** The double-stranded oligonucleotide according to any one of claims 16 to 17, wherein the double-stranded oligonucleotide comprises one or more pairs of:

an antisense strand having a nucleotide sequence with 0 or 1 nucleotide base difference from the sequence as set forth in SEQ ID NO: 908, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 931, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 932, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 933, and a sense strand having a nucleotide sequence with 0 or 1 nucleotide base difference from the sequence as set forth in SEQ ID NO: 887;
an antisense strand having a nucleotide sequence with 0 or 1 nucleotide base difference from the sequence as set forth in SEQ ID NO: 912, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 914, and a sense strand having a nucleotide sequence with 0 or 1 nucleotide base difference from the sequence as set forth in SEQ ID NO: 892;

an antisense strand having a nucleotide sequence with 0 or 1 nucleotide base difference from the sequence as set forth in SEQ ID NO: 927, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 937, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 938, and a sense strand having a nucleotide sequence with 0 or 1 nucleotide base difference from the sequence as set forth in SEQ ID NO: 892;
an antisense strand having a nucleotide sequence with 0 or 1 nucleotide base difference from the sequence as set forth in SEQ ID NO: 940, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 941, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 942, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 943, and a sense strand having a nucleotide sequence with 0 or 1 nucleotide base difference from the sequence as set forth in SEQ ID NO: 897;
an antisense strand having a nucleotide sequence with 0 or 1 nucleotide base difference from the sequence as set forth in SEQ ID NO: 920, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 934, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 935, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 936, and a sense strand having a nucleotide sequence with 0 or 1 nucleotide base difference from the sequence as set forth in SEQ ID NO: 902;
an antisense strand having a nucleotide sequence with 0 or 1 nucleotide base difference from the sequence as set forth in SEQ ID NO: 928, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 929, and a sense strand having a nucleotide sequence with 0 or 1 nucleotide base difference from the sequence as set forth in SEQ ID NO: 907;
an antisense strand having a nucleotide sequence with 0 or 1 nucleotide base difference from the sequence as set forth in SEQ ID NO: 930, and a sense strand having a nucleotide sequence with 0 or 1 nucleotide base difference from the sequence as set forth in SEQ ID NO: 907; and
an antisense strand having a nucleotide sequence with 0 or 1 nucleotide base difference from the sequence as set forth in SEQ ID NO: 939, and a sense strand having a nucleotide sequence with 0 or 1 nucleotide base difference from the sequence as set forth in SEQ ID NO: 907;
optionally, the double-stranded oligonucleotide comprises one or more pairs of:

an antisense strand having a nucleotide sequence as set forth in SEQ ID NO: 908 and a sense strand having a nucleotide sequence as set forth in SEQ ID NO: 887;
an antisense strand having a nucleotide sequence as set forth in SEQ ID NO: 931 and a sense strand having a nucleotide sequence as set forth in SEQ ID NO: 887;
an antisense strand having a nucleotide sequence as set forth in SEQ ID NO: 932 and a sense strand having a nucleotide sequence as set forth in SEQ ID NO: 887;
an antisense strand having a nucleotide sequence as set forth in SEQ ID NO: 933 and a sense strand having a nucleotide sequence as set forth in SEQ ID NO: 887;
an antisense strand having a nucleotide sequence as set forth in SEQ ID NO: 912 and a sense strand having a nucleotide sequence as set forth in SEQ ID NO: 892;
an antisense strand having a nucleotide sequence as set forth in SEQ ID NO: 914 and a sense strand having a

nucleotide sequence as set forth in SEQ ID NO: 892;
an antisense strand having a nucleotide sequence as set forth in SEQ ID NO: 927 and a sense strand having a nucleotide sequence as set forth in SEQ ID NO: 892;
an antisense strand having a nucleotide sequence as set forth in SEQ ID NO: 937 and a sense strand having a nucleotide sequence as set forth in SEQ ID NO: 892;
an antisense strand having a nucleotide sequence as set forth in SEQ ID NO: 938 and a sense strand having a nucleotide sequence as set forth in SEQ ID NO: 892;
an antisense strand having a nucleotide sequence as set forth in SEQ ID NO: 940 and a sense strand having a nucleotide sequence as set forth in SEQ ID NO: 897;
an antisense strand having a nucleotide sequence as set forth in SEQ ID NO: 941 and a sense strand having a nucleotide sequence as set forth in SEQ ID NO: 897;
an antisense strand having a nucleotide sequence as set forth in SEQ ID NO: 942 and a sense strand having a nucleotide sequence as set forth in SEQ ID NO: 897;
an antisense strand having a nucleotide sequence as set forth in SEQ ID NO: 943 and a sense strand having a nucleotide sequence as set forth in SEQ ID NO: 897;
an antisense strand having a nucleotide sequence as set forth in SEQ ID NO: 920 and a sense strand having a nucleotide sequence as set forth in SEQ ID NO: 902;
an antisense strand having a nucleotide sequence as set forth in SEQ ID NO: 934 and a sense strand having a nucleotide sequence as set forth in SEQ ID NO: 902;
an antisense strand having a nucleotide sequence as set forth in SEQ ID NO: 935 and a sense strand having a nucleotide sequence as set forth in SEQ ID NO: 902;
an antisense strand having a nucleotide sequence as set forth in SEQ ID NO: 936 and a sense strand having a nucleotide sequence as set forth in SEQ ID NO: 902;
an antisense strand having a nucleotide sequence as set forth in SEQ ID NO: 928 and a sense strand having a nucleotide sequence as set forth in SEQ ID NO: 907;
an antisense strand having a nucleotide sequence as set forth in SEQ ID NO: 929 and a sense strand having a nucleotide sequence as set forth in SEQ ID NO: 907;
an antisense strand having a nucleotide sequence as set forth in SEQ ID NO: 930 and a sense strand having a nucleotide sequence as set forth in SEQ ID NO: 907; and
an antisense strand having a nucleotide sequence as set forth in SEQ ID NO: 939 and a sense strand having a nucleotide sequence as set forth in SEQ ID NO: 907.

**19.** The double-stranded oligonucleotide according to any one of claims 12 to 14, wherein the antisense strand comprises a nucleotide sequence having 0, 1, 2 or 3 nucleotide base differences from the sequence as set forth in SEQ ID NO: 914;

optionally, the antisense strand comprises a nucleotide sequence having 0 or 1 nucleotide base difference from the sequence as set forth in SEQ ID NO: 914; and
optionally, the antisense strand comprises the sequence as set forth in SEQ ID NO: 914.

**20.** The double-stranded oligonucleotide according to claim 19, wherein the sense strand comprises a nucleotide sequence having 0, 1, 2 or 3 nucleotide base differences from the sequence as set forth in SEQ ID NO: 892;

optionally, the sense strand comprises a nucleotide sequence having 0 or 1 nucleotide base difference from the sequence as set forth in SEQ ID NO: 892; and
optionally, the sense strand comprises the sequence as set forth in SEQ ID NO: 892.

**21.** The double-stranded oligonucleotide according to claim 20, wherein the double-stranded oligonucleotide comprises: an antisense strand having a nucleotide sequence as set forth in SEQ ID NO: 914 or a nucleotide sequence having 0 or 1 nucleotide base difference therefrom, and a sense strand having a nucleotide sequence as set forth in SEQ ID NO: 892 or a nucleotide sequence having 0 or 1 nucleotide base difference therefrom.

**22.** The double-stranded oligonucleotide according to claim 21, wherein the double-stranded oligonucleotide comprises: an antisense strand having a nucleotide sequence as set forth in SEQ ID NO: 914, and a sense strand having a nucleotide sequence as set forth in SEQ ID NO: 892.

**23.** The double-stranded oligonucleotide according to any one of claims 1 to 22, wherein the double-stranded oligonucleotide is siRNA.

**24.** A conjugate comprising the double-stranded oligonucleotide according to any one of claims 1 to 23, and one or more gene delivery carriers;

optionally, the gene delivery carrier is selected from a ligand capable of binding to a cell surface receptor;
optionally, the ligand is selected from a ligand of asialoglycoprotein receptor (ASGPR ligand);
optionally, the ASGPR ligand comprises galactose or a derivative thereof;
the galactose or the derivative thereof is selected from galactose, galactosamine, N-formyl galactosamine, N-acetyl galactosamine, N-propionyl galactosamine, N-n-butyryl galactosamine and N-isobutyryl galactosamine; and
optionally, the number of the ASGPR ligand is one, and the one ASGPR ligand is conjugated to 3'-end of the sense strand of the double-stranded oligonucleotide.

**25.** The conjugate according to claim 24, wherein the conjugate comprises a structure as shown by formula (101):

$$(R_{ligand})_m\text{—Nu}$$

(101) ;

wherein Nu represents the double-stranded oligonucleotide;
each $R_{ligand}$ is independently selected from an ASGPR ligand; and
m is selected from 1, 2, 3 and 4;
optionally, m is 1; and
optionally, m is one, and the one $R_{ligand}$ is conjugated to 3'-end of the sense strand.

**26.** The conjugate according to claim 25, wherein each $R_{ligand}$ is independently selected from a structure as shown by formula (201), a pharmaceutically acceptable salt thereof and a steroisomer thereof:

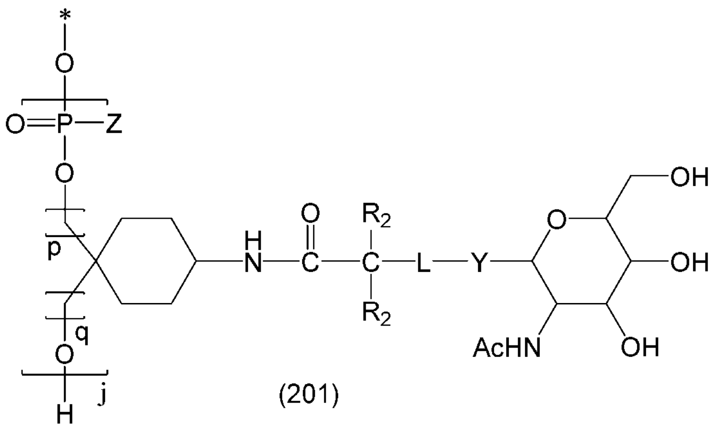

(201) ;

wherein * represents a conjugation site of the ligand on the sense strand or the antisense strand;
j is selected from 1, 2, 3 and 4;
each Z is selected from hydroxy and sulfydryl;
each p is selected from 1, 2 and 3;
each q is selected from 1, 2 and 3;
each $R_2$ is independently selected from H, any optionally substituted $C_{1-6}$ alkyl and optionally substituted $C_{1-6}$ alkoxy;
each L is selected from optionally substituted $C_{2-20}$ alkylene and

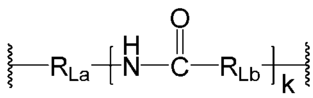

,

wherein $R_{La}$ and $R_{Lb}$ are independently selected from any optionally substituted $C_{1-10}$ alkylene, and k is selected

from 1, 2, 3, 4 and 5; and
each Y is selected from O, S and NH;
optionally, m is selected from 1, 2 or 3;
optionally, m is 3;
optionally, Z is hydroxy;
optionally, p is 1;
optionally, q is 1;
optionally, $R_2$ is H;
optionally, each L is selected from optionally substituted $C_{2-10}$ alkylene and

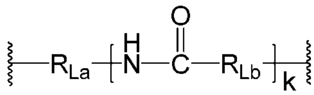,

wherein $R_{La}$ and $R_{Lb}$ are independently selected from any optionally substituted $C_{1-10}$ alkylene, and k is selected from 1, 2 and 3;
optionally, k is 1;
optionally, each L is selected from

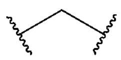, 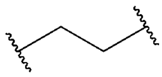, 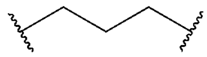,

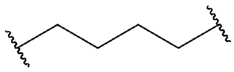, 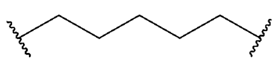, 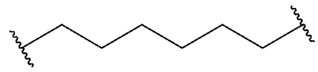,

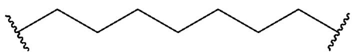, 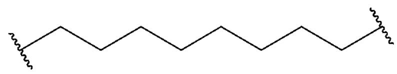,

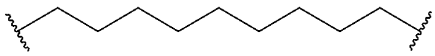, 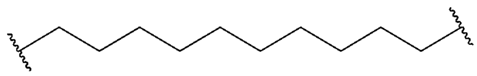,

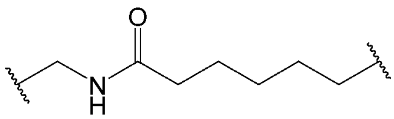, 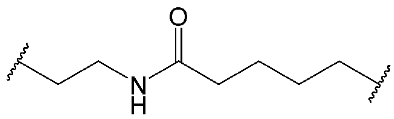,

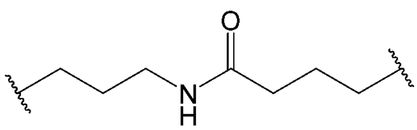, 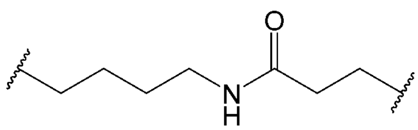

and

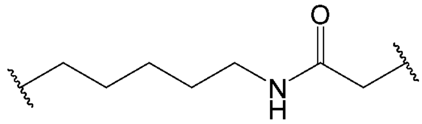;

and
optionally, Y is O.

**27.** The conjugate according to claim 26, wherein each $R_{ligand}$ is independently selected from a structure as shown by

formula (202), a pharmaceutically acceptable salt thereof and a steroisomer thereof:

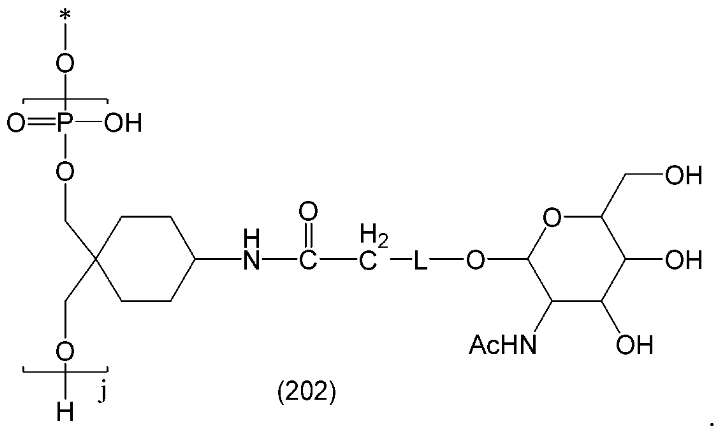

(202)

**28.** The conjugate according to any one of claims 25 to 27, wherein each $R_{ligand}$ is independently selected from a structure as shown by formula (CR01008×3), a pharmaceutically acceptable salt thereof and a steroisomer thereof:

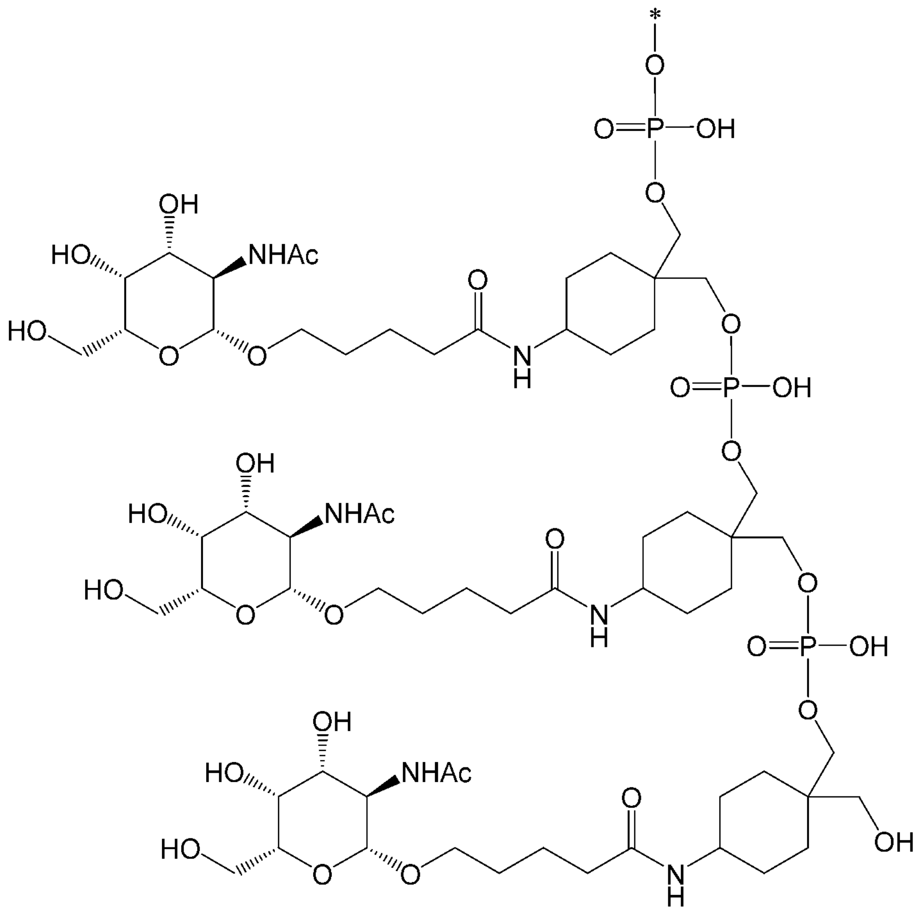

(CR01008X3)

alternatively, each $R_{ligand}$ is independently selected from a structure as shown by formula (CR01013×3), a pharmaceutically acceptable salt thereof and a steroisomer thereof:

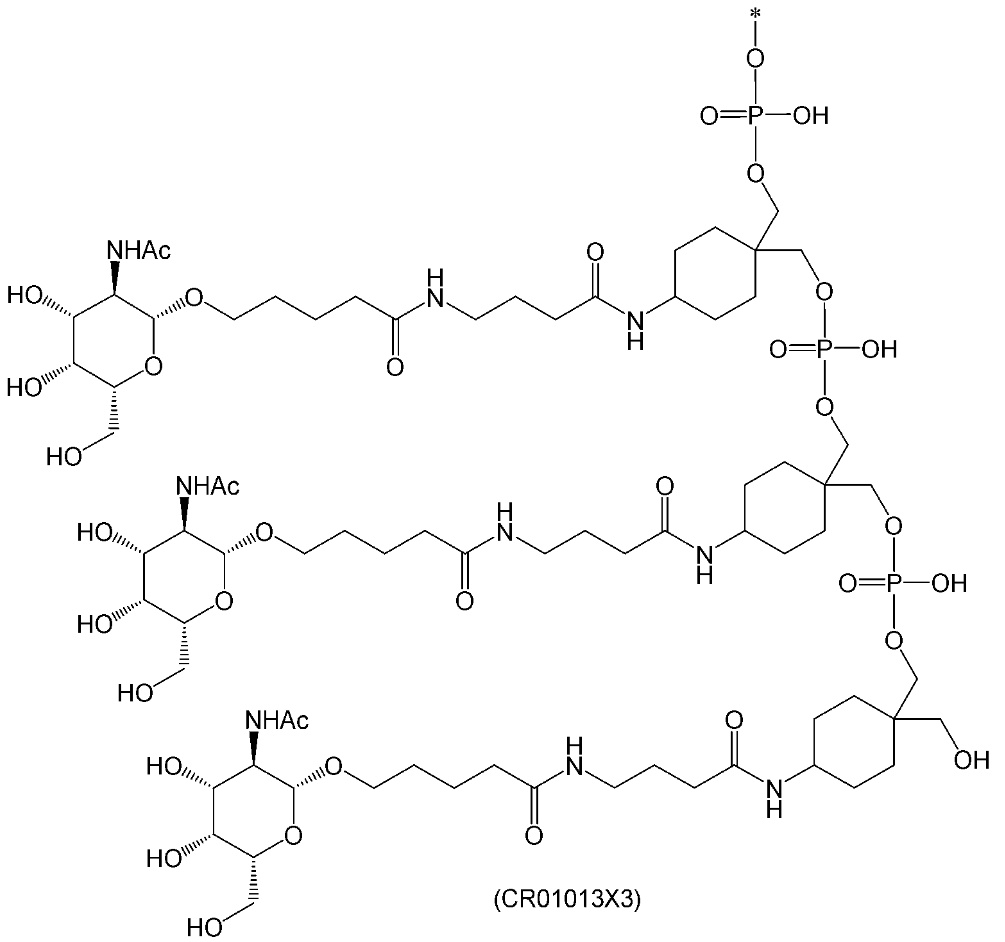

(CR01013X3)

**29.** The conjugate according to claim 28, wherein the conjugate comprises one or more pairs of:

➽Too large Table

| Pair | Sense strand (5'-3') | | Antisense strand (5'-3') | |
|---|---|---|---|---|
| RZ00 2033 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfC(moe)UfUmCmGmsUmsAm | SEQ ID NO: 908 |
| RZ00 2034 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAfUmGmAmGfC(moe)UfUmCmGmsUmsAm | SEQ ID NO: 909 |
| RZ00 2036 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmUmCmAfUmGmAmGfC(moe)UfUmCmGmsUmsAm | SEQ ID NO: 909 |
| RZ00 2037 | CmsGmsAmAmGmCmUfCfAfUfGmA(moe)AmUmAmUmAmUmUm (CR01008×3) | SEQ ID NO: 889_(CR01008×3) | AmsAfsUmAmUmAfUmUmCmAfUmGmAmGfC(moe)UfUmCmGmsUmsAm | SEQ ID NO: 909 |
| RZ00 2038 | CmsGmsAmAmGmCmUfCfAfUfGmAmA(moe)UmAmUmAmUmUm (CR01008×3) | SEQ ID NO: 890(CR01008×3 ) | AmsAfsUmAmUmAfUmUmCmAfUmGmAmGfC(moe)UfUmCmGmsUmsAm | SEQ ID NO: 909 |

(continued)

| Pair | Sense strand (5'-3') | | Antisense strand (5'-3') | |
|---|---|---|---|---|
| RZ00 2039 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmUmCmAfUmGmAmGfC(moe)UfUmCmGmsUmsAm | SEQ ID NO: 909 |
| RZ00 2040 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmT(moe)CmAfUmGmAmGfCmUfUmCmGmsUmsAm | SEQ ID NO: 911 |
| RZ00 2041 | CmsGmsAmAmGmCmUfCfAfUfGmA(moe)AmUmAmUmAmUmUm (CR01008×3) | SEQ ID NO: 889_(CR01008×3) | AmsAfsUmAmUmAfUmT(moe)CmAfUmGmAmGfCmUfUmCmGmsUmsAm | SEQ ID NO: 911 |
| RZ00 2042 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmT(moe)CmAfUmGmAmGfCmUfUmCmGmsUmsAm | SEQ ID NO: 911 |
| RZ00 2043 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmT(moe)CmAfUmGmAmGfCmUfUmCmGmsUmsAm | SEQ ID NO: 911 |
| RZ00 2050 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfC(moe)UfCmUmGmsUmsAm | SEQ ID NO: 912 |
| RZ00 2051 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAfAmUmUmUfC(moe)UfCmUmGmsUmsAm | SEQ ID NO: 913 |
| RZ00 2053 | CmsAmsGmAmG(moe)AmAfAfUfUfCmUmAmCmUmAmCmAmUm (CR01008×3) | SEQ ID NO: 893_(CR01008×3) | AmsUfsGmUmAmGfUmAmGmAfAmUmUmUfC(moe)UfCmUmGmsUmsAm | SEQ ID NO: 913 |
| RZ00 2054 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmAmGmAfAmUmUmUfC(moe)UfCmUmGmsUmsAm | SEQ ID NO: 913 |
| RZ00 2055 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmAmGmAfAmUmUmUfC(moe)UfCmUmGmsUmsAm | SEQ ID NO: 913 |
| RZ00 2056 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmAmGmAfAmUmUmUfC(moe)UfCmUmGmsUmsAm | SEQ ID NO: 913 |
| RZ00 2057 | CmsAmsGmAmG(moe)AmAfAfUfUfCmUmAmCmUmAmCmAmUm (CR01008×3) | SEQ ID NO: 893_(CR01008×3) | AmsUfsGmUmAmGfUmA(moe)GmAfAmUmUmUfCmUfCmUmGmsUmsAm | SEQ ID NO: 915 |

(continued)

| Pair | Sense strand (5'-3') | | Antisense strand (5'-3') | |
|---|---|---|---|---|
| RZ00 2058 | CmsAmsGmAmGmAmAfAfUfUfCmT(moe)AmCmUmAmCmAmUm (CR01008×3) | SEQ ID NO: 894_(CR01008×3) | AmsUfsGmUmAmGfUmA(moe)GmAfAmUmUmUfCmUfCmUmGmsUmsAm | SEQ ID NO: 915 |
| RZ00 2059 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmA(moe)GmAfAmUmUmUfCmUfCmUmGmsUmsAm | SEQ ID NO: 915 |
| RZ00 2060 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmA(moe)GmAfAmUmUmUfCmUfCmUmGmsUmsAm | SEQ ID NO: 915 |
| RZ00 2066 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfT(moe)UfCmUmCmsCmsUm | SEQ ID NO: 916 |
| RZ00 2067 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAfGmCmAmAfT(moe)UfCmUmCmsCmsUm | SEQ ID NO: 917 |
| RZ00 2068 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGfCmAmAfT(moe)UfCmUmCmsCmsUm | SEQ ID NO: 918 |
| RZ00 2069 | GmsAmsGmAmA(moe)UmUfGfCfUfUmCmAmUmAmCmAmAmAm (CR01008×3) | SEQ ID NO: 898_(CR01008×3) | UmsUfsUmGmUmAfUmGmAmAfGmCmAmAfT(moe)UfCmUmCmsCmsUm | SEQ ID NO: 917 |
| RZ00 2070 | GmsAmsGmAmAmUmUfGfCfUfUmC(moe)AmUmAmCmAmAmAm (CR01008×3) | SEQ ID NO: 899_(CR01008×3) | UmsUfsUmGmUmAfUmGmAmAfGmCmAmAfT(moe)UfCmUmCmsCmsUm | SEQ ID NO: 917 |
| RZ00 2071 | GmsAmsGmAmAmUmUfGfCfUfUmCmA(moe)UmAmCmAmAmAm (CR01008×3) | SEQ ID NO: 900_(CR01008×3) | UmsUfsUmGmUmAfUmGmAmAfGmCmAmAfT(moe)UfCmUmCmsCmsUm | SEQ ID NO: 917 |
| RZ00 2072 | GmsAmsGmAmAmUmUfGfCfUfUmCmAmUmAmCmAmA(moe)Am (CR01008×3) | SEQ ID NO: 901_(CR01008×3) | UmsUfsUmGmUmAfUmGmAmAfGmCmAmAfT(moe)UfCmUmCmsCmsUm | SEQ ID NO: 917 |
| RZ00 2073 | GmsAmsGmAmA(moe)UmUfGfCfUfUmCmAmUmAmCmAmAmAm (CR01008×3) | SEQ ID NO: 898_(CR01008×3) | UmsUfsUmGmUmAfUmG(moe)AmAfGmCmAmAfUmUfCmUmCmsCmsUm | SEQ ID NO: 919 |
| RZ00 2074 | GmsAmsGmAmAmUmUfGfCfUfUmC(moe)AmUmAmCmAmAmAm (CR01008×3) | SEQ ID NO: 899_(CR01008×3) | UmsUfsUmGmUmAfUmG(moe)AmAfGmCmAmAfUmUfCmUmCmsCmsUm | SEQ ID NO: 919 |

(continued)

| Pair | Sense strand (5'-3') | | Antisense strand (5'-3') | |
|---|---|---|---|---|
| RZ00 2075 | GmsAmsGmAmAmUmUfGfCfUfUmCmA(moe)UmAmCmAmAmAm (CR01008×3) | SEQ ID NO: 900_(CR01008×3) | UmsUfsUmGmUmAfUmG(moe)AmAfGmCmAmAfUmUfCmUmCmsCmsUm | SEQ ID NO: 919 |
| RZ00 2076 | GmsAmsGmAmAmUmUfGfCfUfUmCmAmUmAmCmAmA(moe)Am (CR01008×3) | SEQ ID NO: 901_(CR01008×3) | UmsUfsUmGmUmAfUmG(moe)AmAfGmCmAmAfUmUfCmUmCmsCmsUm | SEQ ID NO: 919 |
| RZ00 2082 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCfAmGmGmUmGfA(moe)GfUmUmGmsAmsUm | SEQ ID NO: 920 |
| RZ00 2083 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAfGmGmUmGfA(moe)GfUmUmGmsAmsUm | SEQ ID NO: 921 |
| RZ00 2084 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGfGmUmGfA(moe)GfUmUmGmsAmsUm | SEQ ID NO: 922 |
| RZ00 2085 | CmsAmsAmCmT(moe)CmAfCfCfUfGmUmAmAmUmAmAmAmUm (CR01008×3) | SEQ ID NO: 903_(CR01008×3) | AmsUfsUmUmAmUfUmAmCmAfGmGmUmGfA(moe)GfUmUmGmsAmsUm | SEQ ID NO: 921 |
| RZ00 2086 | CmsAmsAmCmUmCmAfCfCfUfGmT(moe)AmAmUmAmAmAmUm (CR01008×3) | SEQ ID NO: 904_(CR01008×3) | AmsUfsUmUmAmUfUmAmCmAfGmGmUmGfA(moe)GfUmUmGmsAmsUm | SEQ ID NO: 921 |
| RZ00 2087 | CmsAmsAmCmUmCmAfCfCfUfGmUmA(moe)AmUmAmAmAmUm (CR01008×3) | SEQ ID NO: 905_(CR01008×3) | AmsUfsUmUmAmUfUmAmCmAfGmGmUmGfA(moe)GfUmUmGmsAmsUm | SEQ ID NO: 921 |
| RZ00 2088 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmAmCmAfGmGmUmGfA(moe)GfUmUmGmsAmsUm | SEQ ID NO: 921 |
| RZ00 2089 | CmsAmsAmCmT(moe)CmAfCfCfUfGmUmAmAmUmAmAmAmUm (CR01008×3) | SEQ ID NO: 903_(CR01008×3) | AmsUfsUmUmAmUfUmA(moe)CmAfGmGmUmGfAmGfUmUmGmsAmsUm | SEQ ID NO: 923 |
| RZ00 2090 | CmsAmsAmCmUmCmAfCfCfUfGmT(moe)AmAmUmAmAmAmUm (CR01008×3) | SEQ ID NO: 904_(CR01008×3) | AmsUfsUmUmAmUfUmA(moe)CmAfGmGmUmGfAmGfUmUmGmsAmsUm | SEQ ID NO: 923 |
| RZ00 2091 | CmsAmsAmCmUmCmAfCfCfUfGmUmA(moe)AmUmAmAmAmUm (CR01008×3) | SEQ ID NO: 905_(CR01008×3) | AmsUfsUmUmAmUfUmA(moe)CmAfGmGmUmGfAmGfUmUmGmsAmsUm | SEQ ID NO: 923 |

(continued)

| Pair | Sense strand (5'-3') | | Antisense strand (5'-3') | |
|---|---|---|---|---|
| RZ00 2092 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmA(moe)CmAfGmGmUmGfAmGfUmUmGmsAmsUm | SEQ ID NO: 923 |
| RZ00 2099 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUfGmAmGfC(moe)UfUmCmGmsUmsAm | SEQ ID NO: 910 |
| RZ00 2100 | CmsGmsAmAmG(moe)CmUfCfAfUfGmAmAmUmAmUmAmUmUm (CR01008×3) | SEQ ID NO: 888_(CR01008×3) | AmsAfsUmAmUmAfUmUmCmAmUfGmAmGfC(moe)UfUmCmGmsUmsAm | SEQ ID NO: 910 |
| RZ00 2101 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmUmCmAmUfGmAmGfC(moe)UfUmCmGmsUmsAm | SEQ ID NO: 910 |
| RZ00 2102 | CmsGmsAmAmGmCmUfCfAfUfGmAmA(moe)UmAmUmAmUmUm (CR01008×3) | SEQ ID NO: 890_(CR01008×3) | AmsAfsUmAmUmAfUmT(moe)CmAmUfGmAmGfCmUfUmCmGmsUmsAm | SEQ ID NO: 924 |
| RZ00 2103 | CmsGmsAmAmGmCmUfCfAfUfGmAmAmUmAmUmAmT(moe)Um (CR01008×3) | SEQ ID NO: 891_(CR01008×3) | AmsAfsUmAmUmAfUmT(moe)CmAmUfGmAmGfCmUfUmCmGmsUmsAm | SEQ ID NO: 924 |
| RZ00 2106 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAfUmUmUfC(moe)UfCmUmGmsUmsAm | SEQ ID NO: 914 |
| RZ00 2107 | CmsAmsGmAmGmAmAfAfUfUfCmUmA(moe)CmUmAmCmAmUm (CR01008×3) | SEQ ID NO: 895_(CR01008×3) | AmsUfsGmUmAmGfUmAmGmAmAfUmUmUfC(moe)UfCmUmGmsUmsAm | SEQ ID NO: 914 |
| RZ00 2108 | CmsAmsGmAmGmAmAfAfUfUfCmUmAmCmUmAmCmA(moe)Um (CR01008×3) | SEQ ID NO: 896_(CR01008×3) | AmsUfsGmUmAmGfUmA(moe)GmAmAfUmUmUfCmUfCmUmGmsUmsAm | SEQ ID NO: 925 |
| RZ00 2112 | CmsAmsAmCmUmCmAfCfCfUfGmUmA(moe)AmUmAmAmAmUm (CR01008×3) | SEQ ID NO: 905_(CR01008×3) | AmsUfsUmUmAmUfUmAmCmAmGfGmUmGfA(moe)GfUmUmGmsAmsUm | SEQ ID NO: 922 |
| RZ00 2113 | CmsAmsAmCmUmCmAfCfCfUfGmUmAmAmUmAmAmA(moe)Um (CR01008×3) | SEQ ID NO: 906_(CR01008×3) | AmsUfsUmUmAmUfUmA(moe)CmAmGfGmUmGfAmGfUmUmGmsAmsUm | SEQ ID NO: 926 |
| RZ00 2115 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfC(moe)UfCmUmGmsUmsAm | SEQ ID NO: 927 |

(continued)

| Pair | Sense strand (5'-3') | | Antisense strand (5'-3') | |
|---|---|---|---|---|
| RZ00 2116 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfAmAfUmUmUmsCmsUm | SEQ ID NO: 928 |
| RZ00 2117 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfA(moe)AfUmUmUmsCmsUm | SEQ ID NO: 929 |
| RZ00 2118 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUmAmGmUfAmGfA(moe)AfUmUmUmsCmsUm | SEQ ID NO: 930 |
| RZ00 2119 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCfAmUmGmAmGfCmUfUmCmGmsUmsAm | SEQ ID NO: 931 |
| RZ00 2120 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfCmUfUmCmGmsUmsAm | SEQ ID NO: 932 |
| RZ00 2121 | CmsGmsAmAmGmCmUfCfAfUfGmAmAmUmAmUmAmUmUm_(CR01008×3) | SEQ ID NO: 887_(CR01008×3) | AmsAfsUmAmUmAfUmUmCmAmUmGfAmGfC(moe)UfUmCmGmsUmsAm | SEQ ID NO: 933 |
| RZ00 2122 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCfAmGmGmUmGfAmGfUmUmGmsAmsUm | SEQ ID NO: 934 |
| RZ00 2123 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfAmGfUmUmGmsAmsUm | SEQ ID NO: 935 |
| RZ00 2124 | CmsAmsAmCmUmCmAfCfCfUfGmUmAmAmUmAmAmAmUm_(CR01008×3) | SEQ ID NO: 902_(CR01008×3) | AmsUfsUmUmAmUfUmAmCmAmGmGfUmGfA(moe)GfUmUmGmsAmsUm | SEQ ID NO: 936 |
| RZ00 2125 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGfAmAmUmUmUfCmUfCmUmGmsUmsAm | SEQ ID NO: 937 |
| RZ00 2126 | CmsAmsGmAmGmAmAfAfUfUfCmUmAmCmUmAmCmAmUm_(CR01008×3) | SEQ ID NO: 892_(CR01008×3) | AmsUfsGmUmAmGfUmAmGmAmAmUfUmUfCmUfCmUmGmsUmsAm | SEQ ID NO: 938 |
| RZ00 2130 | AmsAmsAmUmUmCmUfAfCfUfAmCmAmUmCmUmAmUmAm_(CR01008×3) | SEQ ID NO: 907_(CR01008×3) | UmsAfsUmAmGmAfUmGmUfAmGmUmAmGfAmAfUmUmUmsCmsUm | SEQ ID NO: 939 |

(continued)

| Pair | Sense strand (5'-3') | | Antisense strand (5'-3') | |
|---|---|---|---|---|
| RZ00 2131 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfUmUfCmUmCmsCmsUm | SEQ ID NO: 940 |
| RZ00 2132 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfUmUfCmUmCmsCmsUm | SEQ ID NO: 941 |
| RZ00 2133 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAfAmGmCmAmAfU(moe)UfCmUmCmsCmsUm | SEQ ID NO: 942 |
| RZ00 2134 | GmsAmsGmAmAmUmUfGfCfUfUmCmAmUmAmCmAmAmAm_(CR01008×3) | SEQ ID NO: 897_(CR01008×3) | UmsUfsUmGmUmAfUmGmAmAmGmCfAmAfU(moe)UfCmUmCmsCmsUm | SEQ ID NO: 943 |

optionally, the conjugate comprises one or more pairs of:

| Pair | Sense strand (5'-3') | | Antisense strand (5'-3') | |
|---|---|---|---|---|
| RZ00 2033 | CmsGmsAmAmGmCmUfCfAfUfGmAmAmUmAmUmAmUmUm_(CR01008×3) | SEQ ID NO: 887_(CR01008× 3) | AmsAfsUmAmUmAfUmUmCfAmUmGmAmGfC(moe)UfUmCmGmsUmsAm | SEQ ID NO: 908 |
| RZ00 2050 | CmsAmsGmAmGmAmAfAfUfUfCmUmAmCmUmAmCmAmUm_(CR01008×3) | SEQ ID NO: 892_(CR01008× 3) | AmsUfsGmUmAmGfUmAmGfAmAmUmUmUfC(moe)UfCmUmGmsUmsAm | SEQ ID NO: 912 |
| RZ00 2082 | CmsAmsAmCmUmCmAfCfCfUfGmUmAmAmUmAmAmAmUm_(CR01008×3) | SEQ ID NO: 902_(CR01008× 3) | AmsUfsUmUmAmUfUmAmCfAmGmGmUmGfA(moe)GfUmUmGmsAmsUm | SEQ ID NO: 920 |
| RZ00 2106 | CmsAmsGmAmGmAmAfAfUfUfCmUmAmCmUmAmCmAmUm_(CR01008×3) | SEQ ID NO: 892_(CR01008× 3) | AmsUfsGmUmAmGfUmAmGmAmAfUmUmUfC(moe)UfCmUmGmsUmsAm | SEQ ID NO: 914 |
| RZ00 2115 | CmsAmsGmAmGmAmAfAfUfUfCmUmAmCmUmAmCmAmUm_(CR01008×3) | SEQ ID NO: 892_(CR01008× 3) | AmsUfsGmUmAmGfUmAmGmAmAmUfUmUfC(moe)UfCmUmGmsUmsAm | SEQ ID NO: 927 |
| RZ00 2116 | AmsAmsAmUmUmCmUfAfCfUfAmCmAmUmCmUmAmUmAm_(CR01008×3) | SEQ ID NO: 907_(CR01008× 3) | UmsAfsUmAmGmAfUmGmUmAmGmUfAmGfAmAfUmUmUmsCmsUm | SEQ ID NO: 928 |
| RZ00 2117 | AmsAmsAmUmUmCmUfAfCfUfAmCmAmUmCmUmAmUmAm_(CR01008×3) | SEQ ID NO: 907_(CR01008× 3) | UmsAfsUmAmGmAfUmGmUfAmGmUmAmGfA(moe)AfUmUmUmsCmsUm | SEQ ID NO: 929 |
| RZ00 2118 | AmsAmsAmUmUmCmUfAfCfUfAmCmAmUmCmUmAmUmAm_(CR01008×3) | SEQ ID NO: 907_(CR01008× 3) | UmsAfsUmAmGmAfUmGmUmAmGmUfAmGfA(moe)AfUmUmUmsCmsUm | SEQ ID NO: 930 |
| RZ00 2119 | CmsGmsAmAmGmCmUfCfAfUfGmAmAmUmAmUmAmUmUm_(CR01008×3) | SEQ ID NO: 887_(CR01008× 3) | AmsAfsUmAmUmAfUmUmCfAmUmGmAmGfCmUfUmCmGmsUmsAm | SEQ ID NO: 931 |
| RZ00 2120 | CmsGmsAmAmGmCmUfCfAfUfGmAmAmUmAmUmAmUmUm_(CR01008×3) | SEQ ID NO: 887_(CR01008× 3) | AmsAfsUmAmUmAfUmUmCmAmUmGfAmGfCmUfUmCmGmsUmsAm | SEQ ID NO: 932 |
| RZ00 2121 | CmsGmsAmAmGmCmUfCfAfUfGmAmAmUmAmUmAmUmUm_(CR01008×3) | SEQ ID NO: 887_(CR01008× 3) | AmsAfsUmAmUmAfUmUmCmAmUmGfAmGfC(moe)UfUmCmGmsUmsAm | SEQ ID NO: 933 |

(continued)

| Pair | Sense strand (5'-3') | | Antisense strand (5'-3') | |
|---|---|---|---|---|
| RZ00 2122 | CmsAmsAmCmUmCmAfCfCfUfGmUmAmAmUmAmAmAmUm_(CR01008×3) | SEQ ID NO: 902_(CR01008× 3) | AmsUfsUmUmAmUfUmAmCfAmGmGmUmGfAmGfUmUmGmsAmsUm | SEQ ID NO: 934 |
| RZ00 2123 | CmsAmsAmCmUmCmAfCfCfUfGmUmAmAmUmAmAmAmUm_(CR01008×3) | SEQ ID NO: 902_(CR01008× 3) | AmsUfsUmUmAmUfUmAmCmAmGmGfUmGfAmGfUmUmGmsAmsUm | SEQ ID NO: 935 |
| RZ00 2124 | CmsAmsAmCmUmCmAfCfCfUfGmUmAmAmUmAmAmAmUm (CR01008×3) | SEQ ID NO: 902_(CR01008×3) | AmsUfsUmUmAmUfUmAmCmAmGmGfUmGfA(moe)GfUmUmGmsAmsUm | SEQ ID NO: 936 |
| RZ00 2125 | CmsAmsGmAmGmAmAfAfUfUfCmUmAmCmUmAmCmAmUm_(CR01008×3) | SEQ ID NO: 892_(CR01008× 3) | AmsUfsGmUmAmGfUmAmGfAmAmUmUmUfCmUfCmUmGmsUmsAm | SEQ ID NO: 937 |
| RZ00 2126 | CmsAmsGmAmGmAmAfAfUfUfCmUmAmCmUmAmCmAmUm_(CR01008×3) | SEQ ID NO: 892_(CR01008× 3) | AmsUfsGmUmAmGfUmAmGmAmAmUfUmUfCmUfCmUmGmsUmsAm | SEQ ID NO: 938 |
| RZ00 2130 | AmsAmsAmUmUmCmUfAfCfUfAmCmAmUmCmUmAmUmAm_(CR01008×3) | SEQ ID NO: 907_(CR01008× 3) | UmsAfsUmAmGmAfUmGmUfAmGmUmAmGfAmAfUmUmUmsCmsUm | SEQ ID NO: 939 |
| RZ00 2131 | GmsAmsGmAmAmUmUfGfCfUfUmCmAmUmAmCmAmAmAm_(CR01008×3) | SEQ ID NO: 897_(CR01008× 3) | UmsUfsUmGmUmAfUmGmAfAmGmCmAmAfUmUfCmUmCmsCmsUm | SEQ ID NO: 940 |
| RZ00 2132 | GmsAmsGmAmAmUmUfGfCfUfUmCmAmUmAmCmAmAmAm_(CR01008×3) | SEQ ID NO: 897_(CR01008× 3) | UmsUfsUmGmUmAfUmGmAmAmGmCfAmAfUmUfCmUmCmsCmsUm | SEQ ID NO: 941 |
| RZ00 2133 | GmsAmsGmAmAmUmUfGfCfUfUmCmAmUmAmCmAmAmAm_(CR01008×3) | SEQ ID NO: 897_(CR01008× 3) | UmsUfsUmGmUmAfUmGmAfAmGmCmAmAfU(moe)UfCmUmCmsCmsUm | SEQ ID NO: 942 |
| RZ00 2134 | GmsAmsGmAmAmUmUfGfCfUfUmCmAmUmAmCmAmAmAm_(CR01008×3) | SEQ ID NO: 897_(CR01008× 3) | UmsUfsUmGmUmAfUmGmAmAmGmCfAmAfU(moe)UfCmUmCmsCmsUm | SEQ ID NO: 943 |

; and

optionally, the conjugate comprises the pair of:

➽Too large Table

| Pair | Sense strand (5'-3') | | Antisense strand (5'-3') | |
|---|---|---|---|---|
| RZ00 2106 | CmsAmsGmAmGmAmAfAfUfUf CmUmAmCmUmAmCmAmUm_ (CR01008×3) | SEQ ID NO: 892_(CR01008× 3) | AmsUfsGmUmAmGfUmAmGmA mAfUmUmUfC(moe)UfCmUmG msUmsAm | SEQ ID NO: 914 |

**30.** Use of the double-stranded oligonucleotide according to any one of claims 1 to 23 or the conjugate according to any one of claims 24 to 29, in the manufacture of a medicament for ameliorating, preventing and/or treating a disease or disorder mediated by C3 gene;
optionally, the disease or disorder mediated by C3 gene is selected from: IgA nephropathy, atypical hemolytic uremic syndrome (aHUS), paroxysmal nocturnal hemoglobinuria (PNH), C3 glomerulopathy, lupus nephritis, and membranous nephropathy.

**31.** A pharmaceutical composition comprising the double-stranded oligonucleotide according to any one of claims 1 to 23 and/or the conjugate according to any one of claims 24 to 29.

**32.** A kit comprising the double-stranded oligonucleotide according to any one of claims 1 to 23 and/or the conjugate according to any one of claims 24 to 29.

**33.** A method for inhibiting expression of C3 gene, comprising administering the double-stranded oligonucleotide according to any one of claims 1 to 23, and/or the conjugate according to any one of claims 24 to 29, and/or the pharmaceutical composition according to claim 31, to a subject in need thereof.

**34.** A method for ameliorating, treating and/or preventing a disease or disorder mediated by C3 gene, comprising administering the double-stranded oligonucleotide according to any one of claims 1 to 23, and/or the conjugate according to any one of claims 24 to 29, and/or the pharmaceutical composition according to claim 31, to a subject in need thereof;

optionally, the disease or disorder mediated by C3 gene is associated with the mRNA expression level of C3 gene;
optionally, the disease or disorder is selected from IgA nephropathy, atypical hemolytic uremic syndrome, paroxysmal nocturnal hemoglobinuria (PNH), C3 glomerulopathy, lupus nephritis and membranous nephropathy.

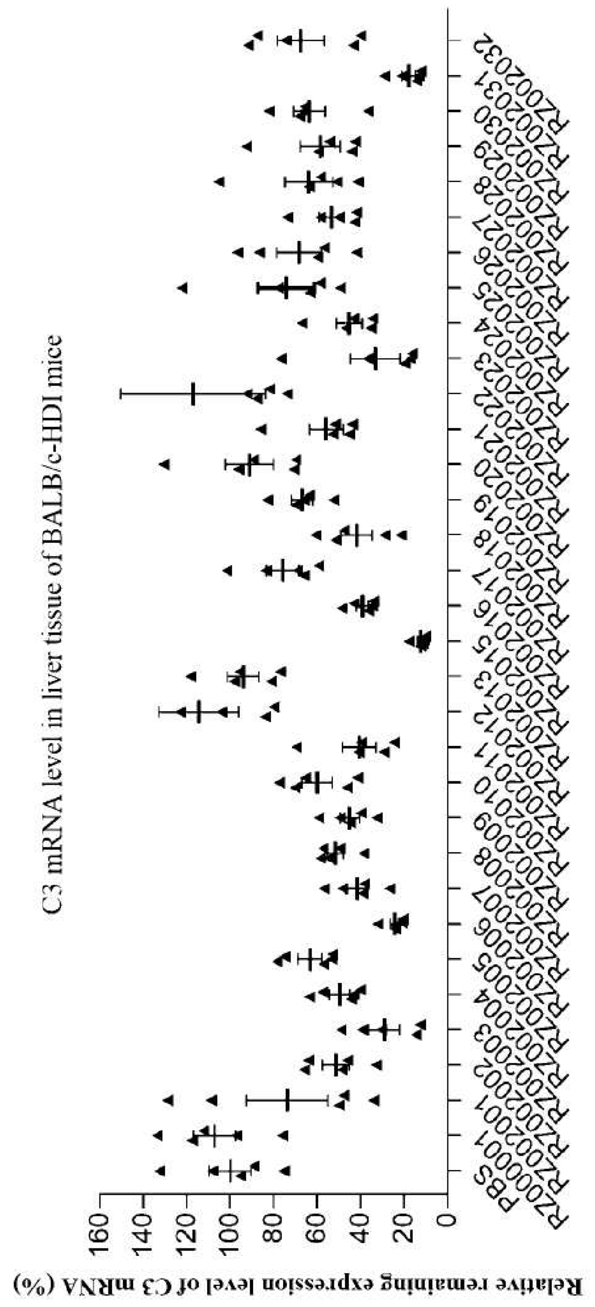
C3 mRNA level in liver tissue of BALB/c-HDI mice
Relative remaining expression level of C3 mRNA (%)
160
140
120
100
80
60
40
20
0
PBS
RZ000001
RZ002001
RZ002002
RZ002003
RZ002004
RZ002005
RZ002006
RZ002007
RZ002008
RZ002009
RZ002010
RZ002011
RZ002012
RZ002013
RZ002015
RZ002016
RZ002017
RZ002018
RZ002019
RZ002020
RZ002021
RZ002022
RZ002023
RZ002024
RZ002025
RZ002026
RZ002027
RZ002028
RZ002029
RZ002030
RZ002031
RZ002032

FIG. 1

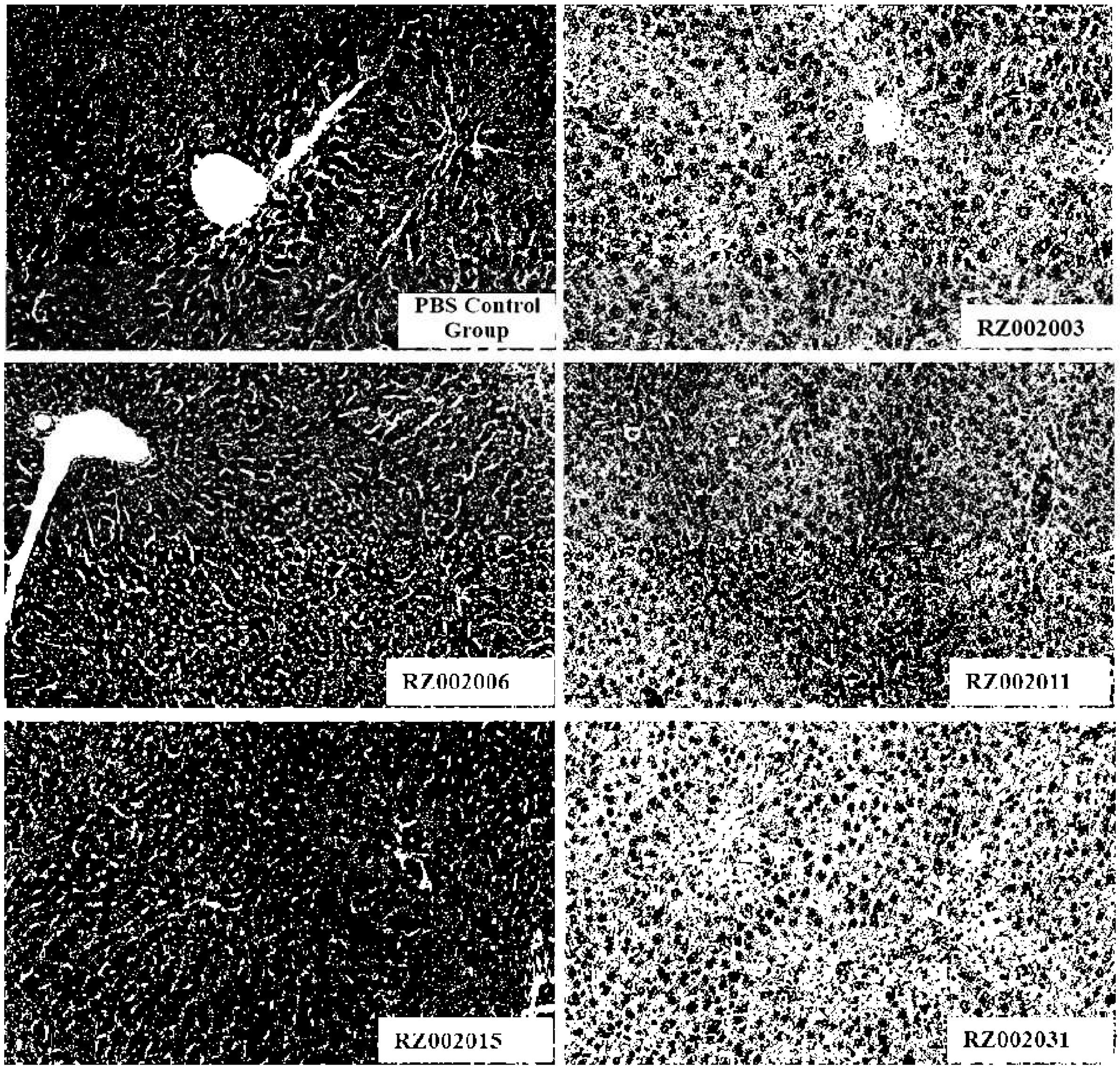
PBS Control Group
RZ002003
RZ002006
RZ002011
RZ002015
RZ002031

FIG. 2

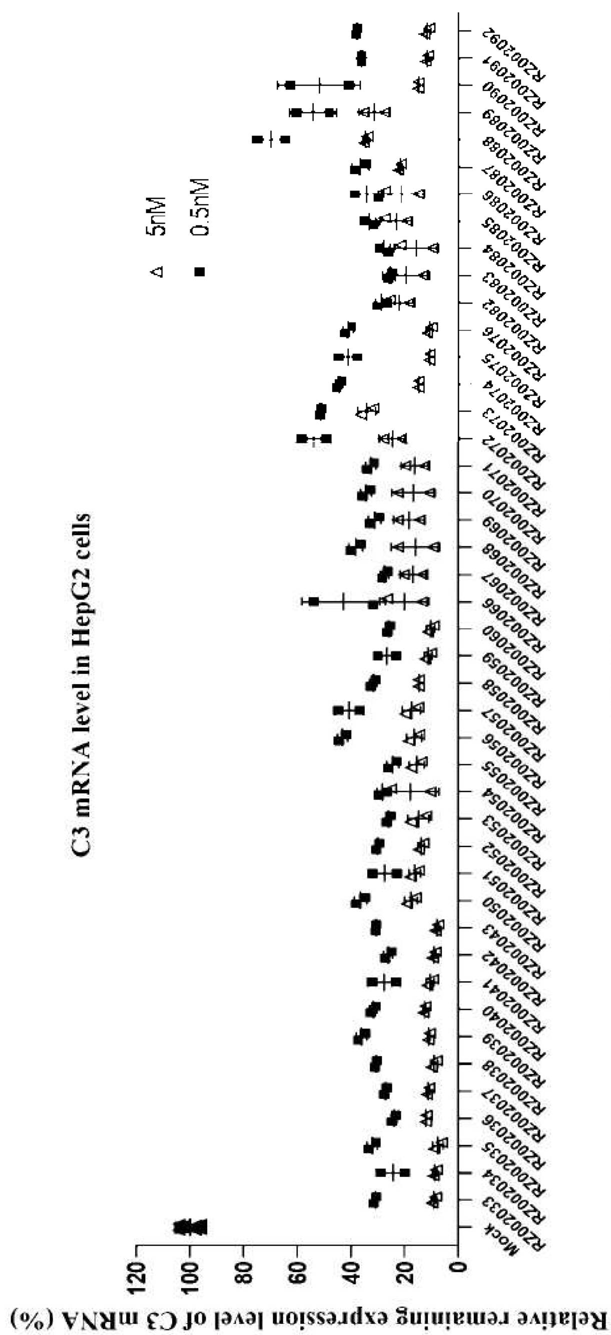
C3 mRNA level in HepG2 cells
Relative remaining expression level of C3 mRNA (%)
120
100
80
60
40
20
0
5nM
0.5nM
Mock
RZ002033
RZ002034
RZ002035
RZ002036
RZ002037
RZ002038
RZ002039
RZ002040
RZ002041
RZ002042
RZ002043
RZ002050
RZ002051
RZ002052
RZ002053
RZ002054
RZ002055
RZ002056
RZ002057
RZ002058
RZ002059
RZ002060
RZ002066
RZ002067
RZ002068
RZ002069
RZ002070
RZ002071
RZ002072
RZ002073
RZ002074
RZ002075
RZ002076
RZ002082
RZ002083
RZ002084
RZ002085
RZ002086
RZ002087
RZ002088
RZ002089
RZ002090
RZ002091
RZ002092

FIG. 3

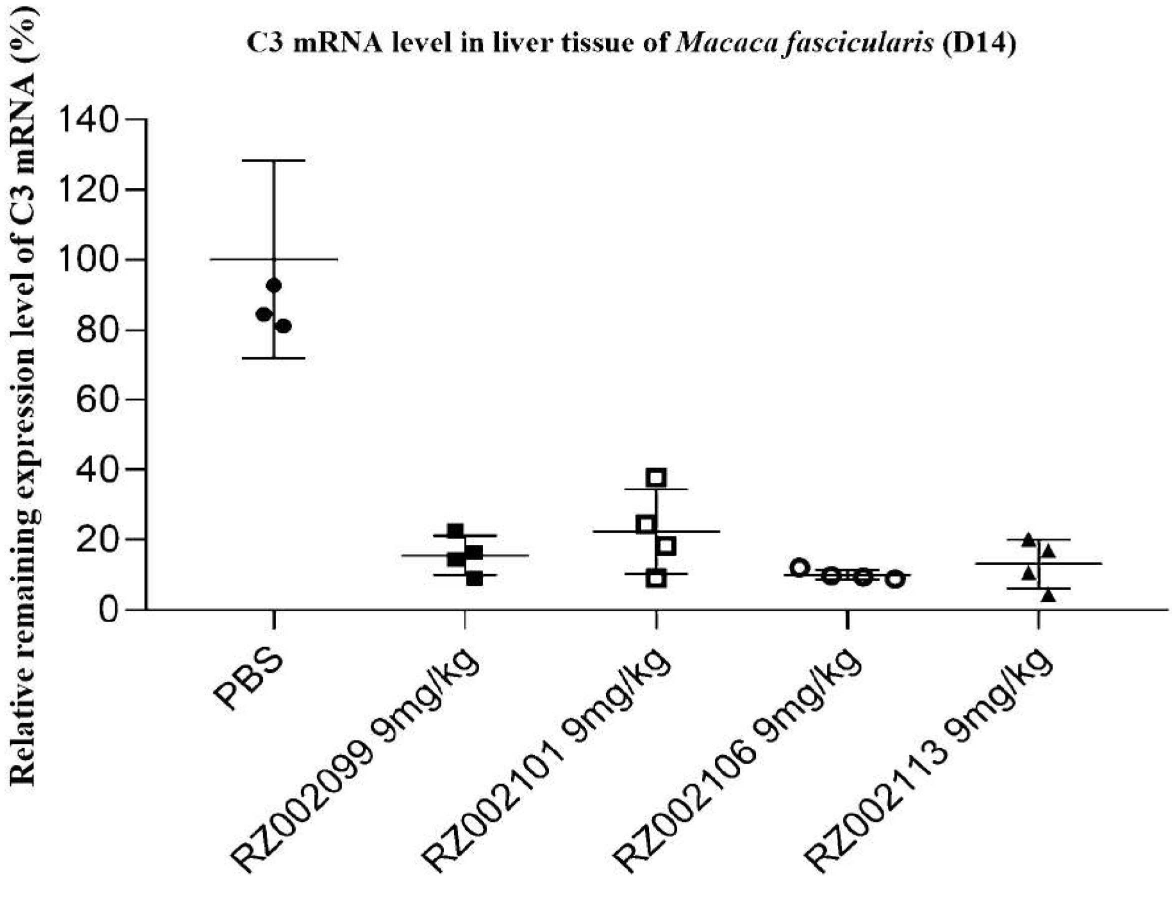
C3 mRNA level in liver tissue of *Macaca fascicularis* (D14)
Relative remaining expression level of C3 mRNA (%)
140
120
100
80
60
40
20
0
PBS
RZ002099 9mg/kg
RZ002101 9mg/kg
RZ002106 9mg/kg
RZ002113 9mg/kg

**FIG. 4**

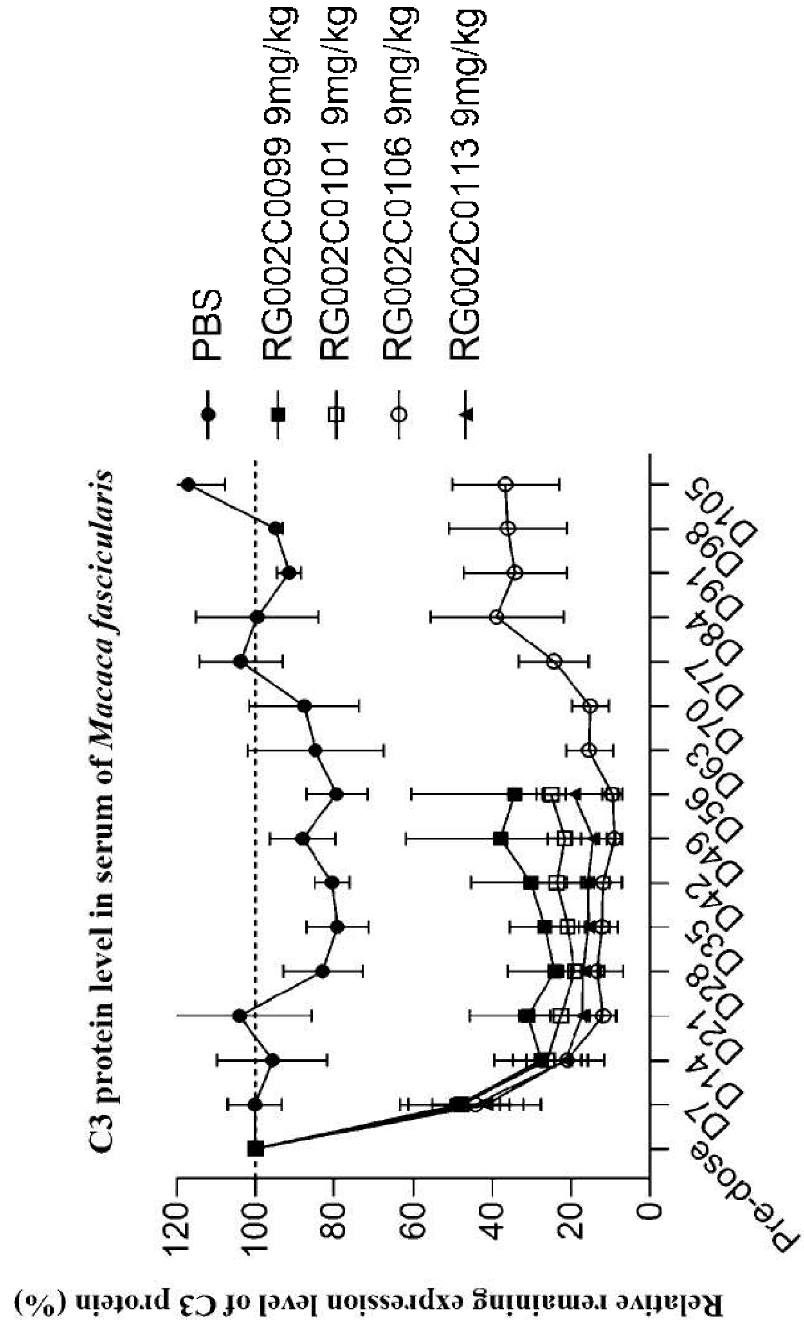
C3 protein level in serum of *Macaca fascicularis*
Relative remaining expression level of C3 protein (%)
120
100
80
60
40
20
0
Pre-dose
D7
D14
D21
D28
D35
D42
D49
D56
D63
D70
D77
D84
D91
D98
D105
PBS
RG002C0099 9mg/kg
RG002C0101 9mg/kg
RG002C0106 9mg/kg
RG002C0113 9mg/kg

FIG. 5

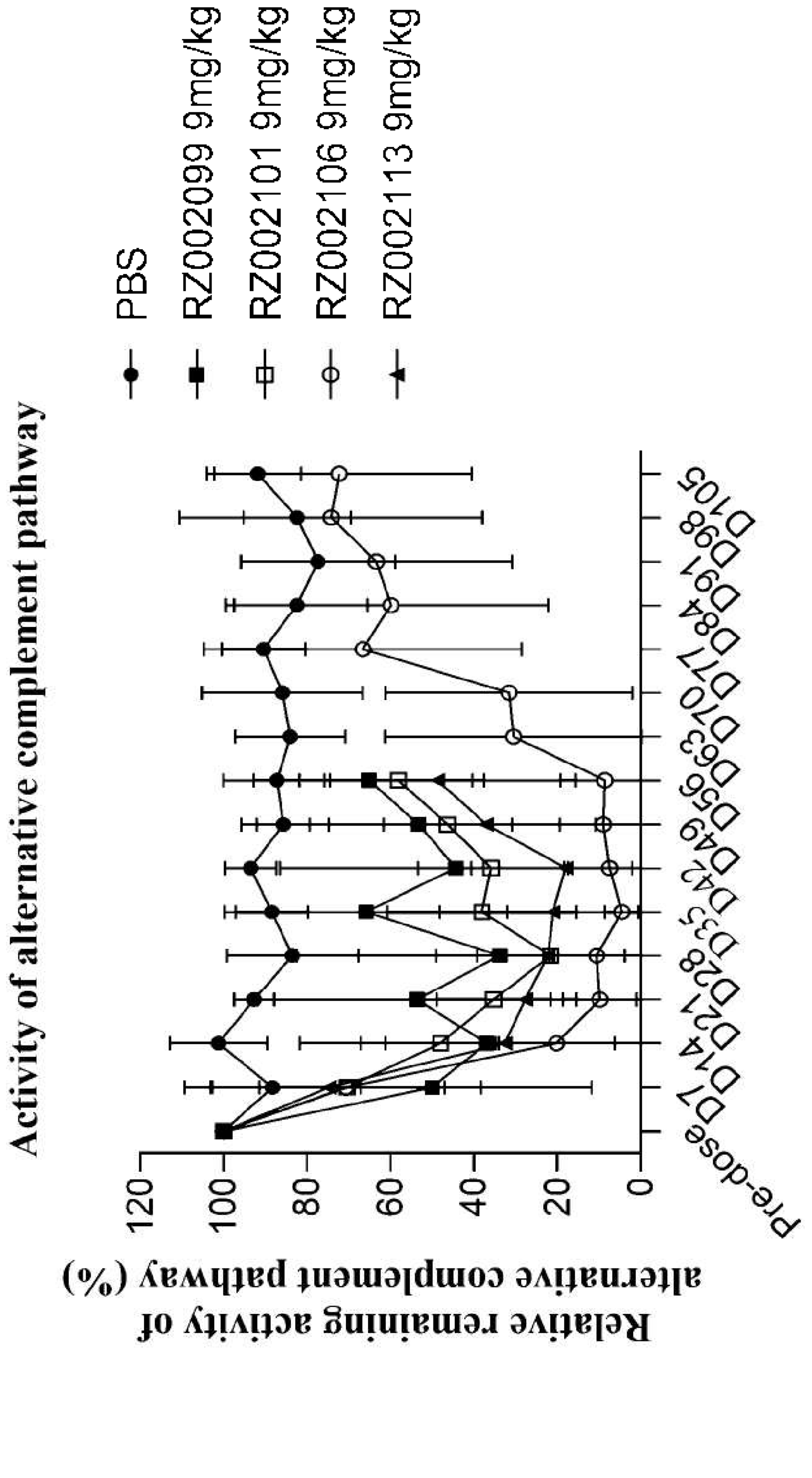
Activity of alternative complement pathway
Relative remaining activity of alternative complement pathway (%)
120
100
80
60
40
20
0
Pre-dose
D7
D14
D21
D28
D35
D42
D49
D56
D63
D70
D77
D84
D91
D98
D105
PBS
RZ002099 9mg/kg
RZ002101 9mg/kg
RZ002106 9mg/kg
RZ002113 9mg/kg

FIG. 6

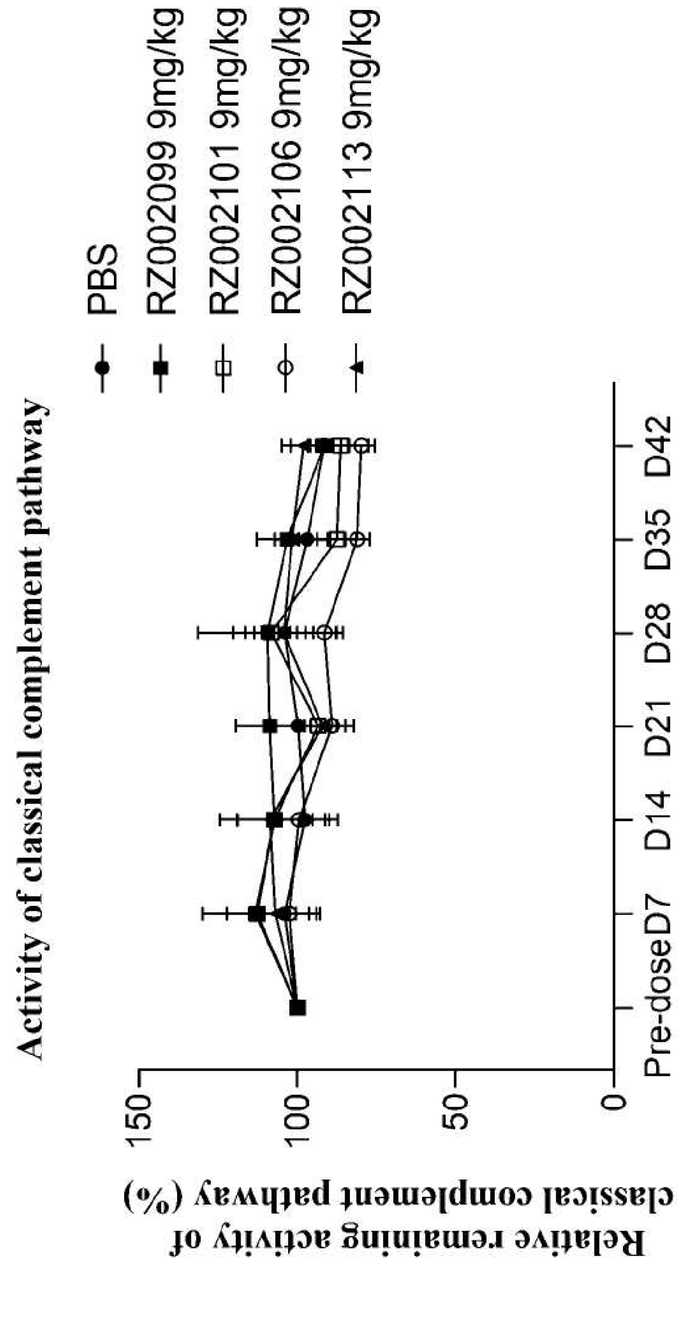
Activity of classical complement pathway
Relative remaining activity of classical complement pathway (%)
150
100
50
0
Pre-dose
D7
D14
D21
D28
D35
D42
PBS
RZ002099 9mg/kg
RZ002101 9mg/kg
RZ002106 9mg/kg
RZ002113 9mg/kg

FIG. 7

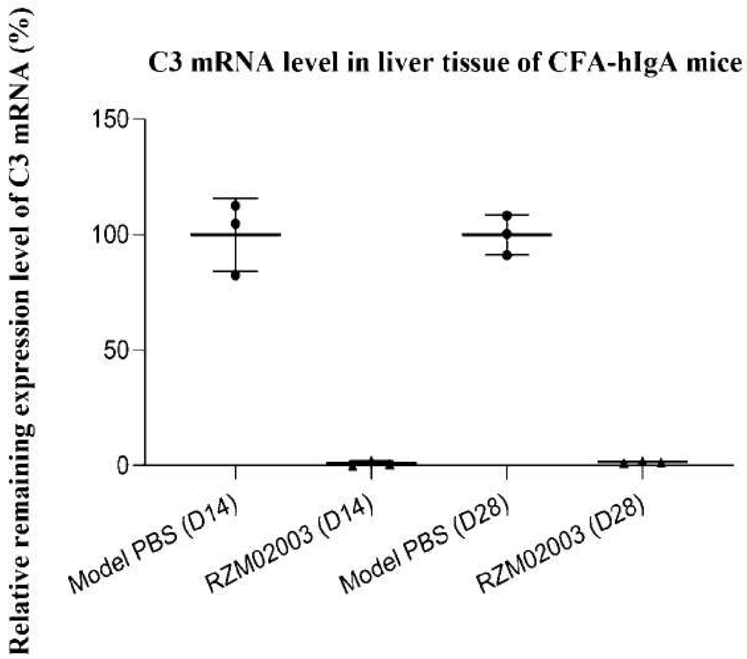
C3 mRNA level in liver tissue of CFA-hIgA mice
Relative remaining expression level of C3 mRNA (%)
150
100
50
0
Model PBS (D14)
RZM02003 (D14)
Model PBS (D28)
RZM02003 (D28)

FIG. 8

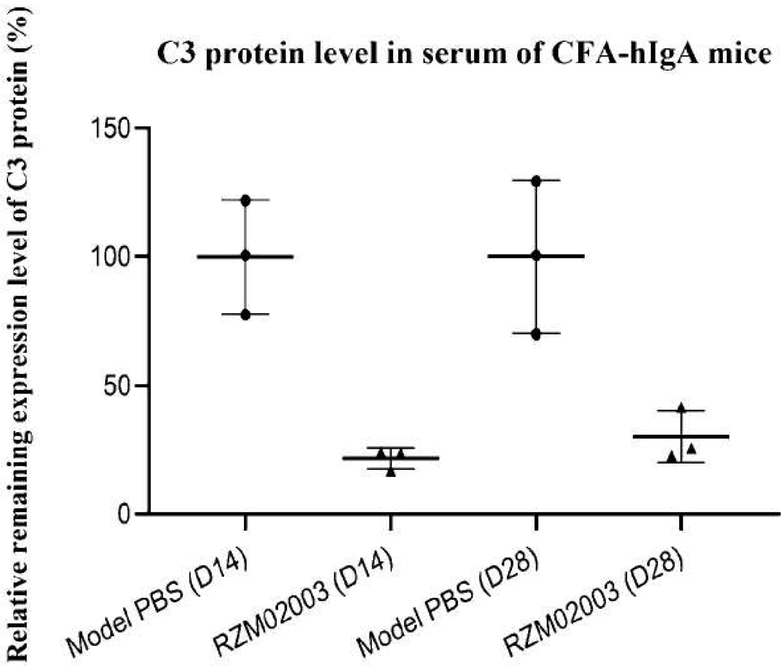
C3 protein level in serum of CFA-hIgA mice
Relative remaining expression level of C3 protein (%)
150
100
50
0
Model PBS (D14)
RZM02003 (D14)
Model PBS (D28)
RZM02003 (D28)

FIG. 9

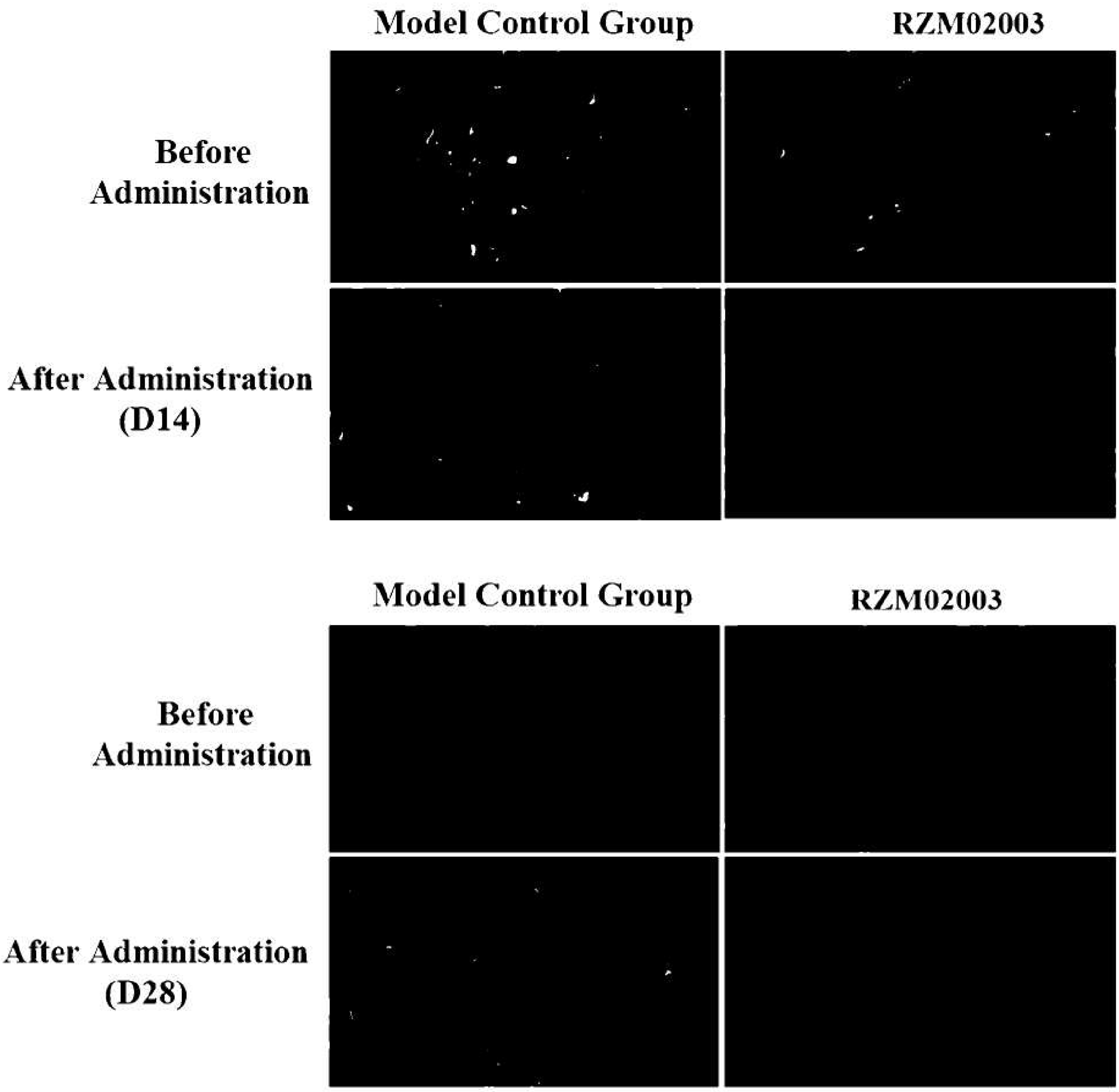
Model Control Group
RZM02003
Before Administration
After Administration (D14)
Model Control Group
RZM02003
Before Administration
After Administration (D28)

FIG. 10

## INTERNATIONAL SEARCH REPORT

International application No.
**PCT/CN2023/116571**

**A. CLASSIFICATION OF SUBJECT MATTER**

C12N15/113(2010.01)i; A61K31/713(2006.01)i; A61K48/00(2006.01)i; A61P5/00(2006.01)i

According to International Patent Classification (IPC) or to both national classification and IPC

**B. FIELDS SEARCHED**

Minimum documentation searched (classification system followed by classification symbols)

IPC: C12N; A61K; A61P

Documentation searched other than minimum documentation to the extent that such documents are included in the fields searched

Electronic data base consulted during the international search (name of data base and, where practicable, search terms used)

CNTXT; ENTXTC; ENTXT; VEN; CJFD; SFNPL; CNKI; PubMed; ISI; Baidu, GenBank, STN, Baidu: siRNA, dsRNA, C3, 补体, 配体, ASGPR, SEQ ID NO: 213, complement, ligand

**C. DOCUMENTS CONSIDERED TO BE RELEVANT**

| Category* | Citation of document, with indication, where appropriate, of the relevant passages | Relevant to claim No. |
|---|---|---|
| X | CN 115176004 A (ALNYLAM PHARMACEUTICALS, INC.) 11 October 2022 (2022-10-11) claims 1-74, and description, paragraphs 440-450 | 1-34 |
| X | CN 110997919 A (SUZHOU RIBO LIFE SCIENCE CO., LTD.) 10 April 2020 (2020-04-10) claims 1-43, and description, page 30 | 1-34 |
| X | CN 116622710 A (BEIJING FOYOU PHARMACEUTICAL CO., LTD.) 22 August 2023 (2023-08-22) claims 1-45 | 1-34 |
| X | CN 114981430 A (SILENCE THERAPEUTICS GMBH) 30 August 2022 (2022-08-30) claims 1-16 | 1-34 |
| A | CN 116497024 A (BEIJING FOYOU PHARMACEUTICAL CO., LTD.) 28 July 2023 (2023-07-28) entire document | 1-34 |
| X | Cristina Zanchi et al. "Therapeutic Small Interfering RNA Targeting Complement C3 in a Mouse Model of C3 Glomerulopathy" *The Journal of Immunology,* Vol. 208, No. (7), 01 April 2022 (2022-04-01), 1772-1781 abstract | 1-34 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 December 2023** | **20 December 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/116571**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No. **PCT/CN2023/116571**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **33, 34**
because they relate to subject matter not required to be searched by this Authority, namely:

Claims 33 and 34 relate to a method for inhibiting C3 gene expression or a method for relieving, treating and/or preventing C3-mediated diseases or conditions, and do not comply with PCT Rule 39.1(iv). A search is carried out on the basis of the corresponding pharmaceutical use thereof.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

Invention 1: a double-stranded oligonucleotide, which has a complementary sequence relating to SEQ ID NO: 213 and is for inhibiting C3 gene expression; and

inventions 2-212: double-stranded oligonucleotides, which have complementary sequences relating to SEQ ID NOs: 214-424 and are for inhibiting C3 gene expression.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **claims 1-34 (the part involving SEQ ID NO: 213)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (July 2022)